(19) Europäisches Patentamt
European Patent Office
Office européen des brevets

(11) EP 1 820 504 A1

(12) EUROPEAN PATENT APPLICATION
published in accordance with Art. 158(3) EPC

(43) Date of publication:
22.08.2007 Bulletin 2007/34

(21) Application number: 05800461.5

(22) Date of filing: 31.10.2005

(51) Int Cl.:
A61K 31/426 (2006.01) A61K 31/415 (2006.01)
A61K 31/4155 (2006.01) A61K 31/42 (2006.01)
A61K 31/421 (2006.01) A61K 31/422 (2006.01)
A61K 31/4245 (2006.01) A61K 31/425 (2006.01)
A61K 31/427 (2006.01) A61K 31/428 (2006.01)
A61K 31/429 (2006.01) A61K 31/433 (2006.01)
A61K 31/4365 (2006.01) A61K 31/437 (2006.01)
A61K 31/4375 (2006.01) A61K 31/44 (2006.01)
A61K 31/4409 (2006.01) A61K 31/4418 (2006.01)
A61K 31/4427 (2006.01) A61K 31/443 (2006.01)

(86) International application number:
PCT/JP2005/019977

(87) International publication number:
WO 2006/051704 (18.05.2006 Gazette 2006/20)

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(30) Priority: 15.11.2004 JP 2004330079
15.11.2004 JP 2004330080
02.06.2005 JP 2005162163
20.07.2005 JP 2005109774

(71) Applicant: TAISHO PHARMACEUTICAL CO., LTD
Tokyo 170-8633 (JP)

(72) Inventors:
- SAITO, Shiuji, c/o TAISHO PHARMACEUTICAL CO. LTD. Toshima-ku, Tokyo 170-8633 (JP)
- OHTA, Hiroshi, c/o TAISHO PHARMACEUTICAL CO. LTD. Toshima-ku, Tokyo 170-8633 (JP)
- ISHIZAKA, Tomoko, TAISHO PHARMACEUTICAL CO. LTD. Toshima-ku, Tokyo 170-8633 (JP)
- YOSHINAGA, Mitsukane, TAISHO PHARMAC. CO. LTD. Toshima-ku, Tokyo 170-8633 (JP)
- TATSUZUKI, Makoto, TAISHO PHARMACEUTICAL CO., LTD. Toshima-ku, Tokyo 170-8633 (JP)
- YOKOBORI, Yuji, c/o TAISHO PHARMACEUTICAL CO. LTD. Toshima-ku, Tokyo 170-8633 (JP)
- TOMISHIMA, Yasumitsu, TAISHO PHARMAC. CO. LTD. Toshima-ku, Tokyo 170-8633 (JP)
- MORITA, Aki, c/o TAISHO PHARMACEUTICAL CO., LTD. Toshima-ku, Tokyo 170-8633 (JP)
- TODA, Yoshihisa, c/o TAISHO PHARMACEUTICAL CO. LTD Toshima-ku, Tokyo 170-8633 (JP)
- TOKUGAWA, Kimiko, TAISHO PHARMACEUTICAL CO. LTD. Toshima-ku, Tokyo 170-8633 (JP)
- KAKU, Ayaka, c/o TAISHO PHARMACEUTICAL CO., LTD. Toshima-ku, Tokyo 170-8633 (JP)
- MURAKAMI, Tomomi, TAISHO PHARMACEUTICAL CO. LTD. Toshima-ku, Tokyo 170-8633 (JP)
- YOSHIMURA, Hiromitsu, TAISHO PHARMAC. CO. LTD. Toshima-ku, Tokyo 170-8633 (JP)
- SEKINE, Shingo, c/o TAISHO PHARMACEUTICAL CO. LTD. Toshima-ku, Tokyo 170-8633 (JP)

EP 1 820 504 A1

• **YOSHIMIZU, Takao,**
**TAISHO PHARMACEUTICAL CO. LTD.**
**Toshima-ku,**
**Tokyo 170-8633 (JP)**

(74) Representative: **HOFFMANN EITLE**
**Patent- und Rechtsanwälte**
**Arabellastrasse 4**
**81925 München (DE)**

(54) **IMINE COMPOUND**

(57) An imine compound represented by the formula:

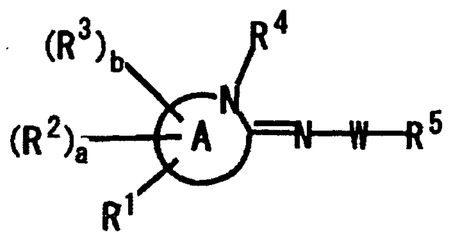

wherein A represents a heterocyclic group; $R^1$, $R^2$, an $R^3$ each represent a hydrogen atom, a halogen atom, a $C_{1-10}$ alkyl group optionally substituted with an aryl group (s) substituted with a halogen atom (s), a $C_{3-10}$ cycloalkyl group, a $C_{1-6}$ haloalkyl group, a $C_{1-10}$ alkoxy group, etc.; $R^4$ represents an optionally substituted $C_{1-10}$ alkyl, $C_{2-6}$ alkenyl, or aryl group; $R^5$ represents a hydrogen atom, a $C_{1-10}$ alkoxy group, a $C_{1-6}$ haloalkyl group, an optionally substituted $C_{1-10}$ alkyl or $C_{2-6}$ alkenyl group, an optionally substituted aryl or heterocyclic group, etc.; W represents -CO-, -CO-CO-, -CO-NH-, -CS-NH-, or $-SO_2-$,
or a cannabinoid-receptor agonist comprising said imine compound as an active ingredient.

The imine compound of the present invention has a cannabinoid-receptor agonist effect, and is useful as a therapeutic or prophylactic drug for pains and autoimmune diseases.

## Description

TECHNICAL FIELD

The present invention relates to an imine compound having a cannabinoid-receptor agonist effect.

BACKGROUND ART

Cannabinoids are substances isolated as a physiologically active component of marihuana in 1960, and having effects such as an analgesic, anti-anxiety, sedation or euphoriant effect. Then, its receptor was found. By virtue of this, endogenous ligands having a cannabinoid-like physiological activity, such as anandamide was discovered.
As the cannabinoid receptor, a cannabinoid type 1 (CB1) receptor was discovered in 1990. It was found that CB1 is distributed over a central nervous system such as brain, and its agonist has an analgesic effect. In 1993, a cannabinoid type 2 (CB2) receptor was discovered. It was found that CB2 is distributed over the tissue and cells of an immune system including blood-system cells such as spleen, lymph node and leucocytes, B cells, T cells, macrophages, and mast cells, and that its agonist has effects such as an immune suppressive effect, an anti-inflammatory effect, and an analgesic effect.
Compounds having a CB1 receptor agonist effect and those having a CB2 receptor agonist effect are disclosed, for example, in Non-Patent Documents 1 and 2, etc.
Imine compounds having analogous structures to those of the compounds according to the present invention are described, for example, in Non-Patent Documents 3 to 8 and Patent Documents 1 to 20, etc. It has been reported that the imine compounds are applied to various usages such as agricultural germicides, herbicides, platelet aggregation inhibitors, therapeutic drugs having a leukocyte infiltration inhibitory effect for various types of inflammations, anti-allergic drugs/anti-inflammatory agents/immunomodulators, and analgesics. However, no reports have been made on a cannabinoid receptor agonist effect caused by an imine compound as an active ingredient.
| | |
|---|---|
| Non-Patent Document 1: | Exp. Opin. Ther. Patent (2002) 12 (10): 1475-1489 |
| Non-Patent Document 2: | Exp. Opin. Ther. Patent (2004) 14 (10): 1435-1452 |
| Non-Patent Document 3: | European Journal of Medicinal Chmistry (1994) 29 (11): 841-854 |
| Non-Patent Document 4: | Journal of Medicinal Chmistry (1966)9(1):151-153 |
| Non-Patent Document 5: | IzVestiya Akademii Nauk SSSR, Seriya Kimicheskaya (1953): 154-162 |
| Non-Patent Document 6: | Farmaco, Edizione Scientifica (1985) 40 (3): 178-189 |
| Non-Patent Document 7: | Journal of Heterocyclic Chemistry (1983) 20 (5): 1153-1154 |
| Non-Patent Document 8: | Journal of Heterocyclic Chemistry (1981) 18 (4): 745-750 |
| Patent Document 1: | WO9215564 |
| Patent Document 2: | EP432600 |
| Patent Document 3: | DE1036326 |
| Patent Document 4: | WO2001055139 |
| Patent Document 5: | WO2000063207 |
| Patent Document 6: | JP2003292485 |
| Patent Document 7: | WO2002002542 |
| Patent Document 8: | WO2003097605 |
| Patent Document 9: | JP2003192591 |
| Patent Document 10: | WO2000017196 |
| Patent Document 11: | WO9842703 |
| Patent Document 12: | WO2002002542 |
| Patent Document 13: | JP02250874 |
| Patent Document 14: | JP62004277 |
| Patent Document 15: | EP40573 |
| Patent Document 16: | JP63203672 |
| Patent Document 17: | JP08081449 |
| Patent Document 18: | WO9703058 |
| Patent Document 19: | WO9404516 |
| Patent Document 20: | JP02229164 |

DISCLOSURE OF THE INVENTION

An object of the present invention is to provide a novel imine compound having a cannabinoid receptor agonist

effect.

The present inventors conducted intensive studies on imine compounds, and found a novel imine compound having a cannabinoid receptor agonist effect. Based on the finding, the present invention was accomplished.

The present invention will be explained below.

According to the present invention, there is provided a cannabinoid-receptor agonist comprising an imine compound represented by Formula (I)

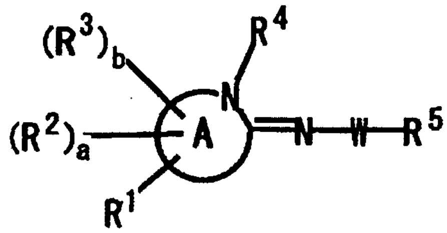

(I)

[where A represents any one of the rings represented by the following formulas (where X represents an oxygen atom or a sulfur atom, and X' represents CH or a nitrogen atom):

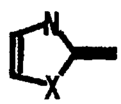 , 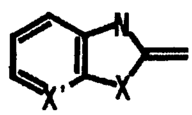 , 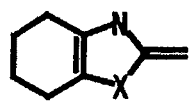 ,

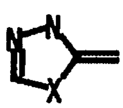 , 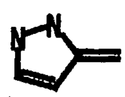 , 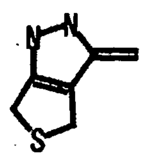 ,

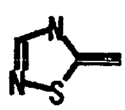 , 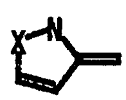 , 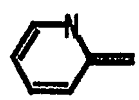 ,

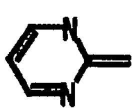 , 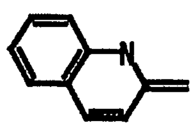 , 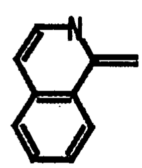

$R^1$ represents

a hydrogen atom;

a halogen atom;

a $C_{1-10}$ alkyl group that may be substituted with an aryl group(s) substituted with a halogen atom(s);

a $C_{3-10}$ cycloalkyl group;

a $C_{2-6}$ alkenyl group;

a $C_{1-6}$ haloalkyl group;
a $C_{1-10}$ alkoxy group;
a carboxyl group;
a $C_{2-6}$ alkoxycarbonyl group;
a hydroxy-$C_{1-6}$ alkyl group;
a group represented by Formula -N($R^6$)$R^7$ (where $R^6$ and $R^7$ each represent a hydrogen atom or a $C_{1-6}$ alkyl group, or $R^6$ and $R^7$, in combination with the adjacent nitrogen atom, form a cyclic amino group);
a group represented by Formula -CON($R^{61}$)$R^{71}$ (where $R^{61}$ and $R^{71}$ each represent a hydrogen atom, or a $C_{1-6}$ alkyl group that may be substituted with a cyclic amino group(s), or $R^{61}$ and $R^{71}$, in combination with the adjacent nitrogen atom, form a cyclic amino group); or
an aryl group that may be substituted with 1 to 3 groups selected from the group consisting of: a $C_{1-6}$ alkyl group, a $C_{1-6}$ haloalkyl group and a halogen atom,
$R^2$ and $R^3$ each represent
a hydrogen atom;
a halogen atom;
a $C_{1-6}$ alkyl group;
a $C_{1-6}$ haloalkyl group; or
an aryl group that may be substituted with 1 to 3 groups selected from the group consisting of: a $C_{1-6}$ alkyl group, a $C_{1-6}$ haloalkyl group and a halogen atom,
$R^4$ represents
a $C_{1-10}$ alkyl group;
a $C_{1-6}$ haloalkyl group;
a $C_{1-10}$ alkyl group or $C_{2-6}$ alkenyl group substituted with: a $C_{3-10}$ cycloalkyl group (s), a $C_{1-6}$ alkoxy group (s), a hydroxyl group(s), an amino group(s), a phthalimide group(s), a cyano group(s), an arylthio group(s), a $C_{2-6}$ alkoxycarbonyl group (s), a carboxyl group(s), a group(s) represented by Formula -CON($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group), or an aryl group(s) that may be substituted with a $C_{1-6}$ haloalkyl group(s), a $C_{2-6}$ alkoxycarbonyl group(s), a carboxyl group(s), or an N-piperidinocarbamoyl group(s);
a $C_{2-6}$ haloalkenyl group;
a $C_{2-6}$ alkynyl group;
a 1,1-dioxothiolanyl group; or
an aryl group,
$R^5$ represents
a hydrogen atom;
a $C_{1-10}$ alkoxy group;
a $C_{1-6}$ alkoxy-$C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, a $C_{2-6}$ alkoxycarbonyl group, a $C_{1-6}$ haloalkyl group, a $C_{1-6}$ alkoxy group that may be substituted with a $C_{1-6}$ alkoxy group(s) or an aryl group(s), a $C_{3-10}$ cycloalkoxy group that may be substituted with 1 to 2 $C_{1-6}$ alkyl groups, an aryl group or aryloxy group that may be substituted with 1 to 5 groups selected from the group consisting of a $C_{1-6}$ alkyl group, a $C_{1-6}$ alkoxy group, a $C_{1-6}$ haloalkyl group, a $C_{1-6}$ haloalkoxy group, an aralkyloxy group, a nitro group and a halogen atom, a heterocyclic group, a phthalimide group, a $C_{1-6}$ alkanoyloxy group, an aralkyloxy group, a $C_{1-6}$ alkylthio group, an arylthio group and a group represented by Formula -N($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with: a $C_{1-6}$ alkyl group(s), a $C_{1-6}$ alkoxy group(s), a $C_{2-6}$ alkoxycarbonyl group (s), a $C_{1-6}$ haloalkyl group(s) or a $C_{1-6}$ haloalkoxy group(s);
an aralkyloxy group; or
a group represented by Formula (II)

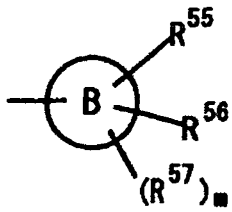

(II)

{where
B represents
a $C_{3-10}$ cycloalkyl group;
an aryl group;
a heterocyclic group;
a $C_{2-6}$ cyclic amino group;
a fluorenyl group;
a phthalimide group;
a 2-oxopyrrolidinyl group;
a group represented by Formula (III)

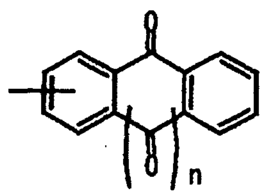

(III)

(where n represents 0 or 1); or
a group represented by Formula (IV)

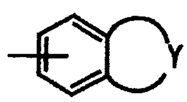

(IV)

(where Y represents $-(CH_2)p-$, $-CO-CH_2-CH_2-$, $-CO-CH_2-CH_2-CH_2-$, $-O-CH_2-CH_2-$, $-O-CH_2-CH=CH-$, or $-O-(CH_2)q-O-$, in which p represents an integer of 2 to 4, and q represents an integer of 1 to 3);
$R^{55}$ represents
a hydrogen atom;
a halogen atom;
a $C_{1-10}$ alkyl group that may be substituted with: an aryl group(s), a heterocyclic group(s) or an aryloxy group(s), each of which may be substituted with a halogen atom(s)
; or with a group(s) represented by Formula $-N(R^{62})R^{72}$ (where
$R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);

a $C_{1-6}$ haloalkyl group;
a $C_{1-10}$ alkoxy group;
a $C_{1-6}$ alkylthio group;
a $C_{1-6}$ alkylsulfonyl group;
an arylsulfonyl group that may be substituted with a $C_{1-6}$ alkyl group(s) or a halogen atom(s);
a $C_{1-6}$ haloalkoxy group;
a $C_{1-6}$ haloalkylthio group;
a $C_{3-10}$ cycloalkyl group;
a $C_{2-6}$ alkenyl group;
a $C_{2-6}$ alkenyloxy group;
a $C_{2-6}$ alkenylthio group;
a $C_{1-6}$ alkoxy-$C_{1-6}$ alkoxy group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group, a cyano group and a nitro group;
a heterocyclic group that may be substituted with a $C_{1-6}$ alkyl group(s) or a $C_{1-6}$ haloalkyl group(s);
an aryloxy group or arylthio group that may be substituted with a halogen atom(s) or a $C_{1-6}$ alkyl group(s);
a group represented by Formula $-N(R^{63})R^{73}$ (where $R^{63}$ and $R^{73}$ each represent a hydrogen atom, a $C_{1-6}$ alkyl group, a $C_{1-6}$ hydroxyalkyl group, a $C_{1-6}$ alkoxy-$C_{1-6}$ alkyl group, an aryl group, a $C_{1-6}$ alkanoyl group, a di-$C_{1-6}$ alkylamino-$C_{2-6}$ alkanoyl group, a benzoyl group, or a heterocyclic group that may be substituted with a $C_{1-6}$ alkyl group (s), or $R^{63}$ and $R^{73}$, in combination with the adjacent nitrogen atom, form a cyclic amino group);
a hydroxyl group;
a cyano group;
a nitro group;
a $C_{1-6}$ alkanoyl group;
a $C_{1-6}$ alkanoyloxy group;
a $C_{1-6}$ alkanoyloxy-$C_{1-6}$ alkyl group;
a $C_{2-6}$ haloalkanoyl group;
a carboxyl group;
a $C_{2-6}$ alkoxycarbonyl group;
a $C_{2-6}$ cyclic amino group that may be substituted with an aryl group(s);
a group represented by Formula $-CON(R^{64})R^{74}$ (where $R^{64}$ and $R^{74}$ each represent a hydrogen atom, a $C_{1-6}$ alkyl group, a $C_{1-6}$
alkoxy-$C_{1-6}$ alkyl group, or a heterocyclic group that may be substituted with a $C_{1-6}$ alkyl group(s), or $R^{64}$ and $R^{74}$, in combination with the adjacent nitrogen atom, form a cyclic amino group);
a group represented by Formula $-SO_2N(R^{62})R^{72}$ (where $R^{62}$ and $R^{72}$ each represent a hydrogen atom, a $C_{1-6}$ alkyl group, or $R^{62}$ and $R^{72}$, in combination with the adjacent nitrogen atom, form a cyclic amino group);
a $C_{1-6}$ alkylsulfenyl group;
a $C_{1-6}$ alkylsulfonyl group that may be substituted with a halogen atom(s); or
an arylsulfonyl group that may be substituted with a halogen atom(s),
$R^{56}$ represents
a hydrogen atom;
a halogen atom;
a $C_{1-10}$ alkyl group that may be substituted with an aryl group(s), a pyridyl group(s), a thienyl group(s) or a heterocyclic group(s), each of which may be substituted with a $C_{1-6}$ alkyl group(s) or a halogen atom(s);
a $C_{1-6}$ haloalkyl group;
a $C_{3-10}$ cycloalkyl group;
a $C_{1-10}$ alkoxy group;
a $C_{2-6}$ alkenyl group;
an aryl group that may be substituted with 1 to 3 groups selected from the group consisting of: a $C_{1-6}$ alkyl group, a halogen atom, a $C_{1-6}$ alkoxy group and a nitro group;
a heterocyclic group that may be substituted with a $C_{1-6}$ alkyl group(s);
a $C_{1-6}$ alkanoyl group;
a $C_{1-6}$ alkylsulfenyl group;
a $C_{1-6}$ alkylsulfonyl group;
an arylsulfonyl group that may be substituted with a halogen atom(s);
a hydroxyl group;
a cyano group; or
a nitro group,

$R^{57}$ represents
a hydrogen atom;
a $C_{1-10}$ alkyl group that may be substituted with a pyridyl group(s) or a thienyl group(s);
a $C_{1-6}$ haloalkyl group;
a $C_{1-10}$ cycloalkyl group;
a halogen atom;
a $C_{2-6}$ alkenyl group;
an aryl group that may be substituted with a halogen atom(s); a $C_{1-10}$ alkoxy group;
a $C_{1-6}$ alkanoyl group; or
a $C_{1-6}$ alkylsulfenyl group, and
m represents an integer of 1 to 3},
a and b each represent 0 or 1, and
W represents -CO-, -CO-CO-, -CO-NH-, -CS-NH-, or $-SO_2-$], or a pharmaceutically acceptable salt thereof, as an active ingredient.

According to another aspect of the present invention, there is provided a cannabinoid-receptor agonist comprising an imine compound represented by the following Formula (I-1)

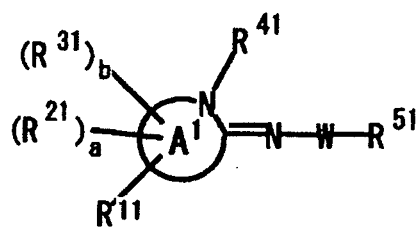

(I-1)

[where $A^1$ represents any one of the rings represented by the following formulas (where X represents an oxygen atom or a sulfur atom):

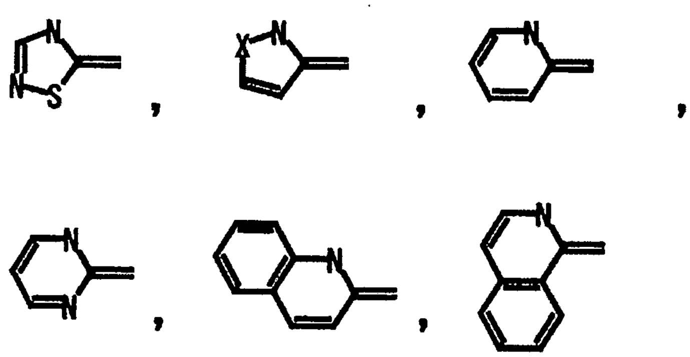

$R^{11}$ represents
a hydrogen atom;
a halogen atom;
a $C_{1-6}$ alkyl group
a $C_{2-6}$ alkenyl group;
a $C_{1-6}$ haloalkyl group;
a $C_{1-6}$ alkoxy group; or
a group represented by Formula $-N(R^6)R^7$ (where $R^6$ and $R^7$ each represent a hydrogen atom or a $C_{1-6}$ alkyl group, or $R^6$ and $R^7$, in combination with the adjacent nitrogen atom, form a cyclic amino group),

$R^{21}$ and $R^{31}$ each represent
a hydrogen atom;
a halogen atom; or
a $C_{1-6}$ alkyl group,
$R^{41}$ represents
a $C_{1-10}$ alkyl group or $C_{2-6}$ alkenyl group that may be substituted with: a halogen atom(s), a $C_{3-10}$ cycloalkyl group(s), an aryl group (s) or a $C_{1-6}$ alkoxy group,
$R^{51}$ represents
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, an aryl group or aryloxy group that may be substituted with 1 to 5 groups selected from the group consisting of a $C_{1-6}$ alkyl group, a $C_{1-6}$ alkoxy group, a $C_{1-6}$ haloalkyl group and a halogen atom, and a heterocyclic group;
a group represented by Formula (II-1)
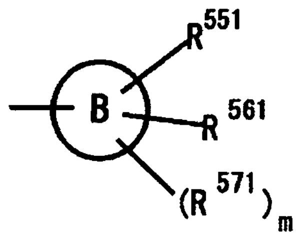

(II-1)

{where
B represents
a $C_{3-10}$ cycloalkyl group;
an aryl group;
a heterocyclic group;
a fluorenyl group; or
a group represented by Formula (IV-1)
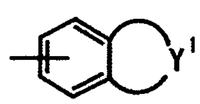

(IV-1)

(where $Y^1$ represents $-(CH_2)p-$, or $-O-(CH_2)q-O-$, in which p represents an integer of 2 to 4, and q represents an integer of 1 to 3),
$R^{551}$ and $R^{561}$ each represent
a hydrogen atom;
a halogen atom;
a $C_{1-10}$ alkyl group that may be substituted with: an aryl group(s), a heterocyclic group(s) or an aryloxy group(s), each of which may be substituted with a halogen atom(s); or with a group represented by Formula $-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;

a $C_{1-6}$ alkylthio group;
a $C_{1-6}$ haloalkoxy group;
a $C_{3-10}$ cycloalkyl group;
a $C_{2-6}$ alkenyl group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group, and a nitro group;
a heterocyclic group that may be substituted with a $C_{1-6}$ alkyl group(s);
an aryloxy group that may be substituted with a halogen atom(s);
a group represented by Formula -N($R^{634}$) $R^{734}$ (where $R^{634}$ and $R^{734}$ each represent a hydrogen atom, a $C_{1-6}$ alkyl group, an aryl group, or a $C_{1-6}$ alkanoyl group, or $R^{634}$ and $R^{734}$, in combination with the adjacent nitrogen atom, form a cyclic amino group);
a hydroxyl group;
a cyano group;
a nitro group;
a $C_{1-6}$ alkanoyl group;
a $C_{2-6}$ haloalkanoyl group;
a $C_{1-6}$ alkylsulfonyl group; or
an arylsulfonyl group that may be substituted with a halogen atom (s),
$R^{571}$ represents
a hydrogen atom;
alkyl group;
a $C_{1-10}$ alkoxy group; or
a halogen atom, and
m represents an integer of 1 to 3}
a and b each represent 0 or 1, and
W represents CO or $SO_2$],
or a pharmaceutically acceptable salt thereof, as an active ingredient.

According to another aspect of the present invention, there is provided an imine compound represented by the following Formula (I-1)

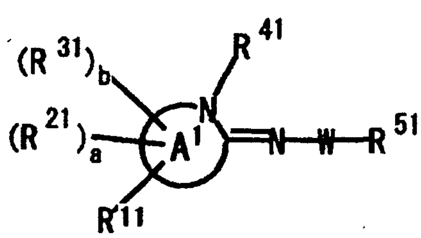

(I-1)

[where $A^1$ represents any one of the rings represented by the following formulas (where X represents an oxygen atom or a sulfur atom):

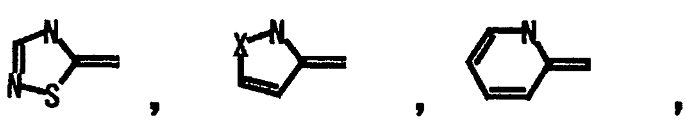

, , ,

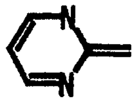 , 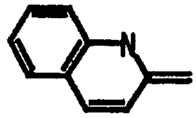 , 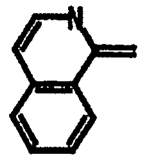

$R^{11}$ represents
a hydrogen atom;
a halogen atom;
a $C_{1-6}$ alkyl group
a $C_{2-6}$ alkenyl group;
a $C_{1-6}$ haloalkyl group;
a $C_{1-6}$ alkoxy group; or
a group represented by Formula $-N(R^6)R^7$ (where $R^6$ and $R^7$ each represent a hydrogen atom or a $C_{1-6}$ alkyl group, or $R^6$ and $R^7$, in combination with the adjacent nitrogen atom, form a cyclic amino group),
$R^{21}$ and $R^{31}$ each represent
a hydrogen atom;
a halogen atom; or
a $C_{1-6}$ alkyl group,
$R^{41}$ represents
a $C_{1-10}$ alkyl group or $C_{2-6}$ alkenyl group that may be substituted with: a halogen atom(s), a $C_{3-10}$ cycloalkyl group(s), an aryl group(s) or a $C_{1-6}$ alkoxy group(s),
$R^{51}$ represents
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, an aryl group or aryloxy group that may be substituted with 1 to 5 groups selected from the group consisting of a $C_{1-6}$ alkyl group, a $C_{1-6}$ alkoxy group, a $C_{1-6}$ haloalkyl group and a halogen atom, and a heterocyclic group;
a group represented by Formula (II-1)
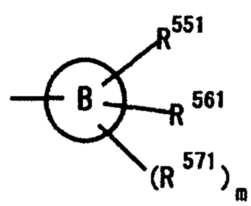

(II-1)

{where
B represents
a $C_{3-10}$ cycloalkyl group;
an aryl group;
a heterocyclic group;
a fluorenyl group; or
a group represented by Formula (IV-1)
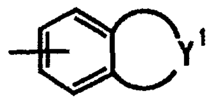

(IV-1)

(where $Y^1$ represents $-(CH_2)p-$, or $-O-(CH_2)q-O-$, in which p represents an integer of 2 to 4, and q represents an integer of 1 to 3),
$R^{551}$ and $R^{561}$ each represent
a hydrogen atom;
a halogen atom;
a $C_{1-10}$ alkyl group that may be substituted with: an aryl group(s), a heterocyclic group(s) or an aryloxy group(s), each of which may be substituted with a halogen atom(s); or with a group(s) represented by Formula $-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ haloalkoxy group
a $C_{3-10}$ cycloalkyl group;
a $C_{2-6}$ alkenyl group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group and a nitro group;
a heterocyclic group that may be substituted with a $C_{1-6}$ alkyl group(s);
an aryloxy group that may be substituted with a halogen atom(s);
a group represented by Formula $-N(R^{634})$ $R^{734}$ (where $R^{634}$ and $R^{734}$ each represent a hydrogen atom, a $C_{1-6}$ alkyl group, an aryl group, or a $C_{1-6}$ alkanoyl group, or $R^{634}$ and $R^{734}$, in combination with the adjacent nitrogen atom, form a cyclic amino group);
a hydroxyl group;
a cyano group;
a nitro group;
a $C_{1-6}$ alkanoyl group;
a $C_{2-6}$ haloalkanoyl group;
a $C_{1-6}$ alkylsulfonyl group; or
an arylsulfonyl group that may be substituted with a halogen atom(s),
$R^{571}$ represents
a hydrogen atom;
a halogen atom;
a $C_{1-10}$ alkyl group; or
a $C_{1-10}$ alkoxy group, and
m represents an integer of 1 to 3}
a and b each represent 0 or 1, and
W represents CO or $SO_2$],
or a pharmaceutically acceptable salt thereof.

According to another aspect of the present invention, there is provided a cannabinoid-receptor agonist comprising an imine compound represented by the Formula (I-2)

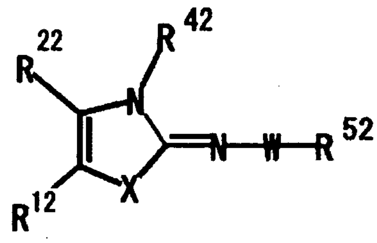

(I-2)

[where
$R^{12}$ and $R^{22}$ each represent
a hydrogen atom;
a halogen atom;
a $C_{1-10}$ alkyl group;
a $C_{1-6}$ haloalkyl group;
a $C_{1-6}$ alkoxy group;
a carboxyl group;
a $C_{2-6}$ alkoxycarbonyl group;
a hydroxy-$C_{1-6}$ alkyl group;
an aryl group that may be substituted with 1 to 3 halogen atoms; or
a group represented by Formula -CON($R^{61}$)$R^{71}$ (where $R^{61}$ and $R^{71}$ each represent a hydrogen atom or a $C_{1-6}$ alkyl group that may be substituted with a cyclic amino group(s), or $R^{61}$ and $R^{71}$, in combination with the adjacent nitrogen atom, form a cyclic amino group), or,
$R^{11}$ and $R^{22}$, in combination with the adjacent carbon atom, form a benzene ring, a pyridine ring or a cyclohexenyl ring, each of which may be substituted with a $C_{1-6}$ alkyl group(s) or a halogen atom(s);
$R^{42}$ represents
a $C_{1-10}$ alkyl group or $C_{2-6}$ alkenyl group that may be substituted with: a halogen atom (s), a cyano group(s), a carboxyl group(s), a $C_{2-6}$ alkoxycarbonyl group(s), a $C_{3-10}$ cycloalkyl group (s), an aryl group(s) that may be substituted with a $C_{1-6}$ haloalkyl group(s), a $C_{1-6}$ haloalkoxy group(s), a
$C_{1-6}$ haloalkylthio group(s), a carboxyl group(s), a $C_{2-6}$ alkoxycarbonyl group(s) or a piperidinocarbamoyl group(s), an arylthio group(s), a $C_{1-6}$ alkoxy group(s), or a group(s) represented by Formula -CON($R^{62}$)$R^{72}$ (where $R^{62}$ and $R^{72}$ each represent a hydrogen atom or a $C_{1-6}$ alkyl group, or $R^{62}$ and $R^{72}$ in combination with the adjacent nitrogen atom, form a cyclic amino group); or
a $C_{2-6}$ alkynyl group,
$R^{52}$ represents
a hydrogen atom;
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, a $C_{2-6}$ alkoxycarbonyl group, a $C_{1-6}$ alkoxy group that may be substituted with a $C_{1-6}$ alkoxy group or an aryl group, a $C_{3-10}$ cycloalkoxy group that may be substituted with a $C_{1-6}$ alkyl group(s), an aryl group or aryloxy group that may be substituted with 1 to 5 groups selected from the group consisting of a $C_{1-6}$ alkyl group, a $C_{1-6}$ haloalkyl group, a $C_{1-6}$ alkoxy group, an aralkyloxy group, a nitro group and a halogen atom, a heterocyclic group, a phthalimide group, a $C_{1-6}$ alkanoyloxy group, an aralkyloxy group, a $C_{1-6}$ alkylthio group, an arylthio group and a group represented by Formula -N($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with: a $C_{1-6}$ alkyl group(s), a $C_{1-6}$ alkoxy group(s), $C_{2-6}$ alkoxycarbonyl group (s), or a $C_{1-6}$ haloalkyl group(s);
an aralkyloxy group;
a group represented by Formula (II-2)

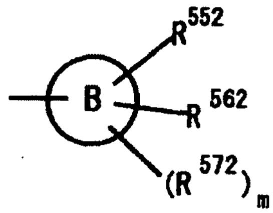

(II-2)

{where
B represents
a $C_{3-10}$ cycloalkyl group;
an aryl group;
a heterocyclic group;
a $C_{2-6}$ cyclic amino group;
a fluorenyl group;
a 2-oxopyrrolidinyl group
a group represented by Formula (III)
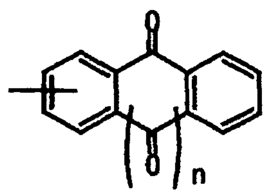

(III)

(where n represents 0 or 1); or
a group represented by Formula (IV-2)
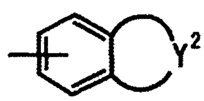

(IV-2)

(where $Y^2$ represents $-(CH_2)p-$, $-CO-CH_2-CH_2-$,$-O-CH_2-CH=CH-$, or $-O-(CH_2)q-O-$, in which p represents an integer of 2 to 4, and q represents an integer of 1 to 3),
$R^{552}$ represents
a hydrogen atom;
a halogen atom;
a $C_{1-10}$ alkyl group that may be substituted with: an aryl group(s) that may be substituted with a halogen atom(s); with an aryloxy group(s); or with a group(s) represented by Formula
$-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;

a $C_{1-10}$ alkoxy group;
a $C_{1-6}$ alkylthio group;
a $C_{1-6}$ alkylsulfonyl group
an arylthio group that may be substituted with a $C_{1-6}$ alkyl group(s) or a halogen atom(s);
an arylsulfonyl group that may be substituted with a $C_{1-6}$ alkyl group(s) or a halogen atom(s);
a $C_{1-6}$ haloalkoxy group;
a $C_{1-6}$ haloalkylthio group;
a $C_{3-10}$ cycloalkyl group;
a $C_{2-6}$ alkenyl group;
a $C_{1-6}$ alkoxy-$C_{1-6}$ alkoxy group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group and a nitro group;
a heterocyclic group that may be substituted with a $C_{1-6}$ alkyl group (s) or a $C_{1-6}$ haloalkyl group(s);
an aryloxy group that may be substituted with a halogen atom(s);
a group represented by Formula $-N(R^{63})R^{73}$ (where $R^{63}$ and $R^{73}$ each represent a hydrogen atom, a $C_{1-6}$ alkyl group, a $C_{1-6}$ hydroxyalkyl group, a $C_{1-6}$ alkoxy-$C_{1-6}$ alkyl group, an aryl group, a $C_{1-6}$ alkanoyl group, or a benzoyl group, or $R^{63}$ and $R^{73}$, in combination with the adjacent nitrogen atom, form a cyclic amino group);
a hydroxyl group;
a cyano group;
a nitro group;
a carboxyl group;
a $C_{2-6}$ alkoxycarbonyl group;
a $C_{2-6}$ cyclic amino group that may be substituted with an aralkyl group(s) or an aryl group(s);
a group represented by Formula $-CON(R^{64})R^{74}$ (where $R^{64}$ and $R^{74}$ each represent a hydrogen atom, a $C_{1-6}$ alkyl group, a $C_{1-6}$ alkoxy-$C_{1-6}$ alkyl group, or a heterocyclic group that may be substituted with a $C_{1-6}$ alkyl group(s), or $R^{64}$ and $R^{74}$, in combination with the adjacent nitrogen atom, form a cyclic amino group);
a group represented by Formula $-SO_2N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ alkylsulfonyl group that may be substituted with a halogen atom(s);
an arylsulfonyl group that may be substituted with a halogen atom(s); or
a 2-oxa-3-oxobicyclo[2.2.1]heptyl group,
$R^{562}$ represents
a hydrogen atom;
a halogen atom;
a $C_{1-10}$ alkyl group;
a $C_{1-6}$ haloalkyl group; or
a $C_{1-6}$ alkoxy group,
$R^{572}$ represents
a hydrogen atom;
a $C_{1-10}$ alkyl group
a $C_{1-6}$ haloalkyl group;
a halogen atom; or
a $C_{1-6}$ alkoxy group;
m represents an integer of 1 to 3}
X represents an oxygen atom or a sulfur atom;
W represents CO or $SO_2$],
or a pharmaceutically acceptable salt thereof, as an active ingredient.

According to another aspect of the present invention, there is provided an imine compound represented by the following Formula (I-2)

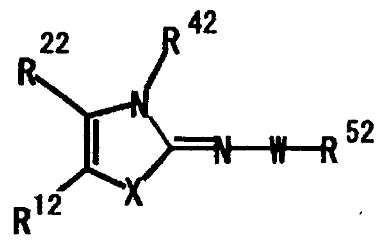

(I-2)

where
W is CO,
$R^{12}$ represents
a halogen atom;
a $C_{1-6}$ alkyl group;
a $C_{1-6}$ haloalkyl group;
a $C_{1-6}$ alkoxy group;
a carboxyl group;
a $C_{2-6}$ alkoxycarbonyl group;
a hydroxy-$C_{1-6}$ alkyl group; or
an aryl group that may be substituted with 1 to 3 halogen atoms; or
a group represented by Formula -CON($R^{61}$)$R^{71}$ (where $R^{61}$ and $R^{71}$ each represent a hydrogen atom(s) or a $C_{1-6}$ alkyl group that may be substituted with a cyclic amino group(s), or $R^{61}$ and $R^{71}$, in combination with the adjacent nitrogen atom, form a cyclic amino group),
$R^{22}$ represents
a hydrogen atom:
a halogen atom;
a $C_{1-10}$ alkyl group;
a $C_{1-6}$ haloalkyl group; or
an aryl group; or
$R^{12}$ and $R^{22}$, in combination with the adjacent carbon atom, form a benzene ring, a pyridine ring or a cyclohexenyl ring, each of which may be substituted with a $C_{1-6}$ alkyl group(s) or a halogen atom(s);
$R^{42}$ represents
a $C_{1-10}$ alkyl group or $C_{2-6}$ alkenyl group substituted with a $C_{3-10}$ cycloalkyl group(s) or a $C_{1-6}$ alkoxy group(s), and
X and $R^{52}$ are as defined above,
or a pharmaceutically acceptable salt thereof.

According to another aspect of the present invention, there is provided a cannabinoid-receptor agonist comprising an imine compound represented by the Formula (I-3)

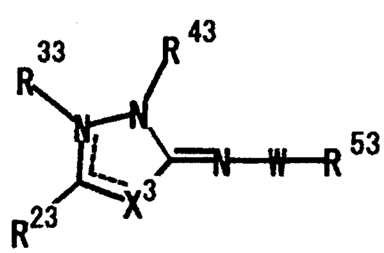

(I-3)

[where the broken line indicates that one of the bonds is a double bond,
$X^3$ represents C($R^{13}$), S or O,

$R^{13}$, $R^{23}$ and $R^{33}$ each represent
a hydrogen atom;
a $C_{1-10}$ alkyl group that may be substituted with an aryl group(s) substituted with a halogen atom(s);
a $C_{1-6}$ haloalkyl group;
a $C_{3-10}$ cycloalkyl group; or
an aryl group or aralkyl group that may be substituted with 1 to 3 halogen atoms, or
in the case where $X^3$ is $C(R^{13})$, $R^{13}$ and $R^{23}$ together represent a group represented by $-CH_2-S-CH_2-$ (with the proviso that, $R^{33}$ is not substituted in the case where $X^3$ is S or O),
$R^{43}$ represents
a 1,1-dioxothiolanyl group; or
a $C_{1-10}$ alkyl group or $C_{2-6}$ alkenyl group that may be substituted with a group(s) selected from the group consisting of: a $C_{3-10}$ cycloalkyl group, a $C_{1-6}$ haloalkyl group and a $C_{1-6}$ alkoxy group; or
an aryl group,
$R^{53}$ represents
a hydrogen atom;
a $C_{1-10}$ alkoxy group;
a $C_{1-6}$ alkoxy-$C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a $C_{1-6}$ alkoxy group, a $C_{3-10}$ cycloalkyl group, a $C_{2-6}$ alkoxycarbonyl group, an aryl group or aryloxy group that may be substituted with 1 to 5 groups selected from the group consisting of a $C_{1-6}$ alkoxy group and a halogen atom, a heterocyclic group, a $C_{1-6}$ alkanoyloxy group, an aralkyloxy group, and a $C_{1-6}$ alkylthio group;
a group represented by Formula (II-3)
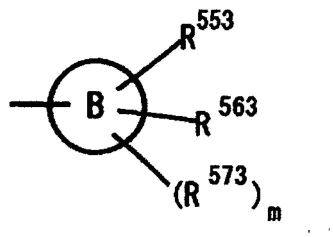

(II-3).

{where B represents
a $C_{3-10}$ cycloalkyl group;
an aryl group;
a heterocyclic group;
a $C_{2-6}$ cyclic amino group;
a group represented by Formula (III)
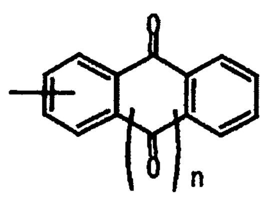

(III)

(where n represents 0 or 1); or
a group represented by Formula (IV-3)
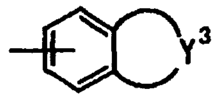

(IV-3)

(where $Y^3$ represents $-O-CH_2-CH=CH-$ or $-O-(CH_2)_q-O-$, in which q represents an integer of 1 to 3),
$R^{553}$ represents
a hydrogen atom;
a halogen atom;
an aryl group;
a $C_{1-10}$ alkyl group;
a $C_{1-6}$ alkanoyloxy-$C_{1-6}$ alkyl group;
a $C_{1-6}$ haloalkyl group;
a $C_{1-10}$ alkoxy group;
a $C_{1-6}$ alkylthio group;
a $C_{2-6}$ alkenyloxy group;
a $C_{2-6}$ alkenylthio group;
a $C_{1-6}$ haloalkoxy group;
a $C_{1-6}$ haloalkylthio group;
an aryl group that may be substituted with 1 to 3 halogen atoms or cyano groups;
a heterocyclic group;
an aryloxy group or arylthio group that may be substituted with a halogen atom(s) or a $C_{1-6}$ alkyl group(s);
a group represented by Formula $-N(R^{633})\ R^{733}$ (where $R^{633}$ and $R^{733}$ each represent a hydrogen atom, a $C_{1-6}$ alkyl group, a $C_{1-6}$ alkanoyl group, a di-$C_{1-6}$ alkylamino-$C_{2-6}$ alkanoyl group, or a heterocyclic group that may be substituted with a $C_{1-6}$ alkyl group(s), or $R^{633}$ and $R^{733}$, in combination with the adjacent nitrogen atom, form a cyclic amino group);
a cyano group;
a nitro group; or
a $C_{2-6}$ alkoxycarbonyl group,
$R^{563}$ represents
a hydrogen atom;
a halogen atom;
a $C_{1-10}$ alkyl group; or
a $C_{1-6}$ haloalkyl group,
$R^{573}$ represents
a hydrogen atom;
a $C_{1-10}$ alkyl group;
a halogen atom; or
a $C_{1-10}$ alkoxy group,
m represents an integer of 1 to 3},
and
W represents -CO-, -CO-CO-, -CO-NH-, -CS-NH- or $-SO_2-$], or a pharmaceutically acceptable salt thereof, as an active ingredient.

According to another aspect of the present invention, there is provided an imine compound represented by Formula (I-3)

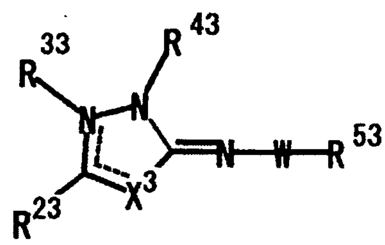

(I-3)

[where the broken line indicates that one of the bonds is a double bond,
$X^3$ represents $C(R^{13})$ , S or O,
$R^{13}$, $R^{23}$ and $R^{33}$ each represent
a hydrogen atom;
a $C_{1-10}$ alkyl group that may be substituted with an aryl group(s) substituted with a halogen atom(s);
a $C_{1-6}$ haloalkyl group;
a $C_{3-10}$ cycloalkyl group; or
an aryl group or aralkyl group that may be substituted with 1 to 3 halogen atoms, or
in the case where $X^3$ is $C(R^{13})$, $R^{13}$ and $R^{23}$ together represent a group represented by $-CH_2-S-CH_2-$ (with the proviso that, $R^{33}$ is not substituted in the case where $X^3$ is S or O),
$R^{43}$ represents
a 1,1-dioxothiolanyl group;
a $C_{1-10}$ alkyl group or $C_{2-6}$ alkenyl group that may be substituted with a group(s) selected from the group consisting of: a $C_{3-10}$ cycloalkyl group, a $C_{1-6}$ haloalkyl group and a $C_{1-6}$ alkoxy group; or
an aryl group,
$R^{53}$ represents
a hydrogen atom;
a $C_{1-10}$ alkoxy group;
a $C_{1-6}$ alkoxy-$C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a $C_{1-6}$ alkoxy group, a $C_{3-10}$ cycloalkyl group, a $C_{2-6}$ alkoxycarbonyl group, an aryl group or aryloxy group that may be substituted with 1 to 5 groups selected from the group consisting of a $C_{1-6}$ alkoxy group and a halogen atom, a heterocyclic group, a $C_{1-6}$ alkanoyloxy group, an aralkyloxy group, and a $C_{1-6}$ alkylthio group;
a group represented by Formula (II-3)

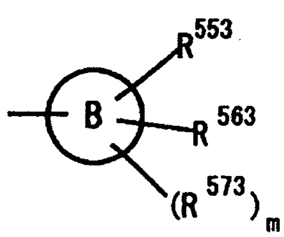

(II-3)

{where B represents
a $C_{3-10}$ cycloalkyl group;
an aryl group;
a heterocyclic group;

a $C_{2-6}$ cyclic amino group;
a group represented by Formula (III)
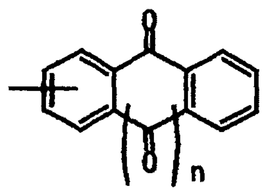

(III)

(where n represents 0 or 1); or
a group represented by Formula (IV-3)
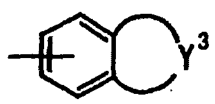

(IV-3)

(where $Y^3$ represents $-O-CH_2-CH=CH-$ or $-O-(CH_2)q-O-$, where q represents an integer of 1 to 3),
$R^{553}$ represents
a hydrogen atom;
a halogen atom;
an aryl group;
a $C_{1-10}$ alkyl group;
a $C_{1-6}$ alkanoyloxy-$C_{1-6}$ alkyl group;
a $C_{1-6}$ haloalkyl group;
a $C_{1-10}$ alkoxy group;
a $C_{1-6}$ alkylthio group;
a $C_{2-6}$ alkenyloxy group;
a $C_{2-6}$ alkenylthio group;
a $C_{1-6}$ haloalkoxy group;
a $C_{1-6}$ haloalkylthio group,
an aryl group that may be substituted with 1 to 3 halogen atoms or cyano groups;
a heterocyclic group;
an aryloxy group or arylthio group that may be substituted with a halogen atom(s) or a $C_{1-6}$ alkyl group(s);
a group represented by Formula $-N(R^{633})$ $R^{733}$ (where $R^{633}$ and $R^{733}$ each represent a hydrogen atom, a $C_{1-6}$ alkyl group, a $C_{1-6}$ alkanoyl group, a di-$C_{1-6}$ alkylamino-$C_{2-6}$ alkanoyl group, or a heterocyclic group that may be substituted with a $C_{1-6}$ alkyl group(s), or $R^{633}$ and $R^{733}$, in combination with the adjacent nitrogen atom, form a cyclic amino group);
a cyano group;
a nitro group; or a $C_{2-6}$ alkoxycarbonyl group;
$R^{563}$ represents
a hydrogen atom;
a halogen atom;
a $C_{1-10}$ alkyl group; or
a $C_{1-6}$ haloalkyl group,
$R^{573}$ represents
a hydrogen atom;
a $C_{1-10}$ alkyl group;

a halogen atom; or
a $C_{1-10}$ alkoxy group, and
m represents an integer of 1 to 3},
and
W represents -CO-, -CO-CO-, -CO-NH-, -CS-NH- or -$SO_2$-], or a pharmaceutically acceptable salt thereof.

In the present invention, $R^4$, $R^{41}$ $R^{42}$ or $R^{43}$ is preferably a $C_{2-6}$ alkenyl group, or a $C_{1-10}$ alkyl group substituted with a $C_{3-10}$ cycloalkyl group(s) or a $C_{1-10}$ alkoxy group(s), and further preferably, a $C_{1-10}$ alkyl group substituted with a $C_{3-10}$ cycloalkyl group(s).

In the present invention, a preferred compound is one where $R^5$, $R^{51}$ $R^{52}$ or $R^{53}$ is a $C_{1-10}$ alkyl group or $C_{2-6}$ alkenyl group that may be substituted with 1 to 3 groups selected from the group consisting of: a $C_{3-10}$ cycloalkyl group, an aryl group that may be substituted with a $C_{1-6}$ haloalkoxy group(s) or a halogen atom (s), a thienyl group, a halogen atom and an aryloxy group; or is a group represented by Formula (II), (II-1), (II-2) or (II-3);
$R^{55}$, $R^{551}$, $R^{552}$ or $R^{553}$ is a hydrogen atom; a halogen atom; a $C_{1-10}$ alkyl group; a $C_{1-6}$ haloalkyl group; a $C_{1-10}$ alkoxy group; a $C_{1-6}$ haloalkoxy group; a $C_{3-10}$ cycloalkyl group; an aryl group; a heterocyclic group that may be substituted with a $C_{1-6}$ alkyl group(s); an aryloxy group; a morpholino group; an arylamino group; a cyano group; a $C_{1-6}$ alkanoyl group; a $C_{2-6}$ haloalkanoyl group; or a $C_{1-6}$ alkylsulfonyl group, $R^{56}$, $R^{561}$ $R^{562}$ or $R^{563}$ is a hydrogen atom; a halogen atom; a $C_{1-6}$ haloalkyl group; or $C_{1-6}$ alkoxy group, and $R^{57}$, $R^{571}$ $R^{572}$ or $R^{573}$ is a hydrogen atom; a halogen atom; a $C_{1-10}$ alkyl group; or a $C_{1-10}$ alkoxy group.

Furthermore, a preferable compound is one where each of $R^5$, $R^{51}$, $R^{52}$ or $R^{53}$ is a group represented by Formula (II), (II-1) (II-2) or (II-3), B is a phenyl group, $R^{55}$, $R^{551}$ $R^{552}$ or $R^{553}$ is a halogen atom; a $C_{1-10}$ alkyl group; a $C_{1-6}$ haloalkyl group; a $C_{1-6}$ alkoxy group; a $C_{1-6}$ haloalkoxy group; a $C_{3-8}$ cycloalkyl group; an aryl group; an aryloxy group; a morpholino group; an arylamino group; a cyano group; a $C_{1-6}$ alkanoyl group; a $C_{2-6}$ haloalkanoyl group; or a $C_{1-6}$ alkylsulfonyl group, $R^{56}$, $R^{561}$, $R^{562}$ or $R^{563}$ is a hydrogen atom; a halogen atom; a $C_{1-6}$ haloalkyl group; or $C_{1-6}$ alkoxy group, and $R^{57}$, $R^{571}$, $R^{572}$ or $R^{573}$ is a hydrogen atom; a halogen atom; a $C_{1-6}$ alkyl group; or $C_{1-6}$ alkoxy group; and m is 1. Moreover, the most preferable compound is one where $R^5$, $R^{51}$, $R^{52}$ or $R^{53}$ is a phenyl group substituted with 1 to 3 groups selected from the group consisting of a halogen atom, a $C_{1-6}$ alkyl group, a $C_{1-6}$ haloalkyl group, a $C_{1-6}$ alkoxy group, a cyano group and a $C_{1-6}$ haloalkoxy group.

In formulas (I), (I-1), (I-2) and (I-3), the double bond made of the carbon atom and the nitrogen atom contained in the group represented by >C=N-CO- is preferably in (Z) configuration.

The imine compounds of the present invention include their prodrugs, hydrates and solvates.

The meaning of the terms used in the present specification will be explained below.
In the present invention, the term "$C_{X-Y}$" means that the group following the term has X to Y carbon atoms.
The term "halogen atom" refers to fluorine, chlorine, bromine or iodine.
The term "$C_{1-6}$ alkyl group" refers to a straight or branched alkyl group having 1 to 6 carbon atoms, and includes, for example, methyl group, ethyl group, n-propyl group, isopropyl group, n-butyl group, 2-butyl group, t-butyl group, 1,1-dimethylethyl group, n-pentyl group, isopentyl group, 1,1-dimethylpropyl group and n-hexyl group.

The term "$C_{1-10}$ alkyl group" refers to a straight or branched alkyl group having 1 to 10 carbon atoms, and includes, in addition to the specific examples mentioned above regarding the "$C_{1-6}$ alkyl group", 1,1,3,3-tetramethylbutyl group, n-nonanyl group and n-decyl group.

The term "$C_{1-6}$ haloalkyl group" refers to an alkyl group wherein the "$C_{1-6}$ alkyl group" as defined above is substituted with one or more halogen atoms, and includes, for example, fluoromethyl group, difluoromethyl group, trifluoromethyl group, 2,2,2-trifluoroethyl group, 2,2,2-trichloroethyl group, pentafluoroethyl group, 3,3,3-trifluoropropyl group, perfluoropropyl group, 4-fluorobutyl group, 4-chlorobutyl group, 4-bromobutyl group and perfluorohexyl group.

The term "$C_{1-6}$ alkoxy group" refers to a straight or branched alkoxy group having 1 to 6 carbon atoms, and includes, for example, methoxy group, ethoxy group, 1-propoxy group, isopropoxy group 1-butoxy group, 1-methyl-1-propoxy group, t-butoxy group and 1-pentyloxy group.

The term "$C_{1-10}$ alkoxy group" refers to a straight or branched alkoxy group having 1 to 10 carbon atoms, and includes, in addition to the specific examples mentioned above regarding the "$C_{1-6}$ alkoxy group", 1,1,3,3-tetramethylbutoxy group and n-decyloxy group.

The term "aryl group" refers to a mono- to tetracyclic aromatic carbocyclic group having 6 to 18 carbon atoms, and includes, for example, phenyl group, naphthyl group, anthoryl group, phenanthrenyl group, tetracenyl group and pyrenyl group. A phenyl group is preferred.

The term "$C_{3-10}$ cycloalkyl group" refers to a cycloalkyl group having 3 to 10 carbon atoms, and includes, for example, cyclopropyl group, cyclobutyl group, cyclopentyl group, cyclohexyl group, cycloheptyl group and adamantyl group.

The term "$C_{2-6}$ alkenyl group" refers to a straight or branched alkyl group having 2 to 6 carbon atoms and one or more double bonds at an arbitrary position of the "alkyl group" as defined above, and includes, for example, vinyl group, 1-propenyl group, 2-propenyl group, 2-butenyl group, 1,3-butadienyl group, 2-pentenyl group, 3-pentel group

and 2-hexenyl group.

The term "$C_{2-6}$ alkynyl group" refers to a straight or branched alkynyl group having 2 to 6 carbon atoms, and includes, for example, ethynyl group. 1-propynyl group and 2-propynyl group.

The term "$C_{2-6}$ alkoxycarbonyl group" refers to a group wherein the alkoxyl group as defined above is attached to a carbonyl group, and includes, for example, methoxycarbonyl group, ethoxycarbonyl group and t-butoxycarbonyl group.

The term "hydroxy-$C_{1-6}$ alkyl group" refers to a group
wherein the $C_{1-6}$ alkyl group as defined above is substituted with 1 to 2 hydroxyl groups and includes, for example, hydroxymethyl group, 2-hydroxyethyl group and 4-hydroxybutyl group.
The term "cyclic amino group" refers to a cyclic amino group having 2 to 6 carbon atoms, and includes, for example, pyrrolidino group, piperidino group, piperazino group, morpholino group and thiomorpholino group. In the present invention, the term thiomorpholino group includes its sulfur-dioxide form.

The term "$C_{1-6}$ haloalkoxy group" refers to an alkoxy group
wherein the "$C_{1-6}$ alkoxyl group" as defined above is substituted with one or more halogen atoms, and includes, for example, fluoromethoxy group difluoromethoxy group, trifluoromethoxy group, 2,2,2-trifluoroethoxy group, 2,2,2-trichloroethoxy group, pentafluoroethoxy group, perfluoropropoxy group, 4-fluorobutoxy group, 4-chlorobutoxy group, 4-bromobutoxy group and perfluorohexyloxy group.

The term "$C_{1-6}$ alkylthio group" refers to a straight or branched alkylthio group having 1 to 6 carbon atoms, and includes, for example, methylthio group, ethylthio group, n-propylthio group, isopropylthio group, n-butylthio group, 2-butylthio group, t-butylthio group, 1,1-dimethylethylthio group, n-pentylthio group, isopentylthio group 1,1-dimethylpropylthio group and n-hexylthio group.

The term "$C_{1-6}$ haloalkylthio group" refers to an alkylthio group wherein the $C_{1-6}$ alkylthio group as defined above is substituted with one or more halogen atoms, and includes, for example, fluoromethylthio group, difluoromethylthio group, trifluoromethylthio group, 2,2,2-trifluoroethylthio group, 2,2,2-trichloroethylthio group, pentafluoroethylthio group, 4-fluorobutylthio group, 4-chlorobutylthio group, 4-bromobutylthio group and perfluorohexylthio group.

Example of "arylthio group" may include phenylthio group and naphthylthio group.

The term "$C_{1-6}$ alkenylthio group" refers to a straight or branched alkenylthio group having 2 to 6 carbon atoms, and includes, for example, vinylthio group, 1-propenylthio group, 2-propenylthio group, 2-butenylthio group, 1,3- butadienylthio group, 2-pentenylthio group, 3-pentenylthio group and 2-hexenylthio group.

The term "$C_{1-6}$ alkanoyl group" refers to a straight or branched alkanoyl group having 1 to 6 carbon atoms, and includes, for example, formyl group, acetyl group, propionyl group, isopropionyl group, butyryl group and pivaloyl group.

The term "$C_{1-6}$ alkanoyloxy group" refers to a group
wherein the $C_{1-6}$ alkanoyl group as defined above is attached to an oxy group, and includes, for example, acetyloxy group, propionyloxy group and pivaloyloxy group.

The term "$C_{1-6}$ alkanoyloxy-$C_{1-6}$ alkyl group" refers to a group wherein the $C_{1-6}$ alkanoyloxy as defined above is attached to a $C_{1-6}$ alkyl group, and includes, for example, acetyloxyethyl group, propionyloxymethyl group and pivaloyloxymethyl group.
The term "$C_{2-6}$ haloalkanoyl group" refers to an alkanoyl group wherein the "$C_{2-6}$ alkanoyl group" is substituted with a halogen atom(s), and includes, for example, fluoroacetyl group, trifluoroacetyl group, 2,2,2-trifluoropropionyl group, 2,2,2-trichloropropionyl group, 4-fluorobutyryl group, 4-chlorobutyryl group and 4-bromobutyryl group.

The term "$C_{1-6}$ alkoxy-$C_{1-6}$ alkoxy group" refers to a group formed by binding two $C_{1-6}$ alkoxy groups, and includes, for example, methoxymethoxy group, methoxypropoxy group, ethoxypropoxy group and heptyloxyethoxy group.

The term "$C_{1-6}$ alkoxy-$C_{1-6}$ alkyl group" refers to a group formed by binding a $C_{1-6}$ alkoxy group and a $C_{1-6}$ alkyl group, and includes, for example, methoxymethyl group, methoxypropyl group, ethoxypropyl group and heptyloxyethyl group.

The term "aryloxy group" refers to a group having an oxygen atom and the aryl group as defined above to be attached via said oxygen atom to another group, and includes, for example, phenoxy group and naphthoxy group.

The term "aralkyl group" refers to a group formed by binding an aryl group and an alkyl group, and includes, for example, a benzyl group, phenethyl group and naphthylmethyl group.

The term "aralkyloxy group" refers to a group formed by binding an aralkyl group and an oxy group, and includes, for example, benzyloxy group, phenethyloxy group and naphthylmethoxy group.

The term "heterocyclic group" refers to a heteromonocyclic group or a fused heterocyclic group containing 1 to 3 atoms selected from the group consisting of a nitrogen atom, oxygen atom and sulfur atom as a ring constituent atom, and includes a saturated heterocyclic group, an aromatic heterocyclic group, and a fused heterocyclic group having a partially saturated aromatic heteromonocyclic group. Furthermore, the fused heterocyclic group having a partially saturated aromatic heteromonocyclic group can be substituted with =O. As the heterocyclic group, a heterocyclic group

having 5 to 10 atoms in the cycle is preferred.

Examples of the saturated heterocyclic group may include aziridinyl group, azetidinyl group, pyrrolidinyl group, imidazolidyl group, pyrazolidinyl group, oxolanyl group, thiolanyl group, piperidinyl group, piperazinyl group and morpholinyl group

Examples of the aromatic heterocyclic group may include pyridyl group, pyridazinyl group, pyrimidinyl group, pyrazinyl group, quinolyl group, isoquinolyl group, thienyl group (for example, 2-thienyl group, 3-thienyl group), pyrrolyl group (for example, 1-pyrrolyl group, 2-pyrrolyl group, 3-pyrrolyl group), thiazolyl group (for example, 2-thiazolyl group, 4-thiazolyl group, 5-thiazolyl group), isothiazolyl group (for example, 3-isothiazolyl group, 4-isothiazolyl group, 5-isothiazolyl group), pyrazolyl group (for example, 1-pyrazolyl group, 3-pyrazolyl group, 4-pyrazolyl group), imidazolyl group (for example, 1-imidazolyl group, 2-imidazolyl group, 3-imidazolyl group), furyl group (for example, 2-furyl group, 3-furyl group), oxazolyl group (for example, 2-oxazolyl group, 4-oxazolyl group, 5-oxazolyl group), isoxazolyl group (for example, 3-isoxazolyl group, 4-isoxazolyl group, 5-isoxazolyl group), oxadiazolyl group (for example, 1,2,3-oxadiazolyl group, 1,3,4-oxadiazolyl group), thiadiazolyl group (for example, 1,2,3-thiadiazolyl bases, 1,3,4-thiadiazolyl group), triazolyl group (for example, 1,2,4-triazolyl group), benzofuranyl group (for example, 2-benzofuranyl group, 3-benzofuranyl group, 4-benzofuranyl group, 5-benzofuranyl group), benzothienyl group (for example, 2-benzothienyl group, 3-benzothienyl group, 4-benzothienyl group, 5-benzothienyl group), indolyl group (for example, 2-indolyl group, 3-indolyl group, 4-indolyl group, 5-indolyl group), benzoxazolyl group (for example, 2-benzoxazolyl group, 4-benzoxazolyl group, 5-benzoxazolyl group, 6-benzoxazolyl group), benzisoxazolyl group (for example, 3-benzo[c]isoxazolyl group, 4-benzo[c]isoxazolyl group, 5-benzo[c]isoxazolyl group, 6-benzo[c]isoxazolyl group, 3-benzo[d]isoxazolyl group, 4-benzo[d]isoxazolyl group, 5-benzo[d]isoxazolyl group, 6-benzo[d] isoxazolyl group), indazolyl group (for example, 3-indazolyl group, 4-indazolyl group, 5-indazolyl group, 6-indazolyl group), benzimidazolyl group (for example, 2-benzimidazolyl group, 4-benzimidazolyl group, 5-benzimidazolyl group, 6-benzimidazolyl group), benzoxadiazolyl group (for example, 4- benzo[1,2,5]oxadiazolyl group, 5-benzo[1,2,5]oxadiazolyl group, 4-benzo[1,2,3]oxadiazolyl group, 5-benzo[1,2,3]oxadiazolyl group), benzothiadiazolyl group (for example, 4- benzo[1,2,5]thiadiazolyl group, 5-benzo[1,2,5]thiadiazolyl group, 4-benzo[1,2,3] thiadiazolyl group, 5-benzo[1,2,3] thiadiazolyl group), indolidinyl group (for example, 1-indolidinyl group, 2-indolidinyl group, 3-indolidinyl group, 5-indolidinyl group), thienopyridyl group (for example, 2-thieno[2,3-b]pyridyl group, 3-thieno [2,3-b]pyridyl group, 5-thieno[2,3-b]pyridyl group, 6-thieno[2,3-b]pyridyl group, 2-thieno[3,2-b]pyridyl group, 3-thieno [3,2-b]pyridyl group, 5-thieno[3,2-b]pyridyl group, 6-thieno[3,2-b]pyridyl group), pyrazolopyridyl group (for example, 2-pyrazolopyridyl group, 3-pyrazolopyridyl group, 5-pyrazolopyridyl group, 6-pyrazolo pyridyl group), imidazopyridyl group (for example, 1-imidazo[1,5-alpyridyl group, 3-imidazo[1,5-a]pyridyl group, 5-imidazo[1,5-a]pyridyl group, 7-imidazo[1,5-a]pyridyl group, 2-imidazo[1,2-a]pyridyl group, 3-imidazo[1,2-a]pyridyl group, 5-imidazo[1,2-a]pyridyl group, 7-imidazo [1,2-a]pyridyl group), imidazopyrazyl group (for example, 1-imidazo[1,5-a]pyrazyl group, 3-imidazo[1,5-a]pyrazyl group, 5-imidazo[1,5-a]pyrazyl group, 8-imidazo[1,5-a]pyrazyl group, 2-imidazo[1,2-a]pyrazyl group, 3-imidazo[1,2-a]pyrazyl group, 5-imidazo[1,2-a]pyrazyl group, 8-imidazo[1,2-a]pyrazyl group), pyrazolopyrimidyl group (for example, 2-pyrazolo [1,5-a]pyrimidyl, 3-pyrazolo[1,5-a]pyrimidyl group, 5-pyrazolo[1,5-a]pyrimidyl group, 6-pyrazolo[1,5-a]pyrimidyl group, 2-pyrazolo[1,5-c]pyrimidyl group, 3-pyrazolo[1,5-c]pyrimidyl group, 4-pyrazolo[1,5-c]pyrimidyl group, 5-pyrazolo[1,5-c] pyrimidyl group), triazolopyrimidyl group (for example, 3-[1,2,3]triazolo[1,5-a]pyrimidyl group, 5-[1,2,3]triazolo[1,5-a] pyrimidyl group, 6-[1,2,3]triazolo[1,5-a]pyrimidyl group, 3-[1,2,3]triazolo[1,5-c]pyrimidyl group, 4-[1,2,3]triazolo[1,5-c] pyrimidyl group, 5-[1,2,3]triazolo[1,5-c]pyrimidyl group, 2-[1,2,4]triazolo[1,5-a]pyrimidyl group, 5-[1,2,4]triazolo [1,5-a] pyrimidyl group, 6-[1,2,4] triazolo[1,5-a]pyrimidyl group, 7-[1,2,4]triazolo[1,5-a]pyrimidyl group, 2-[1,2,4]triazolo[1,5-c] pyrimidyl group, 5-[1,2,4]triazolo[1,5-c]pyrimidyl group, 7-[1,2,4]triazolo[1,5-c]pyrimidyl group, 8-[1,2,4]triazolo[1,5-c]pyrimidyl group), thienothienyl group (for example, 2-thieno[2,3-b]thienyl group, 3-thieno[2,3-b]thienyl group, 2-thieno[3,2-b]thienyl group, 3-thieno[3,2-b]thienyl group), imidazothiazolyl group (for example, 2-imidazo[2,1-b]thiazolyl group, 3-imidazo[2,1-b]thiazolyl group, 5-imidazo[2,1-b]thiazolyl group, 2-imidazo[5,1-b]thiazolyl group, 3-imidazo[5,1-b]thiazolyl group, and 5-imidazo[5,1-b]thiazolyl group).

Examples of the fused heterocyclic group having a partially saturated aromatic heteromonocyclic group may include tetrahydrobenzofuranyl group, tetrahydrobenzothienyl group, tetrahydrobenzopyrrolyl group, 2,3-dihydro-1H-benzofuranyl group, 2,3-dihydro-1H-benzothienyl group, 2,3-dihydro-1H-indolyl group, 2,3-dihydro-1H-indazolyl group, 2,3-dihydro-1H-benzotriazolyl group, 2,3-dihydro-1H-benzoxazolyl group, 2,3-dihydro-1H-benzothiazolyl group, benzo [1,3]oxathiolyl group, benzo[1,3]dioxolyl group, 2H-chromenyl group, chromanyl group, indolynyl group and isoindolynyl group.

Examples of the fused heterocyclic group having the partially saturated monocycle and substituted with =O may include 2-oxo-1,3-dihydro-1H-indolyl ring, 3-oxo-1,2-dihydro-1H-indazolyl ring, 2-oxo-3H-benzoxazolyl ring, 2-oxo-3H-benzothiazolyl ring, 2-oxo-benz[1,3]oxathiolyl ring, 2-oxo-benz[1,3]dioxolyl ring and 2-oxo-chlomenyl ring.

Preferable examples of the heterocyclic ring of B ring may include pyridyl group, pyridazinyl group, pyrimidinyl group, pyrazinyl group, quinolyl group and isoquinolyl group.

The term "$C_{1-6}$ alkylsulfenyl group" refers to a group having SO and the "$C_{1-6}$ alkyl group(s)" as defined above to be attached via said SO to another group, and includes, for example, methylsulfenyl group, ethylsulfenyl group, n-propylsulfenyl group, n-butylsulfenyl group, t-butylsulfenyl group and n-pentylsulfenyl group.

The term "$C_{1-6}$ alkylsulfonyl group that may be substituted with a halogen atom(s)" refers to a group having a sulfonyl group and the "$C_{1-6}$ alkyl group(s)" as defined above or the "$C_{1-6}$ haloalkyl group(s)" as defined above to be attached via said sulfonyl group to another group. Examples thereof may include methylsulfonyl group, ethylsulfonyl group, n-propylsulfonyl group, n-butylsulfonyl group, t-butylsulfonyl group, n-pentylsulfonyl group, fluoromethylsulfonyl group, difluoromethylsulfonyl group, trifluoromethylsulfonyl group, 2,2,2-trifluoroethylsulfonyl group, 2,2,2-trichloroethylsulfonyl group, pentafluoroethylsulfonyl group, 4-fluorobutylsulfonyl group, 4-chlorobutylsulfonyl group and 4-bromobutylsulfonyl group.

The term "arylsulfonyl group that may be substituted with a halogen atom(s)" refers to an arylsulfonyl group wherein the aryl may be substituted with a halogen atom(s). Examples thereof may include phenylsulfonyl group, 4-chlorophenylsulfonyl group, 4-fluorophenylsulfonyl group, 2,4-dibromophenylsulfonyl group, 2,4-difluorophenylsulfonyl group, naphthylsulfonyl group and 6-bromonaphthylsulfonyl group.

The term "prodrug" refers to a compound that is hydrolyzed in vivo to regenerate an imine compound having a cannabinoid receptor agonist effect.

The term "pharmaceutically acceptable salt" refers to an acid addition salt or a base addition salt. Examples of the acid addition salt may include inorganic salts such as hydrochloride, hydrobromate and sulfate, and organic salts such as citrate, oxalate, malate, tartrate, fumarate, maleate, methanesulfonate, ethanesulfonate, benzenesulfonate, para-toluenesulfonate, benzoate, aspartate and glutamate. Examples of the base addition salt may include inorganic base salts such as a sodium salt, calcium salt, magnesium salt, calcium salt and aluminum salt, and organic salts such as an ethanolamine salt, lysine salt, ornithine salt, meglumine salt and trishydroxymethylaminomethane salt, and ammonium salts.

BEST MODE FOR CARRYING OUT THE INVENTION

A compound according to the present invention can be produced by the following (1) to (4) steps.

(1) A compound (Ia) according to the present invention can be produced from an amine compound (V) by a process represented by the following reaction scheme.

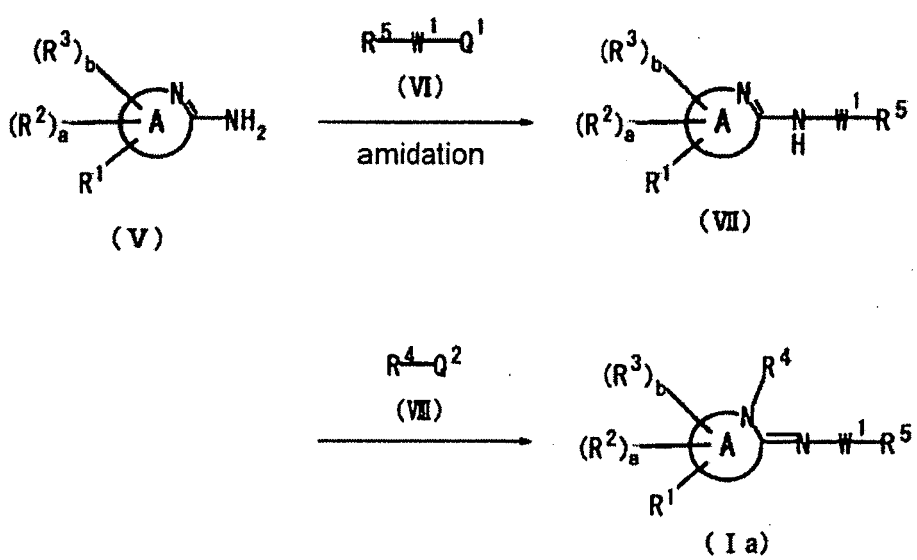

(V) (VI) (VII) (VIII) (Ia)

[where A, $R^1$, $R^2$, $R^3$, $R^4$, $R^5$, a and b are the same as defined above; $Q^1$ represents a hydroxyl group or a halogen atom such as chlorine atom and bromine atom; $Q^2$ represents a leaving group such as a chlorine atom, bromine atom, iodine atom, methanesulfonyloxy group, trifluoromethanesulfonyloxy group or para-toluenesulfonyloxy group; and $W^1$ represents -CO-, -CO-CO-or $-SO_2-$].

Step 1: Production of amido compound (VII)

(i) An amino compound (VII) can be produced by an amidation reaction using an amine compound (V) and a

compound (VI).

When $Q^1$ of a compound (VI) is a halogen atom, the reaction is preferably performed in the presence of a base. Examples of the base to be used may include alkali metal hydroxides (such as lithium hydroxide, sodium hydroxide, potassium hydroxide), alkali metal carbonates (such as lithium carbonate, sodium carbonate, potassium carbonate), alkali metal bicarbonates (such as sodium hydrogencarbonate, potassium hydrogencarbonate), and organic bases (such as triethylamine, diisopropylethylamine, tri-n-butylamine, 1,5-diazabicyclo[4.3.0]-5-nonene, 1,8-diazabicyclo[5.4.0]-7-undecene, pyridine, N,N-dimethylaminopyridine)

As the reaction temperature, a cooling temperature to the boiling point of the solvent or reagent to be used, in particular, a temperature from -20˚C to room temperature is preferable.

The reaction can be performed in the presence or absence of a solvent. Examples of the solvent to be used may include dioxane, tetrahydrofuran, diethyl ether, petroleum ether, n-hexane, cyclohexane, benzene, toluene, xylene, chlorobenzene, pyridine, acetonitrile, ethyl acetate, ethylmethylketone, N,N-dimethylformamide, dimethylsulfoxide, dichloromethane, chloroform, carbon tetrachloride and water.

In the reaction, it is preferable that the type of solvent and reagent to be used and amounts thereof may be appropriately selected, depending on the substrate to be used in the reaction and reaction conditions.

When $Q^1$ of the compound (VI) is a hydroxyl group, a condensing agent is preferably used. Examples of the condensing agent may include acid halogenating agents such as thionyl chloride and oxalyl chloride, alkyl chlorocarbonates such as ethyl chlorocarbonate, carbodiimide compounds such as dicyclohexylcarbodiimide, 1-ethyl-3-(3-dimethylamino)propylcarbodiimide, sulfonyl chloride compounds such as methanesulfonyl chloride, phosphorus compounds such as diphenyl phosphite, diphenylphosphoryl chloride, triphenylphosphine-diethylazodicarboxylate, and N,N'-carbodiimidazole.

The reaction can be performed in the presence or absence of a solvent. Examples of the solvent to be used include methanol, ethanol, n-propanol, isopropanol, n-butanol, t-butanol, dioxane, tetrahydrofuran, diethyl ether, petroleum ether, n-hexane, cyclohexane, benzene, toluene, xylene, chlorobenzene, pyridine, ethyl acetate, N,N-dimethylformamide, dimethylsulfoxide, dichloromethane, chloroform, carbon tetrachloride and water.

In the reaction, it is preferable that the type of solvent and reagent to be used and amounts thereof may be appropriately selected depending on the substrate to be used in the reaction and reaction conditions.

(ii) When $W^1$ of the compound (VI) is -CO- or -CO-CO-, the amide compound (VII) can be produced by using an acid anhydride of the compound (VI) or mixed acid anhydride in place of the compound (VI).

The reaction is preferably performed in the presence of a base. Examples of the base to be used may include alkali metal hydroxides (such as lithium hydroxide, sodium hydroxide, potassium hydroxide), alkali metal carbonates (such as lithium carbonate, sodium carbonate, potassium carbonate), alkali metal bicarbonates (such as sodium hydrogencarbonate, potassium hydrogencarbonate), and organic bases (such as triethylamine, diisopropylethylamine, tri-n-butylamine, 1,5-diazabicyclo[4.3.0]-5-nonene, 1,8-diazabicyclo[5.4.0]-7-undecene, pyridine, N,N-dimethylaminopyridine).

As the reaction temperature, a cooling temperature to the boiling point of the solvent or reagent to be used is preferable.

The reaction can be performed in the presence or absence of a solvent. Examples of the solvent to be used may include methanol, ethanol, n-propanol, isopropanol, n-butanol, t-butanol, dioxane, tetrahydrofuran, diethyl ether, petroleum ether, n-hexane, cyclohexane, benzene, toluene, xylene, chlorobenzene, pyridine, acetonitrile, ethyl acetate, ethylmethylketone, N,N-dimethylformamide, dimethylsulfoxide, dichloromethane, chloroform, carbon tetrachloride, and water.

In the reaction, it is preferable that the type of solvent and reagent to be used and amounts thereof may be appropriately selected depending on the substrate to be used in the reaction and reaction conditions.

Step 2: Production of the compound (Ia) of the present invention

The compound (Ia) of the present invention can be produced by reacting an amide compound (VII) and a compound (VIII).

The reaction is preferably performed in the presence of a base. Examples of the base to be used may include alkali metal hydroxides (such as lithium hydroxide, sodium hydroxide, potassium hydroxide), alkali metal carbonates (such as lithium carbonate, sodium carbonate, potassium carbonate), alkali metal bicarbonates (such as sodium hydrogencarbonate, potassium hydrogencarbonate), alkali metal hydrides (such as sodium hydride, potassium hydride), alkali metals (such as metallic sodium, metallic potassium), organic bases (such as triethylamine, diisopropylethylamine, tri-n-butylamine, 1,5-diazabicyclo[4.3.0]-5-nonene, 1,8-diazabicyclo[5.4.0]-7-undecene, pyridine, N,N-dimethylaminopyridine), alkali metal amides (such as sodium amide), alkali metal alkoxides (such as sodium methoxide, sodium ethoxide, t-butoxy potassium), organometallic compounds (such as n-butyllithium, s-butyllithium, t-butyllithium, lithium diisopropylamide, sodium bis(trimethylsilyl)amide).

As the reaction temperature, a cooling temperature to the boiling point of the solvent or reagent to be used is preferable.

The reaction can be performed in the presence or absence of a solvent. Examples of the solvent to be used may include methanol, ethanol, n-propanol, isopropanol, n-butanol, t-butanol, dioxane, tetrahydrofuran, diethyl ether, petroleum ether, n-hexane, cyclohexane, benzene, toluene, xylene, chlorobenzene, pyridine, ethyl acetate, N,N-dimethylformamide, dimethylsulfoxide, dichloromethane, chloroform, and carbon tetrachloride.

In the reaction, it is preferable that the type of solvent and reagent to be used and amounts thereof may be appropriately selected depending on the substrate to be used in the reaction and reaction conditions.

(2) A compound according to the present invention where W is -CO-NH- or -CS-NH- can be produced by use of an amide compound (VII) where $W^1$ is -CO-NH- or -CS-NH- in a similar manner to the process shown in Step 2 of Section (1).

A compound where $W^1$ of an amide compound (VII) is -CO-NH-or -CS-NH- can be produced by use of an amine compound (V) and a compound: $R^5$-NCO and a compound: $R^5$-NCS.

The reaction can be performed in the presence or absence of a solvent.

As the reaction temperature, a cooling temperature to the boiling point of the solvent or reagent to be used is preferable. Examples of the solvent to be used may include dioxane, tetrahydrofuran, diethyl ether, petroleum ether, n-hexane, cyclohexane, benzene, toluene, xylene, chlorobenzene, pyridine, acetonitrile, ethyl acetate, ethylmethylketone, N,N-dimethylformamide, dimethylsulfoxide, dichloromethane, chloroform, and carbon tetrachloride.

In the reaction, it is preferable that the type of solvent and reagent to be used and amounts thereof may be appropriately selected depending on the substrate to be used in the reaction and reaction conditions.

(3) A compound (I) according to the present invention can be produced by using an imine compound represented by the following formula (IX)

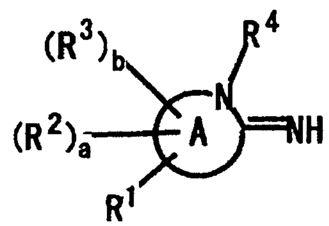

(IX)

(where $R^1$, $R^2$, $R^3$, $R^4$, a and b are the same as defined above) and a compound (VI), a compound: $R^5$-N=C=O or a compound: $R^5$-N=C=S, in the same manner as the process shown in Step 1 of Section (1) or the process shown in Section (2).

An imine compound (IX) can be produced by hydrolyzing a compound (I) according to the present invention. In particular, $R^5$ is preferably a hydrogen atom, a $C_{1-10}$ alkyl group, or a $C_{1-6}$ haloalkyl group. Examples of the hydrolysis reaction may include acid hydrolysis using hydrochloric acid, sulfuric acid, acetic acid, trifluoroacetic acid, methanesulfonic acid, phosphoric acid, and polyphosphoric acid, etc., singly or in any combination; and alkali hydrolysis using lithium hydroxide, sodium hydroxide, potassium hydroxide, lithium carbonate, sodium carbonate, potassium carbonate, sodium hydrogencarbonate, potassium hydrogencarbonate and ammonia, etc.

The reaction can be performed in the presence or absence of a solvent. Examples of the solvent to be used may include methanol, ethanol, n-propanol, isopropanol, n-butanol, t-butanol, dioxane, tetrahydrofuran, diethyl ether, petroleum ether, n-hexane, cyclohexane, benzene, toluene, xylene, chlorobenzene, pyridine, N,N-dimethylformamide, dimethylsulfoxide, dichloromethane, chloroform, carbon tetrachloride and water.

As the reaction temperature, a cooling temperature to the boiling point of the solvent or reagent to be used, particularly 0˚C to 100˚C is preferable.

In the hydrolytic reaction, it is preferable that the type of solvent and reagent to be used and amounts thereof may be appropriately selected depending on the substrate to be used in the reaction and reaction conditions.

(4) A compound according to the present invention represented by the following formula (Ib)

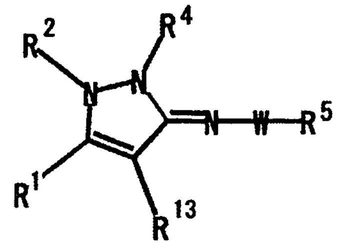

(Ib)

(where $R^1$, $R^2$, $R^3$, $R^4$ , $R^5$, $R^{13}$ and W are the same as defined above) can be produced by the processes shown in the following reaction schemes (i) and (ii).

(i) Pyrazole compound (X)
A compound represented by

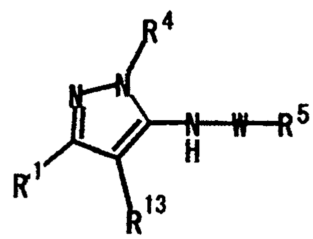

(X)

(where $R^1$, $R^4$, RS , $R^{13}$ and W are the same as defined above) is reacted with a compound: $R^2$- $Q^3$ (where $R^2$ is the same as defined above, $Q^3$ is a leaving group such as a chlorine atom, bromine atom, iodine atom, methanesulfonyloxy group, trifluoromethanesulfonyloxy group or para-toluenesulfonyloxy group), in the presence or absence of a base, to obtain a compound (Ib) according to the present invention.

Examples of the base to be used may include alkali metal hydroxides (such as lithium hydroxide, sodium hydroxide, potassium hydroxide), alkali metal carbonates (such as lithium carbonate, sodium carbonate, potassium carbonate), alkali metal bicarbonates (such as sodium hydrogencarbonate, potassium hydrogencarbonate), alkali metal hydrides (such as sodium hydride, potassium hydride), alkali metals (such as metallic sodium, metallic potassium), organic bases (such as triethylamine, diisopropylethylamine, tri-n-butylamine, 1,5-diazabicyclo[4.3.0]-5-nonene, 1,8-diazabicyclo[5.4.0]-7-undecene, pyridine, N,N-dimethylaminopyridine), alkali metal amides (such as sodium amide), alkali metal alkoxides (such as sodium methoxide, sodium ethoxide, t-butoxy potassium), organometallic compounds (such as n-butyllithium, s-butyllithium, t-butyllithium, lithium diisopropylamide, sodium bis(trimethylsilyl)amide).

As the reaction temperature, a cooling temperature to the boiling point of the solvent or reagent to be used is preferable.

The reaction can be performed in the presence or absence of a solvent. Examples of the solvent to be used may include dioxane, tetrahydrofuran, diethyl ether, petroleum ether, n-hexane, cyclohexane, benzene, toluene, xylene, chlorobenzene, pyridine, ethyl acetate, N,N-dimethylformamide, dimethylsulfoxide, dichloromethane, chloroform, and carbon tetrachloride.

In the reaction, it is preferable that the type of solvent and reagent to be used and amounts thereof may be appropriately selected depending on the substrate to be used in the reaction and reaction conditions.

(ii) The compound: $R^2$- $Q^3$ used in the process shown in reaction scheme (i) of the Step (4) is replaced by a compound: $(R^2O)_2SO_2$ (where $R^2$ is the same as defined above) and the same reaction is performed to obtain a compound (Ib) according to the present invention.

Furthermore, when a compound according to the present invention is produced, depending upon the type of functional group, it is sometimes effective to protect the functional group of a raw material or an intermediate in a production process, or to convert it into a group that can be easily converted into the functional group. Examples of such

a functional group include amino group, hydroxyl group and carboxyl group. Examples of the protecting group include a general protecting group for an amino group, a hydroxyl group, and a carboxyl group. It is preferable that the reaction temperature of protecting and de-protecting procedures, the types of solvent and reagent to be used and amounts thereof are appropriately selected depending upon a substrate to be used in the reaction and reaction conditions thereof.

A compound according to the present invention can be administered orally or parenterally. Examples of dosage form may include tablet, encapsulation, granular, pulvis, powdery, troche, ointment, cream, emulsion, suspension, suppository, and injection forms. These dosage forms can be prepared by a customary preparation technique (for example, a method defined in the 14th revision of the Japanese Pharmacopeia). The dosage form can be appropriately selected depending on the symptom and age of a patient and the therapeutic purpose. When such various types of dosage forms are prepared, conventional excipients (for example, crystalline cellulose, starch, lactose, mannitol), binders (for example, hydroxypropylcellulose, polyvinylpyrrolidone), lubricants (for example, magnesium stearate, talc), and disintegrators (for example, calcium carboxymethylcellulose) can be used.
The dose of a compound according to the present invention is 1 to 2000 mg per day per adult. This is administered once per day or by dividing it several portions. The dosage may be appropriately increased or decreased depending on the age, weight and symptom of a patient.

Examples

The present invention will be more specifically described by way of Examples and Experimental examples below, which should not be construed as limiting the invention.

Example 1

Production of 1-cyclopropylmethyl-2-(trifluoroacetylimino)-1,2-dihydropyridine (Compound No. 1)

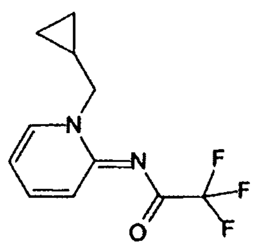

To a solution of 2-aminopyridine (2.0 g) and pyridine (1.9 ml) in chloroform (20 ml), trifluoroacetic anhydride (3.3 ml) was added under ice cooling. The mixture was stirred at room temperature for three days. The reaction solution was washed sequentially with water and saturated brine and dried over anhydrous sodium sulfate. After filtration, the filtrate was concentrated under vacuum. The residue obtained was purified by silica gel column chromatography (developing solvent: n-hexane:ethyl acetate=3:1) to obtain colorless liquid of 2-(trifluoroacetylamino)pyridine (2.3 g).

The obtained 2-(trifluoroacetylamino)pyridine (1.3 g) was dissolved in N,N-dimethylformamide (13 ml). To the solution, sodium iodide (0.01 g), 60% sodium hydride (0.27 g) and cyclopropylmethyl bromide (1.1 g) were added at room temperature. The reaction solution was stirred at 50˚C for 5 hours and returned to room temperature. After water was added, the reaction solution was extracted with ethyl acetate, washed with saturated brine, dried over anhydrous sodium sulfate, and filtrated. Thereafter the filtrate was concentrated under vacuum. The residue obtained was purified by silica gel column chromatography (developing solvent: chloroform:methanol=18:1) to obtain colorless crystals of 1-cyclopropylmethyl-2-(trifluoroacetylimino)-1,2-dihydropyridine (1.4 g).
$^1$H-NMR, MS (ESI) and the melting point are shown in Table 8.

Example 2

Production of 1-cyclopropylmethyl-2-{3-(trifluoromethyl)benzoylimino}-1,2-dihydropyridine (Compound No. 2)

(1) 1-cyclopropylmethyl-2-imino-1,2-dihydropyridine

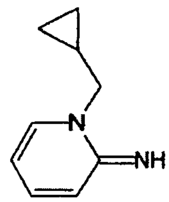

1-cyclopropylmethyl-2-(trifluoroacetylimino)-1,2-dihydropyridine (1.1 g) produced by the process shown in Example 1 was dissolved in methanol (25 ml). To this, an aqueous solution having anhydrous potassium carbonate (1.2 g) dissolved in water (12.5 ml) was added at room temperature and stirred for 2 hours. The reaction solution was extracted with ethyl acetate, dried over anhydrous sodium sulfate, and filtrated. Thereafter, the filtrate was concentrated under vacuum. The residue obtained was purified by silica gel column chromatography (developing solvent: chloroform:methanol=18:1) to obtain a yellow solution of 1-cyclopropylmethyl-2-imino-1,2-dihydropyridine (0.5 g).
$^1$H-NMR (200 MHz, CHLOROFORM-d) d ppm; 0.31-0.37 (m, 2H), 0.58-0.67 (m, 2H), 1.23-1.43 (m, 1H), 3.69 (d, J=6.0 Hz, 2H), 5.70 (m, 1H), 6.29 (d, J=8.0, 1H), 6.76(m, 1H), 7.04 (m, 1H)
MS(ESI)(Positive)m/z; 149 $(M+H)^+$

(2) 1-cyclopropylmethyl-2-{3-(trifluoromethyl)benzoylimino}-1,2-dihydropyridine (Compound No. 2)

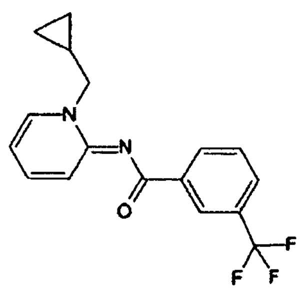

To a solution of 1-cyclopropylmethyl-2-imino-1,2-dihydropyridine (0.20 g) and triethylamine (0.19 ml) in chloroform (2 ml), 3-(trifluoromethyl)benzoyl chloride (0.24 ml) was added under ice cooling. The reaction solution was stirred at room temperature for 17 hours and water was added thereto. The reaction solution was extracted with chloroform, dried over anhydrous sodium sulfate, and filtrated. Thereafter, the filtrate was concentrated under vacuum and the residue obtained was purified by silica gel column chromatography (developing solvent: n-hexane:ethyl acetate=3:1) to obtain a colorless solid substance of 1-cyclopropylmethyl-2-{3-(trifluoromethyl)benzoylimino}-1,2-dihydropyridine (0.44 g).
$^1$H-NMR, MS(ESI) and the melting point are shown in Table 8.

Example 3

Production of 1-cyclopropylmethyl-2-{3-(trifluoromethyl)phenylsulfonylimino}-1,2-dihydropyridine (Compound No. 50)

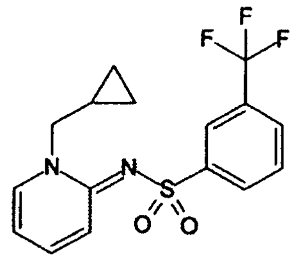

A process in line with the process shown in Example 2 was performed using 3-(trifluoromethyl)phenylsulfonyl chloride in place of 3-(trifluoromethyl)benzoyl chloride to obtain a colorless solid substance of l-cyclopropylmethyl-2-{3-(trifluoromethyl)phenylsulfonylimino}-1,2-dihydropyridine.
$^1$H-NMR, Mass and the melting point are shown in Table 8.

Compounds Nos. 3-49 and 51 to 59 shown in Table 1; compounds Nos. 60, 71 to 107, 116 to 174, 178 to 213, 215 to 342, 343 to 351, and 369 to 372; and compounds Nos. 504 to 515 shown in Table 3 were obtained in line with the methods shown in Examples 1 and 2.

$^1$H-NMR, Mass and the melting points of these compounds are shown in Tables 8, 9 and 11.

Example 4

Production of 5-t-butyl-3-(2-ethoxyethyl)-4-methyl-2-(3-trifluoromethylbenzoyl)imino-2,3-dihydrothiazole (Compound No. 352)

(1) 5-t-butyl-4-methyl-2-(3-trifluoromethylbenzoyl)aminothiazole

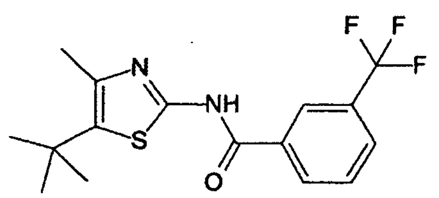

A solution of 2-amino-5-t-butyl-4-methylthiazole (1.0 g), 3-(trifluoromethyl)benzoic acid (1.2 g), 1-ethyl-3-(3-dimethylaminopropyl)carbodiimide monohydrochloride (1.2 g) and 1-hydroxybenzotriazole monohydrate (1.0 g) in N, N,-dimethyl formamide (10 ml) was stirred at room temperature for 48 hours. To the reaction solution, ethyl acetate was added and the reaction solution was washed sequentially with 2M hydrochloric acid, an aqueous 2M sodium hydroxide solution, and saturated brine, dried over anhydrous sodium sulfate and filtrated. Thereafter, the filtrate was concentrated under vacuum to obtain a yellow amorphous substance of 5-t-butyl-4-methyl-2-(3-trifluoromethylbenzoyl)aminothiazole (1.7 g).
$^1$H-NMR (200 MHz, CHLOROFORM-D) d ppm; 1.42(s, 9H), 2.20(s, 3H), 7.55 (t, J=7.5 Hz, 1H), 7.78 (d, J=7.5 Hz, 1H), 8.05 (d, J=7.5 Hz, 1H), 8.16 (s, 1H),
MS(ESI) (Positive)m/z, 343(M+H)

(2) 5-t-butyl-3-(2-ethoxyethyl)-4-methyl-2-(3-trifluoromethylbenzoyl)imino-2,3-dihydrothiazole (Compound No. 352)

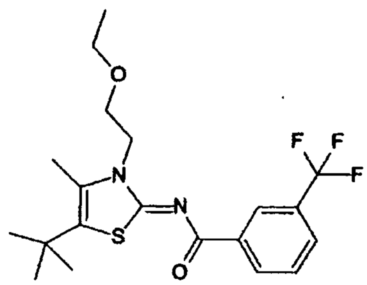

A solution of 5-t-butyl-4-methyl-2-(3-trifluoromethylbenzoyl)aminothiazole (0.15 g), sodium iodide (0.005 g), 60% sodium hydride (0.02 g) and 2-ethoxyethylbromide (0.11g) in N,N-dimethylformamide (1.5 ml) was stirred with heating at 50°C for 5 hours. The reaction solution was returned to room temperature. To the reaction solution, water was added and the reaction solution was extracted with ethyl acetate, washed with saturated brine, dried over anhydrous sodium sulfate, and filtrated. Thereafter, the filtrate was concentrated under vacuum. The residue obtained was purified by silica gel column chromatography (developing solvent: n-hexane:ethyl acetate=5:1) to obtain colorless crystals of 5-t-butyl-3-(2-ethoxyethyl)-4-methyl-2-(3-trifluoromethylbenzoyl)imino-2,3-dihydrothiazole (0.03 g).
$^1$H-NMR, Mass and the melting point are shown in Table 9.

Compounds Nos. 384 to 430, 433, 434 and 438-447, shown in Table 2, were produced in line with the process shown in Example 3.
$^1$H-NMR, Mass and the melting point are shown in Table 10.

Example 5

Production of N-(5-t-butyl-3-cyclopropylmethyl-4-methyl-3H-thiazol-2-ylidene)-1-naphthylsulfonamide (Compound No. 383)

(1) N-(5-t-butyl-4-methylthiazol-2-yl)-1-naphthylsulfonamide

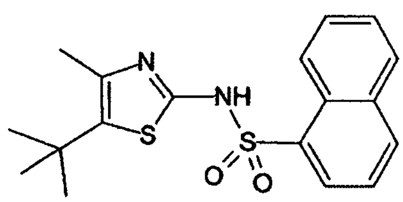

To a solution of 2-amino-5-t-butyl-4-methylthiazole (0.12 g) and N, N-dimethylaminopyridine (catalyst quantity) in pyridine (1.5 ml), 1-naphtylsulfonyl chloride (0.19 g) was added under ice cooling and stirred at room temperature overnight. To the reaction solution, water was added, and a precipitate was obtained by filtration and dried. The crude crystals obtained were recrystallized from chloroform/n-hexane to obtain colorless crystals of N-(5-t-butyl-4-methylthiazol-2-yl)-1-naphthylsulfonamide (0.22 g).
$^1$H-NMR (200 MHz, CHLOROFORM-D) d ppm; 1.43 (s, 9H), 2.36 (s, 3H)
MS (ESI) (Negative)m/z;265(M-H)$^-$

(2) N-(5-t-butyl-3-cyclopropylmethyl-4-methyl-3H-thiazol-2-ylidene)-1-naphthylsulfonamide (compound No. 383)

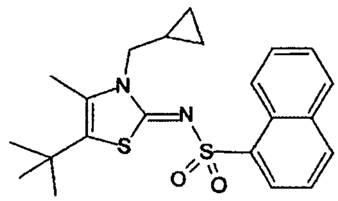

A solution of N-(5-t-butyl-4-methyl-thiazol-2-yl)-1-naphthylsulfonamide (0.20 g), 55% sodium hydride (0.03 g), sodium iodide (catalyst quantity) and (bromomethyl)cyclopropane (0.11 g) in N,N-dimethylformamide (0.5 ml) was stirred at room temperature overnight. To the reaction solution, water was added and the reaction solution was extracted with ethyl acetate, washed sequentially with water and saturated brine, dried over anhydrous magnesium sulfate and filtrated. Thereafter the filtrate was concentrated under vacuum. The residue obtained was purified by silica gel column chromatography (developing solvent: n-hexane:ethyl acetate=4:1) and recrystallized from chloroform/n-hexane to obtain colorless crystals of N-(5-t-butyl-3-cyclopropylmethyl-4-methyl-3H-thiazol-2-ylidene)-1-naphthylsulfonamide (0.12 g). $^1$H-NMR, Mass and the melting point are shown in Table 10.

Example 6

Production of 3-(2-aminoethyl)-5-t-butyl-4-methyl-2-(3-trifluoromethylbenzoyl)imino-2,3-dihydrothiazole (Compound No. 354)

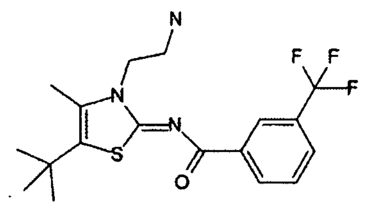

A solution of 3-(2-phthaliminoethyl)-5-t-butyl-4-methyl-2-(3-trifluoromethylbenzoyl)imino-2,3-dihydrothiazole (Compound No. 355) produced line the process shown in Example 4 and hydrazine monohydrate (0.2 ml) in ethanol (6.3 ml) was refluxed under heating for one hour. The reaction solution was returned to room temperature and a precipitate was removed by filtration. To the filtrate, chloroform was added and the resultant solution was washed sequentially with an aqueous 2M sodium hydroxide solution and saturated brine, dried over anhydrous magnesium sulfate, and filtrated. Thereafter, the filtrate was concentrated under vacuum to obtain light yellow crystals of 3-(2-aminoethyl)-5-t-butyl-4-methyl-2-(3-trifluoromethylbenzoyl)imino-2,3-dihydrothiazole (0.12 g). $^1$H-NMR, Mass and the melting point are shown in Table 9.

Example 7

Production of 5-carboxy-3-cyclopropylmethyl-4-methyl-2-(3-trifluoromethylbenzoyl)imino-2,3-dihydrothiazole (Compound No. 357)

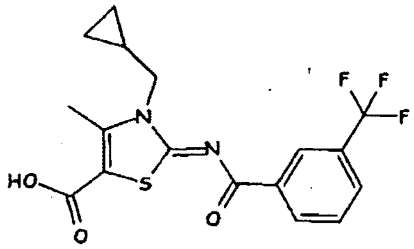

A solution (8 ml) of tetrahydrofuran/ethanol (1:1) containing 5-ethoxycarbonyl-3-cyclopropylmethyl-4-methyl-2-(3- , trifluoromethylbenzoyl)imino-2,3-dihydrothiazole (Compound No. 356)(0.23 g) produced in line with the process shown in Example 4 and a 20% aqueous sodium hydroxide solution (1.25 ml) was stirred at room temperature for one hour. To the reaction solution, 3M hydrochloric acid was added to make the solution acidic and a precipitate was obtained by filtration. The solid substance thus obtained was dissolved in tetrahydrofuran/chloroform, dried over anhydrous magnesium sulfate and filtrated. Thereafter, the filtrate was concentrated under vacuum to obtain a colorless amorphous substance of 5-carboxy-3-cyclopropylmethyl-4-methyl-2-(3-trifluoromethylbenzoyl)imino-2,3-dihydrothiazole (0.20 g).
$^1$H-NMR, Mass and the melting point are shown in Table 9.

Compound No. 214 shown in Table 2 and compounds Nos. 431 and 435 shown in Table 3 were produced in line with the process shown in Example 7.
$^1$H-NMR, Mass and the melting points of these compounds are shown in Tables 9, 10 and 11.

Example 8

Production of 3-cyclopropylmethyl-5-isopropylaminocarbonyl-4-methyl-2-(3-trifluoromethylbenzoyl)imino-2,3-dihydrothiazole (Compound No. 358)

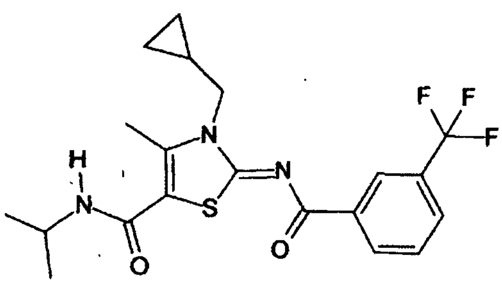

A colorless powdery substance of 3-cyclopropylmethyl-5-isopropylaminocarbonyl-4-methyl-2-(3-trifluoromethylbenzoyl)imino-2,3-dihydrothiazole was obtained in line with the process shown in Example 3 (1) by using 5-carboxy-3-cyclopropylmethyl-4-methyl-2-(3-trifluoromethylbenzoyl)imino-2,3-dihydrothiazole (Compound No. 357) produced in line with the process shown in Example 7.
$^1$H-NMR, Mass and the melting point are shown in Table 9.

Compounds Nos. 359 to 361 were produced in line with the production example shown in Example 8 by using Compound No. 356.

Compound No. 432 was produced similarly, by using compound No. 431.

Compounds Nos. 436 and 437 were produced similarly, by using compound No. 435.

$^1$H-NMR, Mass and the melting points of these compounds are shown in Tables 9 and 10.

Example 9

Production of N-(3-allyl-4,5-diphenyl-3H-thiazol-2-ylidene)benzamide (Compound No. 368)

(1) 1-allyl-3-benzoylthiourea

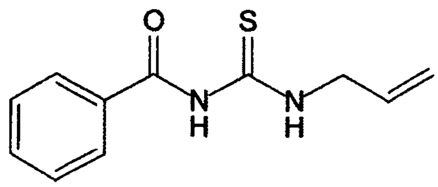

A solution of benzoylisothiocyanate (1.4 g) and allylamine (0.7 ml) in benzene (9 ml) was stirred at room temperature overnight. The reaction solution was concentrated under vacuum and the residue obtained was purified by silica gel column chromatography (developing solvent: n-hexane:ethyl acetate=10:1 to 5:1) to obtain colorless solid substance of 1-allyl-3-benzoylthiourea (1.9 g).

$^1$H-NMR (200 MHz, CHLOROFORM-D) d ppm; 4.30-4.43 (m, 2H), 5.21-5.42(m, 2H), 5.87-6.09 (m, 1H), 7.45-7.69 (m, 3H),7.78-7.90 (m, 2H), 9.00 (s, 1H), 10.80 (s, 1H),
MS(ESI)(Negative)m/z; 219(M-H)$^-$
The melting point was 68 to 69˚C

(2) N-(3-allyl-4,5-diphenyl-3H-thiazol-2-ylidene)benzamide (Compound No. 368)

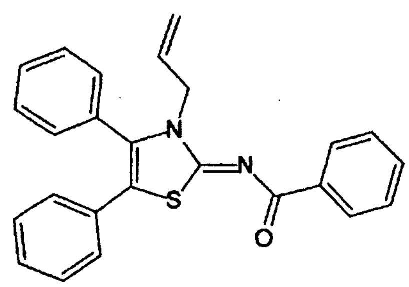

A solution of 1-allyl-3-benzoylthiourea (0.20 g) and 2-bromo-1,2-diphenylethane (0.24 g) in toluene (4.5 ml) was refluxed under heating for 4.5 hours. The solvent was concentrated under vacuum and the residue obtained was purified by silica gel column chromatography (developing solvent: n-hexane:ethyl acetate=30:1 to 20:1) and recrystallized from n-hexane/ethyl acetate to obtain colorless crystals of N-(3-allyl-4,5-diphenyl-3H-thiazol-2-ylidene)benzamide (0.13 g).
$^1$H-NMR, Mass and the melting point are shown in Table 9.

Example 10

Production of N-[1,2-dihydro-1,5-dimethyl-2-(2-ethoxyethyl)pyrazol-3-ylidene]-2-fluoro-3-(trifluoromethyl)benzamide (Compound No. 452)

(1) N-1,5-dimethylpyrazol-3-yl]-2-fluoro-3-(trifluoromethyl)benzamide

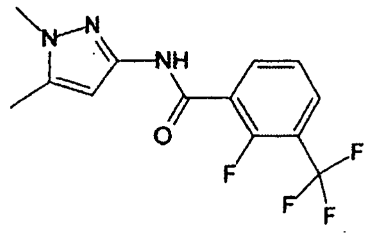

To a solution of 3-amino-1,5-dimethylpyrazole (0.50 g) and triethylamine (0.63 ml) in chloroform (5 ml), 2-fluoro-3-(trifluoromethyl)benzoyl chloride (0.65 ml) was added under ice cooling. The reaction solution was stirred at room temperature for 0.5 hours, washed with an aqueous 2M sodium hydroxide solution, dried over anhydrous sodium sulfate and filtrated. Thereafter, the filtrate was concentrated under vacuum. The residue obtained was washed with n-hexane to obtain a colorless solid substance of N-(1,5-dimethylpyrazol-3-yl)-2-fluoro-3-(trifluoromethyl)benzamide (1.25 g).

$^1$H-NMR(200 MHz, CHLOROFORM-D) d ppm; 2.30 (s, 39H), 3.72 (s, 3H), 6.59 (s, 1H), 7.40 (t, J =7.5 Hz, 1H), 7.78 (t, J = 7.5 Hz, 1H), 8.34 (td, J = 7.5 Hz, 1H), 8.60-8.89. (br. s, 1H)
MS (ESI)(Positive)m/z; 302 (M+H)$^+$

(2) N-[1,2-dihydro-1,5-dimethyl-2-(2-ethoxyethyl)pyrazol-3-ylidene]-2-fluoro-3-(trifluoromethyl)benzamide

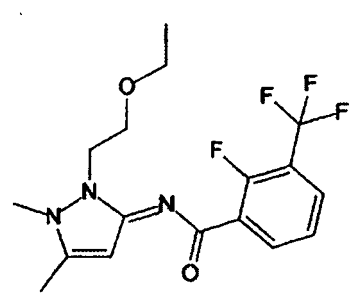

A suspension solution of N-(1,5-dimethylpyrazol-3-yl)-2-fluoro-3-(trifluoromethyl)benzamide (0.50 g), 55% sodium hydride (0.07 g) in N,N-dimethylformamide (5 ml) was stirred at room temperature for 5 minutes. To this mixture, 2-ethoxyethyl bromide (0.38 g) and sodium iodide (catalytic quantity) were added and stirred for 17 hours. To the reaction solution, water was added and the reaction solution was extracted with ethyl acetate and then concentrated under vacuum. The residue obtained was purified by silica gel column chromatography (developing solvent: chloroform:methanol=18:1) to obtain a colorless solid substance of N-[1,2-dihydro-1,5-dimethyl-2-(2-ethoxyethyl)pyrazol-3-ylidene]-2-fluoro-3-(trifluoromethyl)benzamide (0.01 g).
$^1$H-NMR, Mass and the melting points of these compounds are shown in Table 11.

Compounds Nos. 448 to 451,453, 454 and 456 shown in Table 3 were produced in line with the process shown in Example 10.
$^1$H-NMR, Mass and the melting points of these compounds are shown in Table 11.

Example 11

Production of N-{1,2-dihydro-1,5-dimethyl-2-(1,1-dioxotetrahydrothiophen-3-yl)pyrazol-3-ylidene}-2-fluoro-3-(trifluoromethyl)benzamide (Compound No. 455)

(1) N-{2-(1,1-dioxotetrahydrothiophen-3-yl)-5-methylpyrazol-3-yl}-2-fluoro-3-(trifluoromethyl)benzamide

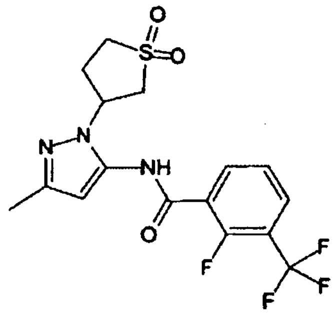

A colorless solid substance of N-{2-(1,1-dioxotetrahydrothiophen-3-yl)-5-methylpyrazol-3-yl}-2-fluoro-3-(trifluoromethyl)benzamide was obtained in line with the process shown in Example 8(1) by using 3-amino-2-(1,1-dioxotetrahydrothiophen-3-yl)-5-methylpyrazole.

$^1$H-NMR (600 MHz, CHLOROFORM-D) d ppm; 2.63-2.69 (m, 1H), 2.75-2.81 (m, 1H), 3.12 (m, 1H), 3.50-3.63 (m, 3H), 4.89 (m, 1H), 6.00 (s, 1H), 7.46(m, 1H), 7.87 (m, 1H), 8.13 (d, J = 13.3 Hz, 1H), 8.33 (m, 1H)
MS(ESI)(Positive)m/z; 406 $(M+H)^+$

(2) N-{1,2-dihydro-1,5-dimethyl-2-(1,1-dioxotetrahydrothiophen-3-yl)pyrazol-3-ylidene}-2-fluoro-3-(trifluoromethyl)benzamide (Compound No. 455)

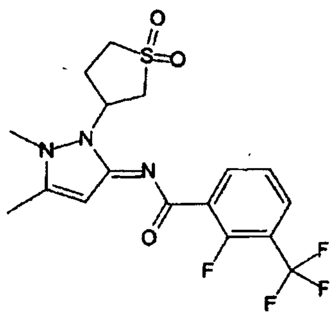

A solution of N-{2-(1,1-dioxotetrahydrothiophen-3-yl)-5-methylpyrazol-3-yl}-2-fluoro-3-(trifluoromethyl)benzamide (0.40 g) and dimethyl sulfate (0.11 ml) in toluene (1.2 ml) was stirred at 80˚C while heating for 47 hours. The reaction solution was returned to room temperature and purified by silica gel column chromatography (developing solvent: chloroform:methanol=20:1) to obtain a colorless amorphous substance of N-{1,2-dihydro-1,5-dimethyl-2-(1,1-dioxotetrahydrothiophen-3-yl)pyrazol-3-ylidene}-2-fluoro-3-(trifluoromethyl)benzamide (0.11g).
$^1$H-NMR, Mass and the melting point are shown in Table 11.

Compounds No. 497 to 499 shown in Table 3 were obtained in accordance with the process shown in Example 11.
$^1$H-NMR, Mass and the melting points thereof are shown in Table 11.

Example 12

Production of N-(5-t-butyl-2-cyclopropylmethyl-1,2-dihydro-1-methylpyrazol-3-ylidene)formamide (Compound No. 457)

(1) 3-amino-5-t-butyl-2-(cyclopropylmethyl)pyrazole

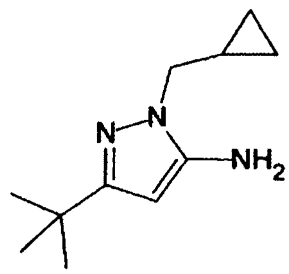

To a solution of cyclopropylmethanol (125 g) and triethylamine (315 ml) in chloroform (500 ml), methanesulfonyl chloride (175 ml) was added under ice cool for 1.5 hours. The mixture was stirred at room temperature for 2 hours. To the reaction solution, water was added and the reaction solution was extracted with chloroform, washed sequentially with water and saturated brine, dried over anhydrous magnesium sulfate, and filtrated. Thereafter, the filtrate was concentrated under vacuum. The residue obtained (232 g) was dissolved in ethanol (500 ml). To this, hydrazine monohydrate (500 g) was added at room temperature and stirred for 17 hours. After the solvent was distilled off under vacuum, the residue was extracted with chloroform, dried over anhydrous magnesium sulfate, and filtrated. The filtrate was concentrated under vacuum. The residue of a yellow oily substance (68 g) obtained was dissolved in ethanol (610 ml). To this, 4,4-dimethyl-3-oxopentanenitrile (99 g) was added at room temperature and then refluxed for 4 hours. After the solvent was removed under vacuum, the residue was purified by silica gel column chromatography (developing solvent: n-hexane:ethyl acetate=5:1) and recrystallized from ethyl acetate/n-hexane to obtain a colorless solid substance of 3-amino-5-t-butyl-2-cyclopropylmethyl)pyrazole (80 g).

$^1$H-NMR (200 MHz, CHLOROFORM-d) d ppm; 0.26-0.41 (m, 2H), 0.49-0.62 (m, 2H), 1.11-1.27 (m, 1H), 1.26 (s, 9H) 3.42 (br. s, 2H), 3.86 (d, J = 6.2 Hz, 2H), 5.42 (s, 1H)
MS(ESI)(Positive) m/z; 194 $(M+H)^+$
Melting point: 69.5-70.5˚C

(2) N-(5-t-butyl-2-cyclopropylmethylpyrazol-3-yl)trifluoroacetamide

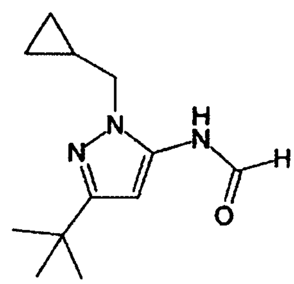

Formic acid (3.9 ml) and acetic anhydride (7.4 ml) were stirred at room temperature for one hour. To this, 3-amino-5-t-butyl-2-(cyclopropylmethyl)pyrazole (5.0 g) was added under ice cooling and stirred at room temperature for one hour. To the reaction solution, an aqueous 2M sodium hydroxide solution was added and the reaction solution was extracted with sodium acetate, washed with water, dried over anhydrous sodium sulfate, and filtrated. Thereafter, the filtrate was concentrated under vacuum to obtain a colorless oily substance of N-(5-t-butyl-2-cyclopropylmethylpyrazol-3-yl)formamide (4.4 g)·

$^1$H-NMR (200 MHz, CHLOROFORM-d) d ppm; 0.27-0.40 (m, 2H), 0.50-0.66 (m, 2H), 1.13-1.27 (m, 1H), 1.29 (s, 9H), 3.91 (d, J = 6.6 Hz, 2H), 5.98 (s, 3H), 6.26 (s, 3H), 8.26-8.31 (m, 3H), 8.31-8.39 (m, 3H)
MS(ESI)(Positive)m/z;222$(M+H)^+$

(3) N-(5-t-butyl-2-cyclopropylmethyl-1,2-dihydro-1-methylpyrazol-3-ylidene)formamide (Compound No. 457)

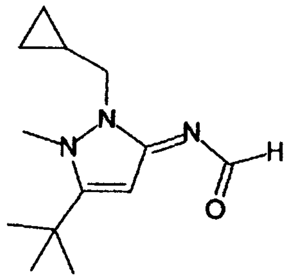

To a solution of N-(5-t-butyl-2-cyclopropylmethylpyrazol-3-yl)formamide (2.0 g) in toluene (6 ml), dimethyl sulfate (0.9 ml) was added and stirred with heating at 50˚C for 48 hours. The reaction solution was returned to room temperature. To this, an aqueous saturated sodium hydrogencarbonate solution was added and the reaction solution was extracted with ethyl acetate, washed sequentially with water and saturated brine, dried over anhydrous magnesium sulfate, and filtrated. Thereafter, the filtrate was concentrated under vacuum. The residue obtained was purified by silica gel column chromatography (developing solvent: chloroform:methanol=18:1) to obtain a colorless solid substance of N-(5-t-butyl-2-cyclopropylmethyl-1,2-dihydro-1-methylpyrazol-3-ylidene)formamide (0.35 g).
$^1$H-NMR, Mass and the melting point are shown in Table 11.

Example 13

Production of N-(5-t-butyl-2-cyclopropylmethyl-1-methylpyrazol-3-yl)trifluoroacetamide (Compound No. 458)

(1) N-(5-t-butyl-2-cyclopropylmethylpyrazol-3-yl)trifluoroacetamide

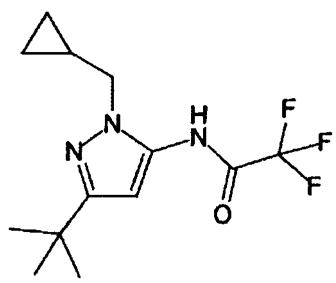

N-(5-t-butyl-2-cyclopropylmethylpyrazol-3-yl) trifluoroacetamide was obtained in line with the process shown in Example 1 by using 3-amino-5-t-butyl-2-(cyclopropylmethyl)pyrazole produced in the process shown in Example 12 (1).
$^1$H-NMR (200 MHz, CHLOROFORM-d) d ppm; 0.32-0.42 (m, 2H), 0.65-0.74 (m, 2H), 1.10-1.36 (m, 1H), 1.30 (s, 9H), 3.99 (d, J = 6.0 Hz, 2H), 6.31 (s, 1H), 8.11-8.23 (brs, 1H)
MS(ESI)(Negative)m/z; 290(M+H)$^+$

(2) N-(5-t-butyl-2-cyclopropylmethyl-1-methylpyrazol-3-yl)trifluoroacetamide (Compound No. 458)

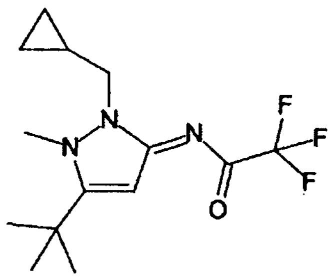

N-(5-t-butyl-2-cyclopropylmethyl-l-methylpyrazol-3-yl)trifluoroacetamide was obtained from N-(5-t-butyl-2-cyclopropylmethylpyrazol-3-yl)trifluoroacetamide in line with the process shown in Example 12 (3).
$^1$H-NMR, Mass and the melting point are shown in Table 11.

Example 14

Production of N-(5-t-butyl-2-cyclopropylmethyl-1,2-dihydro-1-methylpyrazol-3-ylidene)-2-chloro-3-(trifluoromethyl)benzamide (Compound No. 476)

(1) 5-t-butyl-2-cyclopropylmethyl-1,2-dihydro-1-methylpyrazol-3-ylideneamine

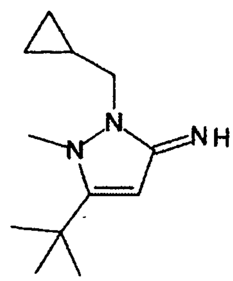

A solution of N-(5-t-butyl-2-cyclopropylmethyl-1,2-dihydro-1-methylpyrazol-3-ylidene)formamide (0.20 g) produced by the process of Example 12 and 12 M hydrochloric acid (0.3 ml) in methanol (3 ml) was stirred at room temperature for 2 hours. To the reaction solution, a 2M aqueous sodium hydroxide solution was added. After the reaction solution was made basic, it was extracted with chloroform, dried over anhydrous sodium sulfate, and filtrated. Thereafter, the filtrate was concentrated under vacuum to obtain a yellow amorphous solid substance of 5-t-butyl-2-cyclopropylmethyl-1,2-dihydro-1-methylpyrazol-3-ylideneamine (0.17 g)
$^1$H-NMR (200 MHz, CHLOROFORM-d) d ppm; 0.28-0.41 (m, 2H), 0.43-0.55 (m, 2H), 0.95-1.17 (m, 1H), 1.29 (s, 9H), 3.16 (s, 3H), 3.62 (d, J= 6.6 Hz, 2H), 5.31 (s, 1H)
MS(ESI)(Negative)m/z; 208(M+H)$^+$

(2) N-(5-t-butyl-2-cyclopropylmethyl-1,2-dihydro-1-methylpyrazol-3-ylidene)-2-chloro-3-(trifluoromethyl)benzamide (Compound No. 476)

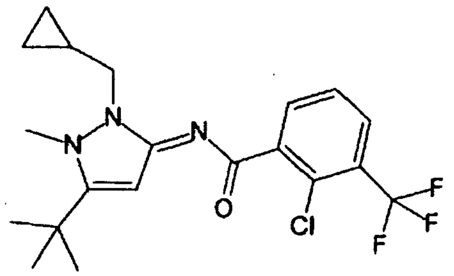

A solution of 2-chloro-3-trifluoromethyl benzoic acid (0.13 g) and thionyl chloride (0.07 ml) and N,N-dimethylformamide (0.01 ml) in tetrahydrofuran (2 ml) was refluxed under heating for 0.5 hours. The solvent was distilled off under vacuum. To the residue, chloroform (1 ml) was added to form a chloroform solution. The chloroform solution was added to a solution of 5-t-butyl-2-cyclopropylmethyl-1-methyl-1,2-dihydropyrazol-3-ylideneamine (0.10 g) and triethylamine (0.10 ml) in chloroform (2 ml) at room temperature and stirred for 17 hours. The reaction solution was washed sequentially with a saturated aqueous sodium hydrogencarbonate solution and a saturated aqueous sodium chloride solution, dried over anhydrous magnesium sulfate, and filtrated. Thereafter, the filtrate was concentrated under vacuum. The residue obtained was purified by silica gel column chromatography (developing solvent: ethyl acetate:methanol=5:1) to obtain a colorless solid substance of N-(5-t-butyl-2-cyclopropylmethyl-1,2-dihydro-1-methylpyrazol-3-ylidene)-2-chloro-3-(trifluoromethyl)benzamide (0.05 g).
$^1$H-NMR, Mass and the melting point are shown in Table 11.

Compounds Nos. 459 to 473, 475, 477 to 496 and 503 shown in Table 3 were produced in line with the process shown in Table 14.
$^1$H-NMR, Mass and the melting points of these compounds are shown in Table 11.

Example 15

Production of N-(5-t-butyl-2-cyclopropylmethyl-1,2-dihydro-1-methylpyrazol-3-ylidene)-2-fluoro-3-(trifluoromethyl)benzamide (Compound No. 474)

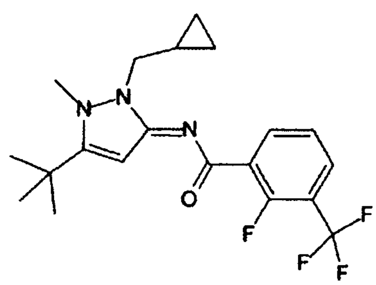

To a solution of 3-amino-5-t-butyl-2-(cyclopropylmethyl)pyrazole (12.8 g) and triethylamine (9.2 ml) in chloroform (120 ml), 2-fluoro-3-(trifluoromethyl)benzoyl chloride (15.0 g) was added under ice cooling and stirred at room temperature for one hour. The reaction solution was washed with an aqueous 2M sodium hydroxide solution, dried over anhydrous sodium sulfate, and filtrated. Thereafter, the filtrate was concentrated under vacuum. The residue obtained was purified by silica gel column chromatography (developing solvent: n-hexane:ethyl acetate=3:1) to obtain a colorless solid substance (18.3 g). The obtained solid substance (4.4 g) was dissolved in toluene (90 ml). To this, dimethyl sulfate (3.2 ml) was added and stirred with heating at 80°C for 17 hours. The reaction solution was returned at room temperature. An aqueous saturated sodium hydrogencarbonate solution was added to this, the reaction solution was extracted with ethyl acetate, washed' sequentially with water and saturated brine, dried over anhydrous sodium sulfate and filtrated. Thereafter, the filtrate was concentrated under vacuum. The residue obtained was purified by silica gel column chromatography (developing solvent: chloroform:methanol=18:1) to obtain a colorless solid substance of N-(5-t-butyl-2-cyclopropylmethyl-1,2-dihydro-1-methylpyrazol-3-ylidene)-2-fluoro-3-(trifluoromethyl)benzamide (0.3 g).
$^1$H-NMR, Mass and the melting point are shown in Table 11.

Example 16

Production of N-(5-t-butyl-2-cyclobutylmethyl-1,2-dihydro-1-methylpyrazol-3-ylidene)-2-fluoro-3-(trifluoromethyl)benzamide (Compound No. 482)

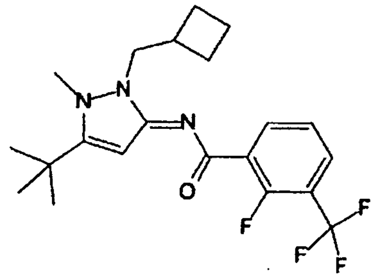

N-(5-t-butyl-2-cyclobutylmethyl-1,2-dihydro-1-methylpyrazol-3-ylidene)-2-fluoro-3-(trifluoromethyl) benzamide was obtained in line with the process shown in Example 15 by using 3-amino-5-t-butyl-2-(cyclobutylmethyl)pyrazole produced in accordance with the process shown in Example 12 (1).
$^1$H-NMR, Mass and the melting point are shown in Table 11.
The compounds Nos. 481 and 483 to 496 shown in Table 3 were produced in line with the process shown in Example 16.
$^1$H-NMR, Mass and the melting points of these compounds are shown in Table 11.

Example 17

Production of N-(5-t-butyl-2-cyclopropylmethyl-1,2-dihydro-1-methylpyrazol-3-ylidene)-3,5-difluorophenyloxoacetamide (Compound No. 500)

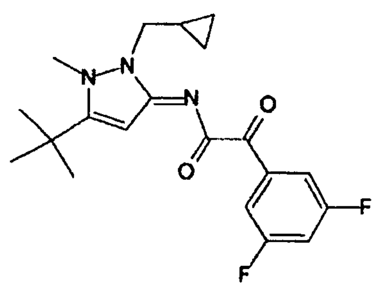

A mixture of 5-t-butyl-2-cyclopropylmethyl-1,2-dihydro-1-methylpyrazol-3-ylideneamine (0.080 g) produced by the process shown in Example 14(1) and ethyl 3,5-difluorophenyloxoacetate (0.2 ml) was stirred with heating at 110˚C for 7 hours. The mixture was returned to room temperature and separated and purified by silica gel column chromatography (developing solvent: chloroform: methanol=30:1) and sequentially by thin-layer chromatography (developing solvent: n-hexane: ethyl acetate=1:10) to obtain a light brown amorphous substance of N-(5-t-butyl-2-cyclopropylmethyl-1,2-dihydro-1-methylpyrazol-3-ylidene)-3,5-difluorophenyloxoacetamide (0.5 mg).
$^1$H-NMR, Mass and the melting point are shown in Table 11.

Example 18

Production of 1-(5-t-butyl-2-cyclopropylmethyl-1,2-dihydro-1-methylpyrazol-3-ylidene)-3-{4-chloro-2-(trifluoromethyl) phenyl}urea (Compound No. 501)

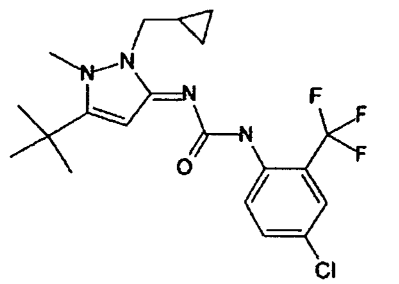

A solution of 5-t-butyl-2-cyclopropylmethyl-1,2-dihydro-1-methylpyrazol-3-ylideneamine (0.050 g) produced by the process shown in Example 14(1) and 4-chloro-2-(trifluoromethyl)phenylisocyanate (0.064 g) in tetrahydrofuran (2 ml) was stirred at room temperature for 20 hours. To the reaction solution, water was added and the reaction solution was extracted with ethyl acetate, washed sequentially with water and saturated brine, dried over anhydrous magnesium sulfate, and filtrated. Thereafter, the filtrate was concentrated under vacuum. The residue obtained was separated and purified by thin-layer column chromatography (developing solvent: chloroform:methanol=25:1) to obtain a light yellow powdery substance of 1-(5-t-butyl-2-cyclopropylmethyl-1,2-dihydro-1-methylpyrazol-3-ylidene)-3-{4-chloro-2-(trifluoromethyl)phenyl}urea (0.001 g).
$^1$H-NMR, Mass and the melting point are shown in Table 11.

Example 19

Production of 1-(5-t-butyl-2-cyclopropylmethyl-1,2-dihydro-1-methylpyrazol-3-ylidene)-3-(4-fluorophenyl)thiourea (Compound No. 502)

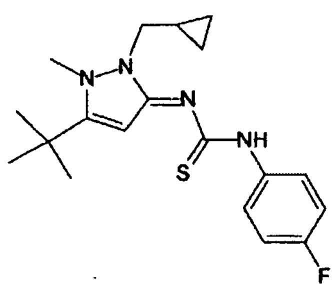

1-(5-t-butyl-2-cyclopropylmethyl-1,2-dihydro-1-methylpyrazol-3-ylidene)-3-(4-fluorophenyl)thiourea was obtained in accordance with the process shown in Example 18.
$^1$H-NMR, Mass and the melting point are shown in Table 11.

Compounds Nos. 1001 to 1431, 2001 to 2678, 3001 to 3158 and 3159 to 3327 shown in Tables 4 to 7 were produced in accordance with the process shown in Example 2 (2) or Example 14(2) by using imine compounds (1-cyclopropylmethyl-2-imino-1,2-dihydropyridine, 3-cyclopropylmethyl-2,3-dihydro-4,5-dimethylthiazol-2-ylideneamine, 5-t-butyl-2,3-dihydro-3,4-dimethylthiazol-2-ylideneamine, 5-t-butyl-2,3-dihydro-3-ethyl-4-methylthiazol-2-ylideneamine, 5-t-butyl-2,3-dihydro-3-(2-methoxyethyl)-4-methylthiazol-2-ylideneamine, 5-t-butyl-3-cyclopropylmethyl-2,3-dihydro-4-methylthiazol-2-ylideneamine, 5-t-butyl-2-cyclopropylmethyl-1,2-dihydro-1-methylpyrazol-3-ylideneamine, and 5-t-butyl-3-cyclopropylmethyl-2,3-dihydro-1,3,4-thiadiazol-2-ylideneamine).

The Mass of the compounds obtained is shown in Tables 12-15.

Table 1

| Compound No. | chemical structure | Compound No. | chemical structure |
|---|---|---|---|
| 1 | 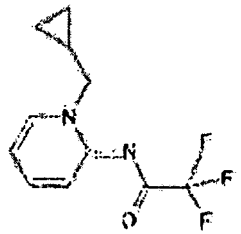 | 6 | 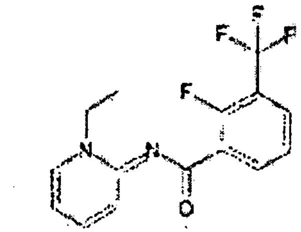 |
| 2 | 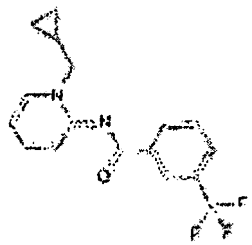 | 7 | 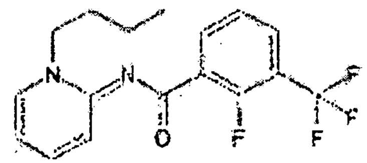 |
| 3 | 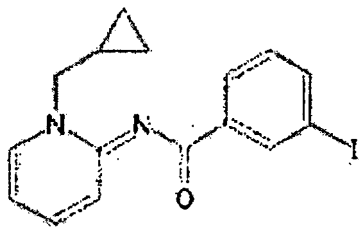 | 8 | 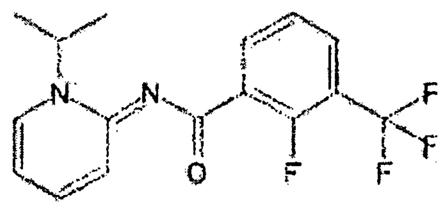 |
| 4 | 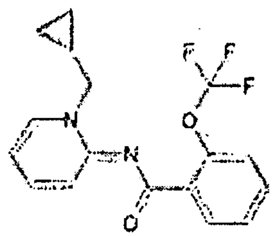 | 9 | 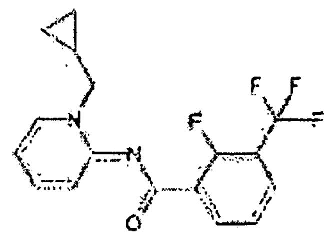 |

(continued)

| Compound No. | chemical structure | Compound No. | chemical structure |
|---|---|---|---|
| 5 | 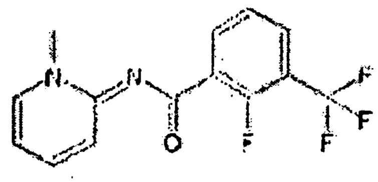 | 10 | 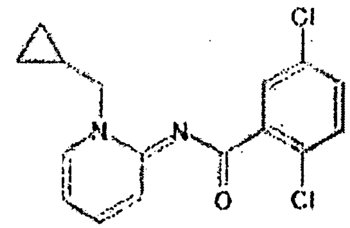 |
| 11 | 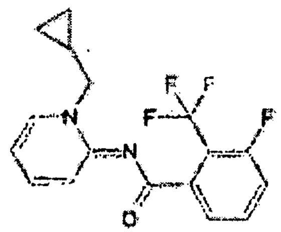 | 16 | 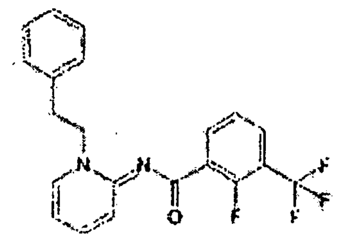 |
| 12 | 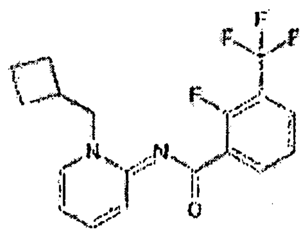 | 17 | 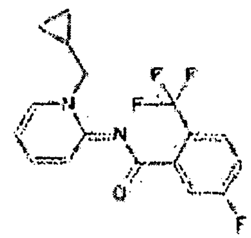 |
| 13 | 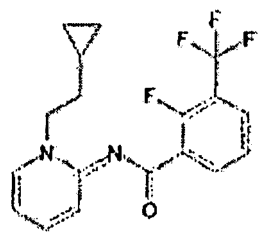 | 18 | 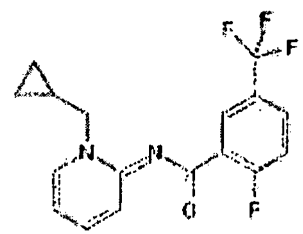 |

(continued)

| Compound No. | chemical structure | Compound No. | chemical structure |
|---|---|---|---|
| 14 | 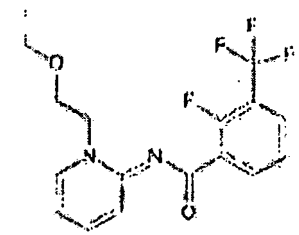 | 19 | 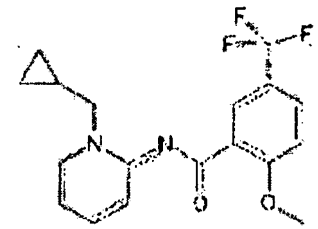 |
| 15 | 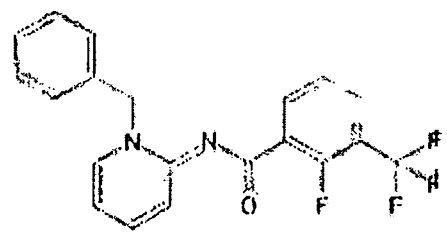 | 20 | 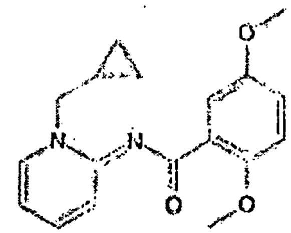 |
| 21 | 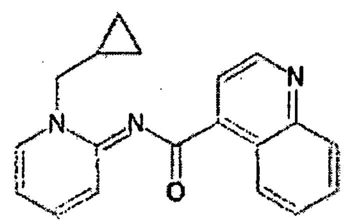 | 26 | 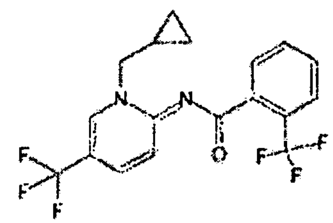 |
| 22 | 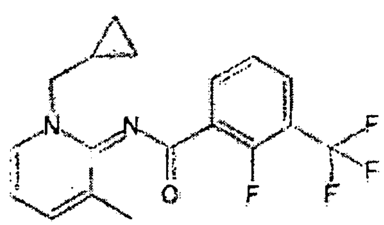 | 27 | 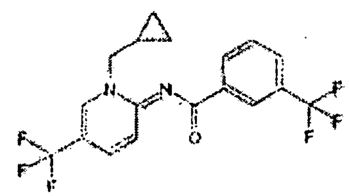 |

(continued)

| Compound No. | chemical structure | Compound No. | chemical structure |
|---|---|---|---|
| 23 | 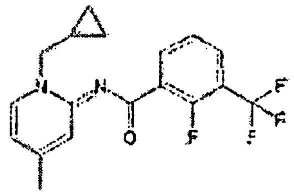 | 28 | 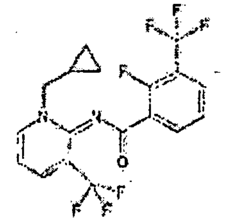 |
| 24 | 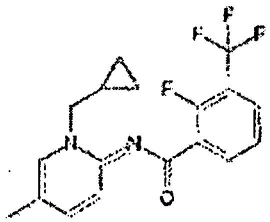 | 29 | 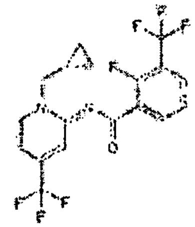 |
| 25 | 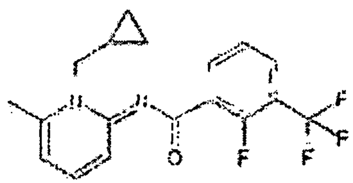 | 30 | 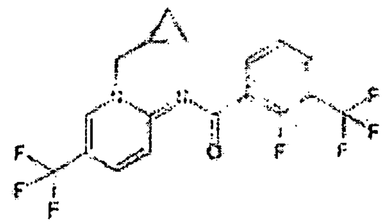 |
| 31 | 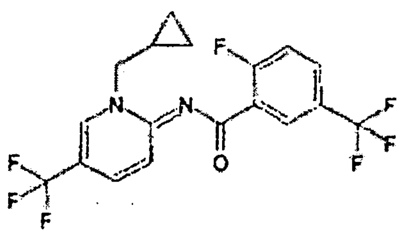 | 36 | 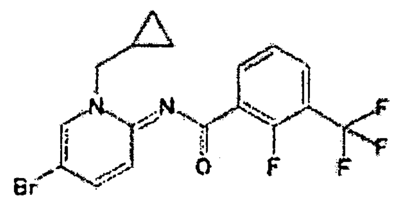 |

(continued)

| Compound No. | chemical structure | Compound No. | chemical structure |
|---|---|---|---|
| 32 | 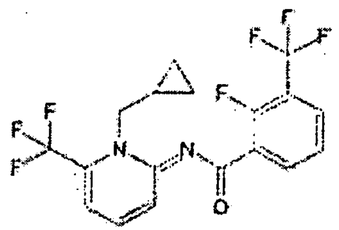 | 37 | 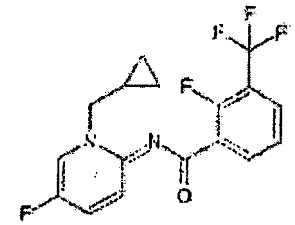 |
| 33 | 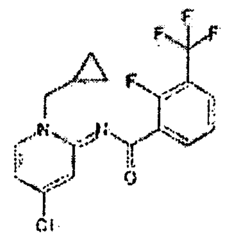 | 38 | 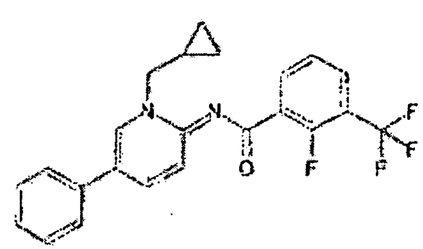 |
| 34 | 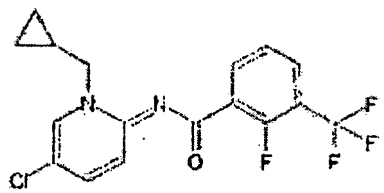 | 39 | 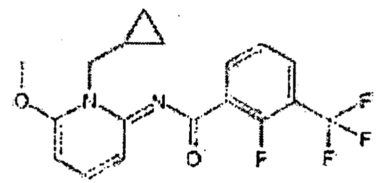 |
| 35 | 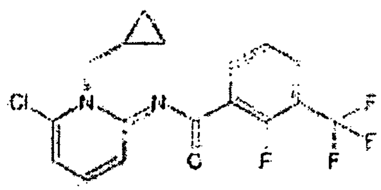 | 40 | 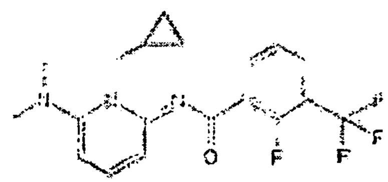 |

(continued)

| Compound No. | chemical structure | Compound No. | chemical structure |
|---|---|---|---|
| 41 | 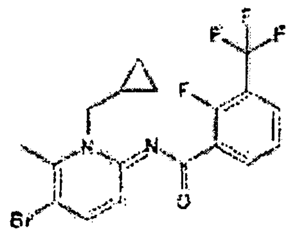 | 46 | 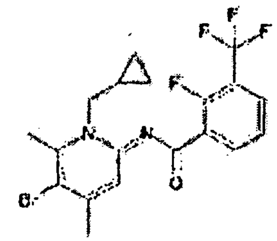 |
| 42 | 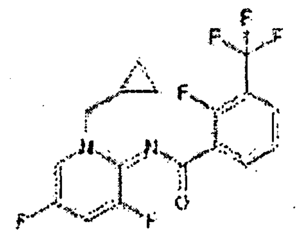 | 47 | 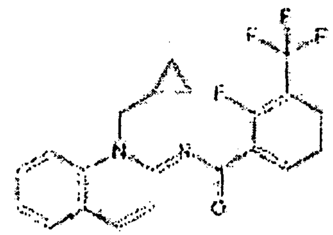 |
| 43 | 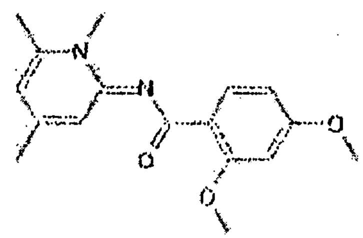 | 48 | 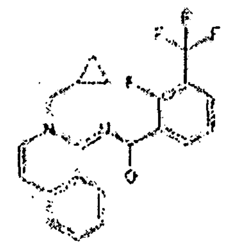 |
| 44 | 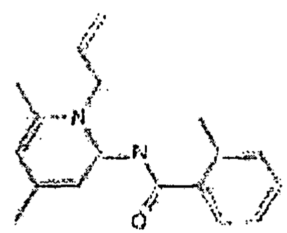 | 49 | 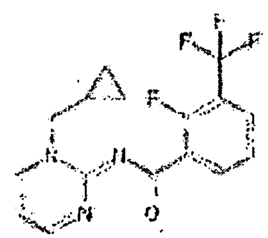 |

(continued)

| Compound No. | chemical structure | Compound No. | chemical structure |
|---|---|---|---|
| 45 | 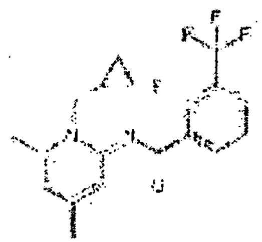 | 50 | 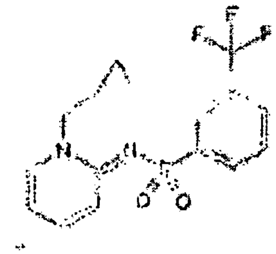 |
| 51 | 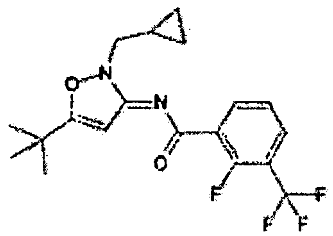 | 56 | 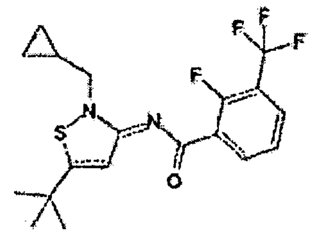 |
| 52 | 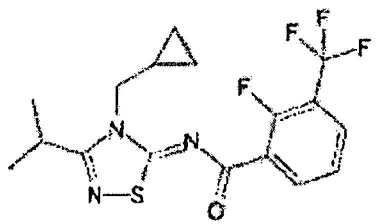 | 57 | 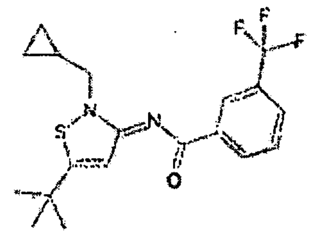 |
| 53 | 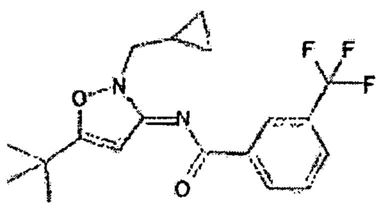 | 58 | 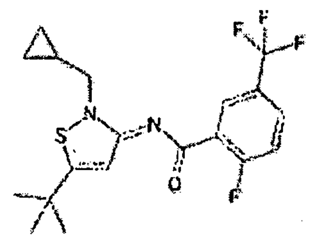 |

(continued)

| Compound No. | chemical structure | Compound No. | chemical structure |
|---|---|---|---|
| 54 | 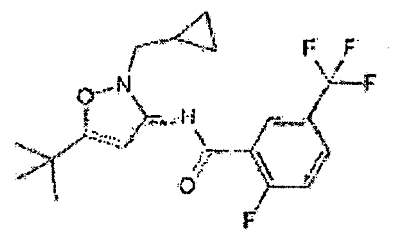 | 59 | 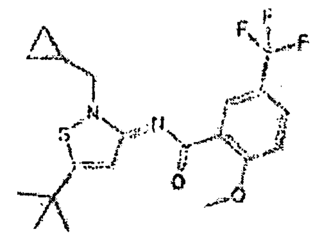 |
| 55 | 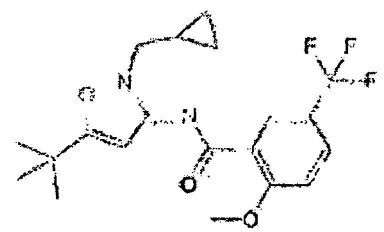 | | |

Table 2

| Example No. | chemical structure | Example No. | chemical structure |
|---|---|---|---|
| 60 | 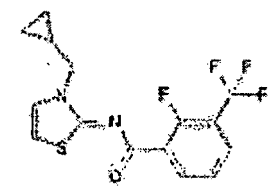 | 66 | 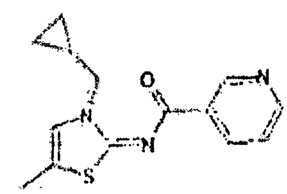 |
| 61 | 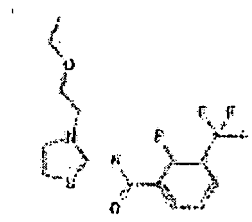 | 67 | 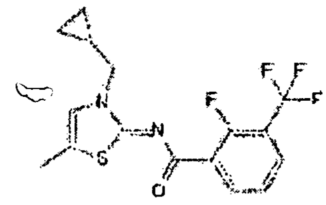 |
| 62 | 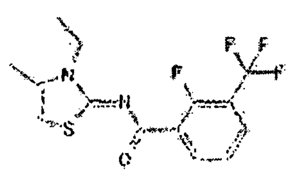 | 68 | 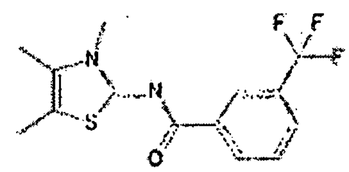 |
| 63 | 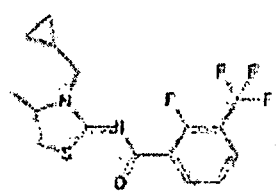 | 69 | 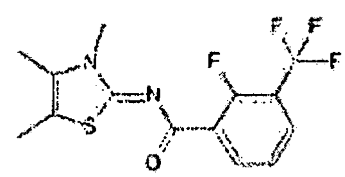 |
| 64 | 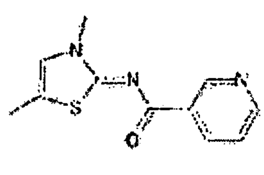 | 70 | 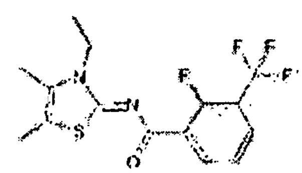 |
| 65 | 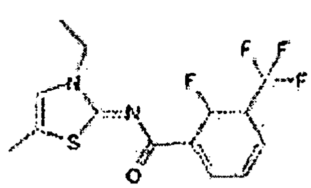 | 71 | 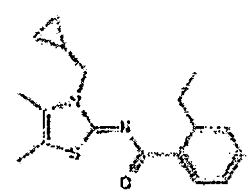 |
| 72 | 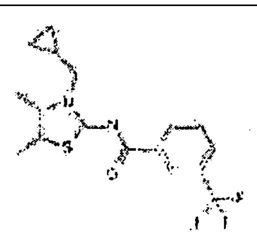 | 78 | 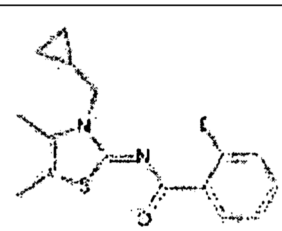 |

(continued)

| Example No. | chemical structure | Example No. | chemical structure |
|---|---|---|---|
| 73 | 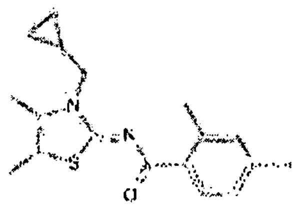 | 79 | 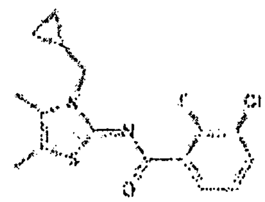 |
| 74 | 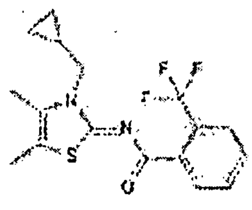 | 80 | 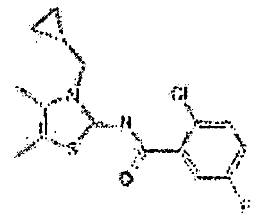 |
| 75 | 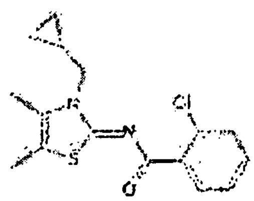 | 81 | 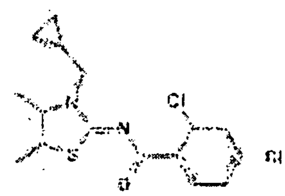 |
| 76 | 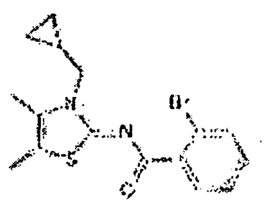 | 82 | 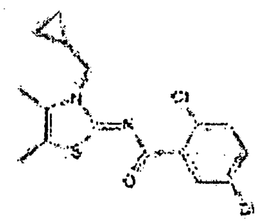 |
| 77 | 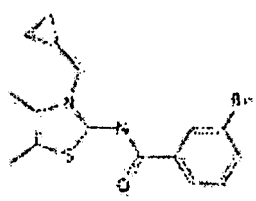 | 83 | 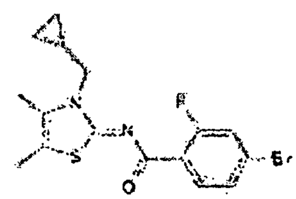 |
| 84 | 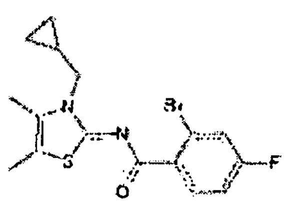 | 90 | 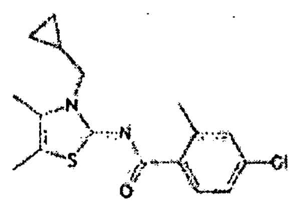 |
| 85 | 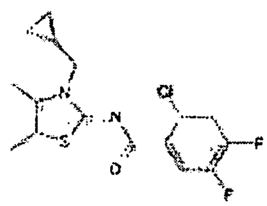 | 91 | 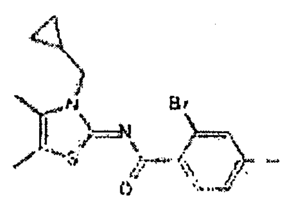 |

(continued)

| Example No. | chemical structure | Example No. | chemical structure |
|---|---|---|---|
| 86 | 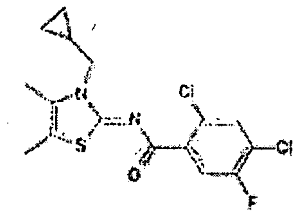 | 92 | 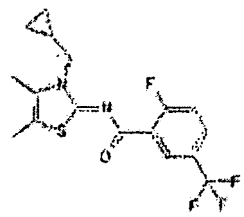 |
| 87 | 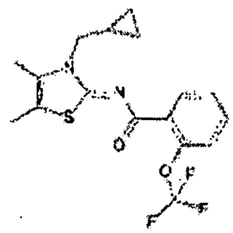 | 93 | 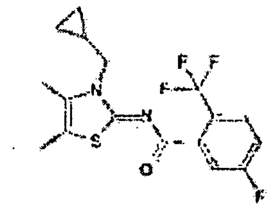 |
| 88 | 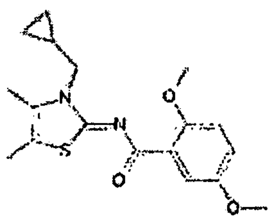 | 94 | 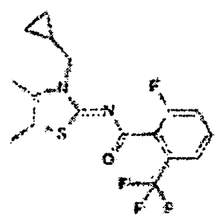 |
| 89 | 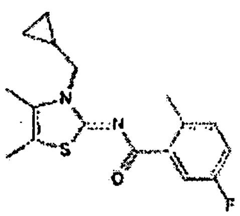 | 95 | 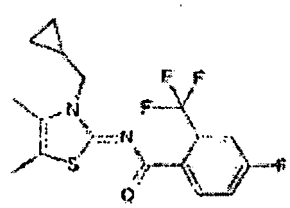 |
| 96 | 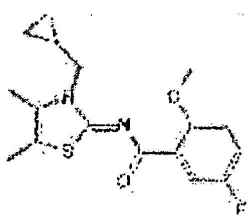 | 102 | 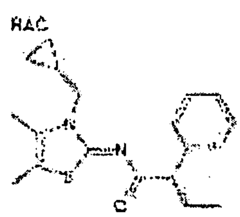 |
| 97 | 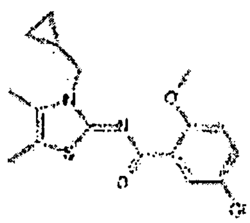 | 103 | 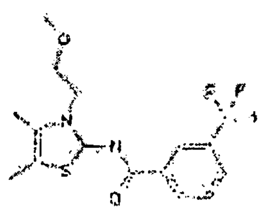 |
| 98 | 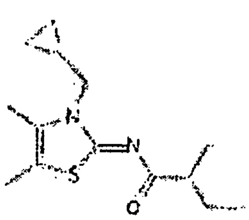 | 104 | 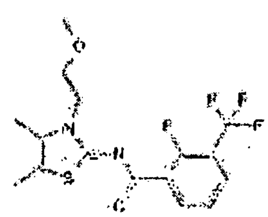 |

(continued)

| Example No. | chemical structure | Example No. | chemical structure |
|---|---|---|---|
| 99 | 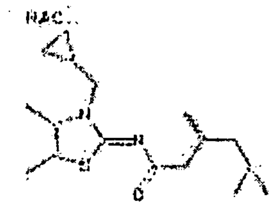 | 105 | 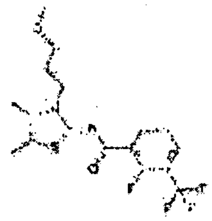 |
| 100 | 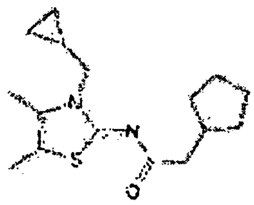 | 106 | 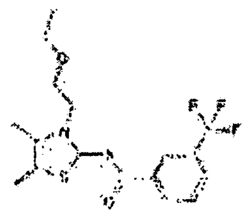 |
| 101 | 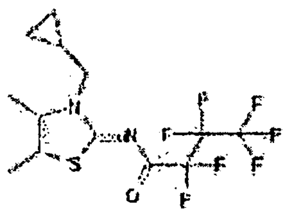 | 107 | 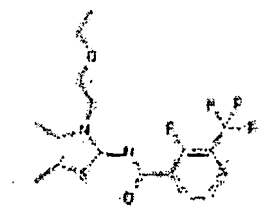 |
| 108 | 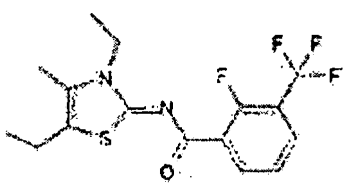 | 114 | 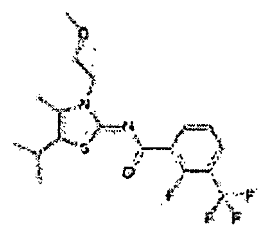 |
| 109 | 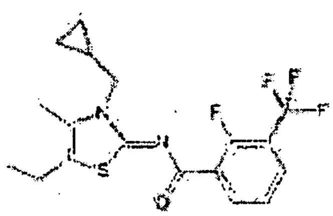 | 115 | 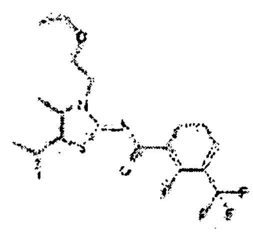 |
| 110 | 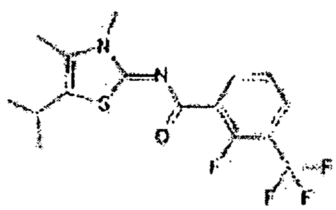 | 116 | 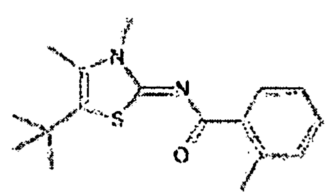 |
| 111 | 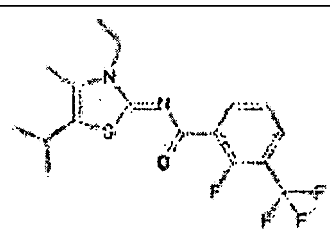 | 117 | 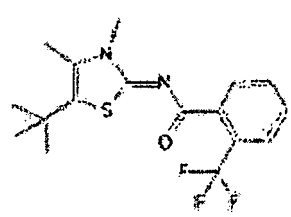 |

(continued)

| Example No. | chemical structure | Example No. | chemical structure |
|---|---|---|---|
| 112 | 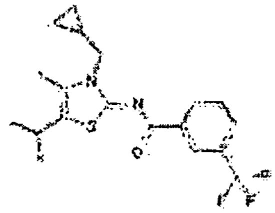 | 118 | 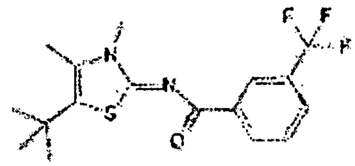 |
| 113 | 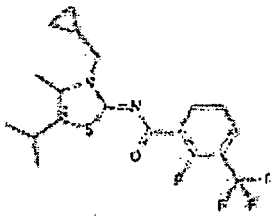 | 119 | 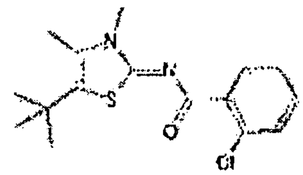 |
| 120 | 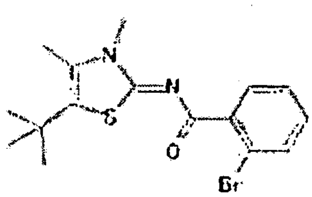 | 126 | 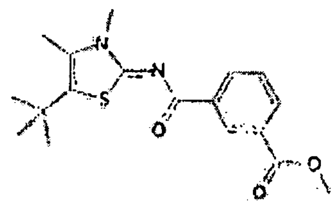 |
| 121 | 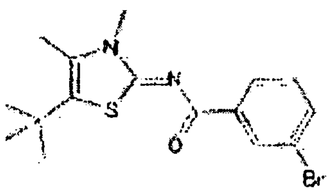 | 127 | 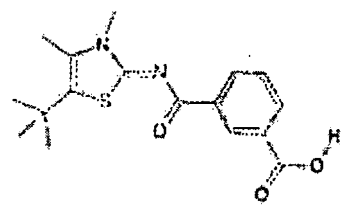 |
| 122 | 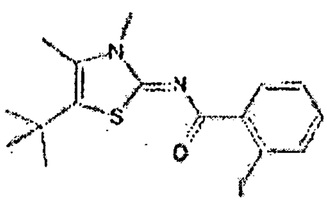 | 128 | 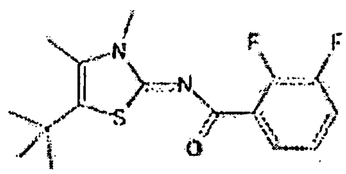 |
| 123 | 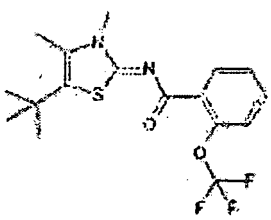 | 129 | 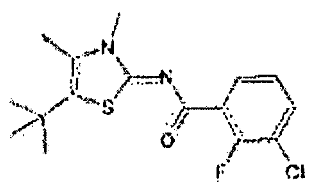 |
| 124 | 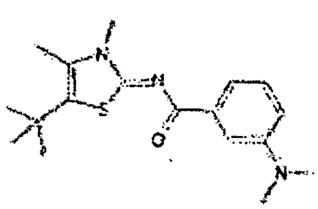 | 130 | 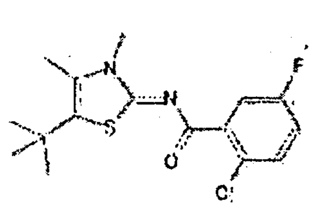 |

(continued)

| Example No. | chemical structure | Example No. | chemical structure |
|---|---|---|---|
| 125 | 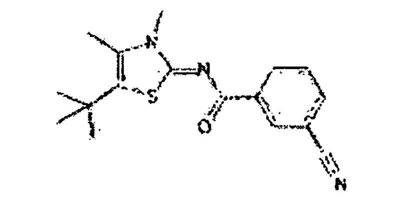 | 131 | 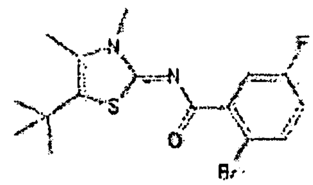 |
| 132 | 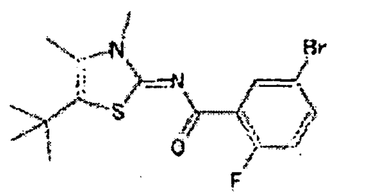 | 138 | 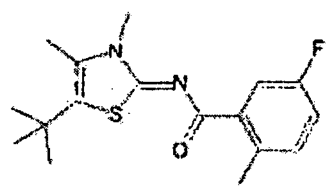 |
| 133 | 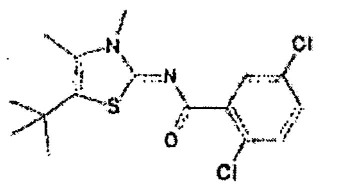 | 139 | 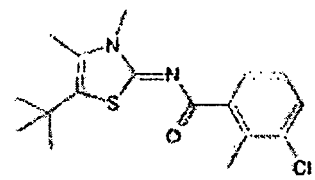 |
| 134 | 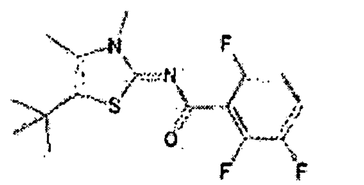 | 140 | 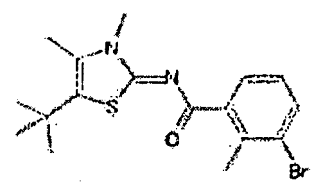 |
| 135 | 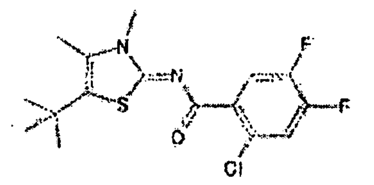 | 141 | 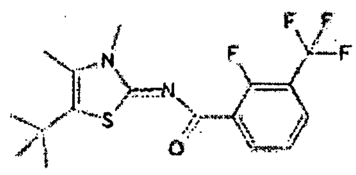 |
| 136 | 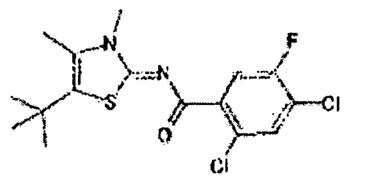 | 142 | 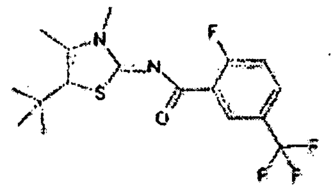 |
| 137 | 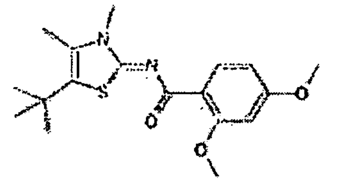 | 143 | 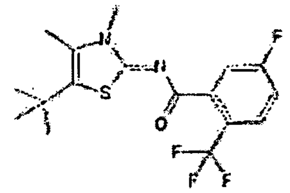 |

(continued)

| Example No. | chemical structure | Example No. | chemical structure |
|---|---|---|---|
| 144 | 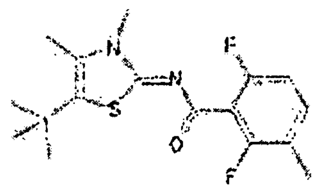 | 150 | 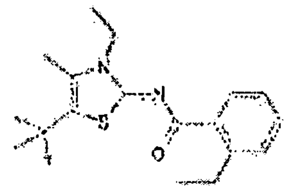 |
| 145 | 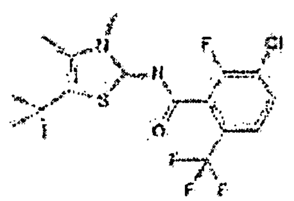 | 151 | 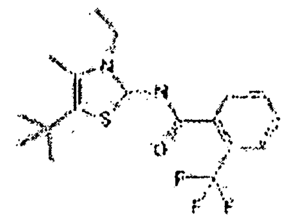 |
| 146 | 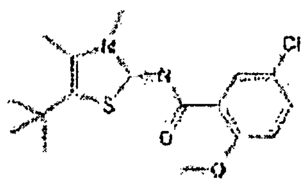 | 152 | 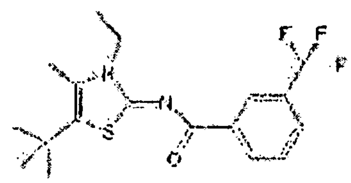 |
| 147 | 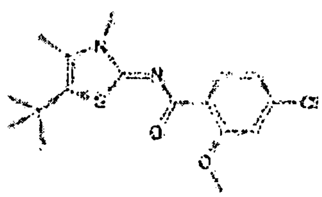 | 153 | 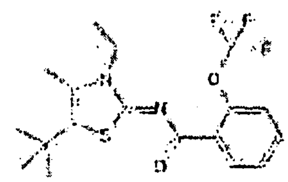 |
| 148 | 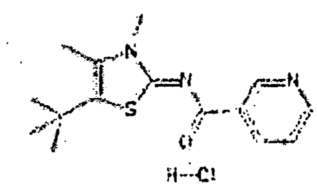 | 154 | 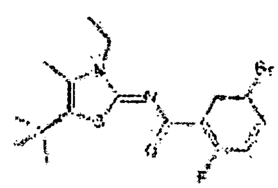 |
| 149 | 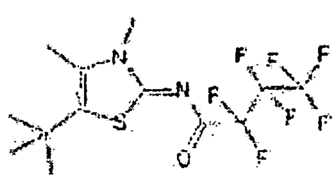 | 155 | 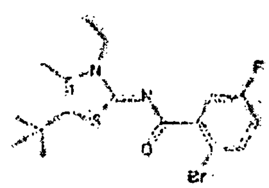 |
| 156 | 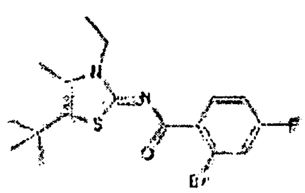 | 162 | 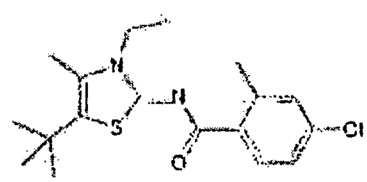 |

(continued)

| Example No. | chemical structure | Example No. | chemical structure |
|---|---|---|---|
| 157 | 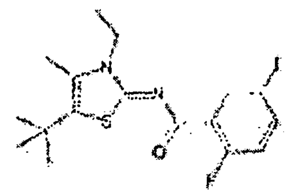 | 163 | 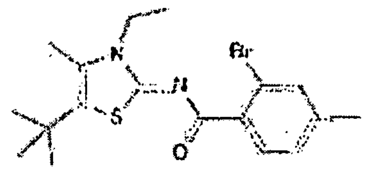 |
| 158 | 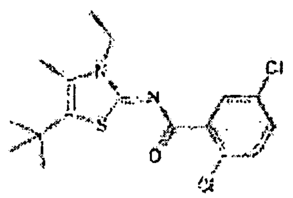 | 164 | 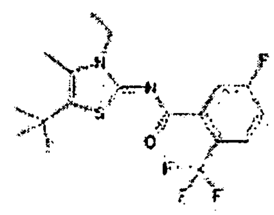 |
| 159 | 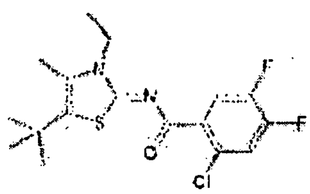 | 165 | 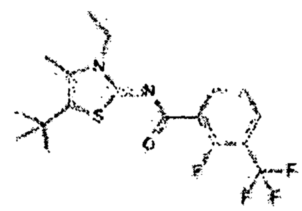 |
| 160 | 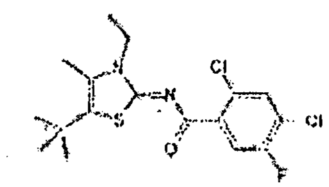 | 166 | 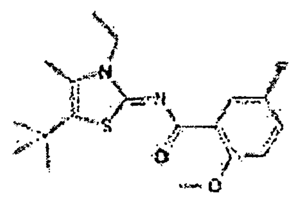 |
| 161 | 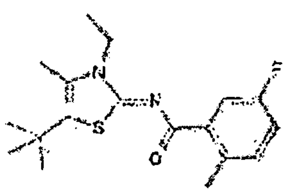 | 167 | 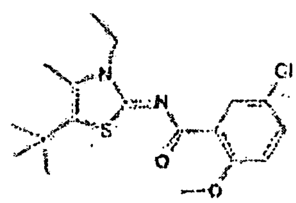 |
| 168 | 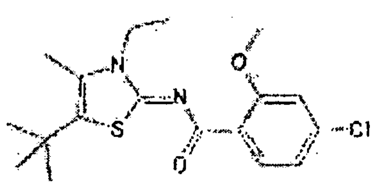 | 174 | 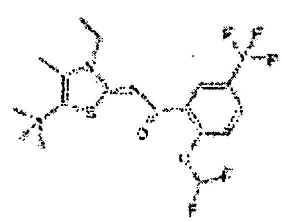 |
| 169 | 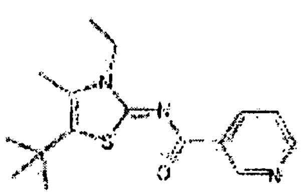 | 175 | 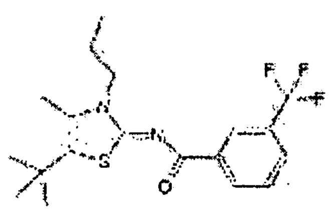 |

(continued)

| Example No. | chemical structure | Example No. | chemical structure |
|---|---|---|---|
| 170 | 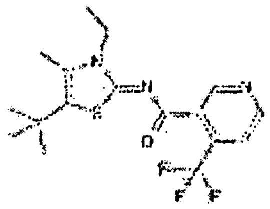 | 176 | 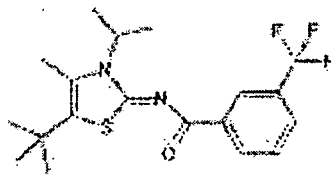 |
| 171 | 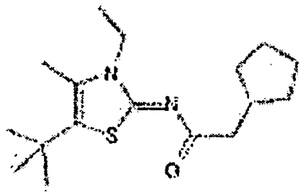 | 177 | 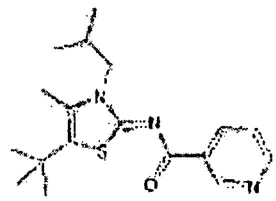 |
| 172 | 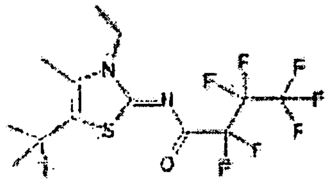 | 178 | 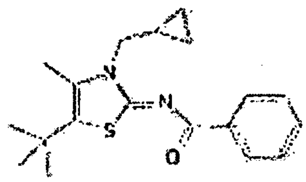 |
| 173 | 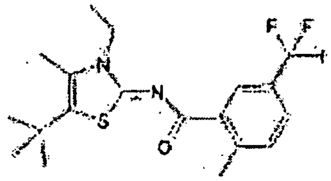 | 179 | 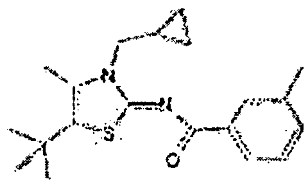 |
| 180 | 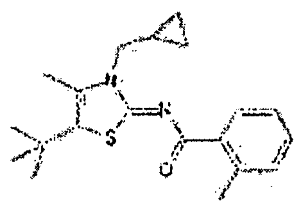 | 186 | 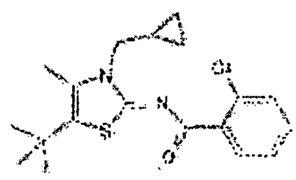 |
| 181 | 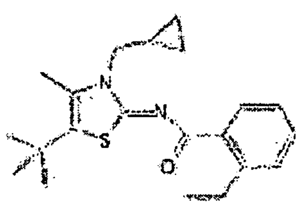 | 187 | 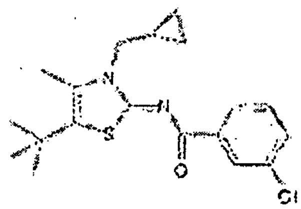 |
| 182 | 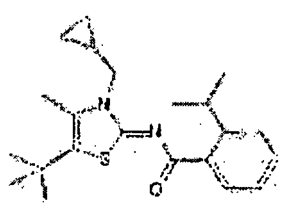 | 188 | 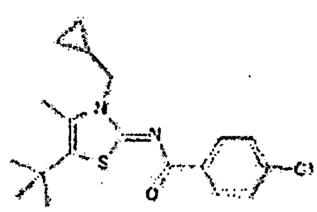 |

(continued)

| Example No. | chemical structure | Example No. | chemical structure |
|---|---|---|---|
| 183 | 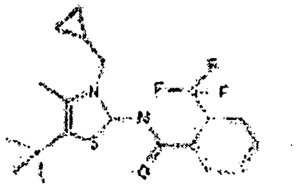 | 189 | 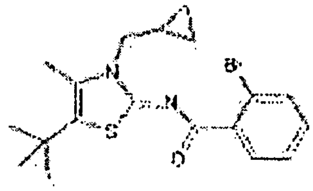 |
| 184 | 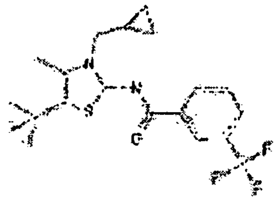 | 190 | 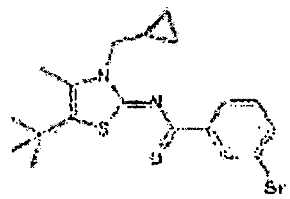 |
| 185 | 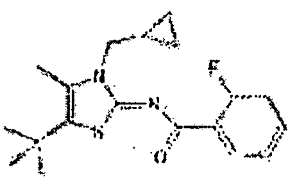 | 191 | 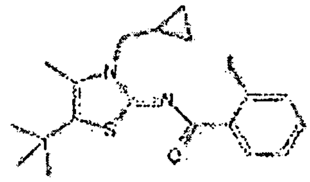 |
| 192 | 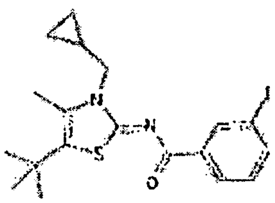 | 198 | 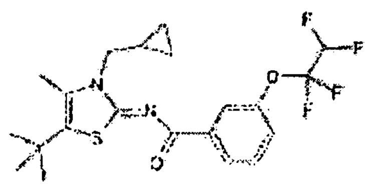 |
| 193 | 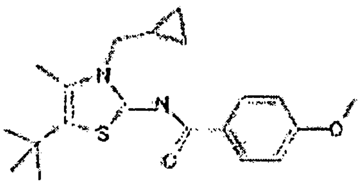 | 199 | 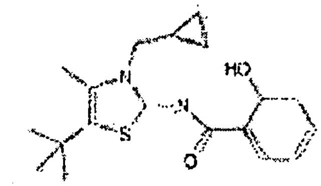 |
| 194 | 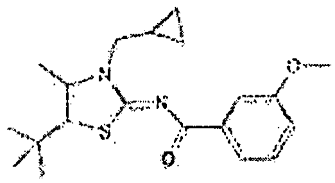 | 200 | 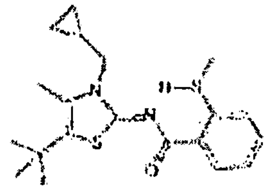 |
| 195 | 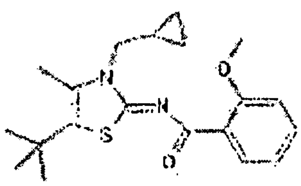 | 201 | 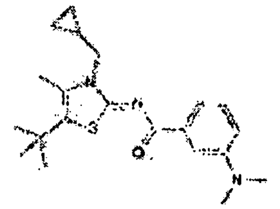 |

(continued)

| Example No. | chemical structure | Example No. | chemical structure |
|---|---|---|---|
| 196 | 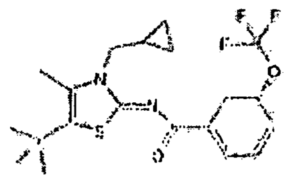 | 202 | 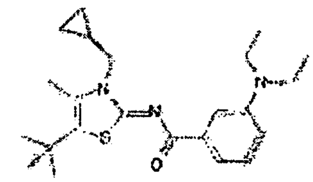 |
| 197 | 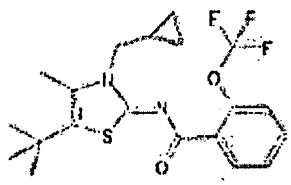 | 203 | 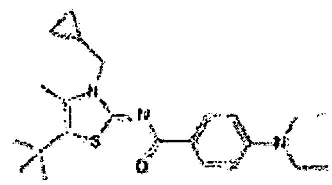 |
| 204 | 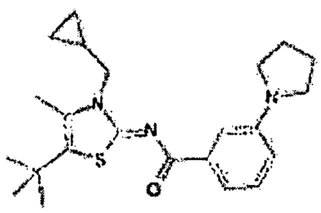 | 210 | 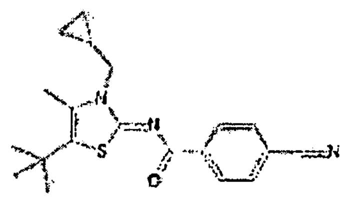 |
| 205 | 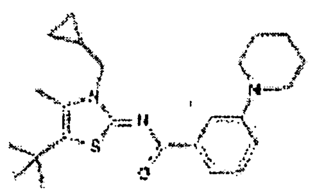 | 211 | 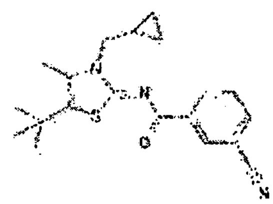 |
| 206 | 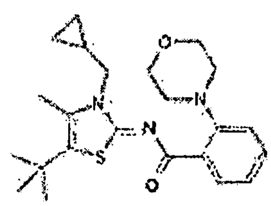 | 212 | 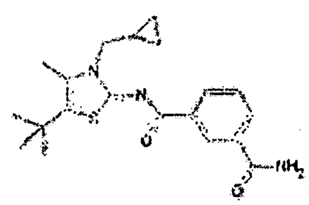 |
| 207 | 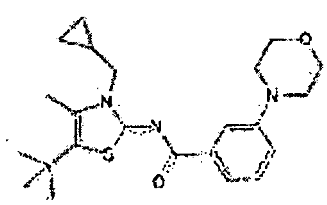 | 213 | 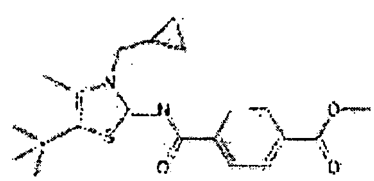 |
| 208 | 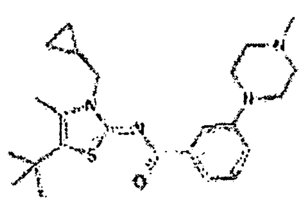 | 214 | 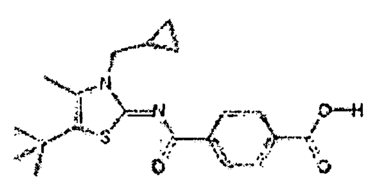 |

(continued)

| Example No. | chemical structure | Example No. | chemical structure |
|---|---|---|---|
| 209 | 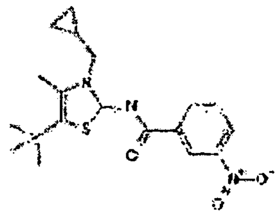 | 215 | 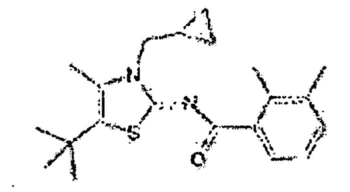 |
| 216 | 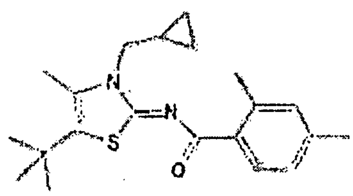 | 222 | 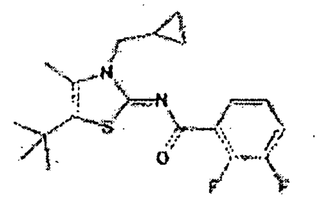 |
| 217 | 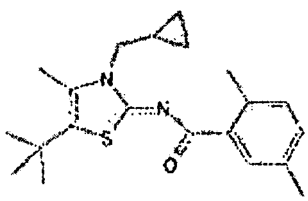 | 223 | 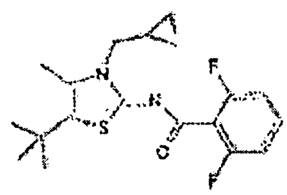 |
| 218 | 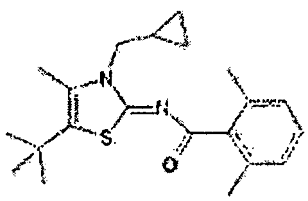 | 224 | 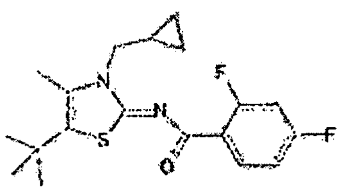 |
| 219 | 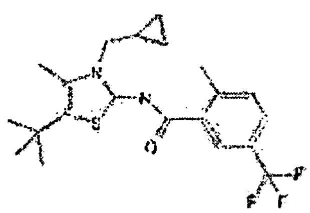 | 225 | 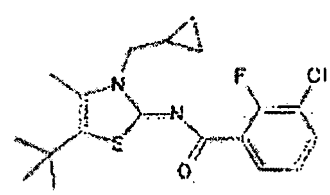 |
| 220 | 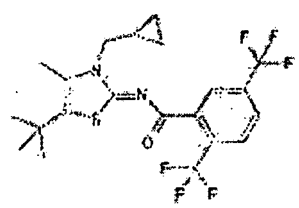 | 226 | 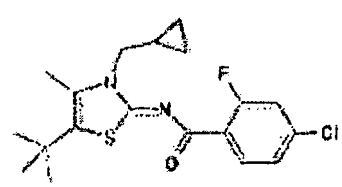 |
| 221 | 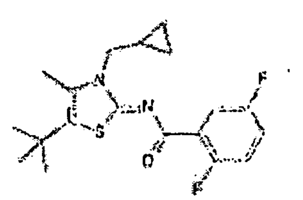 | 227 | 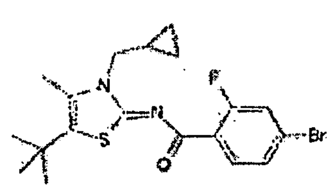 |

(continued)

| Example No. | chemical structure | Example No. | chemical structure |
|---|---|---|---|
| 228 | 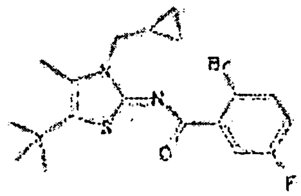 | 234 | 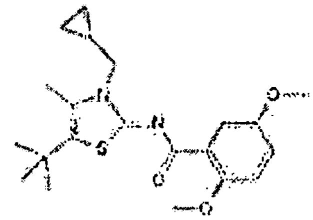 |
| 229 | 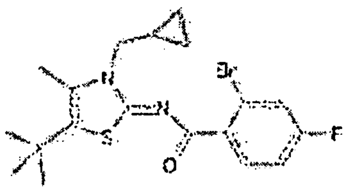 | 235 | 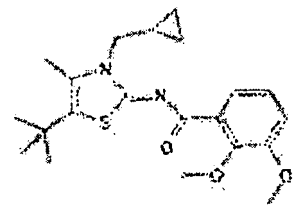 |
| 230 | 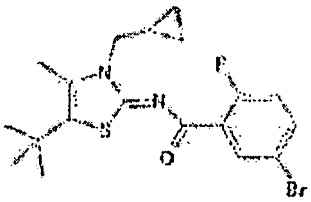 | 236 | 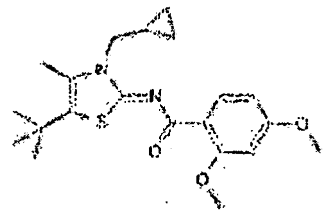 |
| 231 | 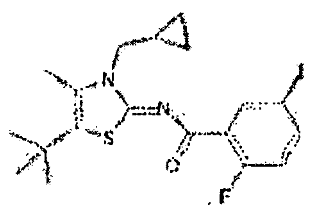 | 237 | 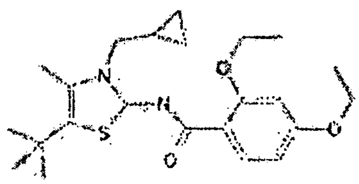 |
| 232 | 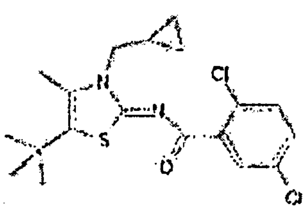 | 238 | 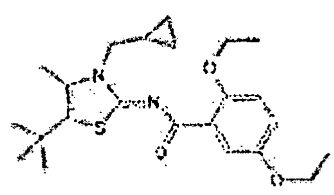 |
| 233 | 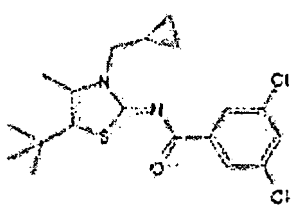 | 239 | 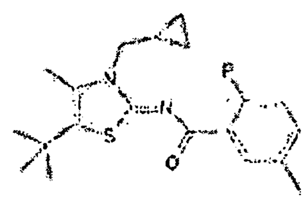 |
| 240 | 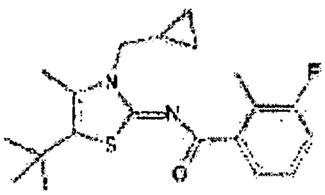 | 246 | 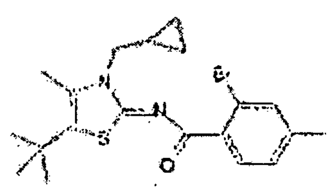 |

(continued)

| Example No. | chemical structure | Example No. | chemical structure |
|---|---|---|---|
| 241 | 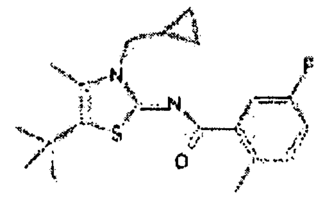 | 247 | 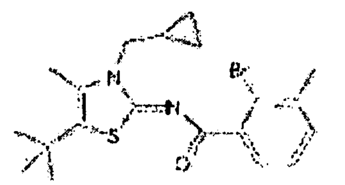 |
| 242 | 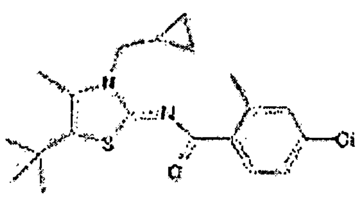 | 248 | 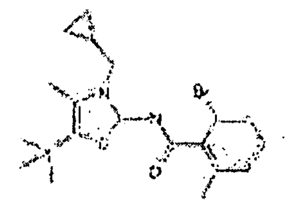 |
| 243 | 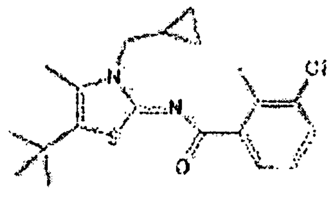 | 249 | 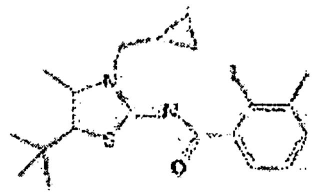 |
| 244 | 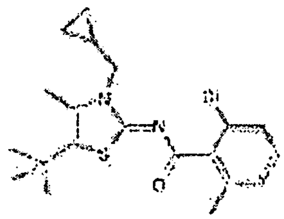 | 250 | 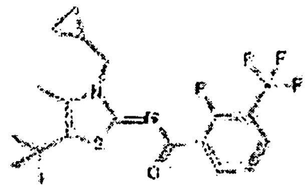 |
| 245 | 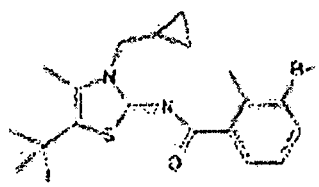 | 251 | 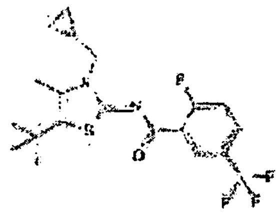 |
| 252 | 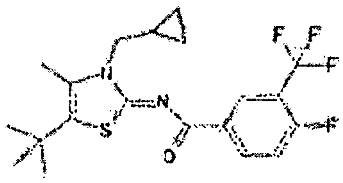 | 258 | 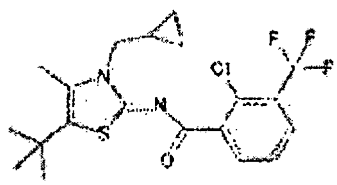 |
| 253 | 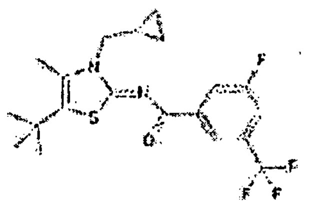 | 259 | 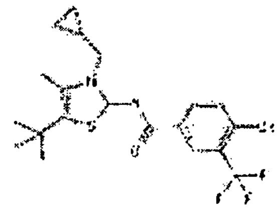 |

(continued)

| Example No. | chemical structure | Example No. | chemical structure |
|---|---|---|---|
| 254 | 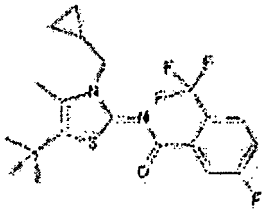 | 260 | 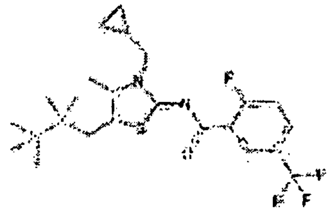 |
| 255 | 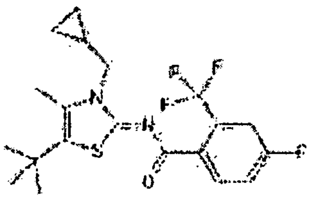 | 261 | 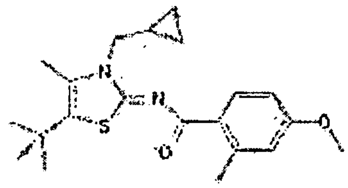 |
| 256 | 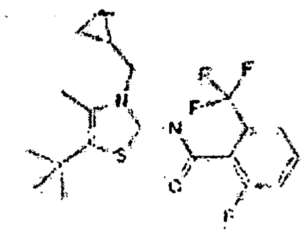 | 262 | 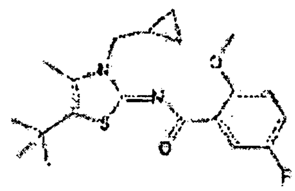 |
| 257 | 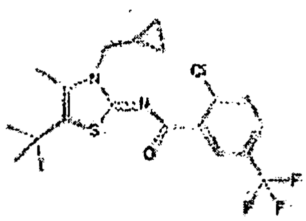 | 263 | 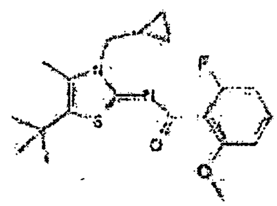 |
| 264 | 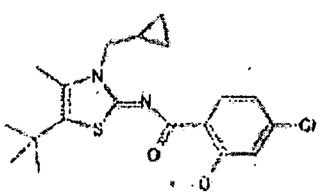 | 270 | 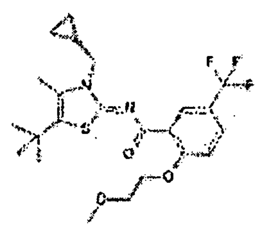 |
| 265 | 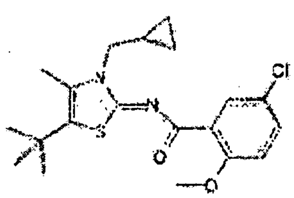 | 271 | 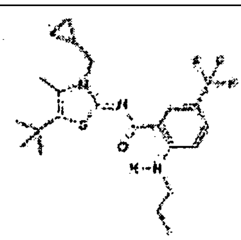 |
| 266 | 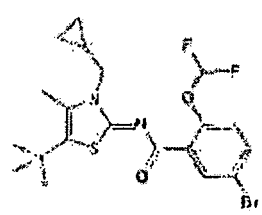 | 272 | 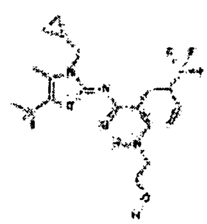 |

(continued)

| Example No. | chemical structure | Example No. | chemical structure |
|---|---|---|---|
| 267 | 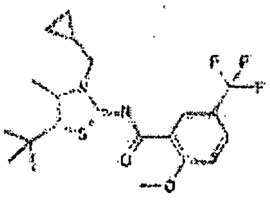 | 273 | 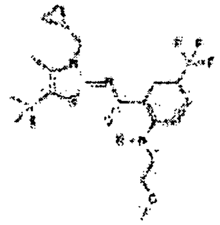 |
| 268 | 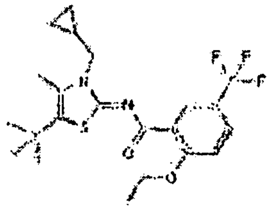 | 274 | 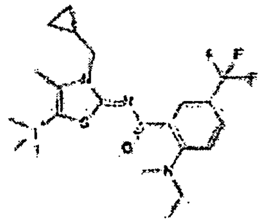 |
| 269 | 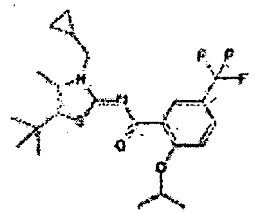 | 275 | 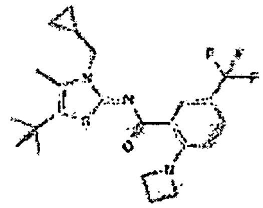 |
| 276 | 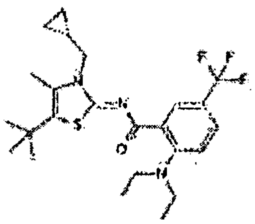 | 282 | 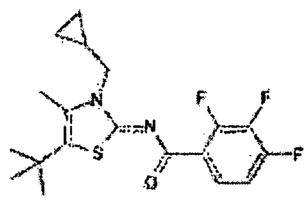 |
| 277 | 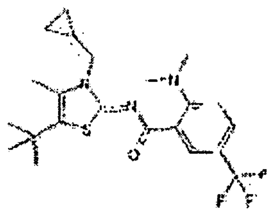 | 283 | 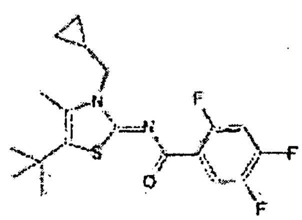 |
| 278 | 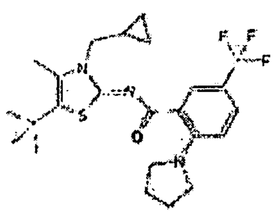 | 284 | 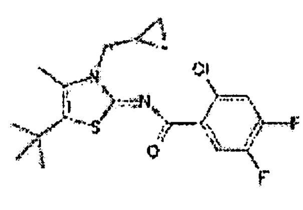 |
| 279 | 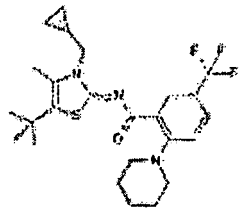 | 285 | 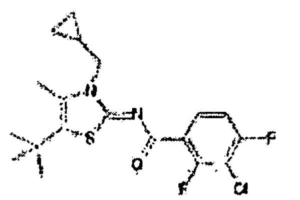 |

(continued)

| Example No. | chemical structure | Example No. | chemical structure |
|---|---|---|---|
| 280 | 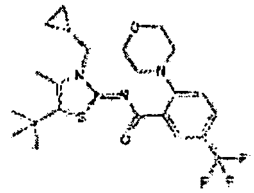 | 286 | 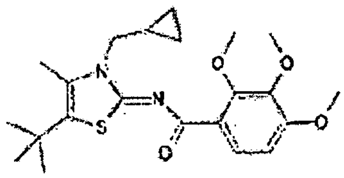 |
| 281 | 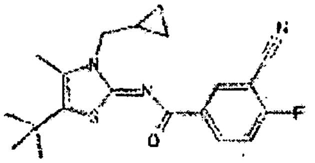 | 287 | 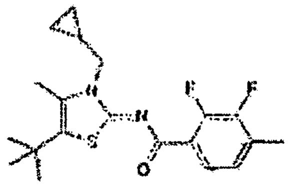 |
| 288 | 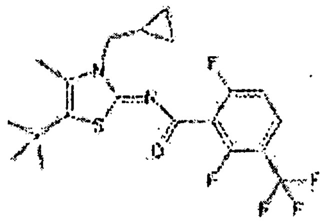 | 294 | 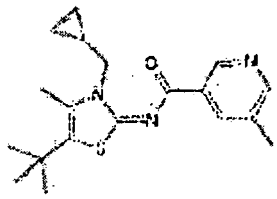 |
| 289 | 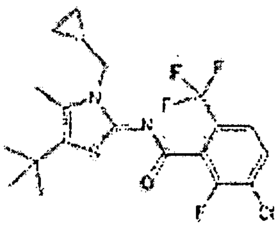 | 295 | 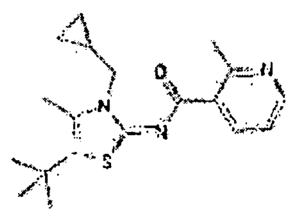 |
| 290 | 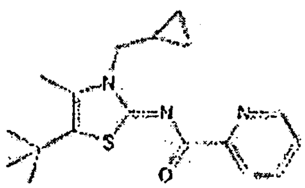 | 296 | 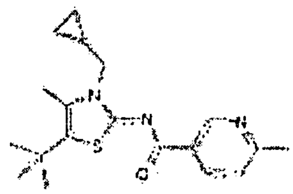 |
| 291 | 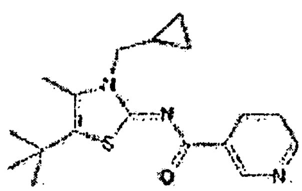 | 297 | 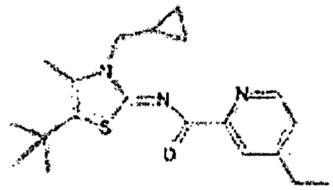 |
| 292 | 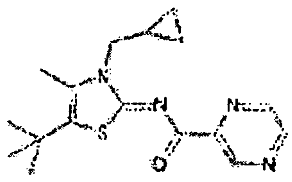 | 298 | 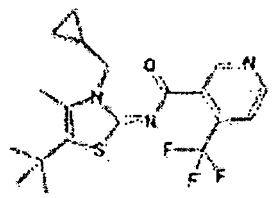 |

(continued)

| Example No. | chemical structure | Example No. | chemical structure |
|---|---|---|---|
| 293 | 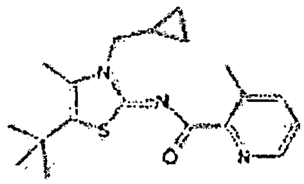 | 299 | 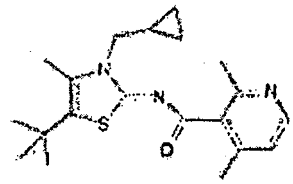 |
| 300 | 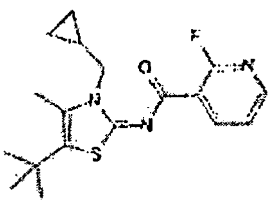 | 306 | 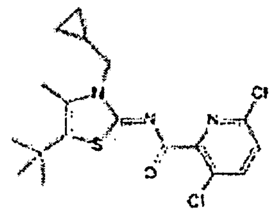 |
| 301 | 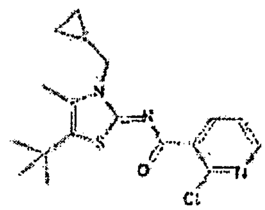 | 307 | 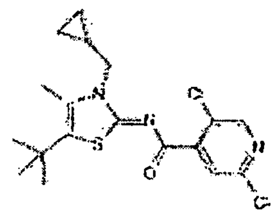 |
| 302 | 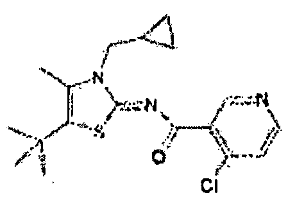 | 308 | 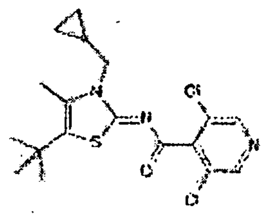 |
| 303 | 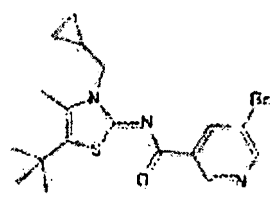 | 309 | 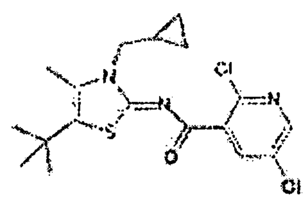 |
| 304 | 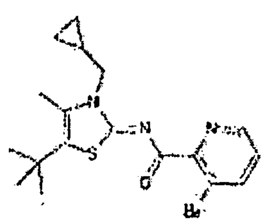 | 310 | 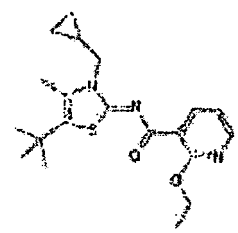 |
| 305 | 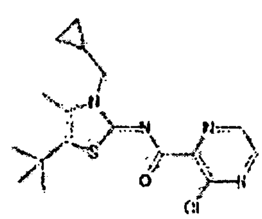 | 311 | 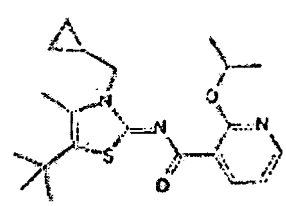 |

(continued)

| Example No. | chemical structure | Example No. | chemical structure |
|---|---|---|---|
| 312 | 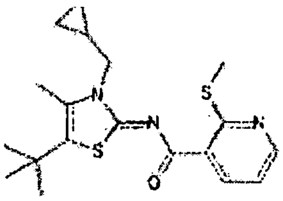 | 318 | 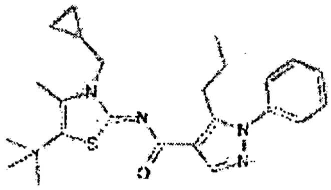 |
| 313 | 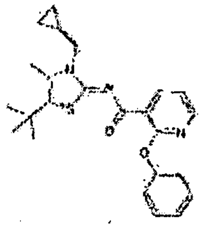 | 319 | 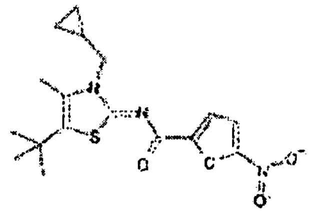 |
| 314 | 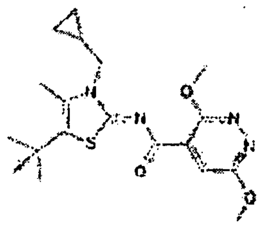 | 320 | 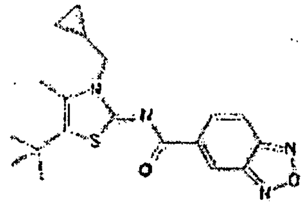 |
| 315 | 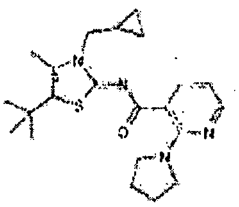 | 321 | 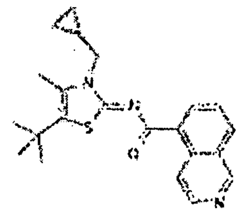 |
| 316 | 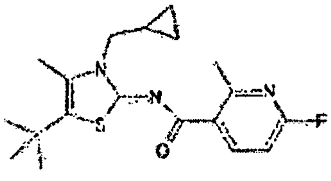 | 322 | 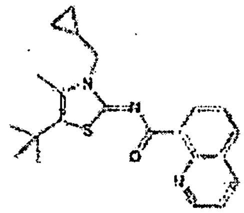 |
| 317 | 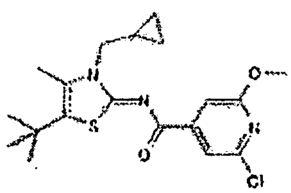 | 323 | 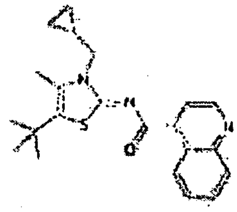 |
| 324 | 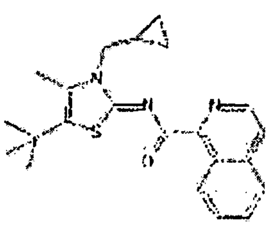 | 330 | 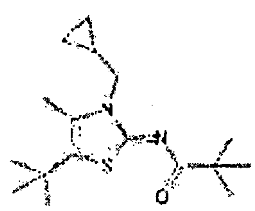 |

(continued)

| Example No. | chemical structure | Example No. | chemical structure |
|---|---|---|---|
| 325 | 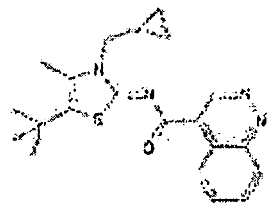 | 331 | 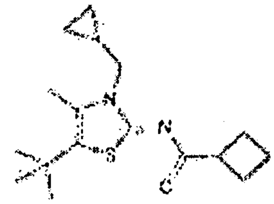 |
| 326 | 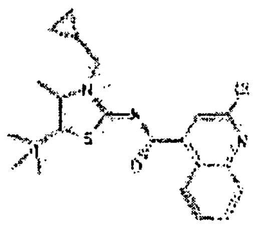 | 332 | 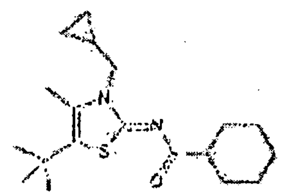 |
| 327 | 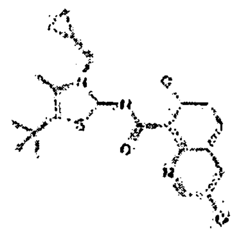 | 333 | 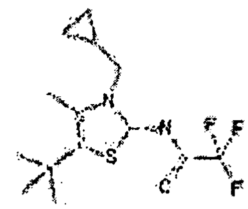 |
| 328 | 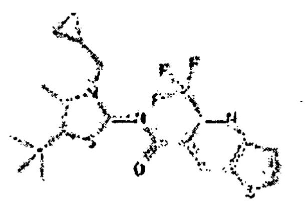 | 334 | 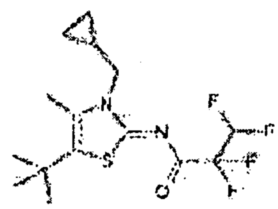 |
| 329 | 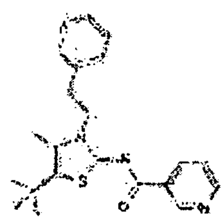 | 335 | 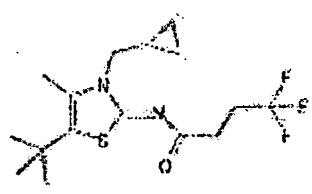 |
| 336 | 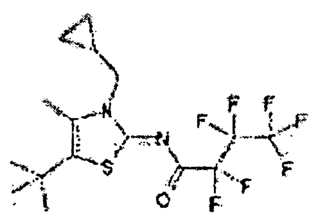 | 342 | 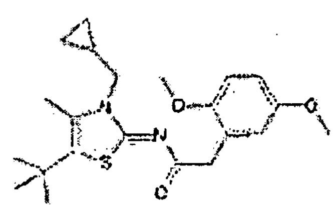 |
| 337 | 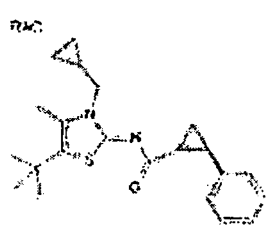 | 343 | 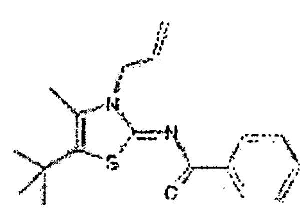 |

(continued)

| Example No. | chemical structure | Example No. | chemical structure |
|---|---|---|---|
| 338 | 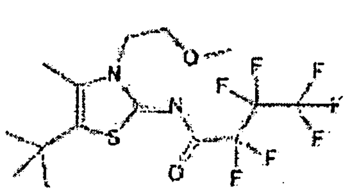 | 344 | 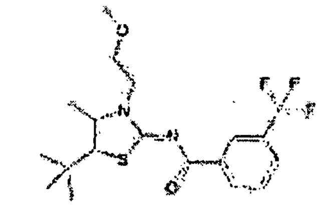 |
| 339 | 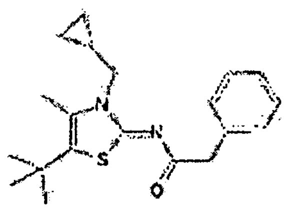 | 345 | 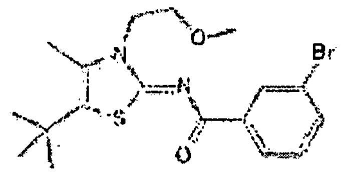 |
| 340 | 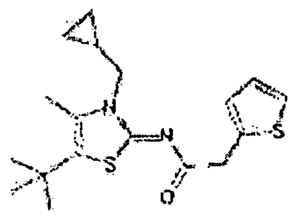 | 346 | 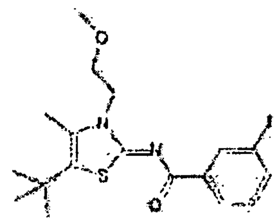 |
| 341 | 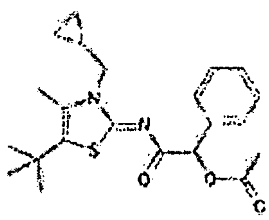 | 347 | 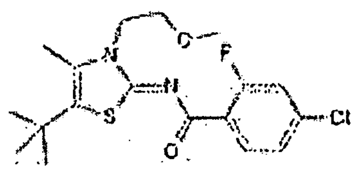 |
| 348 | 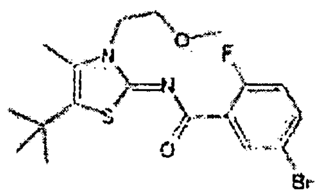 | 354 | 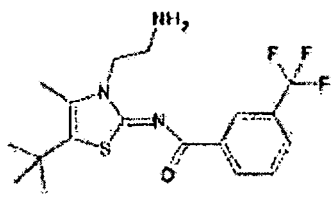 |
| 349 | 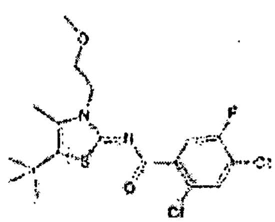 | 355 | 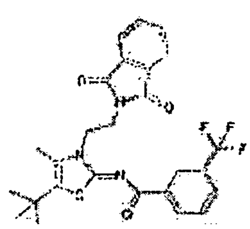 |
| 350 | 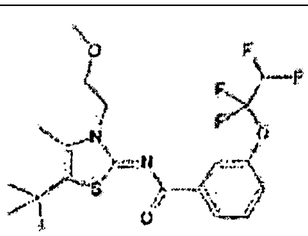 | 356 | 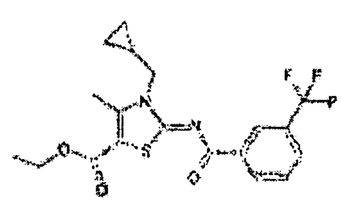 |

(continued)

| Example No. | chemical structure | Example No. | chemical structure |
|---|---|---|---|
| 351 | 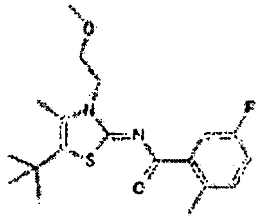 | 357 | 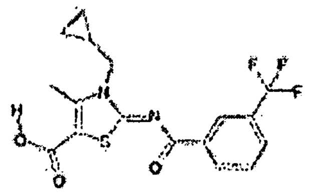 |
| 352 | 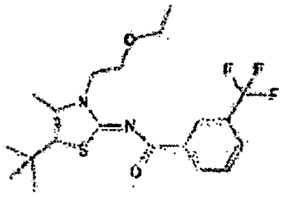 | 358 | 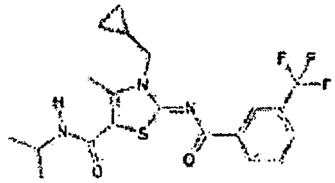 |
| 353 | 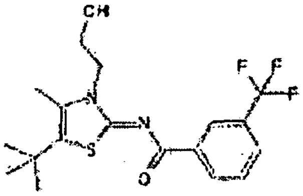 | 359 | 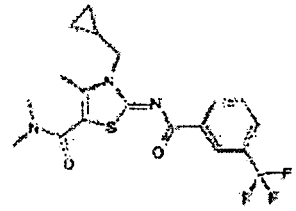 |
| 360 | 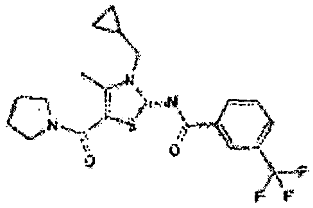 | 366 | 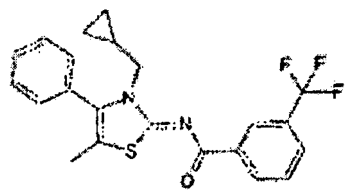 |
| 361 | 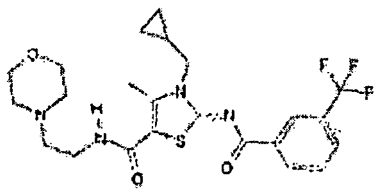 | 367 | 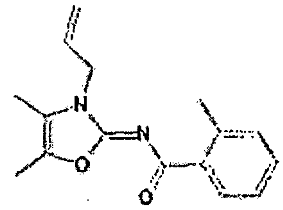 |
| 362 | 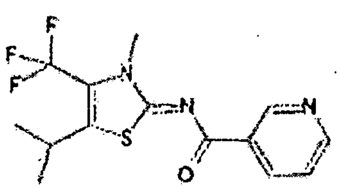 | 368 | 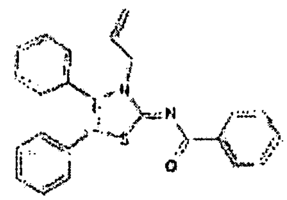 |
| 363 | 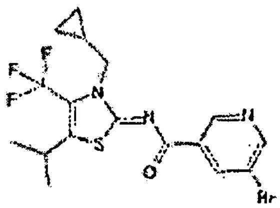 | 369 | 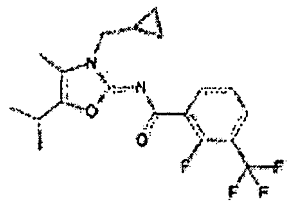 |

(continued)

| Example No. | chemical structure | Example No. | chemical structure |
|---|---|---|---|
| 364 | 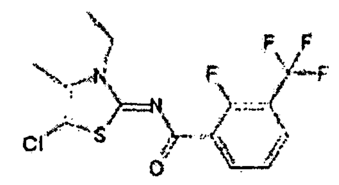 | 370 | 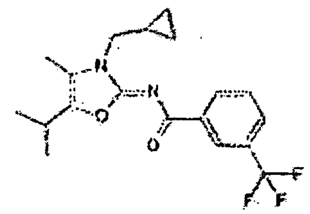 |
| 365 | 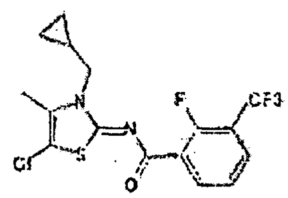 | 371 | 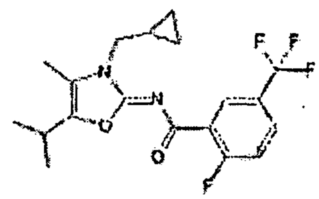 |
| 372 | 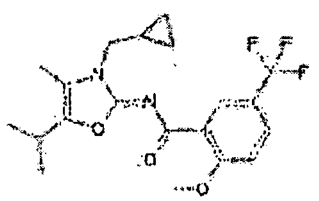 | 378 | 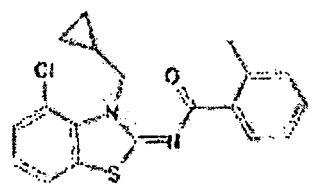 |
| 373 | 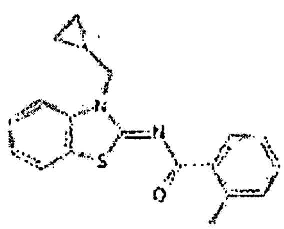 | 379 | 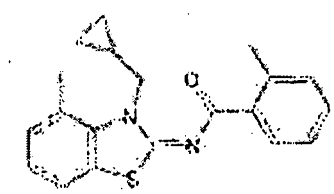 |
| 374 | 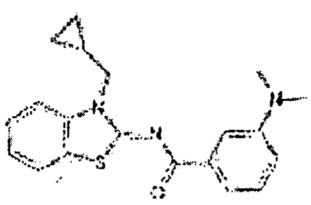 | 380 | 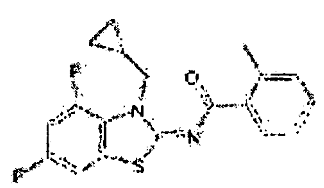 |
| 375 | 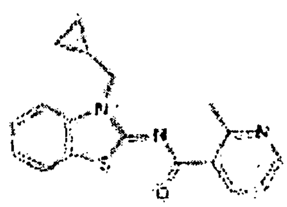 | 381 | 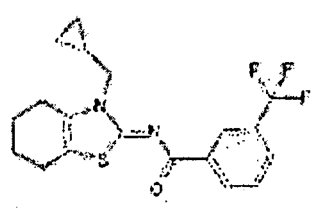 |
| 376 | 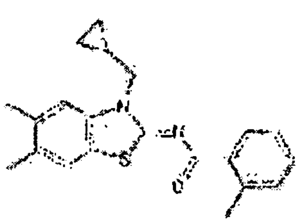 | 382 | 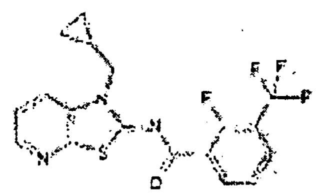 |

(continued)

| Example No. | chemical structure | Example No. | chemical structure |
|---|---|---|---|
| 377 | 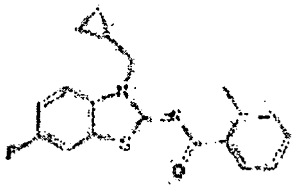 | 383 | 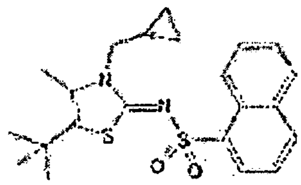 |
| 384 | 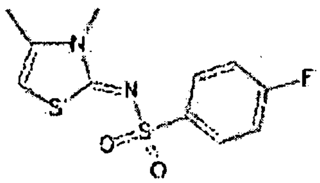 | 390 | 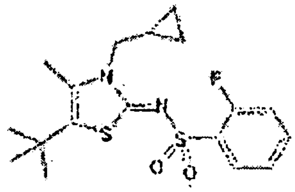 |
| 385 | 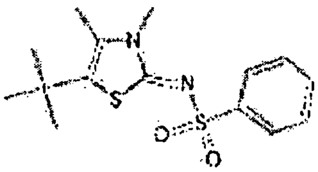 | 391 | 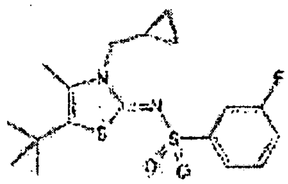 |
| 386 | 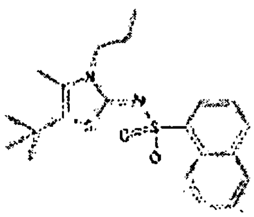 | 392 | 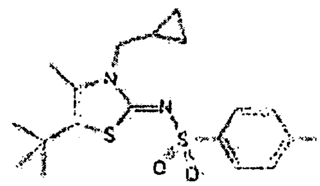 |
| 387 | 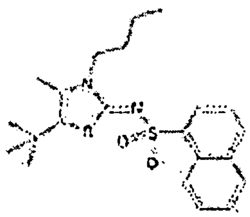 | 393 | 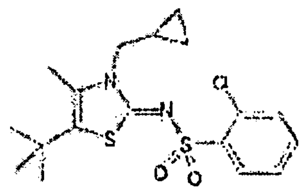 |
| 388 | 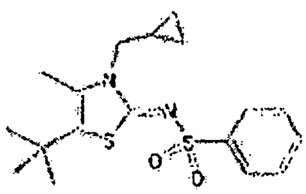 | 394 | 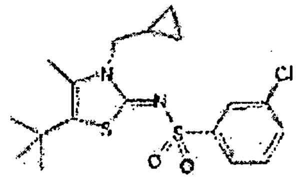 |
| 389 | 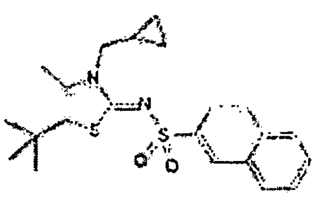 | 395 | 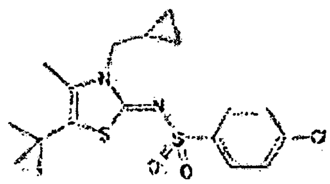 |

(continued)

| Example No. | chemical structure | Example No. | chemical structure |
|---|---|---|---|
| 396 | 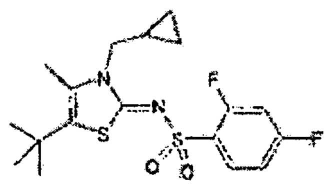 | 402 | 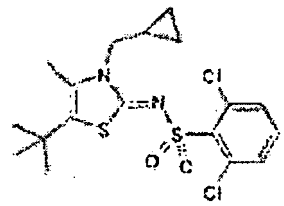 |
| 397 | 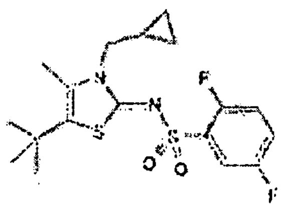 | 403 | 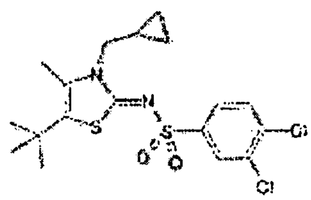 |
| 398 | 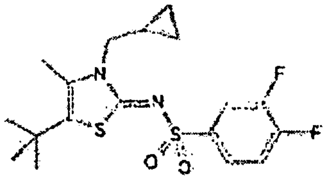 | 404 | 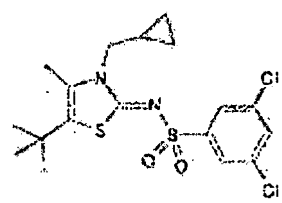 |
| 399 | 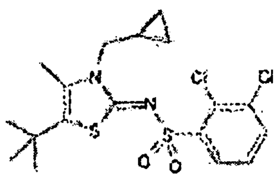 | 405 | 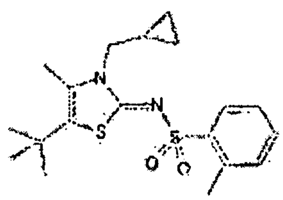 |
| 400 | 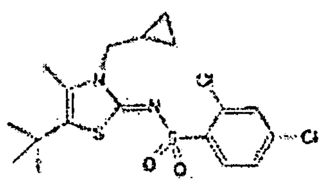 | 406 | 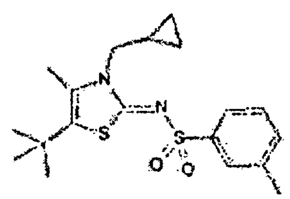 |
| 401 | 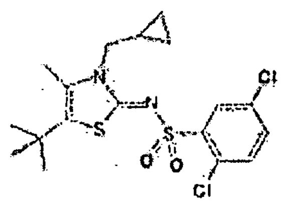 | 407 | 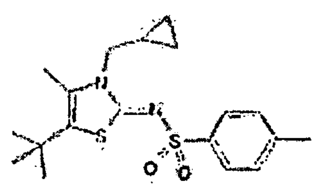 |
| 408 | 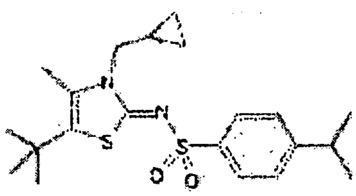 | 414 | 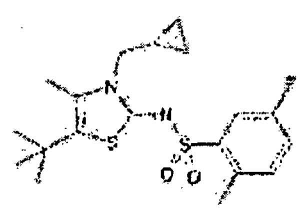 |

(continued)

| Example No. | chemical structure | Example No. | chemical structure |
|---|---|---|---|
| 409 | 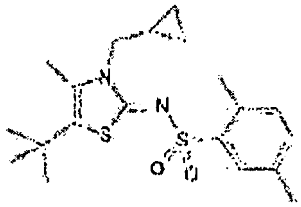 | 415 | 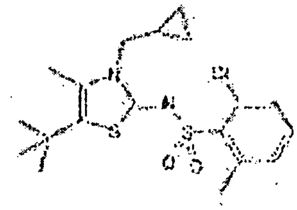 |
| 410 | 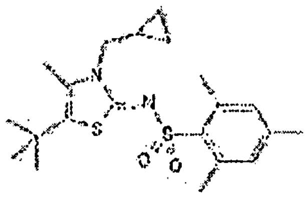 | 416 | 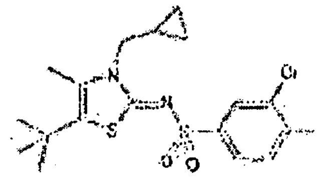 |
| 411 | 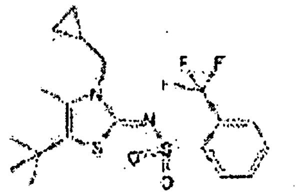 | 417 | 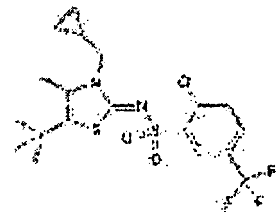 |
| 412 | 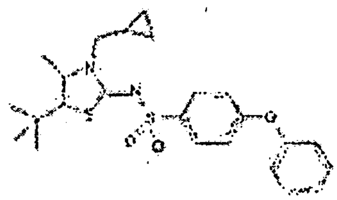 | 418 | 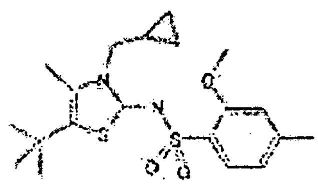 |
| 413 | 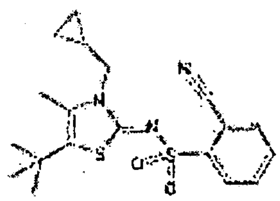 | 419 | 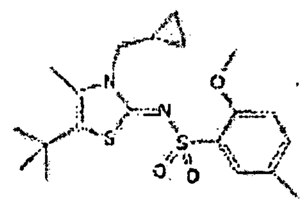 |
| 420 | 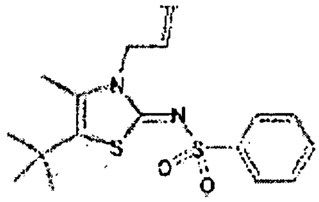 | 426 | 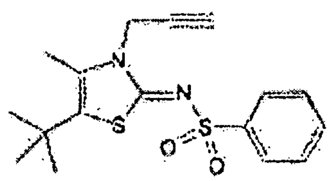 |
| 421 | 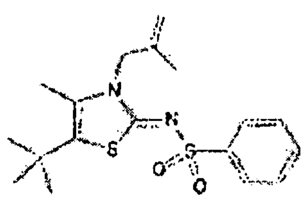 | 427 | 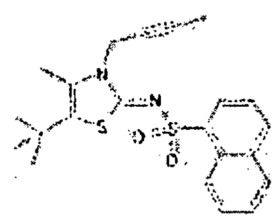 |

(continued)

| Example No. | chemical structure | Example No. | chemical structure |
|---|---|---|---|
| 422 | 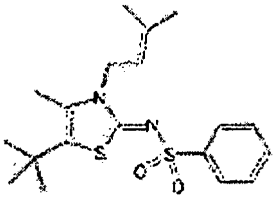 | 428 | 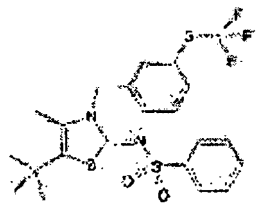 |
| 423 | 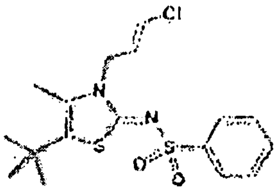 | 429 | 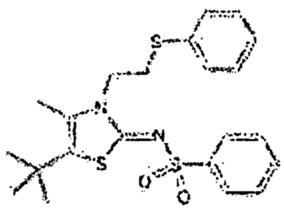 |
| 424 | 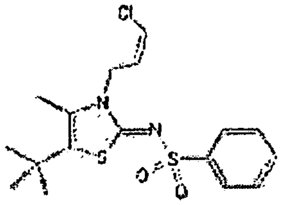 | 430 | 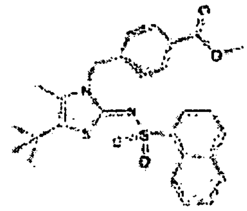 |
| 425 | 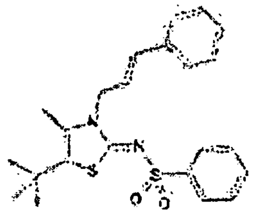 | 431 | 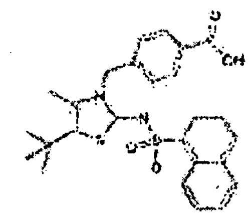 |
| 432 | 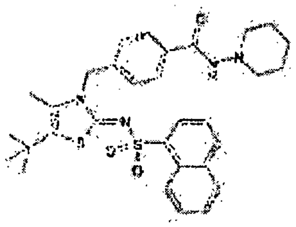 | 438 | 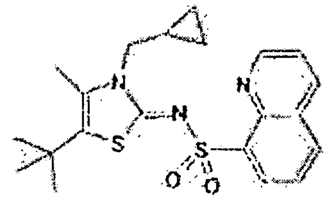 |
| 433 | 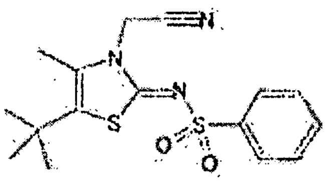 | 439 | 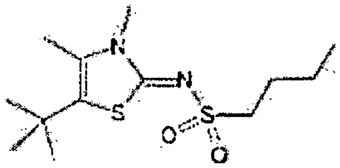 |
| 434 | 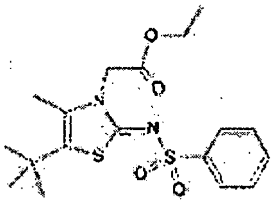 | 440 | 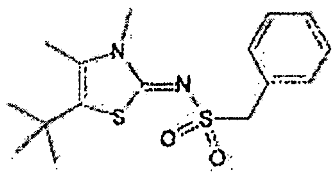 |

(continued)

| Example No. | chemical structure | Example No. | chemical structure |
|---|---|---|---|
| 435 | 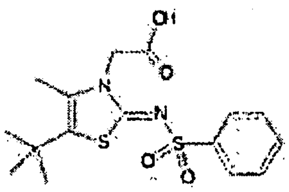 | 441 | 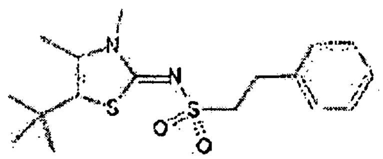 |
| 436 | 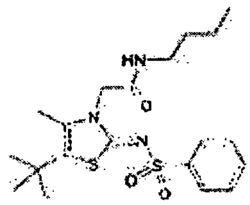 | 442 | 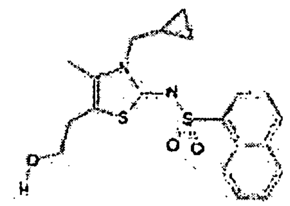 |
| 437 | 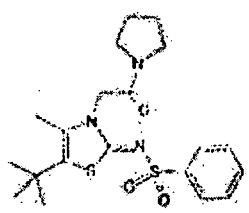 | 443 | 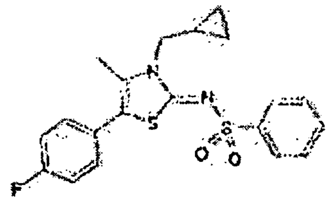 |
| 444 | 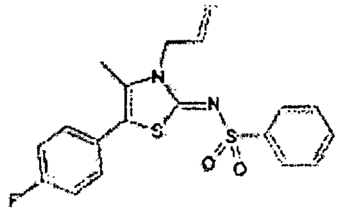 | | |
| 445 | 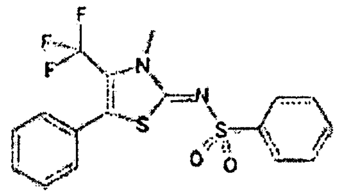 | | |
| 446 | 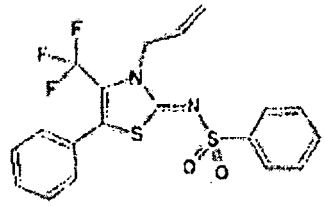 | | |
| 447 | 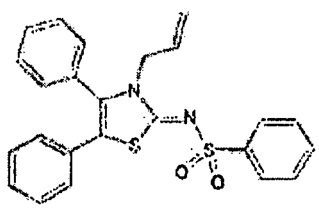 | | |

Table 3

| Example No. | chemical structure | Example No. | chemical structure |
|---|---|---|---|
| 448 | 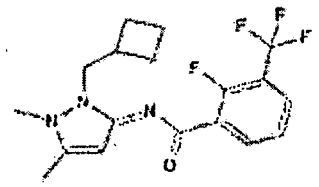 | 453 | 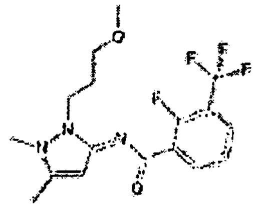 |
| 449 | 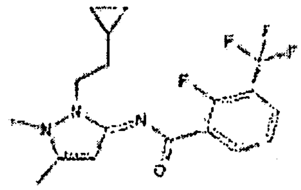 | 454 | 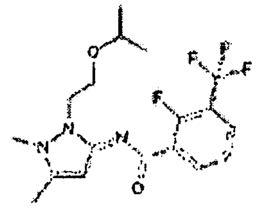 |
| 450 | 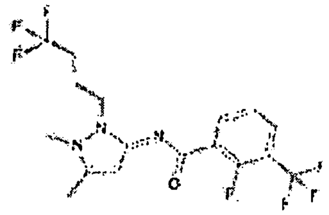 | 455 | 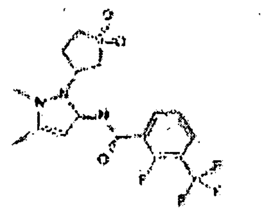 |
| 451 | 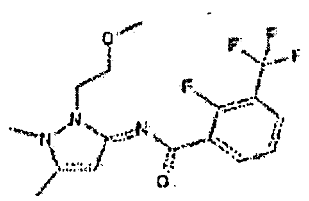 | 456 | 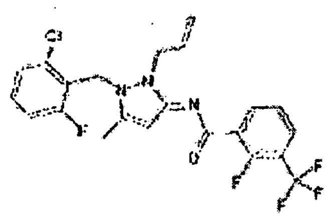 |
| 452 | 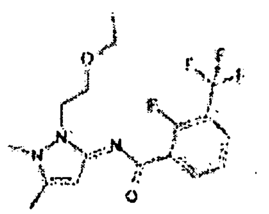 | 457 | 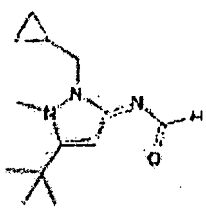 |
| 458 | 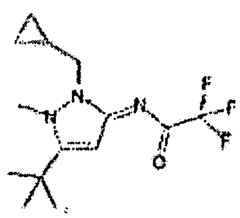 | 463 | 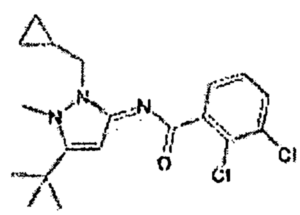 |

(continued)

| Example No. | chemical structure | Example No. | chemical structure |
|---|---|---|---|
| 459 | 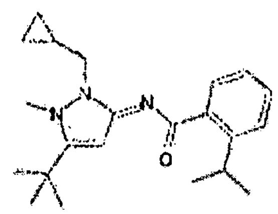 | 464 | 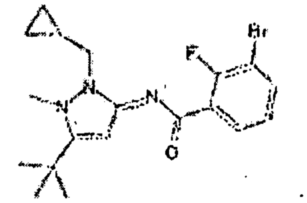 |
| 460 | 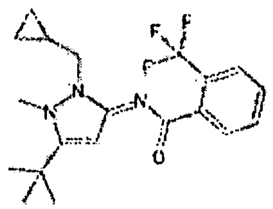 | 465 | 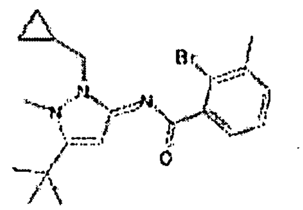 |
| 461 | 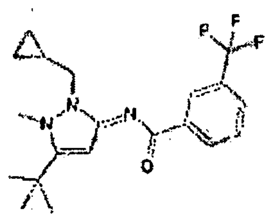 | 466 | 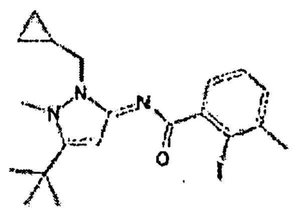 |
| 462 | 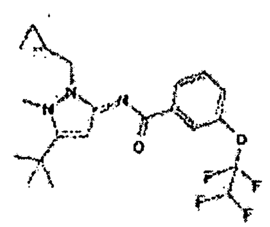 | 467 | 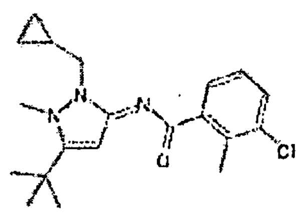 |
| 468 | 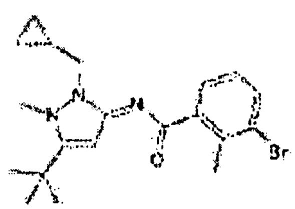 | 473 | 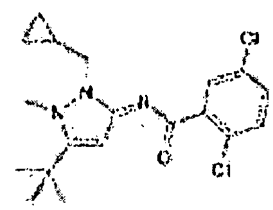 |
| 469 | 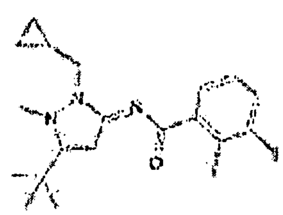 | 474 | 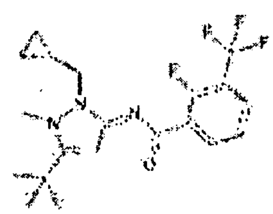 |

(continued)

| Example No. | chemical structure | Example No. | chemical structure |
|---|---|---|---|
| 470 | 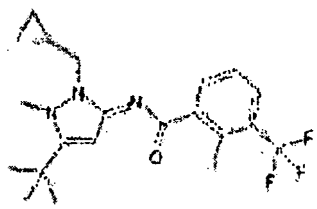 | 475 | 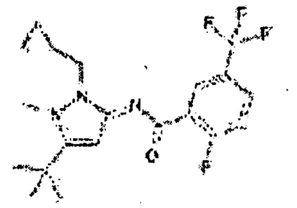 |
| 471 | 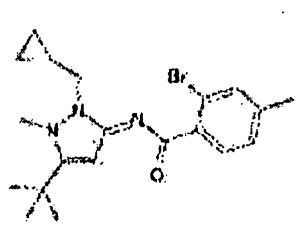 | 476 | 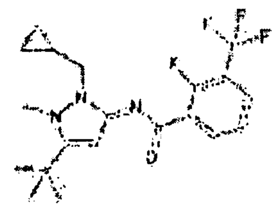 |
| 472 | 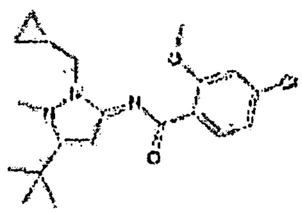 | 477 | 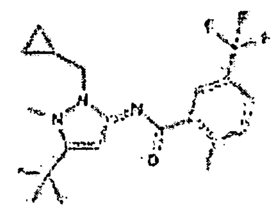 |
| 478 | 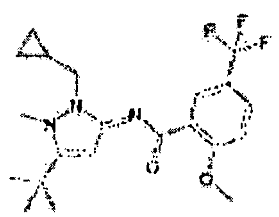 | 483 | 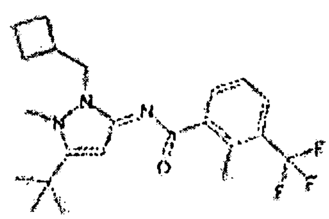 |
| 479 | 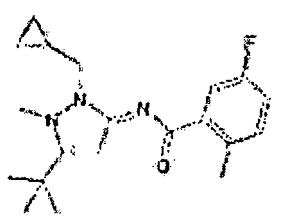 | 484 | 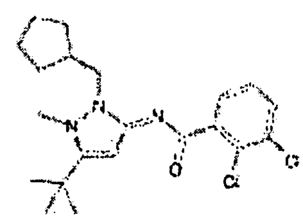 |
| 480 | 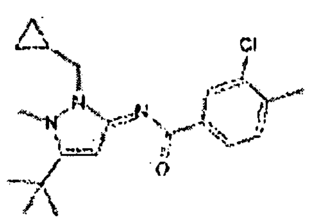 | 485 | 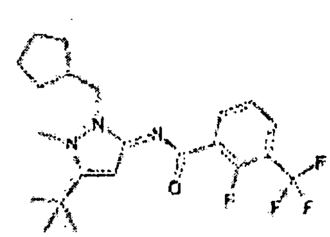 |

(continued)

| Example No. | chemical structure | Example No. | chemical structure |
|---|---|---|---|
| 481 | 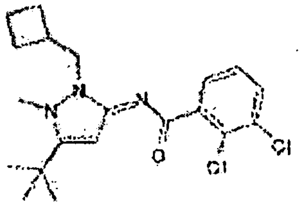 | 486 | 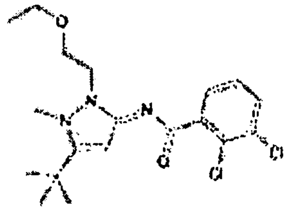 |
| 482 | 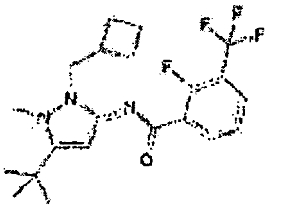 | 487 | 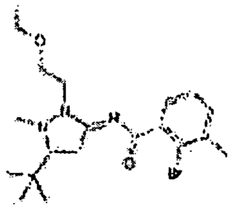 |
| 488 | 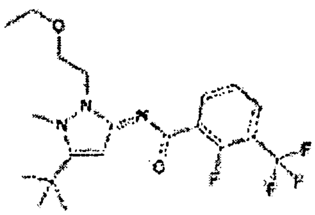 | 493 | 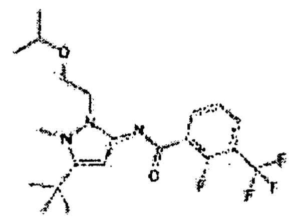 |
| 489 | 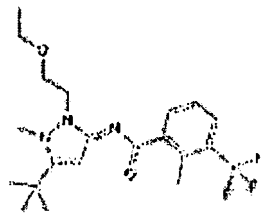 | 494 | 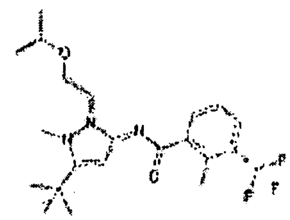 |
| 490 | 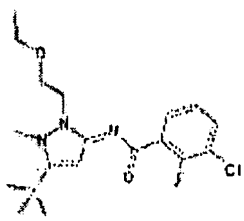 | 495 | 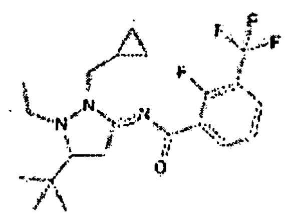 |
| 491 | 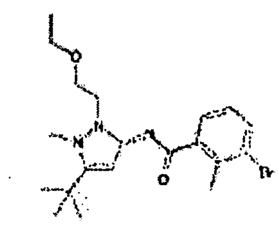 | 496 | 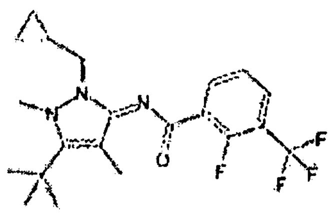 |

(continued)

| Example No. | chemical structure | Example No. | chemical structure |
|---|---|---|---|
| 492 | 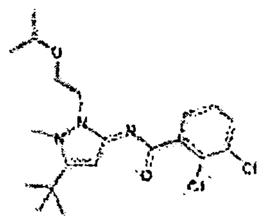 | 497 | 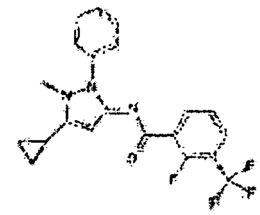 |
| 498 | 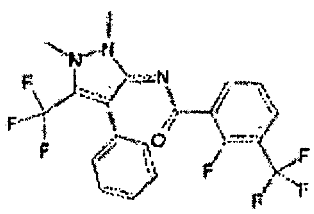 | 503 | 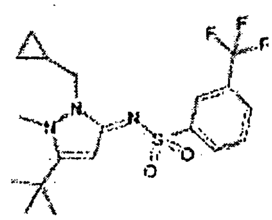 |
| 499 | 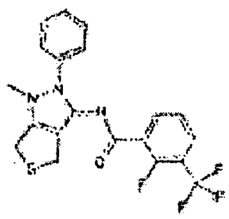 | 504 | 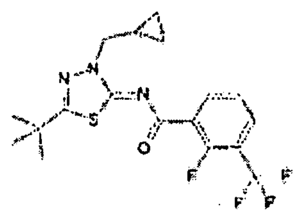 |
| 500 | 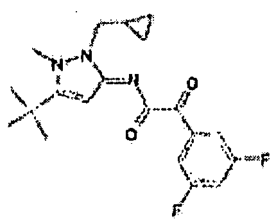 | 505 | 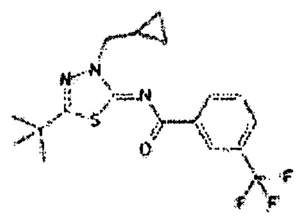 |
| 501 | 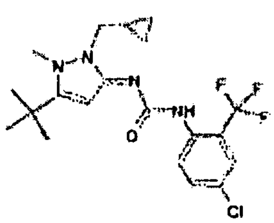 | 506 | 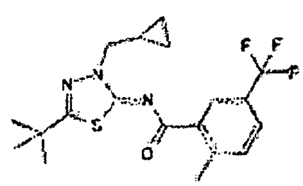 |
| 502 | 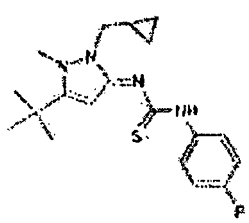 | 507 | 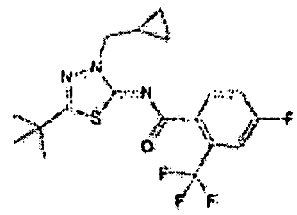 |

(continued)

| Example No. | chemical structure | Example No. | chemical structure |
|---|---|---|---|
| 508 | 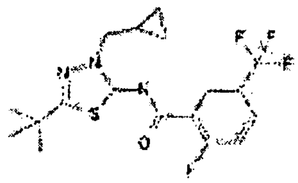 | 513 | 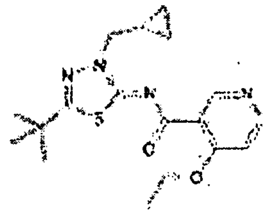 |
| 509 | 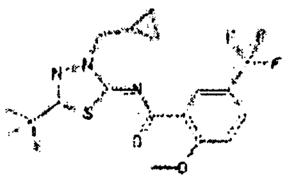 | 514 | 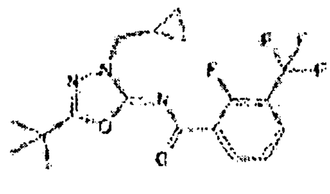 |
| 510 | 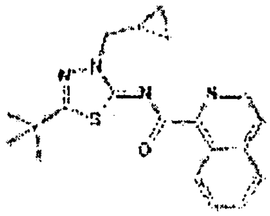 | 515 | 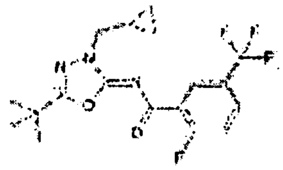 |
| 511 | 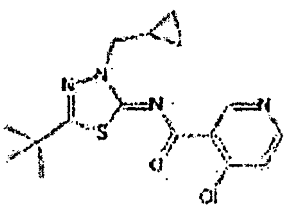 | | |
| 512 | 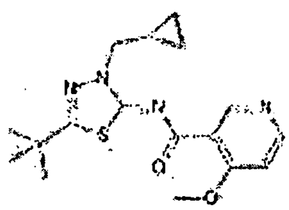 | | |

Table 4

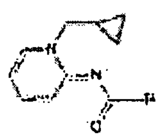

(continued)

| Compound No. | R | Compound No. | R |
|---|---|---|---|
| 1001 | 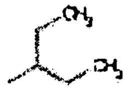 | 1008 | 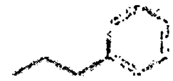 |
| 1002 | 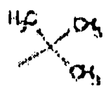 | 1009 | 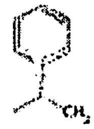 |
| 1003 | 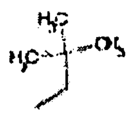 | 1010 | 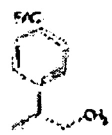 |
| 1004 | 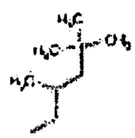 | 1011 | 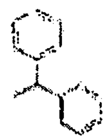 |
| 1005 | 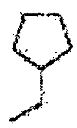 | 1012 | 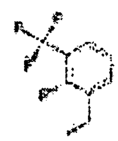 |
| 1006 | 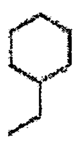 | 1013 | 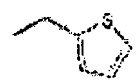 |
| 1007 | 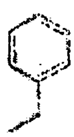 | 1014 | 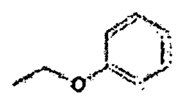 |
| 1015 | 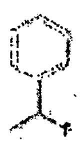 | 1023 | 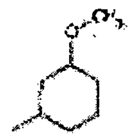 |
| 1016 | 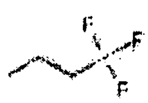 | 1024 | 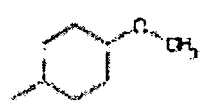 |
| 1017 | 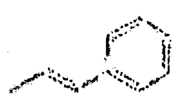 | 1025 | 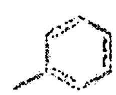 |

(continued)

| Compound No. | R | Compound No. | R |
|---|---|---|---|
| 1018 | 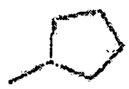 | 1026 | 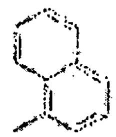 |
| 1019 | 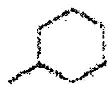 | 1027 | 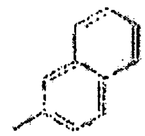 |
| 1020 | 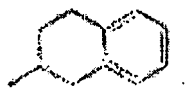 | 1028 | 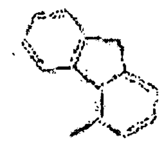 |
| 1021 | 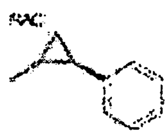 | 1029 | 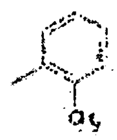 |
| 1022 | 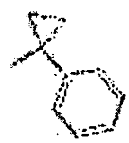 | 1030 | 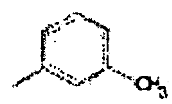 |
| 1031 | 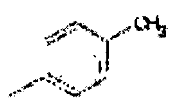 | 1039 | 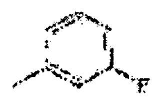 |
| 1032 | 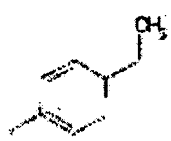 | 1040 | 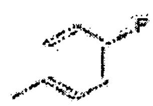 |
| 1033 | 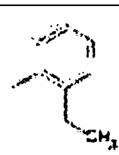 | 1041 | 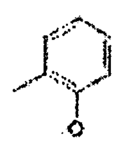 |
| 1034 | 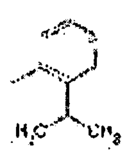 | 1042 | 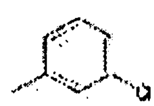 |
| 1035 | 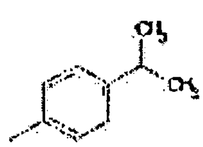 | 1043 | 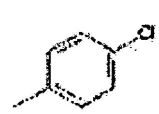 |

(continued)

| Compound No. | R | Compound No. | R |
|---|---|---|---|
| 1036 | 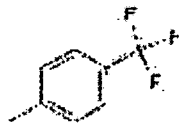 | 1044 | 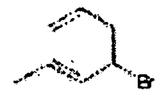 |
| 1037 | 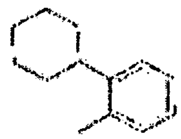 | 1045 | 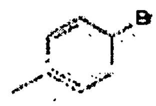 |
| 1038 | 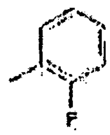 | 1046 | 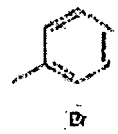 |
| 1047 | 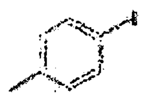 | 1055 | 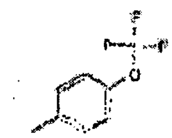 |
| 1048 | 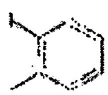 | 1056 | 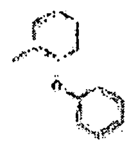 |
| 1049 | 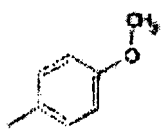 | 1057 | 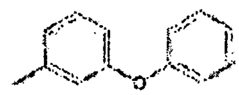 |
| 1050 | 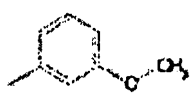 | 1050 | 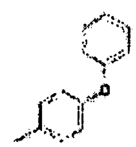 |
| 1051 | 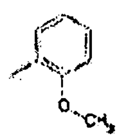 | 1059 | 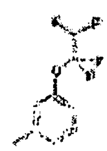 |
| 1052 | 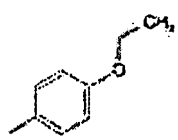 | 1060 | 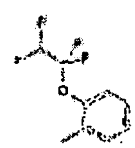 |
| 1053 | 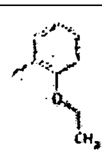 | 1061 | 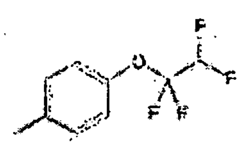 |

(continued)

| Compound No. | R | Compound No. | R |
|---|---|---|---|
| 1054 | 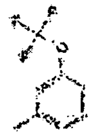 | 1062 | 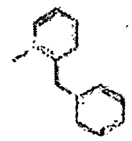 |
| 1063 | 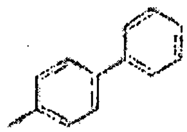 | 1071 | 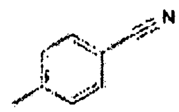 |
| 1064 | 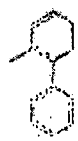 | 1072 | 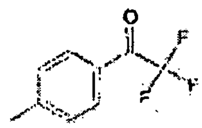 |
| 1065 | 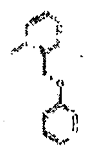 | 1073 | 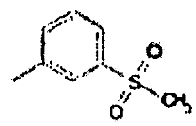 |
| 1066 | 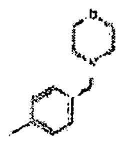 | 1074 | 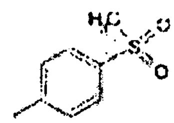 |
| 1067 | 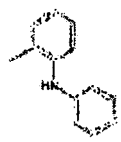 | 1075 | 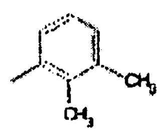 |
| 1068 | 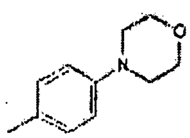 | 1076 | 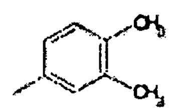 |
| 1069 | 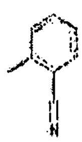 | 1077 | 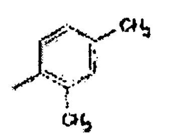 |
| 1070 | 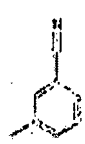 | 1078 | 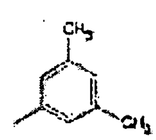 |
| 1079 | 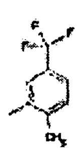 | 1087 | 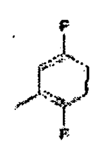 |

(continued)

| Compound No. | R | Compound No. | R |
|---|---|---|---|
| 1080 | 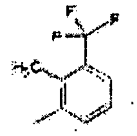 | 1088 | 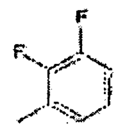 |
| 1081 | 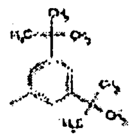 | 1089 | 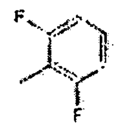 |
| 1082 | 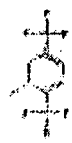 | 1090 | 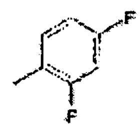 |
| 1083 | 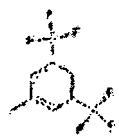 | 1091 | 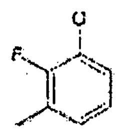 |
| 1084 | 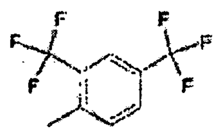 | 1092 | 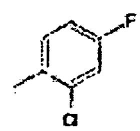 |
| 1085 | 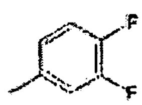 | 1093 | 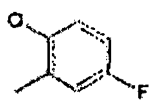 |
| 1086 | 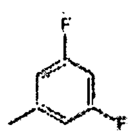 | 1094 | 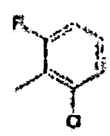 |
| 1095 | 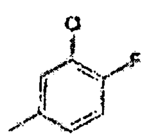 | 1103 | 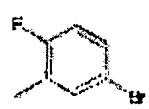 |
| 1096 | 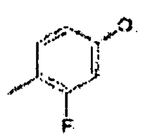 | 1104 | 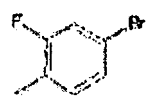 |
| 1097 | 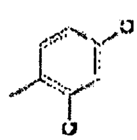 | 1105 | 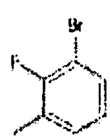 |

(continued)

| Compound No. | R | Compound No. | R |
|---|---|---|---|
| 1098 | 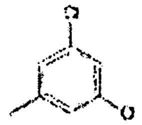 | 1106 | 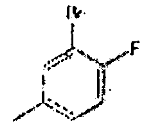 |
| 1099 | 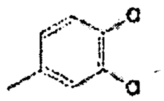 | 1107 | 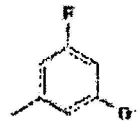 |
| 1100 | 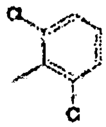 | 1108 | 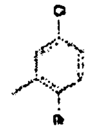 |
| 1101 | 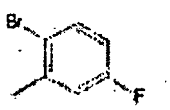 | 1109 | 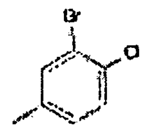 |
| 1102 | 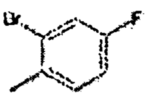 | 1110 | 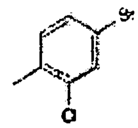 |
| 1111 | 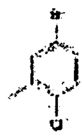 | 1119 | 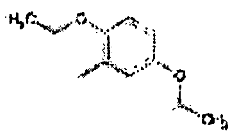 |
| 1112 | 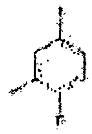 | 1120 | 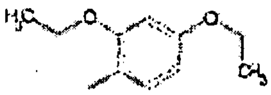 |
| 1113 | 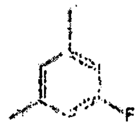 | 1121 | 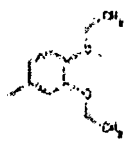 |
| 1114 | 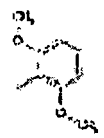 | 1122 | 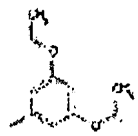 |
| 1115 | 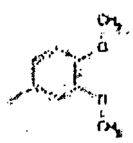 | 1123 | 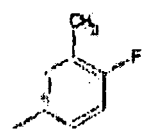 |

(continued)

| Compound No. | R | Compound No. | R |
|---|---|---|---|
| 1116 | 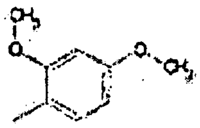 | 1124 | 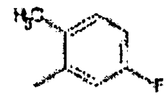 |
| 1117 | 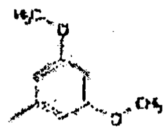 | 1125 | 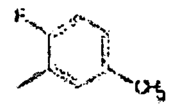 |
| 1118 | 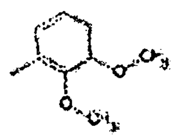 | 1126 | 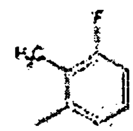 |
| 1127 | 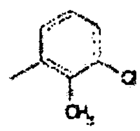 | 1135 | 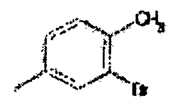 |
| 1128 | 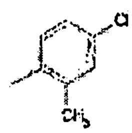 | 1136 | 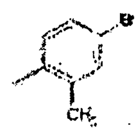 |
| 1129 | 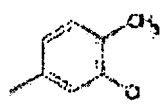 | 1137 | 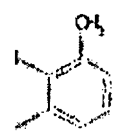 |
| 1130 | 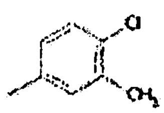 | 1138 | 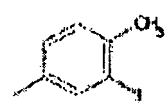 |
| 1131 | 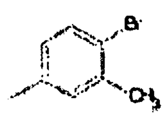 | 1139 | 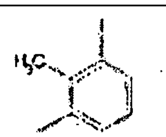 |
| 1132 | 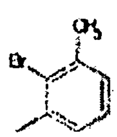 | 1140 | 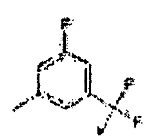 |
| 1133 | 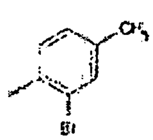 | 1141 | 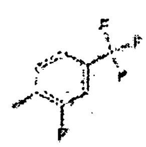 |

(continued)

| Compound No. | R | Compound No. | R |
|---|---|---|---|
| 1134 | 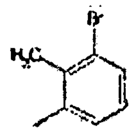 | 1142 | 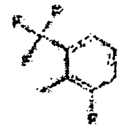 |
| 1143 | 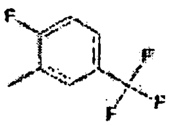 | 1151 | 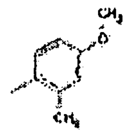 |
| 1144 | 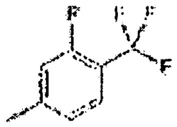 | 1152 | 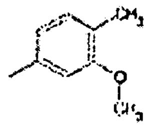 |
| 1145 | 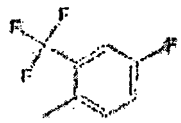 | 1153 | 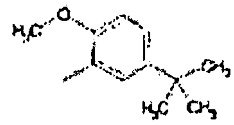 |
| 1146 | 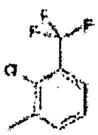 | 1154 | 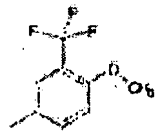 |
| 1147 | 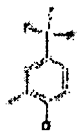 | 1155 | 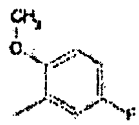 |
| 1148 | 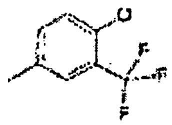 | 1156 | 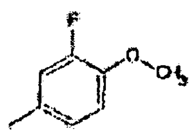 |
| 1149 | 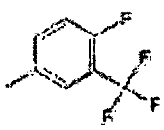 | 1157 | 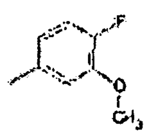 |
| 1150 | 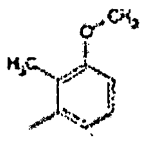 | 1158 | 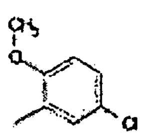 |
| 1150 | 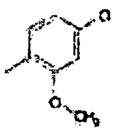 | 1167 | 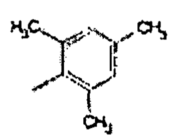 |

(continued)

| Compound No. | R | Compound No. | R |
|---|---|---|---|
| 1160 | 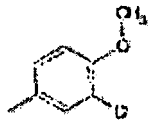 | 1168 | 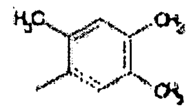 |
| 1101 | 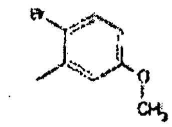 | 1169 | 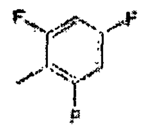 |
| 1162 | 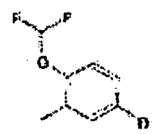 | 1170 | 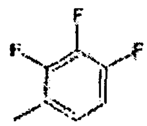 |
| 1163 | 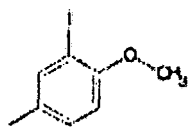 | 1171 | 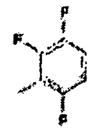 |
| 1164 | 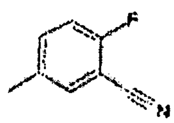 | 1172 | 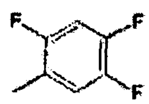 |
| 1165 | 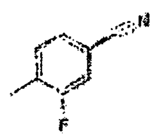 | 1173 | 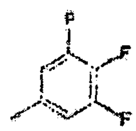 |
| 1166 | 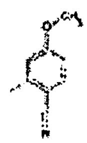 | 1174 | 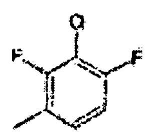 |
| 1175 | 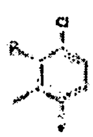 | 1183 | 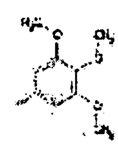 |
| 1176 | 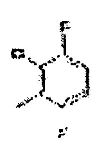 | 1184 | 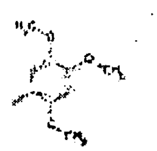 |
| 1177 | 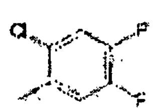 | 1185 | 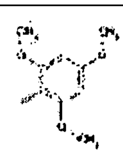 |

(continued)

| Compound No. | R | Compound No. | R |
|---|---|---|---|
| 1178 | 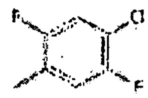 | 1186 | 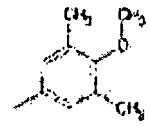 |
| 1179 | 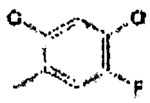 | 1187 | 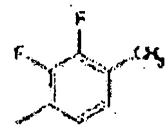 |
| 1180 | 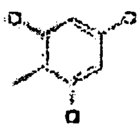 | 1188 | 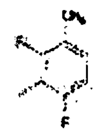 |
| 1181 | 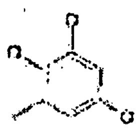 | 1189 | 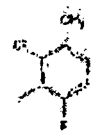 |
| 1182 | 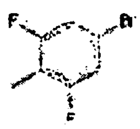 | 1190 | 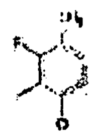 |
| 1191 | 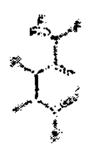 | 1199 | 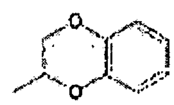 |
| 1192 | 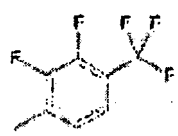 | 1200 | 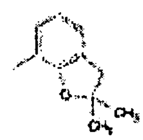 |
| 1193 | 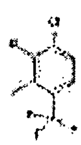 | 1201 | 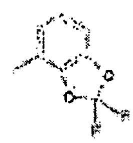 |
| 1194 | 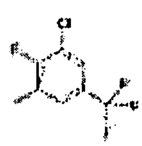 | 1202 | 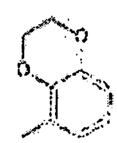 |
| 1195 | 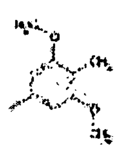 | 1203 | 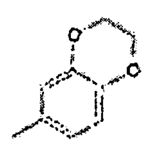 |

(continued)

| Compound No. | R | Compound No. | R |
|---|---|---|---|
| 1196 | 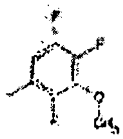 | 1204 | 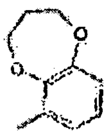 |
| 1197 | 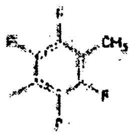 | 1205 | 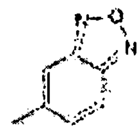 |
| 1108 | 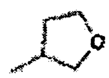 | 1206 | 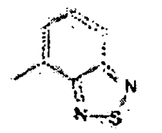 |
| 1207 | 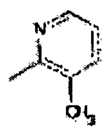 | 1215 | 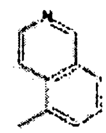 |
| 1208 | 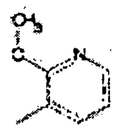 | 1216 | 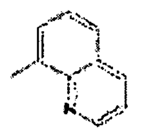 |
| 1209 | 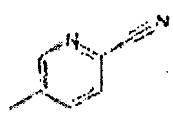 | 1217 | 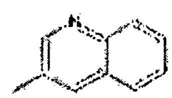 |
| 1210 | 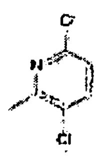 | 1218 | 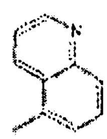 |
| 1211 | 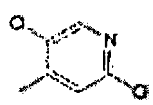 | 1219 | 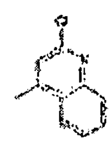 |
| 1212 | 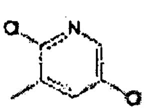 | 1220 | 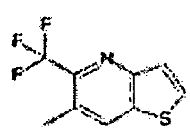 |
| 1213 | 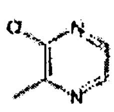 | 1221 | 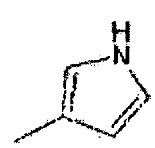 |

(continued)

| Compound No. | R | Compound No. | R |
|---|---|---|---|
| 1214 | 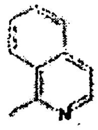 | 1222 | 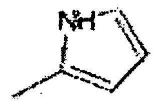 |
| 1223 | 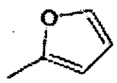 | 1231 | 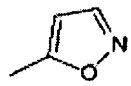 |
| 1224 | 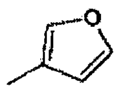 | 1232 | 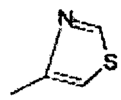 |
| 1225 | 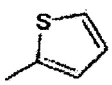 | 1233 | 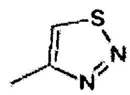 |
| 1226 | 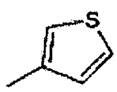 | 1234 | 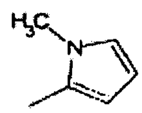 |
| 1227 | 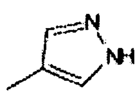 | 1235 | 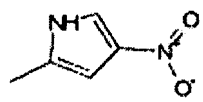 |
| 1228 | 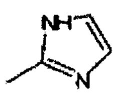 | 1236 | 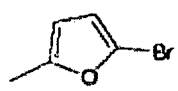 |
| 1229 | 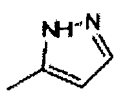 | 1237 | 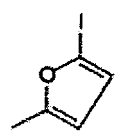 |
| 1230 | 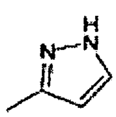 | 1238 | 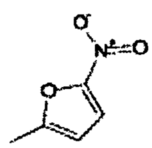 |
| 1239 | 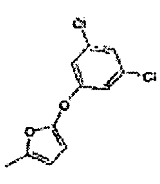 | 1247 | 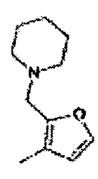 |
| 1240 | 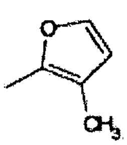 | 1248 | 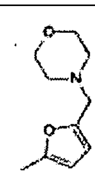 |

(continued)

| Compound No. | R | Compound No. | R |
|---|---|---|---|
| 1241 | 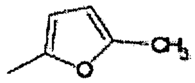 | 1249 | 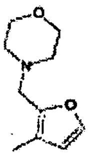 |
| 1242 | 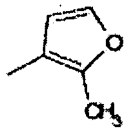 | 1250 | 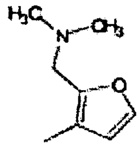 |
| 1243 | 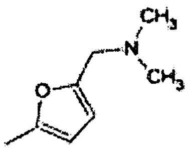 | 1251 | 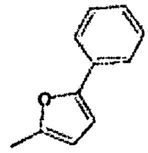 |
| 1244 | 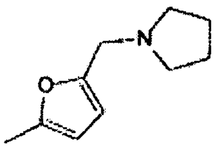 | 1252 | 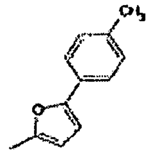 |
| 1245 | 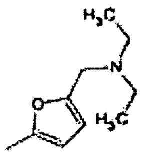 | 1253 | 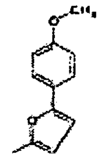 |
| 1246 | 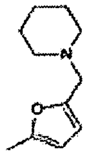 | 1254 | 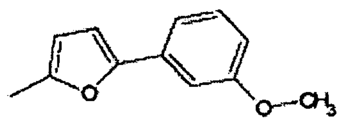 |
| 1255 | 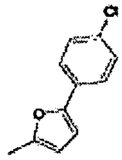 | 1263 | 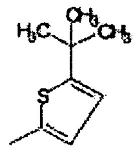 |
| 1256 | 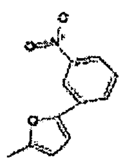 | 1264 | 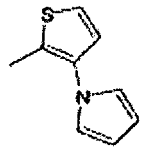 |
| 1257 | 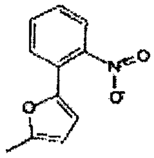 | 1265 | 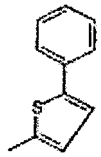 |

(continued)

| Compound No. | R | Compound No. | R |
|---|---|---|---|
| 1259 | 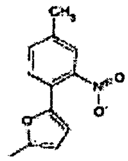 | 1266 | 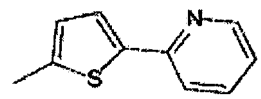 |
| 1259 | 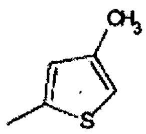 | 1267 | 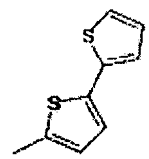 |
| 1260 | 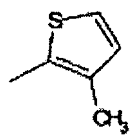 | 1268 | 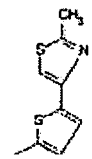 |
| 1261 | 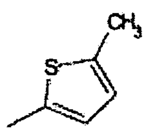 | 1269 | 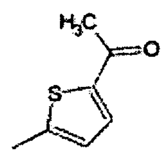 |
| 1262 | 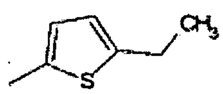 | 1270 | 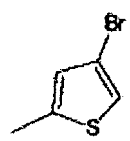 |
| 1271 | 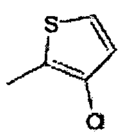 | 1279 | 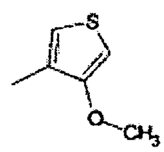 |
| 1272 | 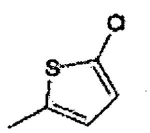 | 1280 | 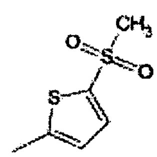 |
| 1273 | 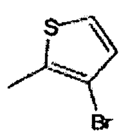 | 1281 | 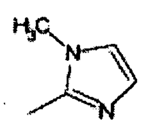 |
| 1274 | 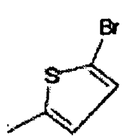 | 1282 | 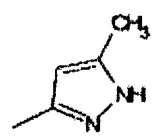 |

(continued)

| Compound No. | R | Compound No. | R |
|---|---|---|---|
| 1275 | 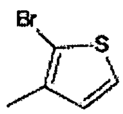 | 1283 | 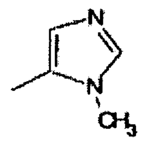 |
| 1276 | 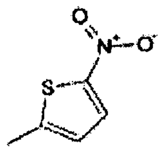 | 1284 | 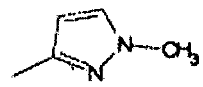 |
| 1277 | 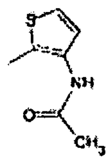 | 1285 | 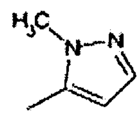 |
| 1278 | 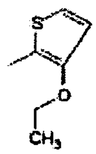 | 1286 | 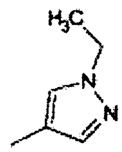 |
| 1287 | 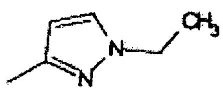 | 1295 | 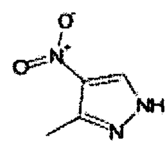 |
| 1288 | 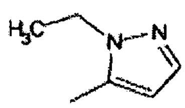 | 1296 | 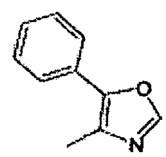 |
| 1289 | 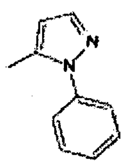 | 1207 | 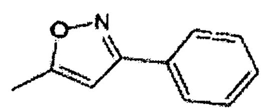 |
| 1290 | 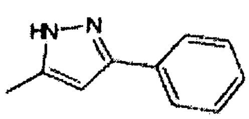 | 1298 | 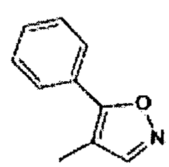 |
| 1291 | 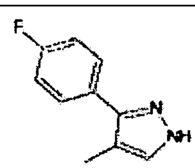 | 1299 | 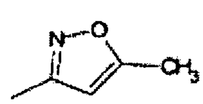 |

(continued)

| Compound No. | R | Compound No. | R |
|---|---|---|---|
| 1292 | 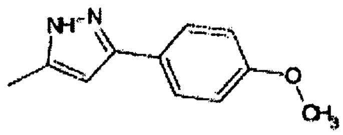 | 1300 | 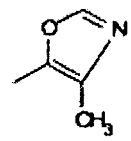 |
| 1293 | 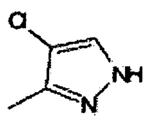 | 1301 | 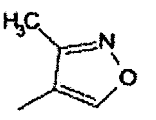 |
| 1294 | 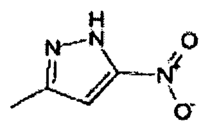 | 1302 | 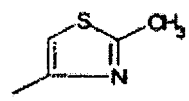 |
| 1303 | 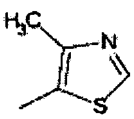 | 1311 | 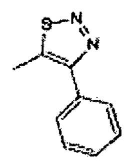 |
| 1304 | 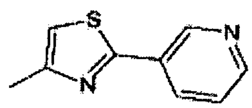 | 1312 | 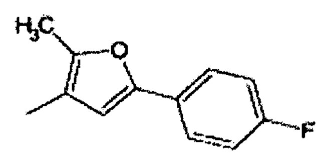 |
| 1305 | 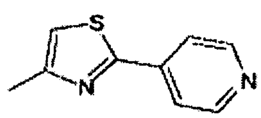 | 1313 | 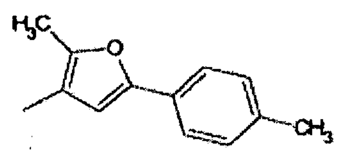 |
| 1308 | 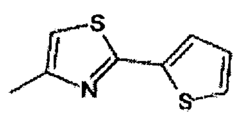 | 1314 | 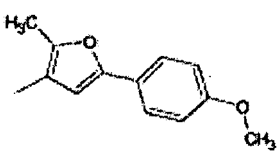 |
| 1307 | 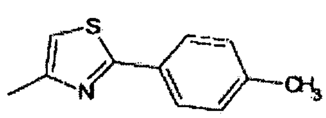 | 1315 | 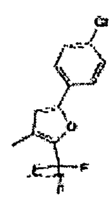 |
| 1308 | 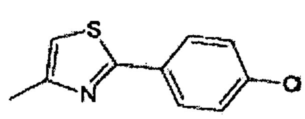 | 1316 | 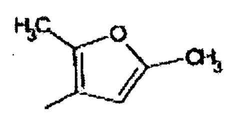 |
| 1369 | 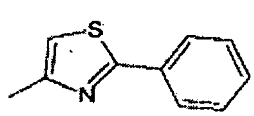 | 1317 | 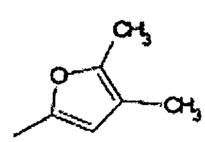 |

(continued)

| Compound No. | R | Compound No. | R |
|---|---|---|---|
| 1310 | 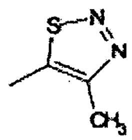 | 1318 | 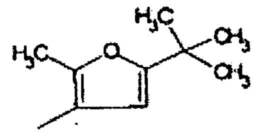 |
| 1319 | 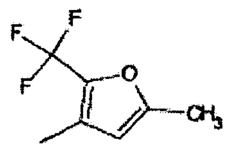 | 1327 | 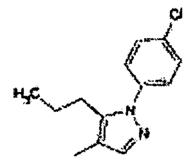 |
| 1320 | 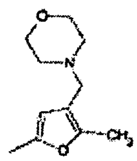 | 1328 | 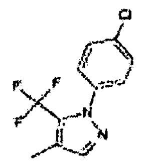 |
| 1321 | 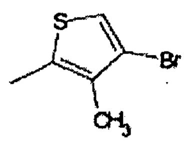 | 1329 | 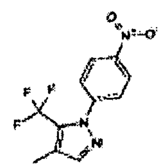 |
| 1322 | 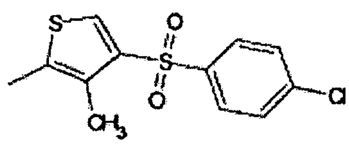 | 1310 | 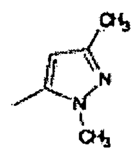 |
| 1323 | 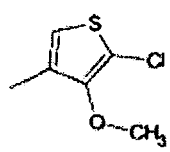 | 1311 | 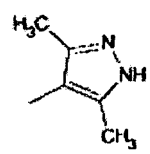 |
| 1324 | 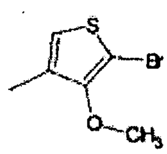 | 1332 | 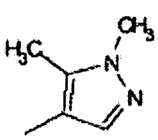 |
| 1325 | 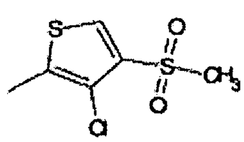 | 1333 | 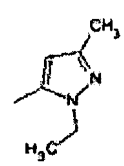 |
| 1326 | 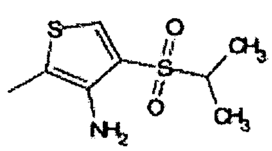 | 1334 | 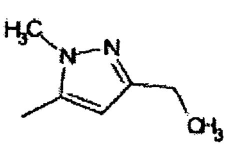 |

(continued)

| Compound No. | R | Compound No. | R |
|---|---|---|---|
| 1335 | 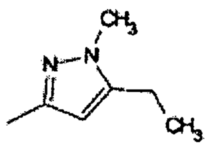 | 1343 | 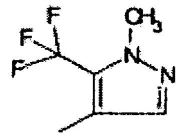 |
| 1336 | 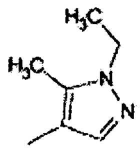 | 1344 | 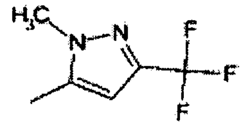 |
| 1337 | 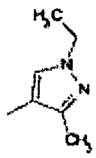 | 1345 | 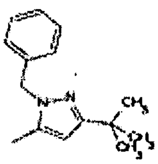 |
| 1338 | 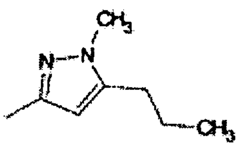 | 1346 | 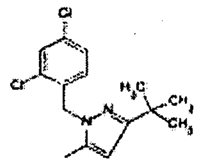 |
| 1339 | 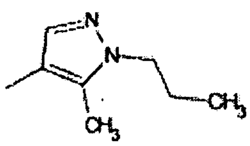 | 1347 | 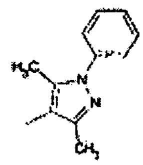 |
| 1340 | 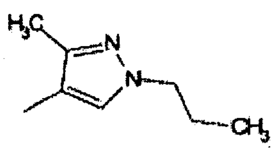 | 1348 | 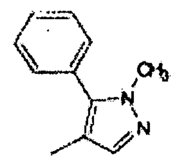 |
| 1341 | 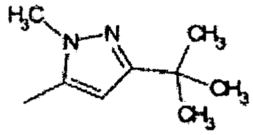 | 1349 | 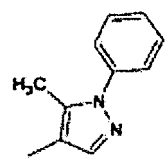 |
| 1342 | 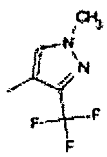 | 1350 | 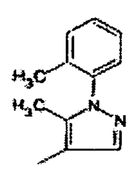 |
| 1351 | 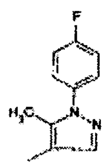 | 1359 | 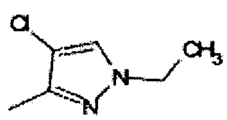 |

(continued)

| Compound No. | R | Compound No. | R |
|---|---|---|---|
| 1352 | 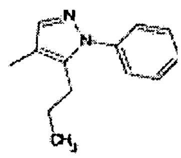 | 1360 | 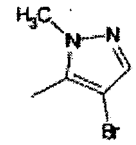 |
| **1353** | 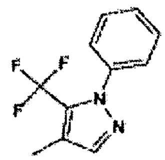 | 1361 | 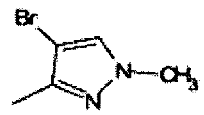 |
| 1354 | 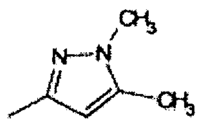 | 1362 | 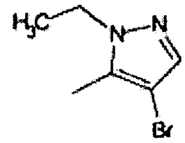 |
| 1355 | 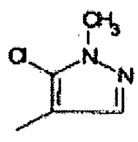 | 1363 | 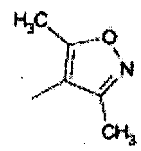 |
| 1356 | 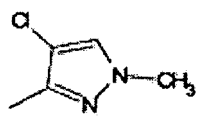 | 1364 | 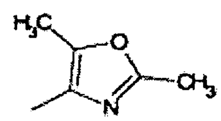 |
| 1357 | 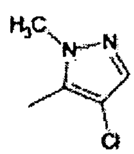 | 1365 | 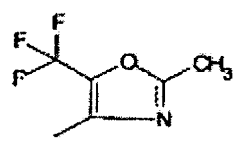 |
| 1358 | 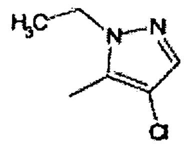 | 1366 | 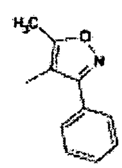 |
| 1387 | 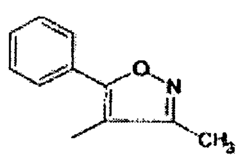 | 1375 | 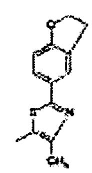 |
| 1368 | 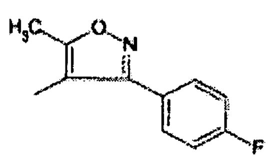 | 1376 | 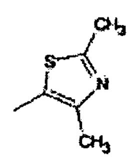 |

(continued)

| Compound No. | R | Compound No. | R |
|---|---|---|---|
| 1369 | 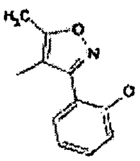 | 1377 | 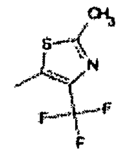 |
| 1370 | 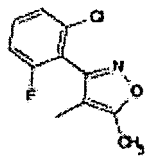 | 1378 | 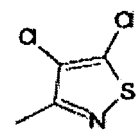 |
| 1371 | 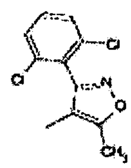 | 1378 | 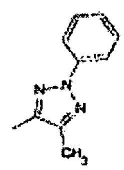 |
| 1372 | 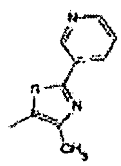 | 1380 | 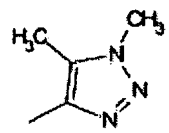 |
| 1373 | 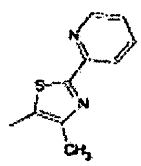 | 1381 | 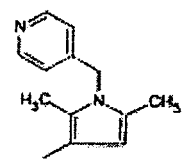 |
| 1374 | 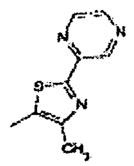 | 1382 | 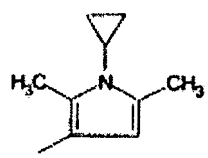 |
| 1383 | 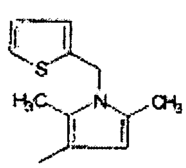 | 1391 | 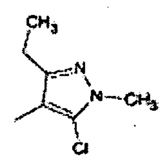 |
| 1384 | 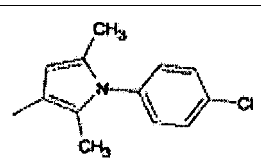 | 1392 | 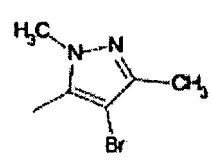 |
| 1385 | 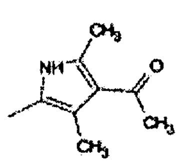 | 1393 | 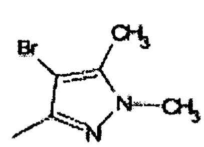 |

(continued)

| Compound No. | R | Compound No. | R |
|---|---|---|---|
| 1388 | 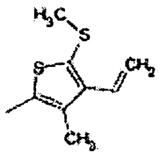 | 1394 | 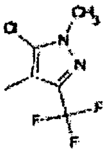 |
| 1387 | 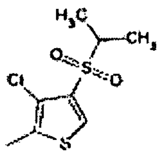 | 1395 | 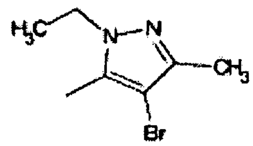 |
| 1388 | 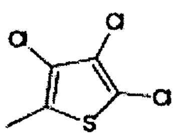 | 1396 | 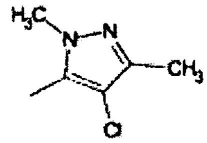 |
| 1389 | 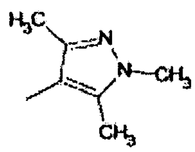 | 1397 | 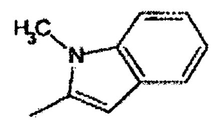 |
| 1390 | 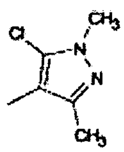 | 1398 | 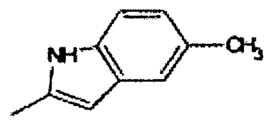 |
| 1399 | 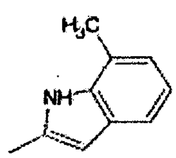 | 1407 | 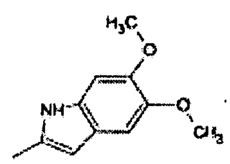 |
| 1400 | 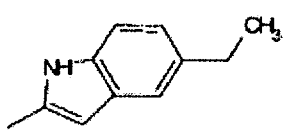 | 1408 | 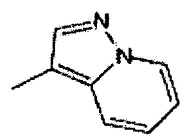 |
| 1401 | 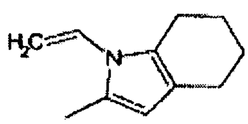 | 1409 | 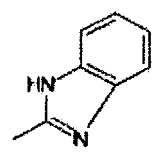 |
| 1402 | 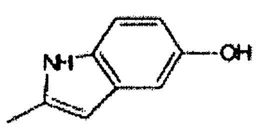 | 1410 | 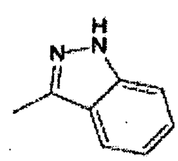 |

(continued)

| Compound No. | R | Compound No. | R |
|---|---|---|---|
| 1403 | 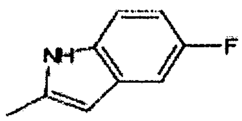 | 1411 | 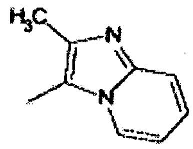 |
| 1404 | 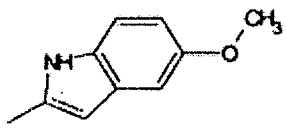 | 1412 | 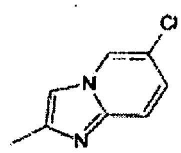 |
| 1405 | 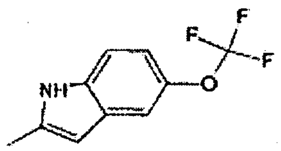 | 1413 | 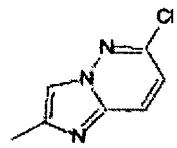 |
| 1406 | 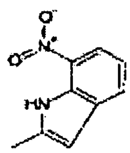 | 1414 | 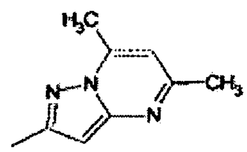 |
| 1415 | 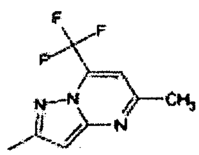 | 1424 | 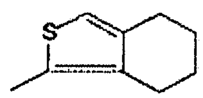 |
| 1416 | 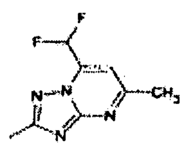 | 1425 | 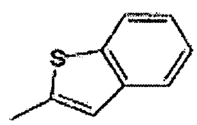 |
| 1417 | 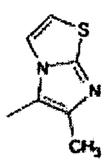 | 1426 | 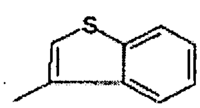 |
| 1418 | 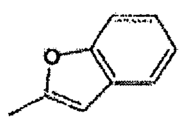 | 1427 | 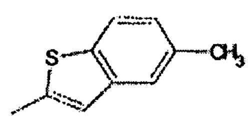 |
| 1419 | 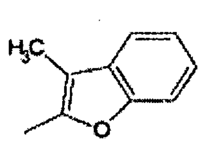 | 1428 | 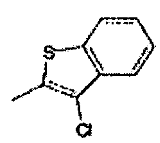 |

(continued)

| Compound No. | R | Compound No. | R |
|---|---|---|---|
| 1420 | 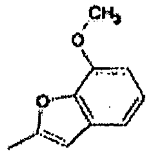 | 1429 | 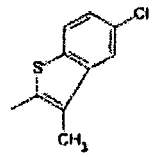 |
| 1421 | 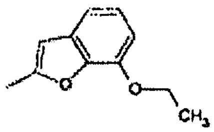 | 1430 | 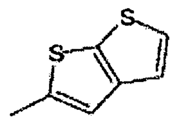 |
| 1422 | 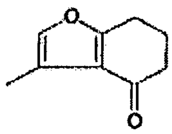 | 1431 | 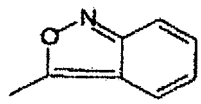 |
| 1423 | 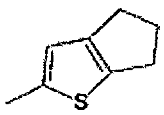 | | |

Table 5-1)

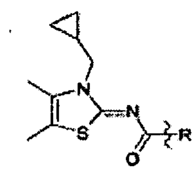

| Compound No. | R | Compound No. | R | Compound No. | R |
|---|---|---|---|---|---|
| 2001 | 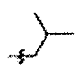 | 2010 | 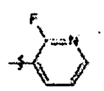 | 2019 | 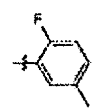 |
| 2002 | 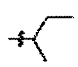 | 2011 | 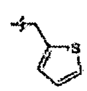 | 2020 | 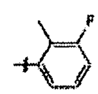 |
| 2003 | 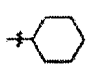 | 2012 | 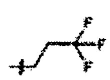 | 2021 | 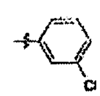 |
| 2004 | 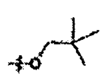 | 2013 | 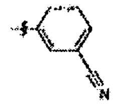 | 2022 | 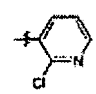 |

(continued)

| Compound No. | R | Compound No. | R | Compound No. | R |
|---|---|---|---|---|---|
| 2005 | 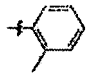 | 2014 | 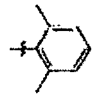 | 2023 | 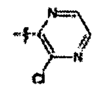 |
| 2006 | 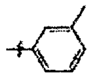 | 2015 | 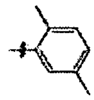 | 2024 | 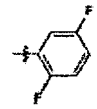 |
| 2007 | 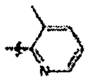 | 2016 | 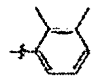 | 2025 | 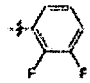 |
| 2008 | 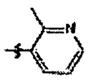 | 2017 | 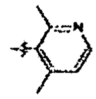 | 2026 | 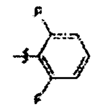 |
| 2009 | 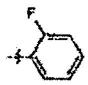 | 2018 | 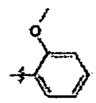 | 2027 | 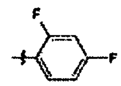 |
| 2028 | 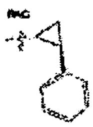 | 2038 | 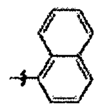 | 2048 | 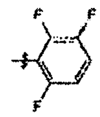 |
| 2029 | 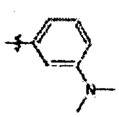 | 2039 | 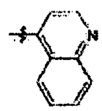 | 2049 | 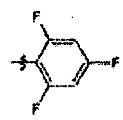 |
| 2030 | 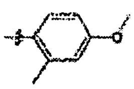 | 2040 | 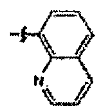 | 2050 | 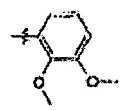 |
| 2031 | 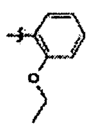 | 2041 | 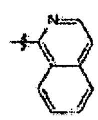 | 2051 | 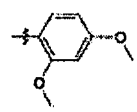 |
| 2032 | 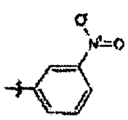 | 2042 | 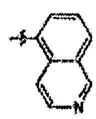 | 2052 | 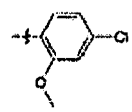 |
| 2033 | 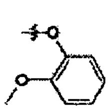 | 2043 | 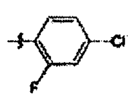 | 2053 | 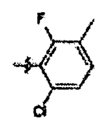 |
| 2034 | 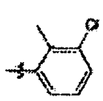 | 2044 | 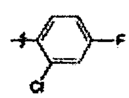 | 2054 | 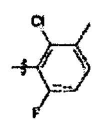 |

(continued)

| Compound No. | R | Compound No. | R | Compound No. | R |
|---|---|---|---|---|---|
| 2035 | 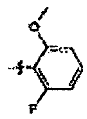 | 2045 | 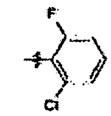 | 2055 | 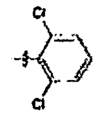 |
| 2036 | 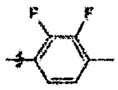 | 2048 | 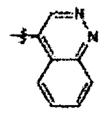 | 2056 | 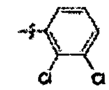 |
| 2037 | 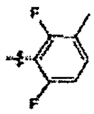 | 2047 | 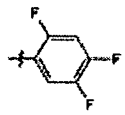 | 2057 | 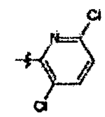 |
| 2058 | 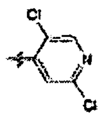 | 2068 | 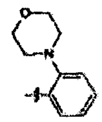 | 2078 | 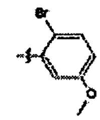 |
| 2059 | 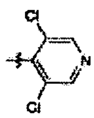 | 2069 | 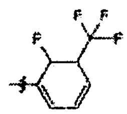 | 2079 | 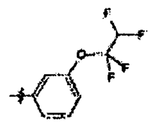 |
| 2060 | 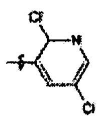 | 2070 | 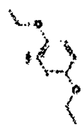 | 2080 | 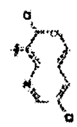 |
| 2061 | 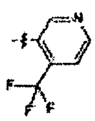 | 2071 | 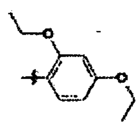 | 2081 | 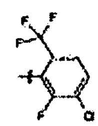 |
| 2062 | 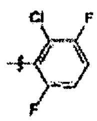 | 2072 | 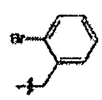 | 2082 | 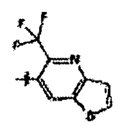 |
| 2063 | 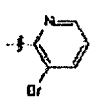 | 2073 | 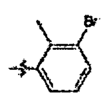 | 2083 | 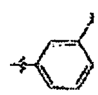 |
| 2064 | 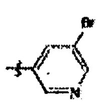 | 2074 | 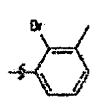 | 2084 | 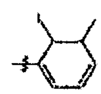 |
| 2065 | 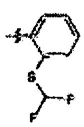 | 2075 | 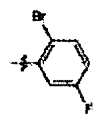 | 2085 | 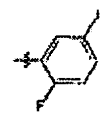 |

(continued)

| Compound No. | R | Compound No. | R | Compound No. | R |
|---|---|---|---|---|---|
| 2066 | 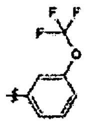 | 2076 | 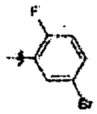 | | |
| 2067 | 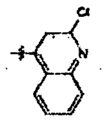 | 2077 | 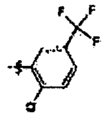 | | |

Table 5-2)

| Compound No. | R | compound No. | R | Compound No. | R |
|---|---|---|---|---|---|
| 2088 | 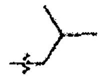 | 2094 | 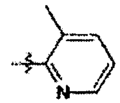 | 2102 | 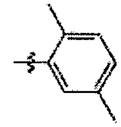 |
| 2087 | 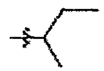 | 2095 | 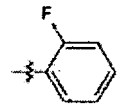 | 2103 | 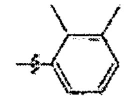 |
| 2088 | 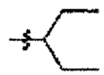 | 2096 | 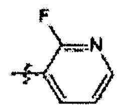 | 2104 | 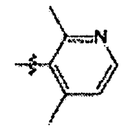 |
| 2089 | 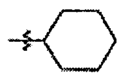 | 2097 | 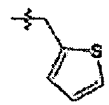 | 2105 | 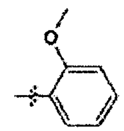 |
| 2090 | 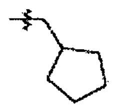 | 2098 | 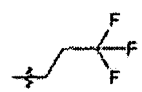 | 2106 | 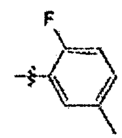 |
| 2091 | 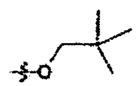 | 2099 | 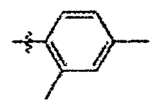 | 2107 | 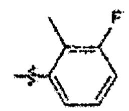 |
| 2092 | 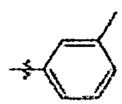 | 2100 | 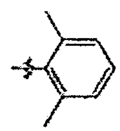 | 2108 | 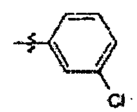 |

(continued)

| Compound No. | R | compound No. | R | Compound No. | R |
|---|---|---|---|---|---|
| 2093 | 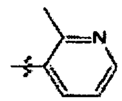 | 2101 | 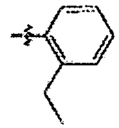 | 2109 | 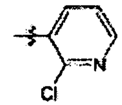 |
| 2110 | 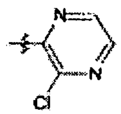 | 2118 | 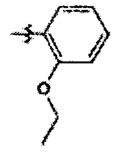 | 2126 | 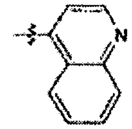 |
| 2111 | 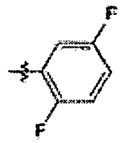 | 2119 | 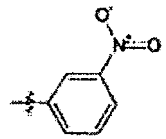 | 2127 | 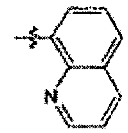 |
| 2112 | 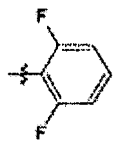 | 2120 | 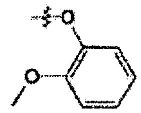 | 2128 | 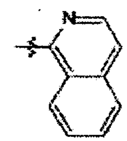 |
| 2113 | 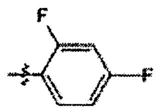 | 2121 | 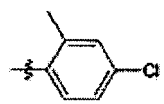 | 2129 | 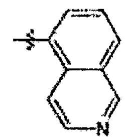 |
| 2114 | 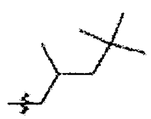 | 2122 | 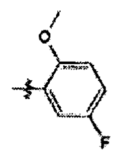 | 2130 | 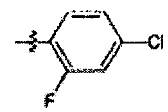 |

(continued)

| Compound No. | R | compound No. | R | Compound No. | R |
|---|---|---|---|---|---|
| 2115 | 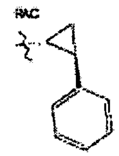 | 2123 | 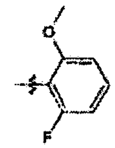 | 2131 | 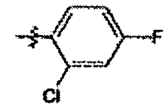 |
| 2116 | 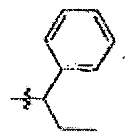 | 2124 | 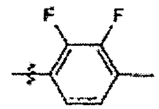 | 2132 | 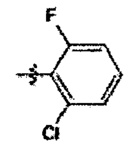 |
| 2117 | 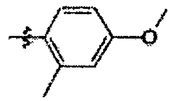 | 2125 | 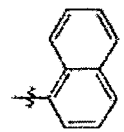 | 2133 | 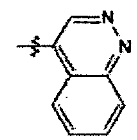 |
| 2134 | 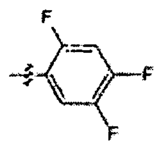 | 2142 | 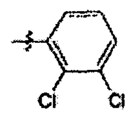 | 2150 | 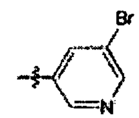 |
| 2135 | 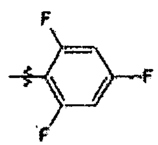 | 2143 | 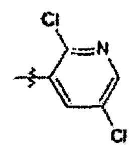 | 2151 | 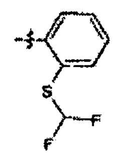 |
| 2138 | 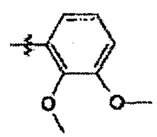 | 2144 | 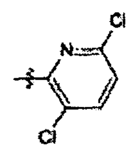 | 2152 | 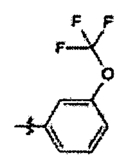 |

(continued)

| Compound No. | R | compound No. | R | Compound No. | R |
|---|---|---|---|---|---|
| 2137 | 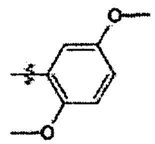 | 2145 | 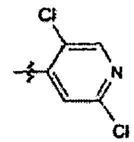 | 2153 | 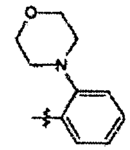 |
| 2138 | 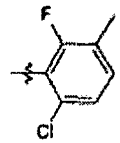 | 2146 | 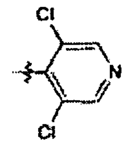 | 2154 | 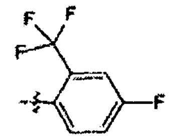 |
| 2139 | 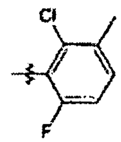 | 2147 | 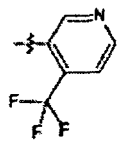 | 2155 | 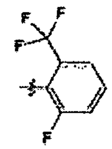 |
| 2140 | 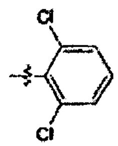 | 2148 | 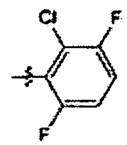 | 2156 | 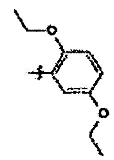 |
| 2141 | 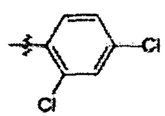 | 2149 | 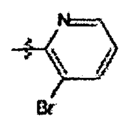 | 2157 | 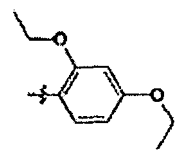 |
| 2158 | 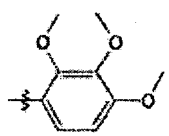 | 2163 | 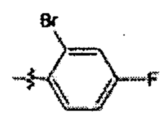 | 2168 | 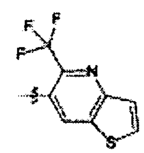 |

(continued)

| Compound No. | R | compound No. | R | Compound No. | R |
|---|---|---|---|---|---|
| 2159 | 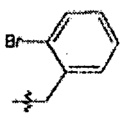 | 2164 | 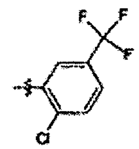 | 2169 | 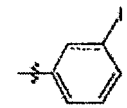 |
| 2160 | 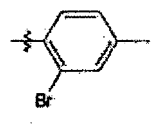 | 2165 | 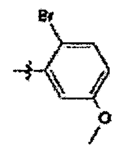 | 2170 | 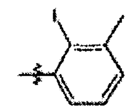 |
| 2161 | 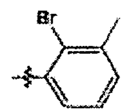 | 2166 | 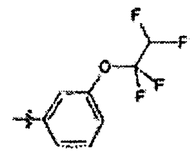 | 2171 | 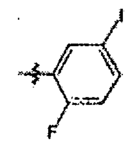 |
| 2162 | 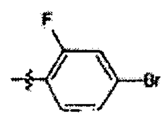 | 2167 | 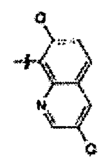 | | |

Table 5-3)

| 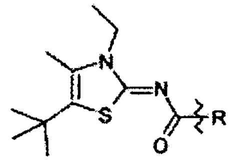 | | | | | |
|---|---|---|---|---|---|
| Compound No. | R | Compound No. | R | Compound No. | R |
| 2172 | 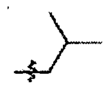 | 2180 | 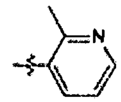 | 2188 | 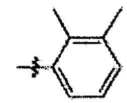 |
| 2173 | 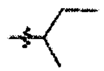 | 2181 | 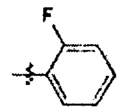 | 2189 | 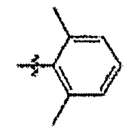 |
| 2174 | 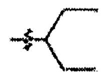 | 2182 | 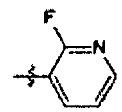 | 2190 | 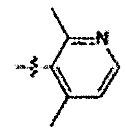 |
| 2175 | 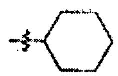 | 2183 | 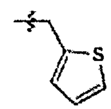 | 2191 | 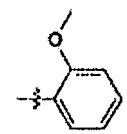 |
| 2176 | 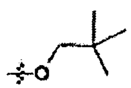 | 2184 | 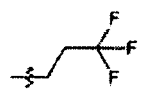 | 2192 | 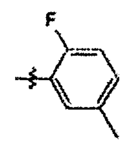 |

(continued)

| Compound No. | R | Compound No. | R | Compound No. | R |
|---|---|---|---|---|---|
| 2177 | 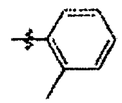 | 2185 | 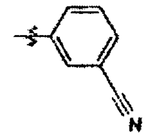 | 2193 | 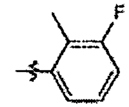 |
| 2178 | 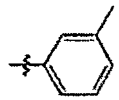 | 2186 | 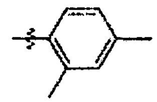 | 2194 | 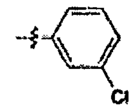 |
| 2179 | 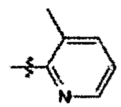 | 2187 | 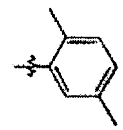 | 2195 | 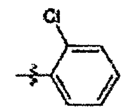 |
| 2196 | 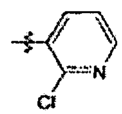 | 2204 | 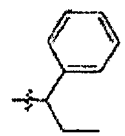 | 2212 | 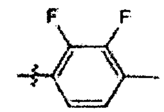 |
| 2197 | 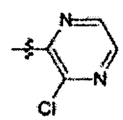 | 2205 | 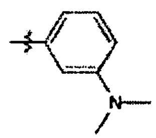 | 2213 | 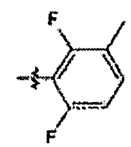 |
| 2198 | 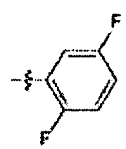 | 2206 | 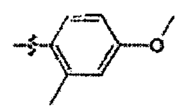 | 2214 | 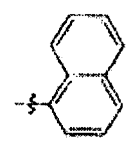 |

(continued)

| Compound No. | R | Compound No. | R | Compound No. | R |
|---|---|---|---|---|---|
| 2199 | 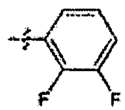 | 2207 | 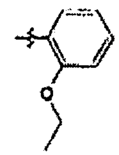 | 2215 | 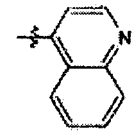 |
| 2200 | 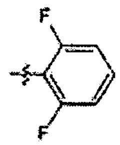 | 2208 | 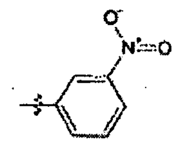 | 2216 | 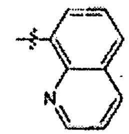 |
| 2201 | 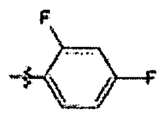 | 2209 | 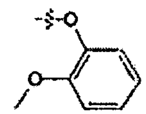 | 2217 | 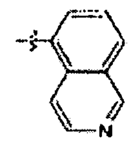 |
| 2202 | 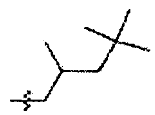 | 2210 | 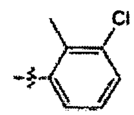 | 2218 | 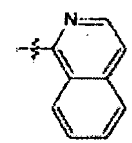 |
| 2203 | 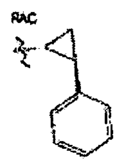 | 2211 | 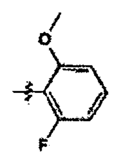 | 2219 | 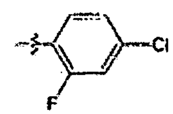 |
| 2220 | 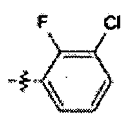 | 2228 | 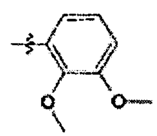 | 2236 | 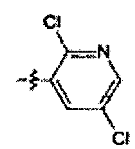 |

(continued)

| Compound No. | R | Compound No. | R | Compound No. | R |
|---|---|---|---|---|---|
| 2221 | 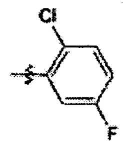 | 2229 | 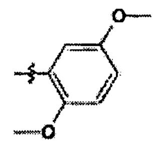 | 2237 | 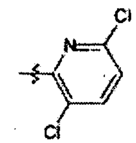 |
| 2222 | 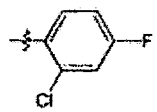 | 2230 | 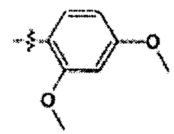 | 2238 | 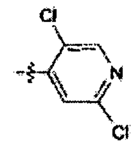 |
| 2223 | 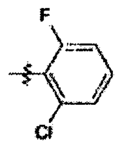 | 2231 | 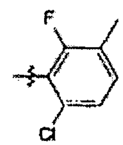 | 2239 | 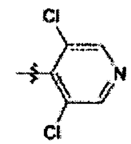 |
| 2224 | 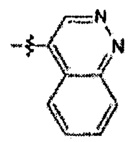 | 2232 | 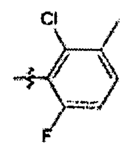 | 2240 | 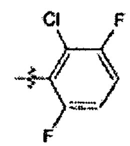 |
| 2225 | 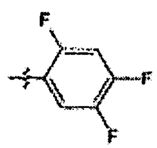 | 2233 | 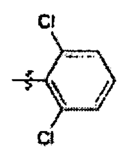 | 2241 | 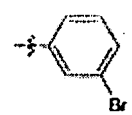 |
| 2226 | 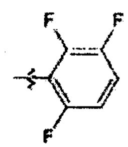 | 2234 | 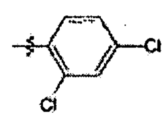 | 2242 | 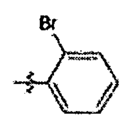 |

(continued)

| Compound No. | R | Compound No. | R | Compound No. | R |
|---|---|---|---|---|---|
| 2227 | 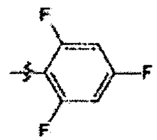 | 2235 | 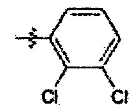 | 2243 | 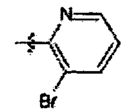 |
| 2244 | 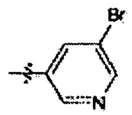 | 2252 | 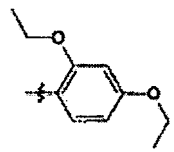 | 2260 | 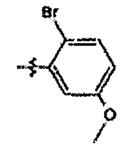 |
| 2245 | 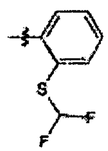 | 2253 | 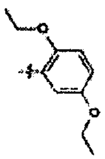 | 2261 | 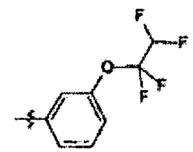 |
| 2246 | 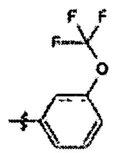 | 2254 | 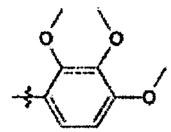 | 2262 | 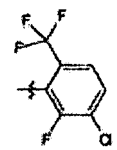 |
| 2247 | 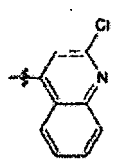 | 2255 | 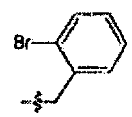 | 2263 | 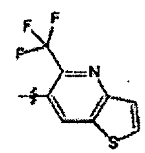 |
| 2248 | 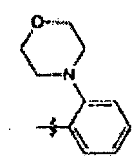 | 2256 | 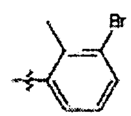 | 2264 | 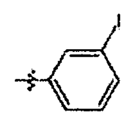 |

(continued)

| Compound No. | R | Compound No. | R | Compound No. | R |
|---|---|---|---|---|---|
| 2249 | 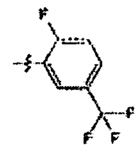 | 2257 | 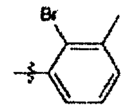 | 2265 | 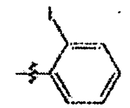 |
| 2250 | 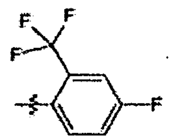 | 2258 | 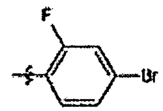 | 2266 | 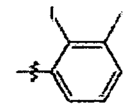 |
| 2251 | 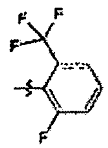 | 2259 | 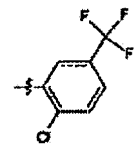 | | |

Table 5-4)

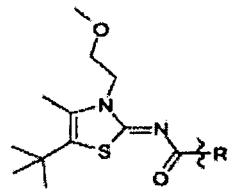

| Compound No. | R | Compound No. | R | Compound No. | R |
|---|---|---|---|---|---|
| 2267 | 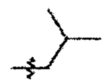 | 2275 | 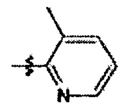 | 2283 | 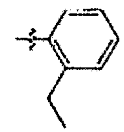 |
| 2268 | 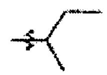 | 2276 | 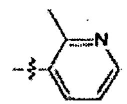 | 2284 | 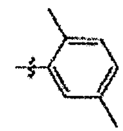 |
| 2269 | 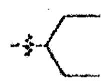 | 2277 | 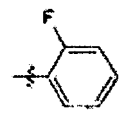 | 2285 | 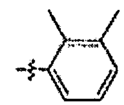 |
| 2270 | 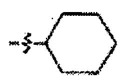 | 2278 | 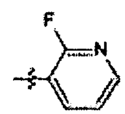 | 2286 | 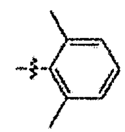 |
| 2271 | 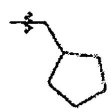 | 2279 | 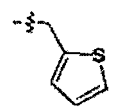 | 2287 | 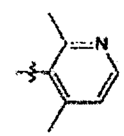 |

(continued)

| Compound No. | R | Compound No. | R | Compound No. | R |
|---|---|---|---|---|---|
| 2272 | 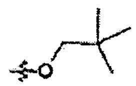 | 2280 | 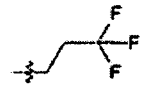 | 2288 | 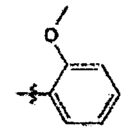 |
| 2273 | 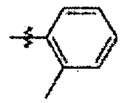 | 2281 | 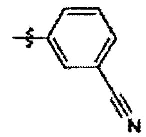 | 2289 | 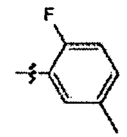 |
| 2274 | 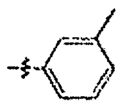 | 2282 | 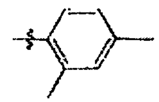 | 2290 | 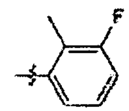 |
| 2291 | 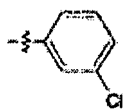 | 2299 | 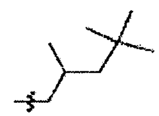 | 2307 | 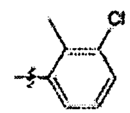 |
| 2292 | 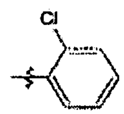 | 2300 | 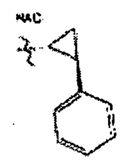 | 2308 | 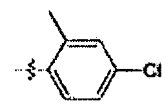 |
| 2293 | 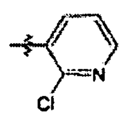 | 2301 | 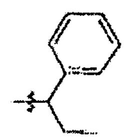 | 2309 | 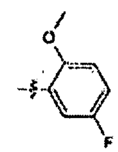 |

(continued)

| Compound No. | R | Compound No. | R | Compound No. | R |
|---|---|---|---|---|---|
| 2294 | 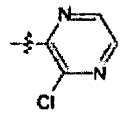 | 2302 | 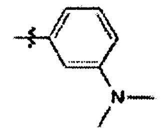 | 2310 | 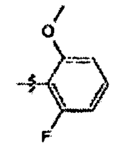 |
| 2295 | 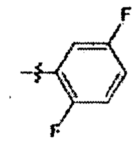 | 2303 | 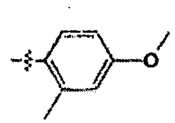 | 2311 | 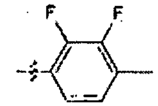 |
| 2296 | 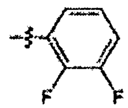 | 2304 | 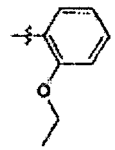 | 2312 | 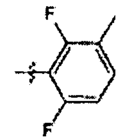 |
| 2297 | 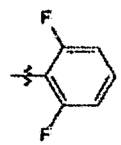 | 2305 | 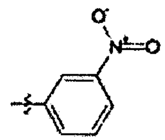 | 2313 | 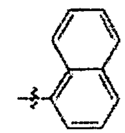 |
| 2298 | 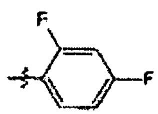 | 2306 | 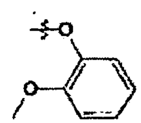 | 2314 | 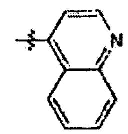 |
| 2315 | 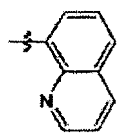 | 2323 | 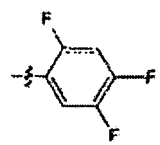 | 2331 | 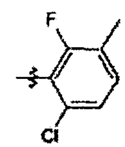 |

(continued)

| Compound No. | R | Compound No. | R | Compound No. | R |
|---|---|---|---|---|---|
| 2316 | 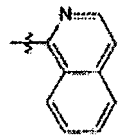 | 2324 | 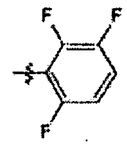 | 2332 | 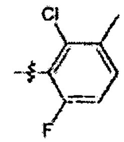 |
| 2317 | 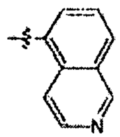 | 2325 | 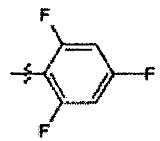 | 2333 | 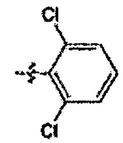 |
| 2318 | 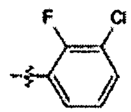 | 2326 | 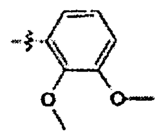 | 2334 | 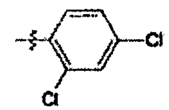 |
| 2319 | 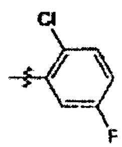 | 2327 | 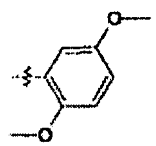 | 2335 | 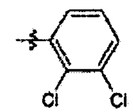 |
| 2320 | 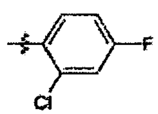 | 2328 | 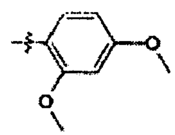 | 2336 | 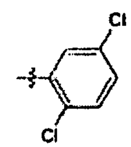 |
| 2321 | 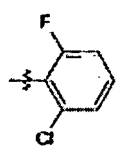 | 2329 | 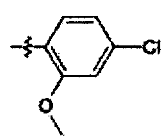 | 2337 | 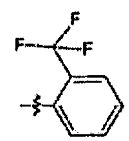 |

(continued)

| Compound No. | R | Compound No. | R | Compound No. | R |
|---|---|---|---|---|---|
| 2322 | 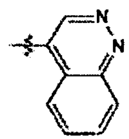 | 2330 | 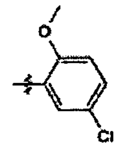 | 2338 | 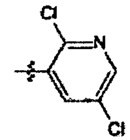 |
| 2339 | 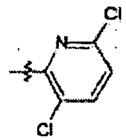 | 2347 | 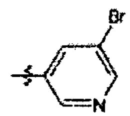 | 2355 | 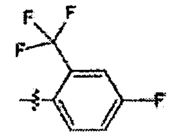 |
| 2340 | 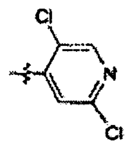 | 2348 | 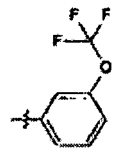 | 2356 | 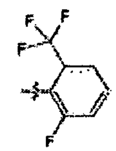 |
| 2341 | 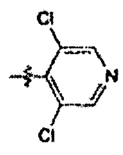 | 2349 | 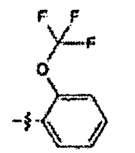 | 2357 | 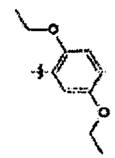 |
| 2342 | 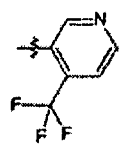 | 2350 | 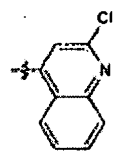 | 2358 | 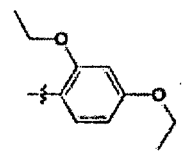 |
| 2343 | 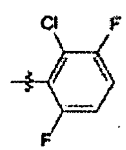 | 2351 | 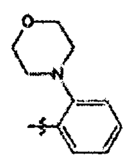 | 2359 | 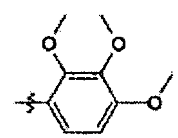 |

(continued)

| Compound No. | R | Compound No. | R | Compound No. | R |
|---|---|---|---|---|---|
| 2344 | 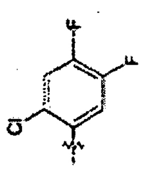 | 2352 | 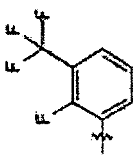 | 2360 | 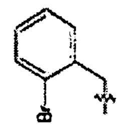 |
| 2345 | 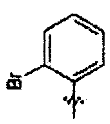 | 2353 | 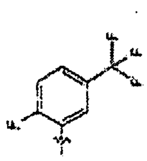 | 2361 | 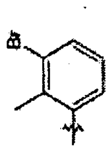 |
| 2346 | 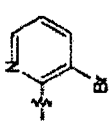 | 2354 | 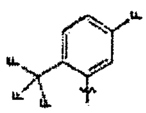 | 2382 | 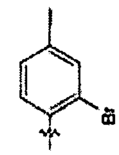 |
| 2363 | 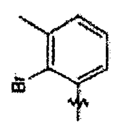 | 2367 | 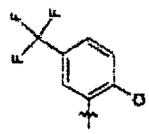 | 2371 | 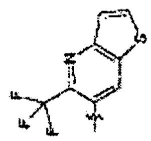 |
| 2364 | 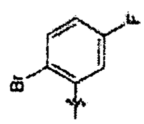 | 2368 | 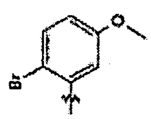 | 2372 | 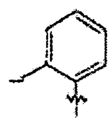 |
| 2365 | 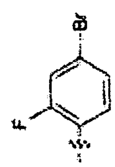 | 2369 | 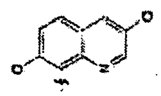 | 2373 | 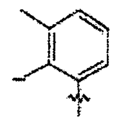 |

(continued)

| Compound No. | R | Compound No. | R | Compound No. | R |
|---|---|---|---|---|---|
| 2366 | 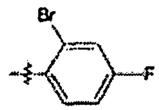 | 2370 | 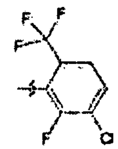 | 2374 | 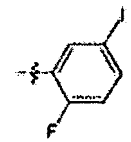 |

Table 5-5)

| 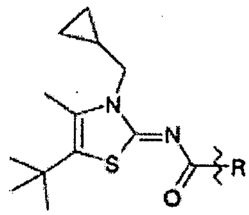 | | | |
|---|---|---|---|
| Compound No. | R | Compound No. | R |
| 2375 | 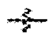 | 2383 | 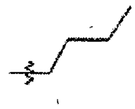 |
| 2376 | 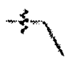 | 2384 | 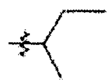 |
| 2377 | 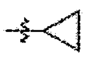 | 2385 | 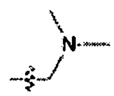 |
| 2378 | 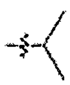 | 2386 | 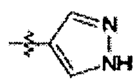 |
| 2379 | 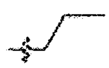 | 2387 | 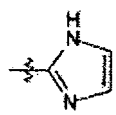 |
| 2380 | 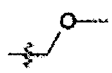 | 2388 | 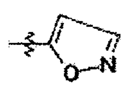 |
| 2381 | 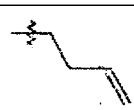 | 2389 | 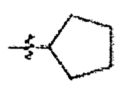 |
| 2382 | 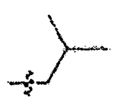 | 2390 | 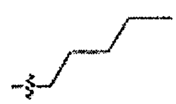 |
| 2391 | 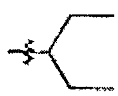 | 2399 | 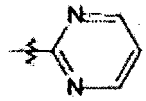 |
| 2392 | 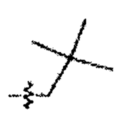 | 2400 | 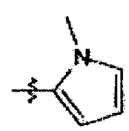 |

(continued)

| Compound No. | R | Compound No. | R |
|---|---|---|---|
| 2393 | 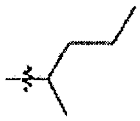 | 2401 | 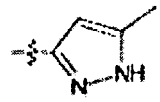 |
| 2394 | 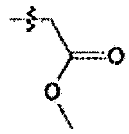 | 2402 | 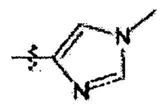 |
| 2395 | 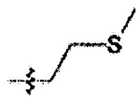 | 2403 | 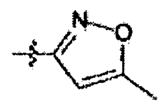 |
| 2396 | 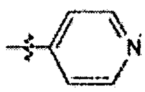 | 2404 | 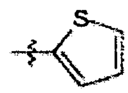 |
| 2397 | 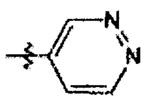 | 2405 | 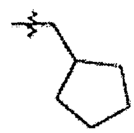 |
| 2398 | 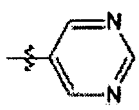 | 2406 | 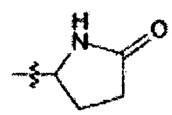 |
| 2407 | 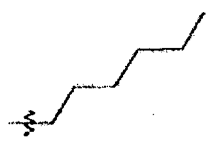 | 2415 | 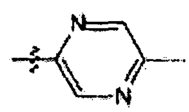 |
| 2408 | 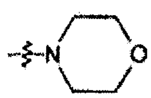 | 2416 | 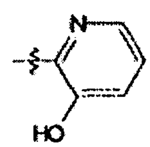 |
| 2409 | 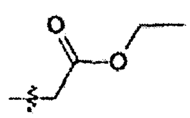 | 2417 | 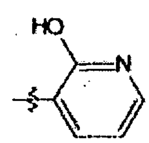 |
| 2410 | 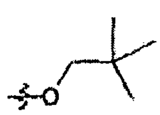 | 2418 | 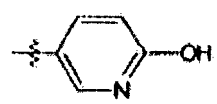 |
| 2411 | 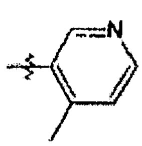 | 2419 | 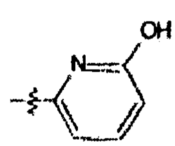 |

(continued)

| Compound No. | R | Compound No. | R |
|---|---|---|---|
| 2412 | 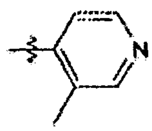 | 2420 | 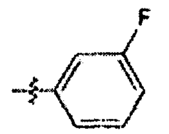 |
| 2413 | 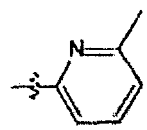 | 2421 | 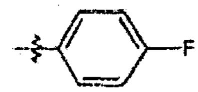 |
| 2414 | 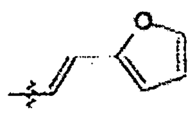 | 2422 | 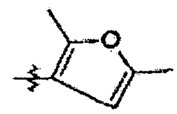 |
| 2423 | 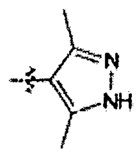 | 2431 | 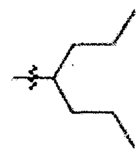 |
| 2424 | 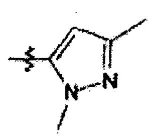 | 2432 | 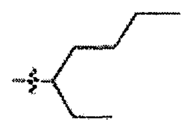 |
| 2425 | 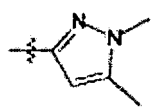 | 2433 | 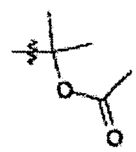 |
| 2426 | 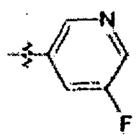 | 2434 | 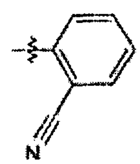 |
| 2427 | 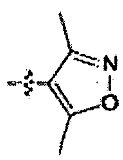 | 2435 | 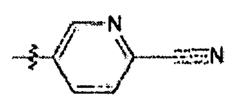 |
| 2428 | 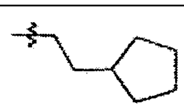 | 2436 | 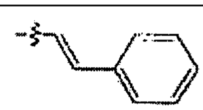 |
| 2429 | 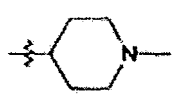 | 2437 | 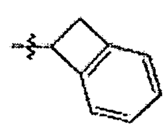 |

(continued)

| Compound No. | R | Compound No. | R |
|---|---|---|---|
| 2430 | 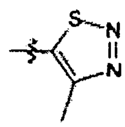 | 2438 | 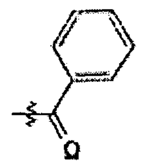 |
| 2439 | 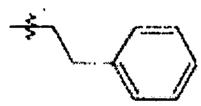 | 2447 | 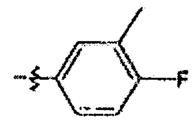 |
| 2440 | 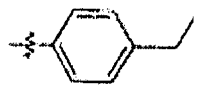 | 2448 | 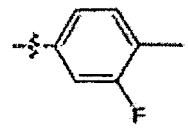 |
| 2441 | 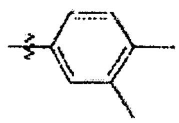 | 2449 | 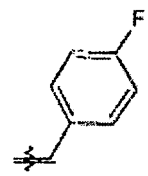 |
| 2442 | 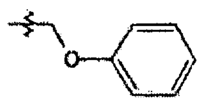 | 2450 | 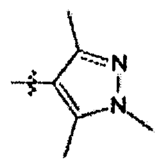 |
| 2443 | 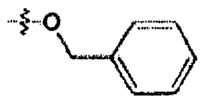 | 2451 | 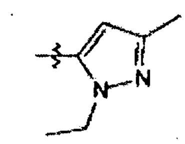 |
| 2444 | 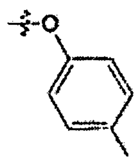 | 2452 | 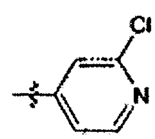 |
| 2445 | 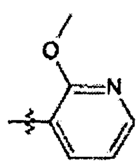 | 2453 | 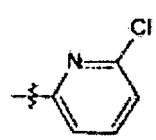 |
| 2446 | 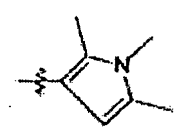 | 2454 | 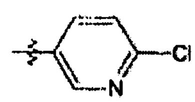 |

(continued)

| Compound No. | R | Compound No. | R |
|---|---|---|---|
| 2455 | 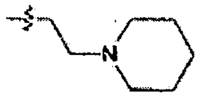 | 2463 | 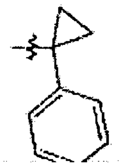 |
| 2456 | 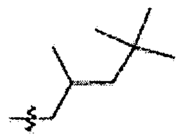 | 2464 | 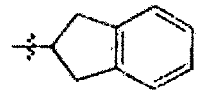 |
| 2457 | 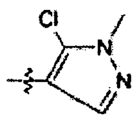 | 2465 | 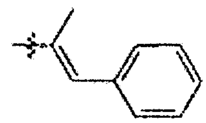 |
| 2458 | 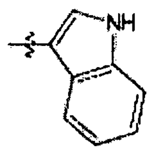 | 2466 | 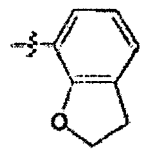 |
| 2459 | 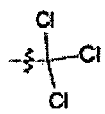 | 2467 | 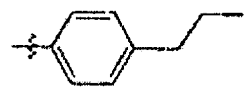 |
| 2460 | 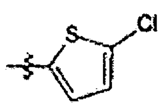 | 2468 | 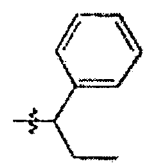 |
| 2461 | 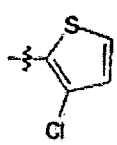 | 2469 | 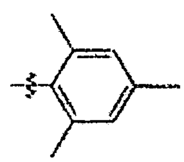 |
| 2462 | 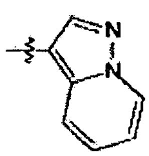 | 2470 | 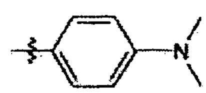 |
| 2471 | 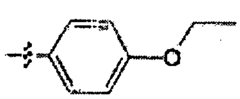 | 2479 | 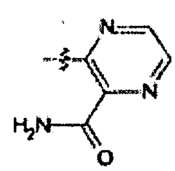 |

(continued)

| Compound No. | R | Compound No. | R |
|---|---|---|---|
| 2472 | 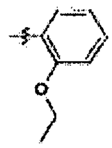 | 2480 | 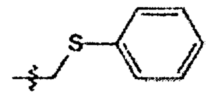 |
| 2473 | 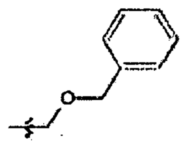 | 2481 | 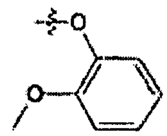 |
| 2474 | 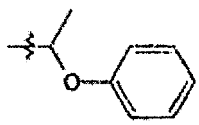 | 2482 | 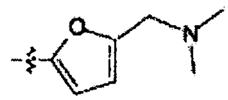 |
| 2475 | 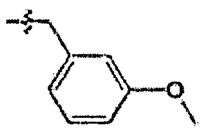 | 2483 | 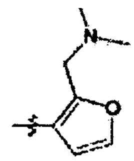 |
| 2476 | 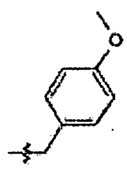 | 2484 | 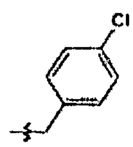 |
| 2477 | 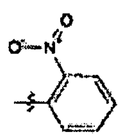 | 2485 | 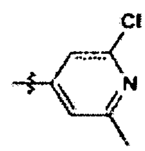 |
| 2478 | 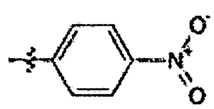 | 2486 | 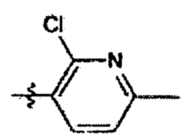 |
| 2487 | 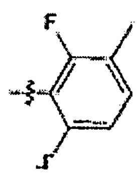 | 2495 | 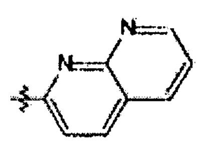 |
| 2488 | 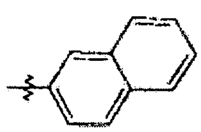 | 2496 | 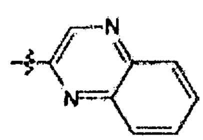 |

(continued)

| Compound No. | R | Compound No. | R |
|---|---|---|---|
| 2489 | 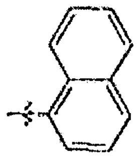 | 2497 | 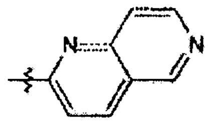 |
| 2490 | 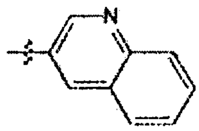 | 2498 | 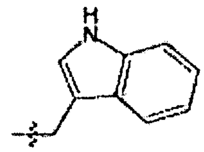 |
| 2491 | 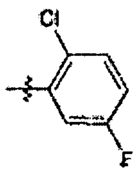 | 2499 | 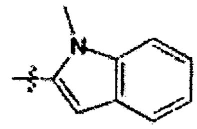 |
| 2492 | 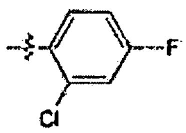 | 2500 | 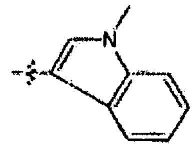 |
| 2493 | 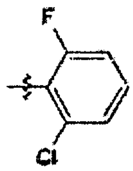 | 2501 | 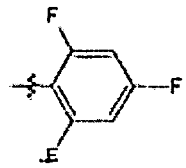 |
| 2494 | 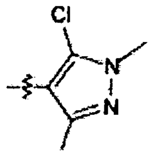 | 2502 | 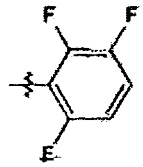 |
| 2503 | 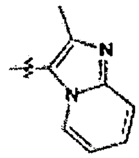 | 2511 | 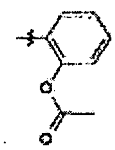 |
| 2504 | 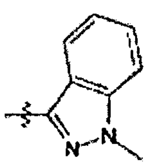 | 2512 | 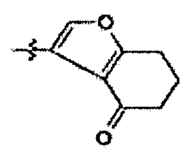 |

(continued)

| Compound No. | R | Compound No. | R |
|---|---|---|---|
| 2505 | 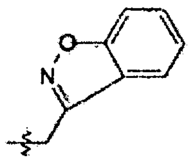 | 2513 | 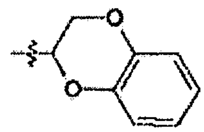 |
| 2506 | 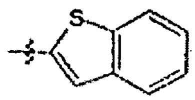 | 2514 | 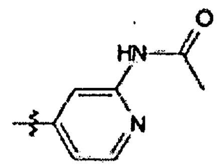 |
| 2507 | 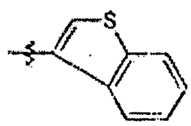 | 2515 | 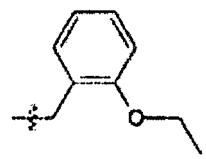 |
| 2508 | 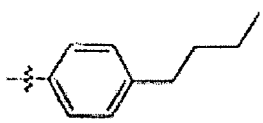 | 2516 | 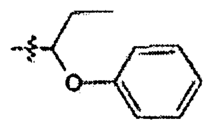 |
| 2509 | 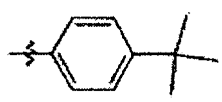 | 2517 | 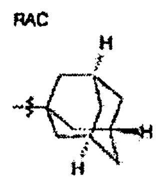 |
| 2510 | 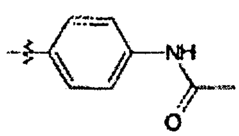 | 2518 | 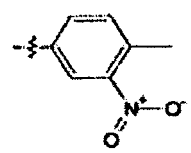 |
| 2519 | 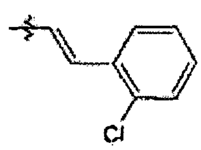 | 2527 | 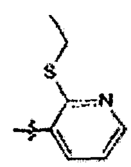 |
| 2520 | 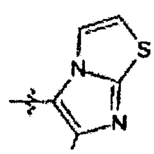 | 2528 | 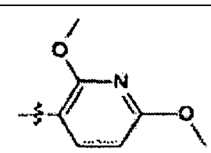 |
| 2521 | 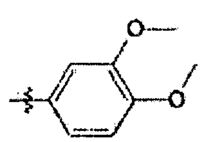 | 2529 | 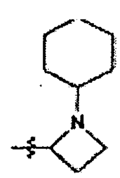 |

(continued)

| Compound No. | R | Compound No. | R |
|---|---|---|---|
| 2522 | 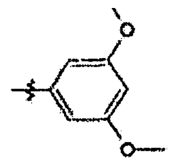 | 2530 | 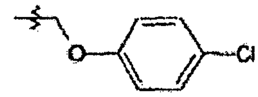 |
| 2523 | 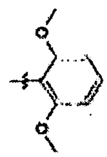 | 2531 | 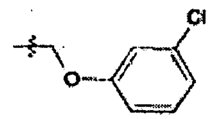 |
| 2524 | 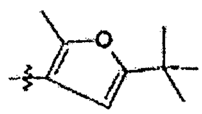 | 2532 | 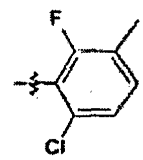 |
| 2525 | 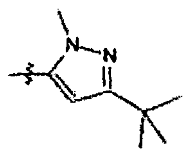 | 2533 | 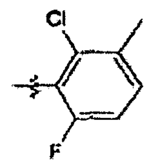 |
| 2526 | 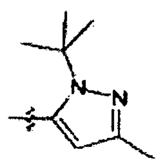 | 2534 | 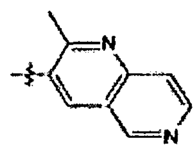 |
| 2535 | 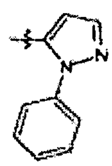 | 2543 | 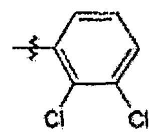 |
| 2536 | 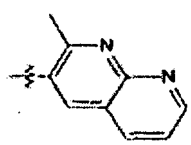 | 2544 | 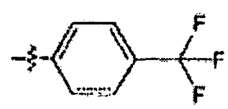 |
| 2537 | 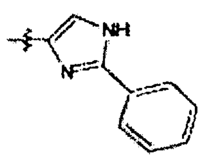 | 2545 | 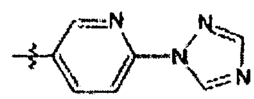 |
| 2538 | 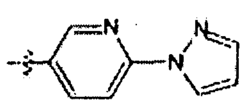 | 2546 | 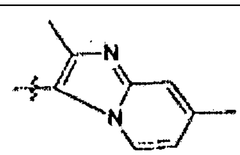 |

(continued)

| Compound No. | R | Compound No. | R |
|---|---|---|---|
| 2539 | 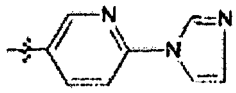 | 2547 | 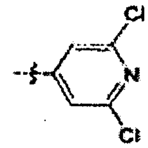 |
| 2540 | 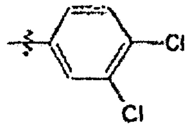 | 2548 | 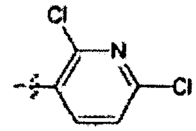 |
| 2541 | 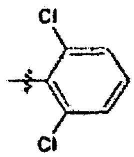 | 2549 | 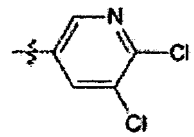 |
| 2542 | 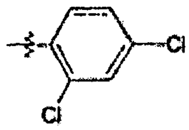 | 2550 | 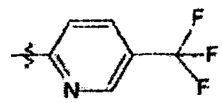 |
| 2551 | 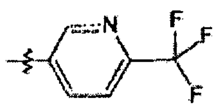 | 2559 | 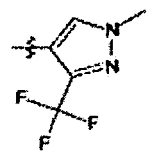 |
| 2552 | 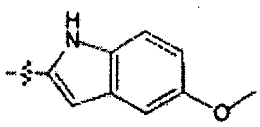 | 2560 | 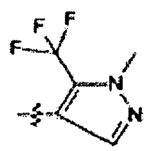 |
| 2553 | 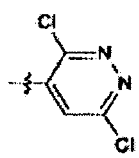 | 2561 | 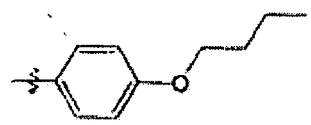 |
| 2554 | 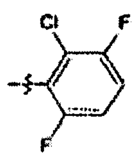 | 2562 | 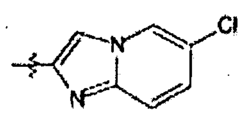 |
| 2555 | 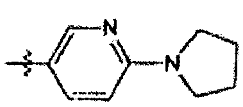 | 2563 | 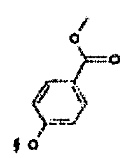 |

(continued)

| Compound No. | R | Compound No. | R |
|---|---|---|---|
| 2556 | 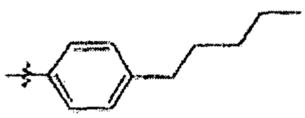 | 2564 | 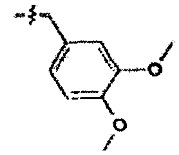 |
| 2557 | 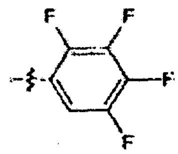 | 2565 | 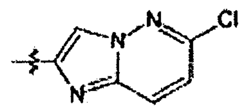 |
| 2558 | 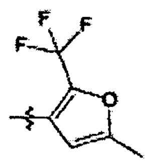 | 2566 | 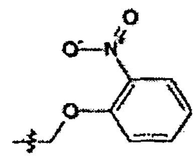 |
| 2567 | 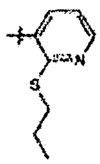 | 2575 | 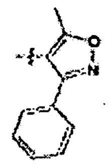 |
| 2568 | 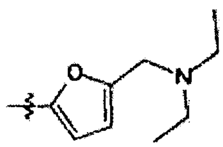 | 2576 | 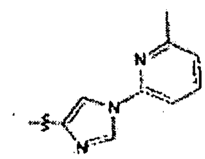 |
| 2569 | 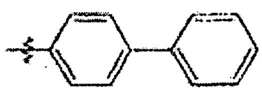 | 2577 | 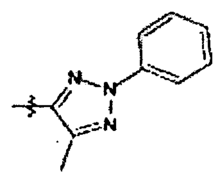 |
| 2570 | 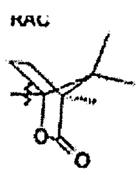 | 2578 | 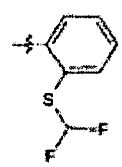 |
| 2571 | 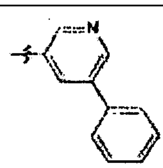 | 2579 | 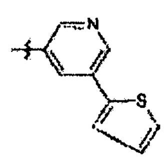 |
| 2572 | 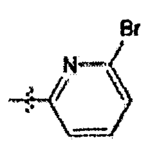 | 2580 | 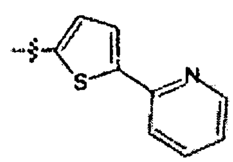 |

(continued)

| Compound No. | R | Compound No. | R |
|---|---|---|---|
| 2573 | 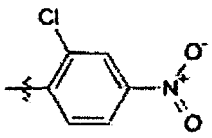 | 2581 | 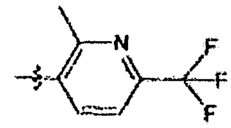 |
| 2574 | 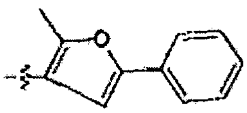 | 2582 | 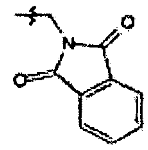 |
| 2583 | 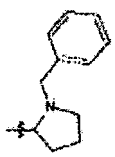 | 2591 | 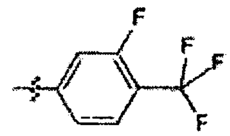 |
| 2584 | 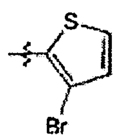 | 2592 | 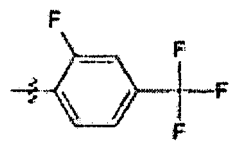 |
| 2585 | 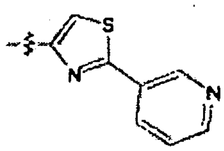 | 2593 | 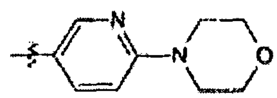 |
| 2586 | 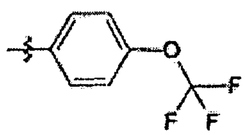 | 2594 | 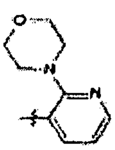 |
| 2587 | 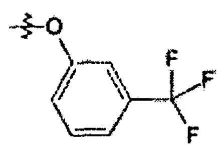 | 2595 | 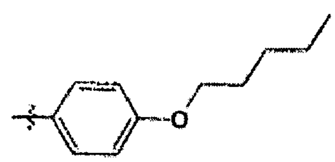 |
| 2588 | 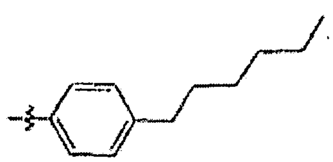 | 2596 | 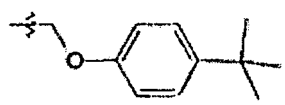 |
| 2589 | 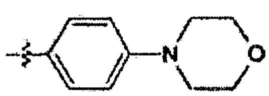 | 2597 | 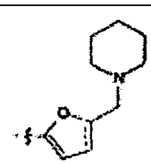 |

(continued)

| Compound No. | R | Compound No. | R |
|---|---|---|---|
| 2590 | 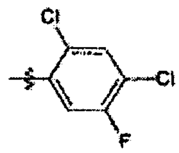 | 2598 | 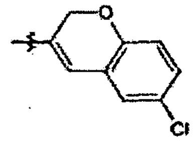 |
| 2599 | 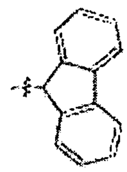 | 2607 | 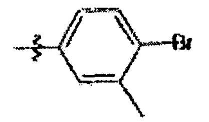 |
| 2600 | 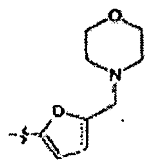 | 2608 | 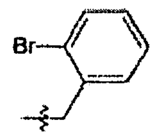 |
| 2601 | 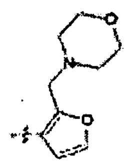 | 2609 | 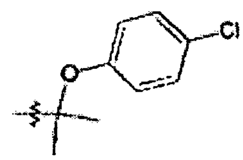 |
| 2602 | 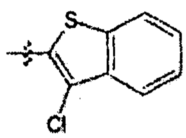 | 2610 | 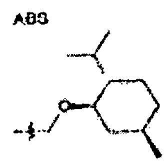 |
| 2603 | 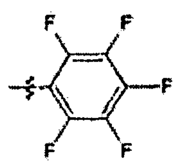 | 2611 | 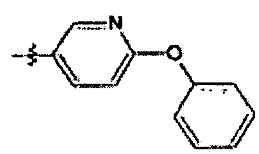 |
| 2604 | 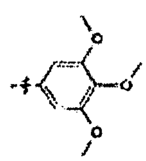 | 2612 | 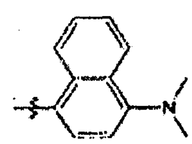 |
| 2605 | 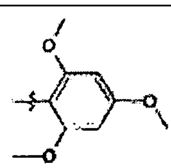 | 2613 | 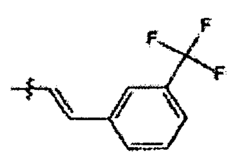 |
| 2606 | 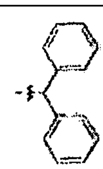 | 2614 | 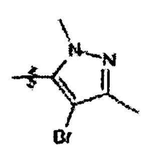 |

(continued)

| Compound No. | R | Compound No. | R |
|---|---|---|---|
| 2615 | 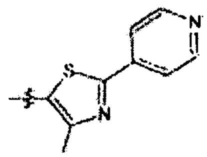 | 2623 | 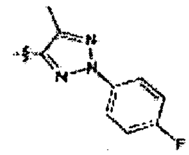 |
| 2616 | 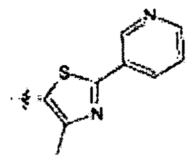 | 2624 | 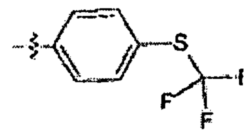 |
| 2617 | 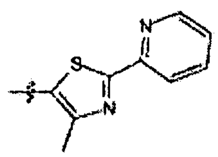 | 2625 | 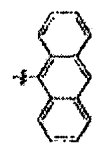 |
| 2618 | 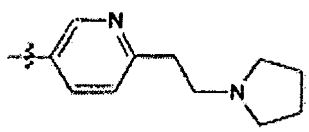 | 2626 | 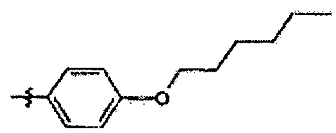 |
| 2619 | 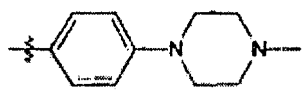 | 2627 | 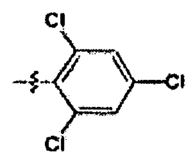 |
| 2620 | 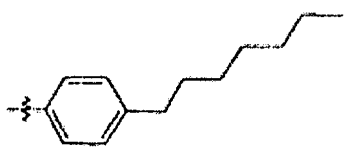 | 2628 | 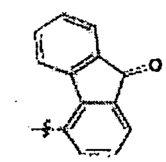 |
| 2621 | 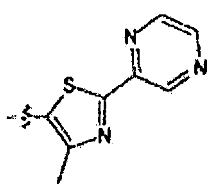 | 2629 | 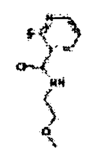 |
| 2622 | 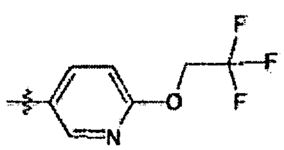 | 2630 | 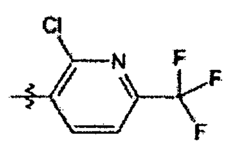 |
| 2631 | 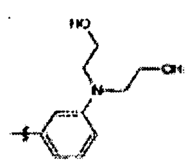 | 2639 | 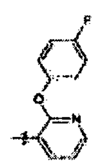 |

(continued)

| Compound No. | R | Compound No. | R |
|---|---|---|---|
| 2632 | 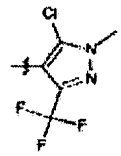 | 2640 | 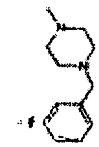 |
| 2633 | 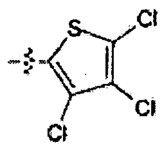 | 2641 | 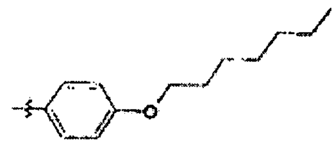 |
| 2634 | 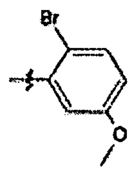 | 2642 | 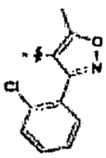 |
| 2635 | 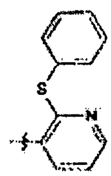 | 2643 | 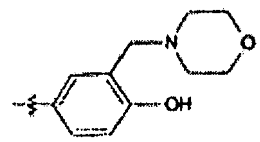 |
| 2636 | 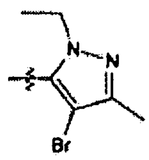 | 2644 | 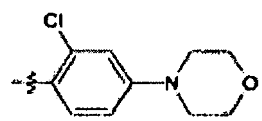 |
| 2637 | 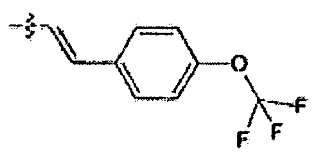 | 2645 | 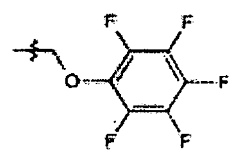 |
| 2638 | 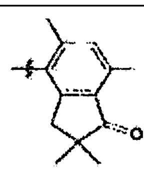 | 2646 | 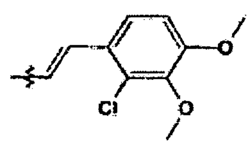 |
| 2647 | 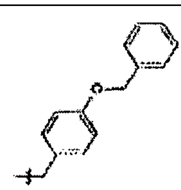 | 2655 | 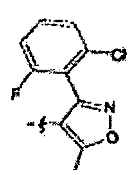 |
| 2648 | 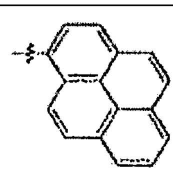 | 2656 | 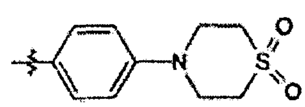 |

(continued)

| Compound No. | R | Compound No. | R |
|---|---|---|---|
| 2649 | 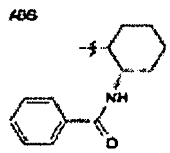 | 2657 | 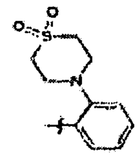 |
| 2650 | 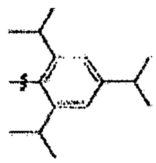 | 2658 | 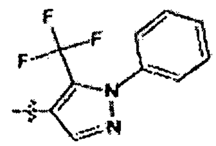 |
| 2651 | 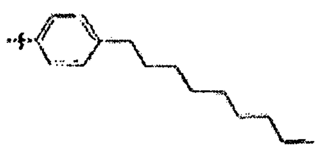 | 2659 | 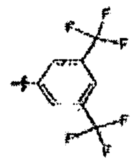 |
| 2652 | 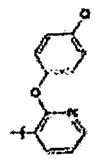 | 2660 | 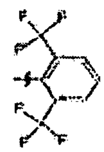 |
| 2653 | 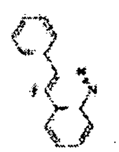 | 2661 | 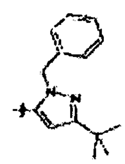 |
| 2654 | 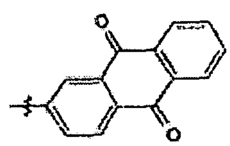 | 2662 | 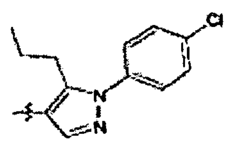 |
| 2663 | 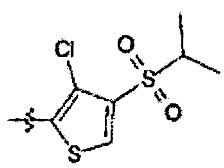 | 2671 | 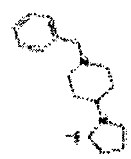 |
| 2664 | 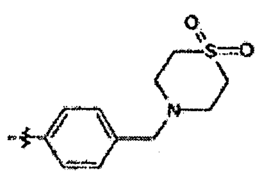 | 2672 | 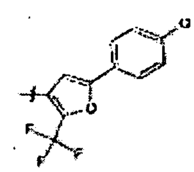 |
| 2665 | 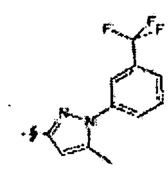 | 2673 | 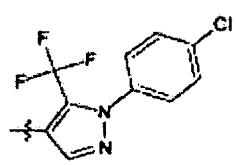 |

(continued)

| Compound No. | R | Compound No. | R |
|---|---|---|---|
| 2666 | 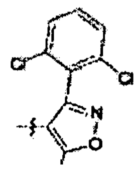 | 2674 | 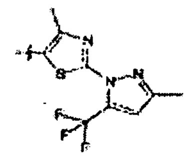 |
| 2667 | 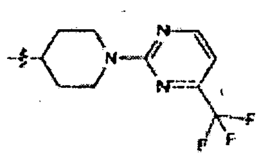 | 2675 | 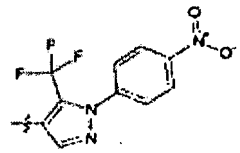 |
| 2668 | 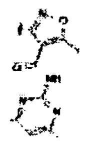 | 2676 | 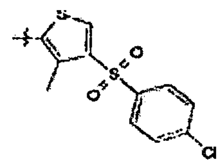 |
| 2669 | 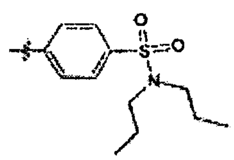 | 2677 | 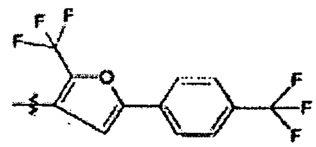 |
| 2670 | 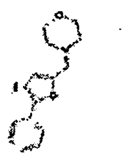 | 2678 | 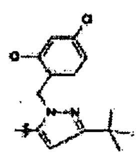 |

Table 6

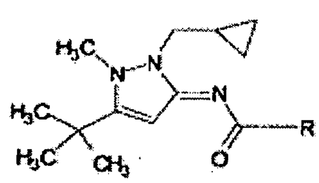

| Compound No. | R | Compound No. | R |
|---|---|---|---|
| 3001 | 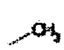 | 3009 | 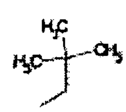 |
| 3002 | 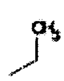 | 3010 | 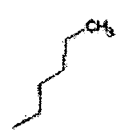 |

(continued)

| Compound No. | R | Compound No. | R |
|---|---|---|---|
| 3003 | 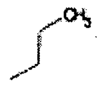 | 3011 | 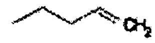 |
| 3004 | 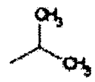 | 3012 | 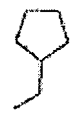 |
| 3005 | 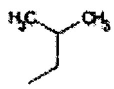 | 3013 | 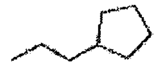 |
| 3006 | 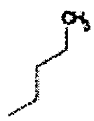 | 3014 | 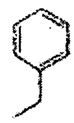 |
| 3007 | 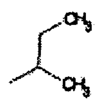 | 3015 | 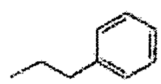 |
| 3008 | 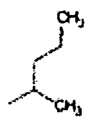 | 3016 | 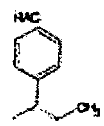 |
| 3017 | 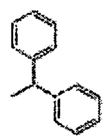 | 3025 | 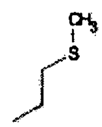 |
| 3018 | 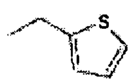 | 3026 | 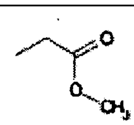 |
| 3019 | 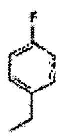 | 3027 | 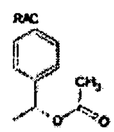 |
| 3020 | 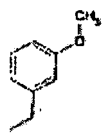 | 3028 | 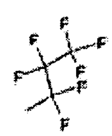 |
| 3021 | 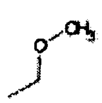 | 3029 | 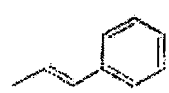 |

(continued)

| Compound No. | R | Compound No. | R |
|---|---|---|---|
| 3022 | 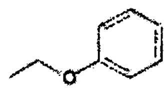 | 3030 | 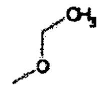 |
| 3023 | 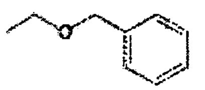 | 3031 | 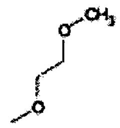 |
| 3024 | 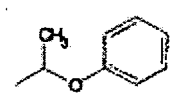 | 3032 | 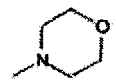 |
| 3033 | 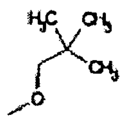 | 3041 | 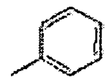 |
| 3034 | 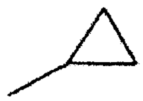 | 3042 | 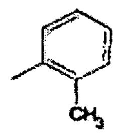 |
| 3035 | 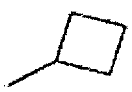 | 3043 | 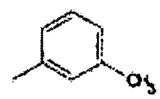 |
| 3036 | 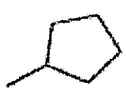 | 3044 | 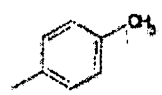 |
| 3037 | 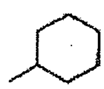 | 3045 | 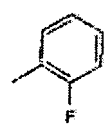 |
| 3038 | 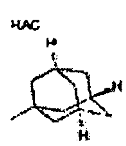 | 3046 | 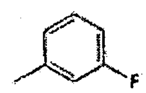 |
| 3039 | 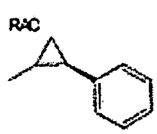 | 3047 | 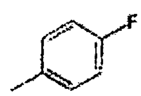 |

(continued)

| Compound No. | R | Compound No. | R |
|---|---|---|---|
| 3040 | 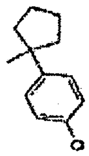 | 3048 | 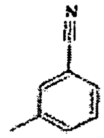 |
| 3049 | 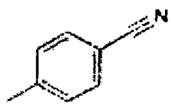 | 3057 | 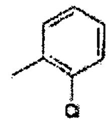 |
| 3050 | 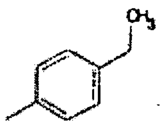 | 3058 | 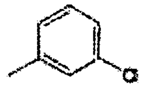 |
| 3051 | 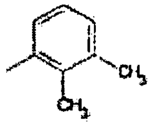 | 3059 | 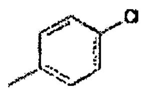 |
| 3052 | 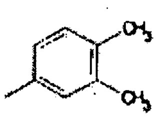 | 3060 | 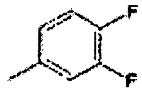 |
| 3053 | 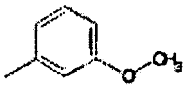 | 3061 | 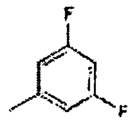 |
| 3054 | 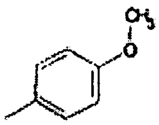 | 3062 | 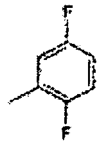 |
| 3055 | 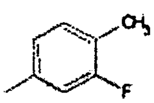 | 3063 | 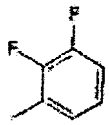 |
| 3056 | 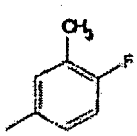 | 3064 | 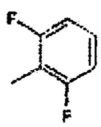 |
| 3065 | 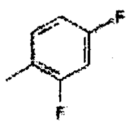 | 3073 | 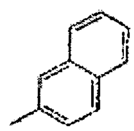 |

(continued)

| Compound No. | R | Compound No. | R |
|---|---|---|---|
| 3066 | 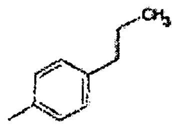 | 3074 | 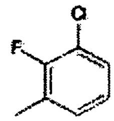 |
| 3067 | 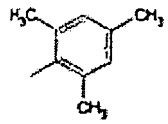 | 3075 | 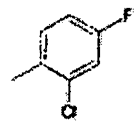 |
| 3068 | 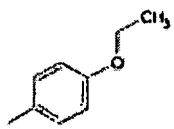 | 3076 | 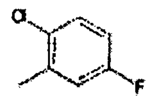 |
| 3069 | 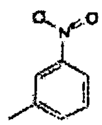 | 3077 | 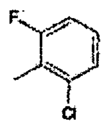 |
| 3070 | 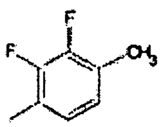 | 3078 | 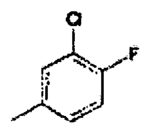 |
| 3071 | 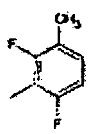 | 3079 | 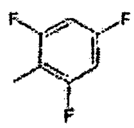 |
| 3072 | 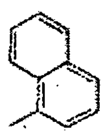 | 3080 | 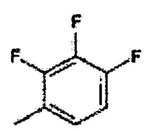 |
| 3081 | 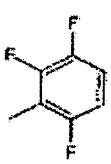 | 3089 | 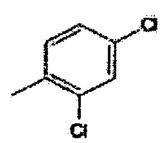 |
| 3082 | 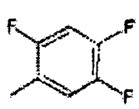 | 3090 | 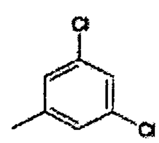 |
| 3083 | 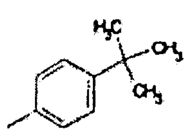 | 3091 | 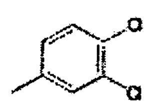 |

(continued)

| Compound No. | R | Compound No. | R |
|---|---|---|---|
| 3084 | 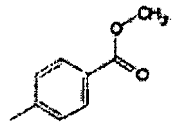 | 3092 | 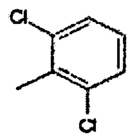 |
| 3085 | 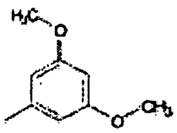 | 3093 | 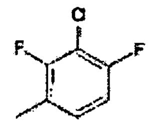 |
| 3086 | 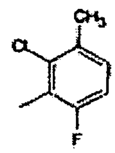 | 3094 | 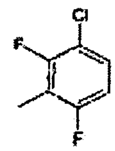 |
| 3087 | 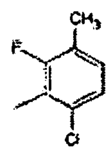 | 3095 | 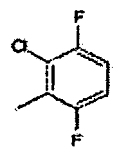 |
| 3088 | 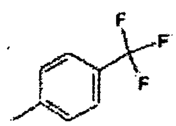 | 3096 | 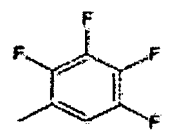 |
| 3097 | 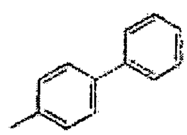 | 3105 | 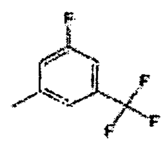 |
| 3098 | 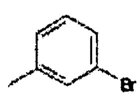 | 3106 | 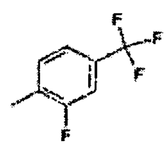 |
| 3099 | 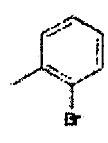 | 3107 | 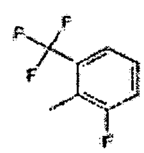 |
| 3100 | 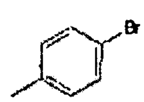 | 3108 | 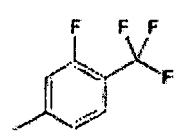 |

(continued)

| Compound No. | R | Compound No. | R |
|---|---|---|---|
| 3101 | 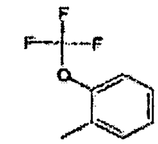 | 3109 | 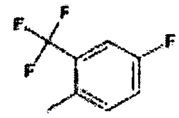 |
| 3102 | 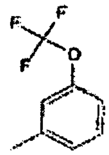 | 3110 | 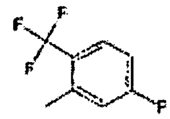 |
| 3103 | 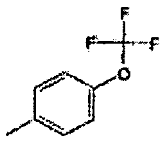 | 3111 | 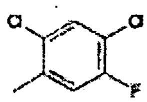 |
| 3104 | 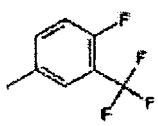 | 3112 | 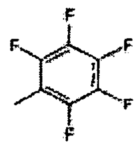 |
| 3113 | 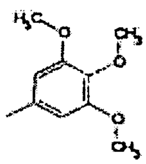 | 3121 | 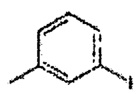 |
| 3114 | 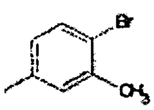 | 3122 | 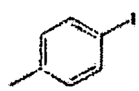 |
| 3115 | 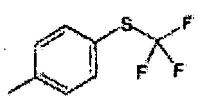 | 3123 | 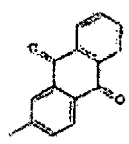 |
| 3116 | 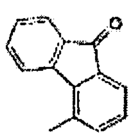 | 3124 | 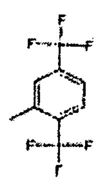 |
| 3117 | 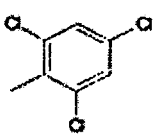 | 3125 | 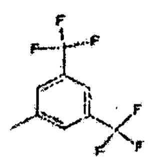 |

(continued)

| Compound No. | R | Compound No. | R |
|---|---|---|---|
| 3118 | 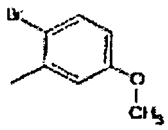 | 3126 | 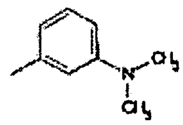 |
| 3119 | 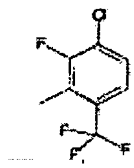 | 3127 | 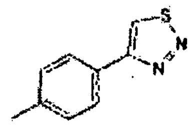 |
| 3120 | 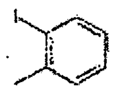 | 3128 | 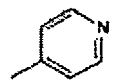 |
| 3129 | 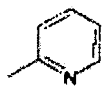 | 3137 | 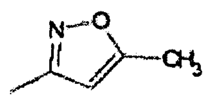 |
| 3130 | 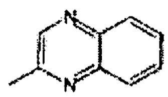 | 3138 | 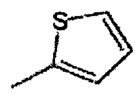 |
| 3131 | 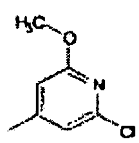 | 3139 | 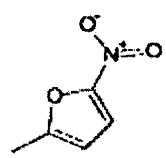 |
| 3132 | 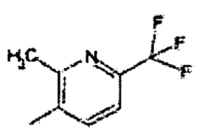 | 3140 | 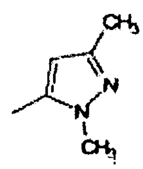 |
| 3133 | 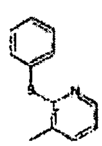 | 3141 | 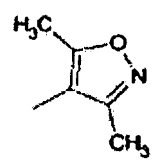 |
| 3134 | 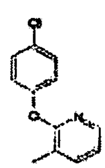 | 3142 | 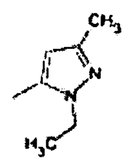 |
| 3135 | 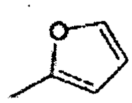 | 3143 | 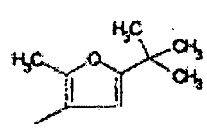 |

(continued)

| Compound No. | R | Compound No. | R |
|---|---|---|---|
| 3136 | 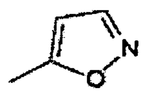 | 3144 | 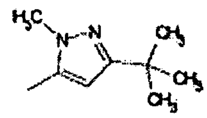 |
| 3145 | 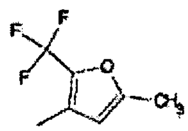 | 3152 | 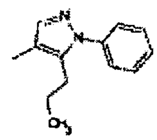 |
| 3146 | 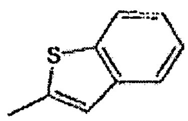 | 3153 | 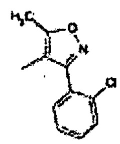 |
| 3147 | 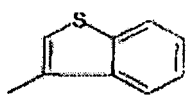 | 3154 | 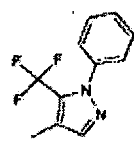 |
| 3148 | 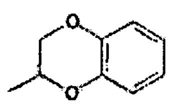 | 3155 | 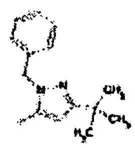 |
| 3149 | 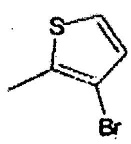 | 3156 | 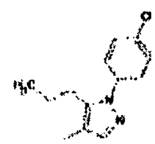 |
| 3150 | 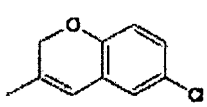 | 3157 | 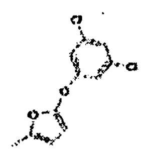 |
| 3151 | 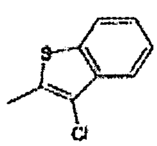 | 3158 | 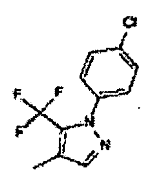 |

Table 7

| 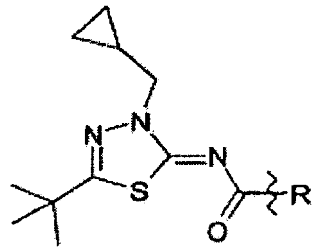 | | | |
|---|---|---|---|
| Compound No. | R | Compound No. | R |
| 3159 | 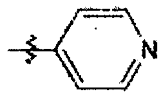 | 3165 | 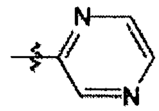 |
| 3160 | 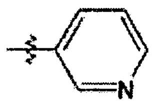 | 3166 | 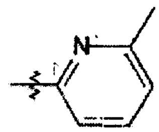 |
| 3161 | 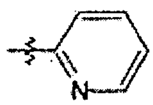 | 3167 | 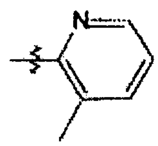 |
| 3162 | 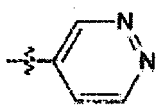 | 3188 | 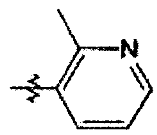 |
| 3163 | 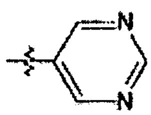 | 3169 | 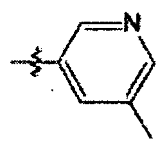 |
| 3164 | 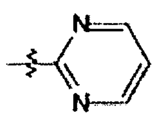 | 3170 | 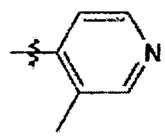 |
| 3171 | 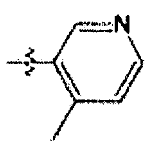 | 3179 | 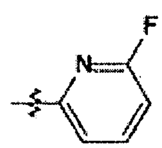 |
| 3172 | 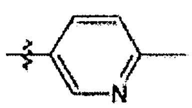 | 3180 | 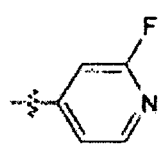 |

(continued)

| Compound No. | R | Compound No. | R |
|---|---|---|---|
| 3173 | 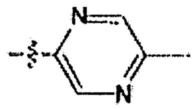 | 3181 | 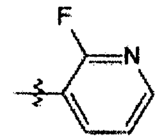 |
| 3174 | 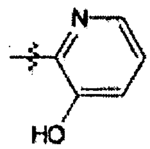 | 3182 | 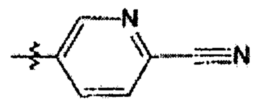 |
| 3175 | 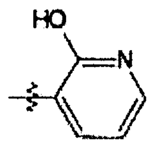 | 3183 | 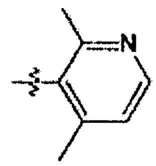 |
| 3176 | 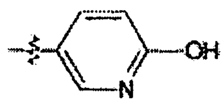 | 3184 | 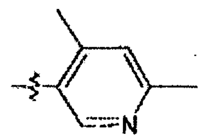 |
| 3177 | 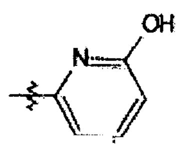 | 3185 | 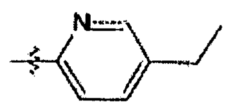 |
| 3178 | 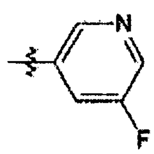 | 3186 | 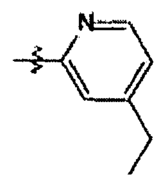 |
| 3187 | 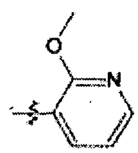 | 3195 | 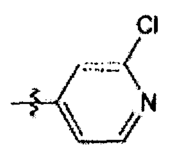 |
| 3188 | 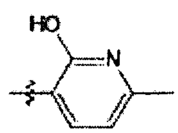 | 3196 | 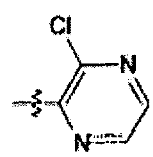 |
| 3189 | 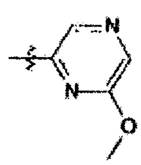 | 3197 | 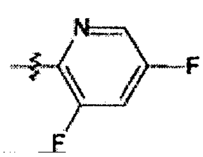 |

(continued)

| Compound No. | R | Compound No. | R |
|---|---|---|---|
| 3190 | 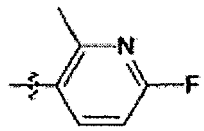 | 3198 | 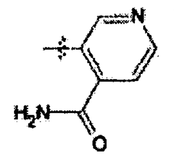 |
| 3191 | 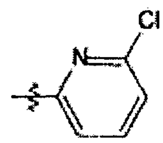 | 3199 | 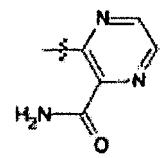 |
| 3192 | 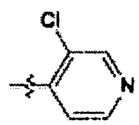 | 3200 | 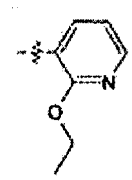 |
| 3193 | 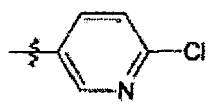 | 3201 | 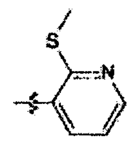 |
| 3194 | 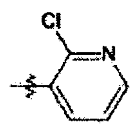 | 3202 | 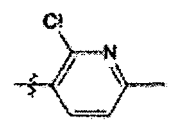 |
| 3203 | 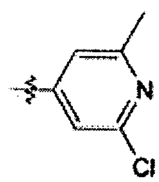 | 3211 | 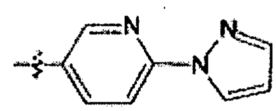 |
| 3204 | 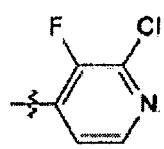 | 3212 | 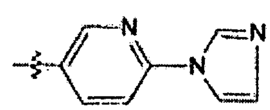 |
| 3205 | 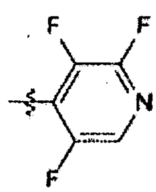 | 3213 | 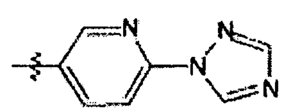 |
| 3206 | 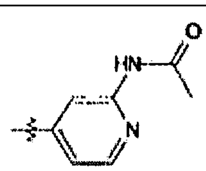 | 3214 | 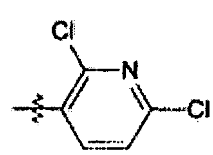 |

(continued)

| Compound No. | R | Compound No. | R |
|---|---|---|---|
| 3207 | 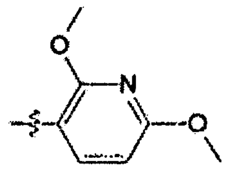 | 3215 | 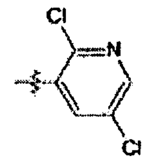 |
| 3208 | 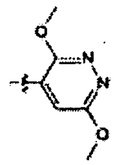 | 3216 | 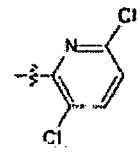 |
| 3209 | 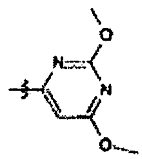 | 3211 | 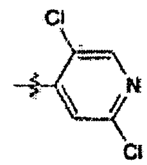 |
| 3210 | 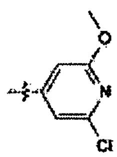 | 3218 | 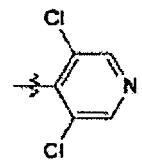 |
| 3219 | 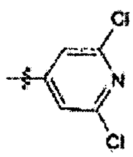 | 3227 | 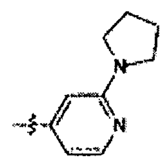 |
| 3220 | 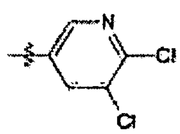 | 3228 | 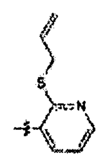 |
| 3221 | 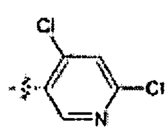 | 3229 | 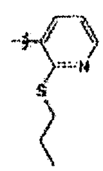 |
| 3222 | 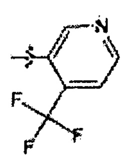 | 3230 | 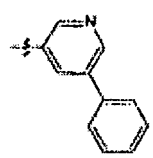 |
| 3223 | 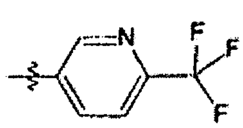 | 3231 | 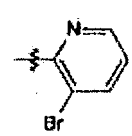 |

(continued)

| Compound No. | R | Compound No. | R |
|---|---|---|---|
| 3224 | 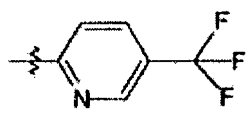 | 3232 | 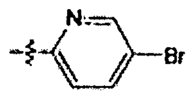 |
| 3225 | 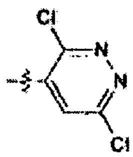 | 3233 | 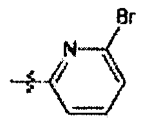 |
| 3226 | 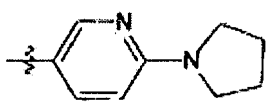 | 3234 | 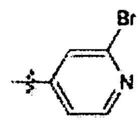 |
| 3235 | 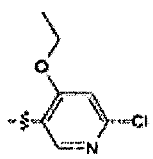 | 3243 | 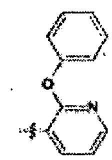 |
| 3236 | 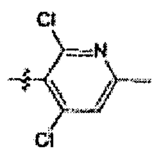 | 3244 | 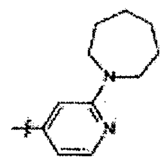 |
| 3237 | 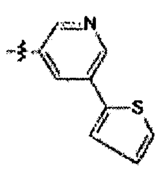 | 3245 | 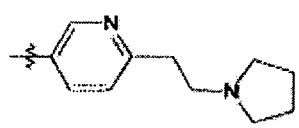 |
| 3238 | 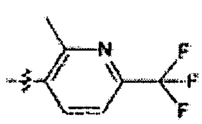 | 3246 | 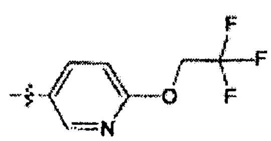 |
| 3239 | 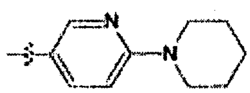 | 3247 | 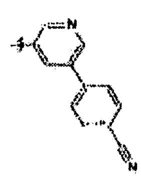 |
| 3240 | 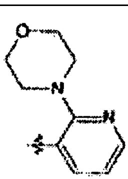 | 3248 | 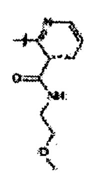 |

(continued)

| Compound No. | R | Compound No. | R |
|---|---|---|---|
| 3241 | 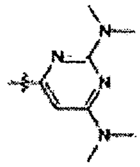 | 3249 | 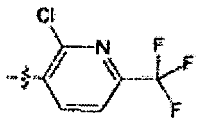 |
| 3242 | 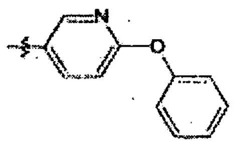 | 3250 | 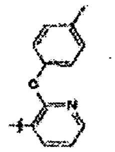 |
| 3251 | 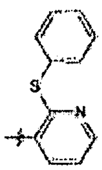 | 3259 | 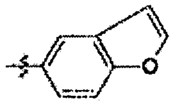 |
| 3252 | 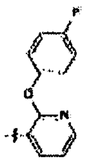 | 3260 | 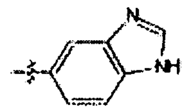 |
| 3253 | 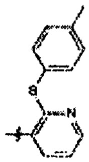 | 3261 | 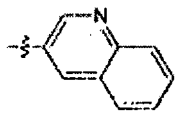 |
| 3254 | 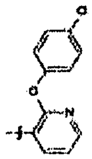 | 3262 | 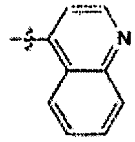 |
| 3255 | 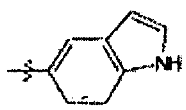 | 3263 | 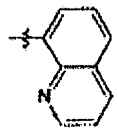 |
| 3256 | 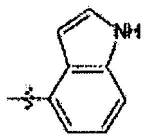 | 3264 | 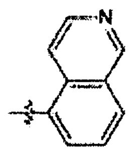 |
| 3257 | 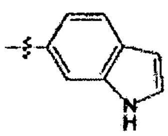 | 3265 | 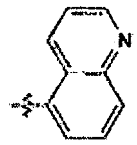 |

(continued)

| Compound No. | R | Compound No. | R |
|---|---|---|---|
| 3258 | 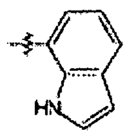 | 3266 | 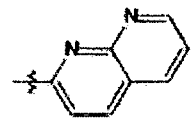 |
| 3267 | 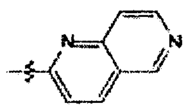 | 3275 | 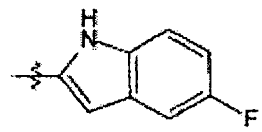 |
| 3268 | 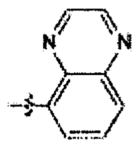 | 3276 | 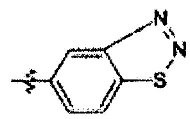 |
| 3269 | 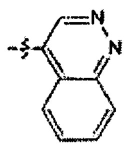 | 3277 | 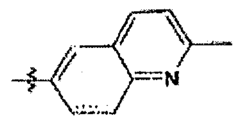 |
| 3270 | 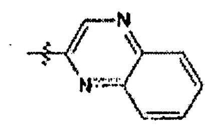 | 3278 | 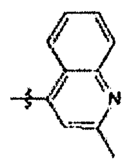 |
| 3271 | 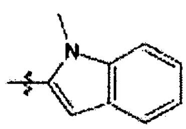 | 3279 | 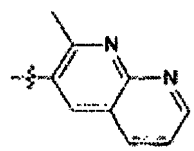 |
| 3272 | 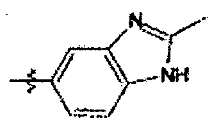 | 3280 | 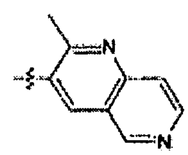 |
| 3273 | 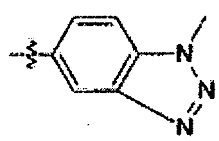 | 3281 | 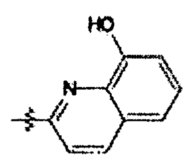 |
| 3274 | 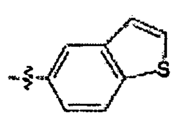 | 3282 | 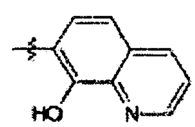 |

(continued)

| Compound No. | R | Compound No. | R |
|---|---|---|---|
| 3283 | 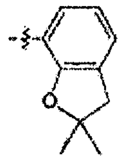 | 3291 | 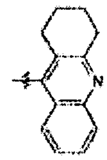 |
| 3284 | 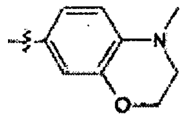 | 3292 | 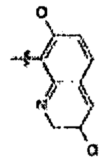 |
| 3285 | 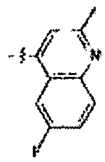 | 3293 | 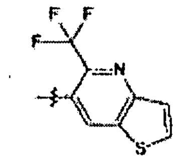 |
| 3286 | 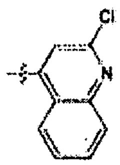 | 3294 | 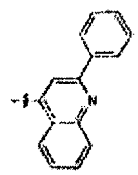 |
| 3287 | 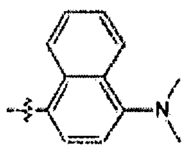 | 3295 | 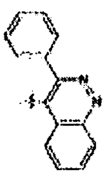 |
| 3288 | 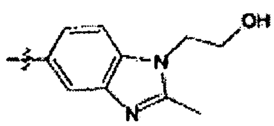 | 3296 | 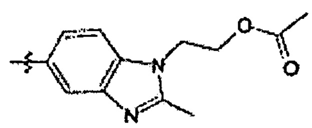 |
| 3289 | 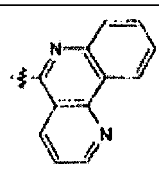 | 3297 | 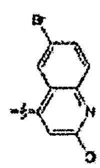 |
| 3290 | 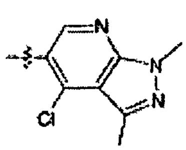 | 3298 | 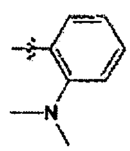 |
| 3299 | 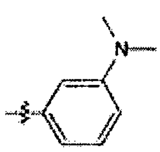 | 3307 | 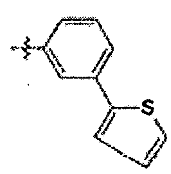 |

(continued)

| Compound No. | R | Compound No. | R |
|---|---|---|---|
| 3300 | 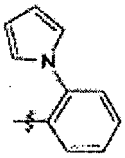 | 3308 | 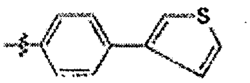 |
| 3301 | 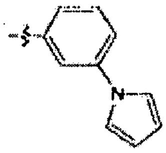 | 3309 | 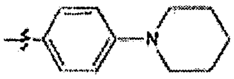 |
| 3302 | 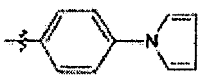 | 3310 | 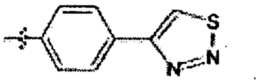 |
| 3303 | 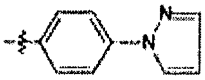 | 3311 | 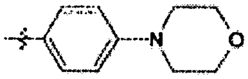 |
| 3304 | 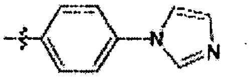 | 3312 | 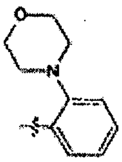 |
| 3305 | 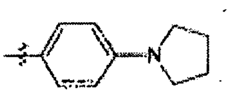 | 3313 | 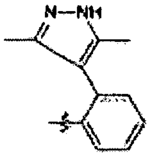 |
| 3306 | 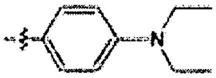 | 3314 | 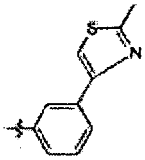 |
| 3315 | 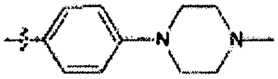 | 3323 | 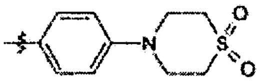 |
| 3316 | 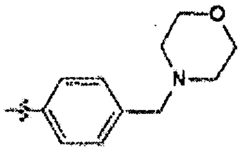 | 3324 | 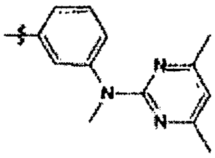 |

(continued)

| Compound No. | R | Compound No. | R |
|---|---|---|---|
| 3317 | 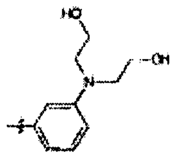 | 3325 | 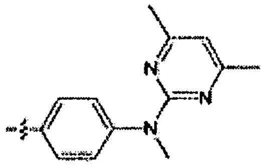 |
| 3318 | 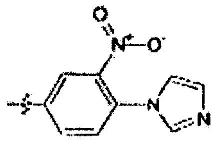 | 3326 | 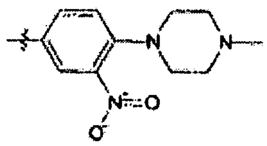 |
| 3319 | 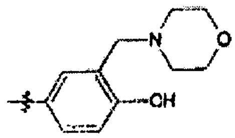 | 3327 | 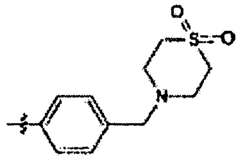 |
| 3320 | 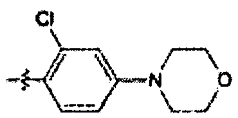 | | |
| 3321 | 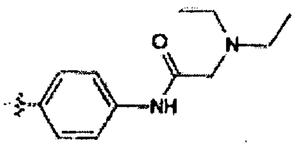 | | |
| 3322 | 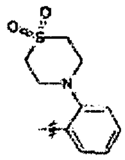 | | |

Table 8

| Compound No. | $^1$H-NMR | Mass | m.p. (˚C) |
|---|---|---|---|
| 1 | $^1$HNMR(200MHz,CHLOROFORM-d)dppm0.44-0.53(m,2H)0.61-0.71(m, 2H)1.30-1.47(m,1H)4.25(d, J=7.31Hz,2H)6.82-6.92(m,1H)7.73-7.895 (m,2H)8.44-8.51(m,1H) | ESI(Pos)245 $(M+H)^+$ | 90-91 |
| 2 | $^1$HNMR(200MHz,CHLOROFORM-d)dppm0.42-0.55(m,2H)0.62-0.78(m, 2H)1.40-1.60(m,1H)4.24(d, J=7.47Hz,2H)6.56-6.69(m,1H)7.45-7.75 (m,4H)8.38-8.59(m,3H) | ESI(Pos)321 $(M+H)^+$ | 105.5-106.5 |
| 3 | $^1$HNMR(200MHz,CHLOROFORM-d)dppm0.42-0.76(m,4H)1.39-1.56(m, 1H)4.22(d,J=7.0Hz,2H)6.53-6.65 (m,1H)7.06-7.22(m,1H)7.52-7.81 (m,3H)8.17-8.27(m.1H)8.32-8.43(m.1H)8.58-8.65(m,1H) | ESI(Pos)379-$(M+H)^+$ | - |
| 4 | $^1$HNMR(200MHz,CHLOROFORM-D)dppm0.53-0.73(m,2H)1.23-1.53(m, 1H)4.14(d.J=7.47Hz,2H) 6.53-6.68(m,1H)7.20-7.46(m,3H)7.49-7.64 (m,1H)7.64-7.77(m,1H)7.85-8.03(m,1H)8.22-8.37(m,1H) | ESI(Pos)337 $(M+H)^+$ | - |
| 5 | $^1$HNMR(200MHz,CHLOROFORM-d)dppm3.90(s,3H)6.56-6.70(m,1H) 7.18-7.31(m,1H)7.58-7.71(m,3H) 8.17-8.29(m,1H)8.37-8.48(m,1H) | ESI(Pos)299 $(M+H)^+$ | 146-147 |
| 6 | $^1$HNMR(200MHz,CHLOROFORM-d)dppm1.49(t,J=7.3Hz,3H)4.40(q,J=7.3 Hz,2H)6.60-6.71(m,1H) 7.18-7.31(m,1H)7.56-7.69(m,3H)8.16-8.27 (m,1H)8.39-8.48(m,1H) | ESI(Pos)313 $(M+H)^+$ | 93-95 |
| 7 | 1HNMR(200MHz,CHLOROFORM-d)dppm0.97(t,J=7.3Hz,3H)1.41(dt, J=14.8,7.3Hz,2H)1.77-1.97(m,2H) 4.34(t,J=7.5Hz,2H)6.55-6.70(m,1 H)7.17-7.31(m,1H)7.55-7.70(m,3H)8.15-8.28(m,1H)8,41-8.50(m,1 H) | ESI(Pos)341 $(M+H)^+$ | 51-52 |
| 8 | $^1$HNMR(200MHz,CHLOROFORM-d)dppm1.47(d,J=6.6Hz,6H)5.84-6.06 (m,1H)6.64-6.77(m,1H)7.18-7.32 (m,1H)7.53-7.78(m,2H)8.14-8.45 (m,2H) | ESI(Pos)327 $(M+H)^+$ | 86-88 |
| 9 | $^1$H NMR (200 MHz, CHLOROFORM-d) d ppm 0.38 - 0.74 (m, 4 H) 1.32 - 1.53 (m, 1 H) 4.22 (d, J=7.03 Hz, 2 H) 6.61 - 6.72 (m, 1 H) 7.18 - 7.31 (m, 1 H) 7.54 - 7.76 (m, 3 H) 8.13 - 8.27 (m, 1 H) 8.37 - 8.48 (m, 1 H) | ESI(Pos) 339 $(M+H)^+$ | 58.5-59 |
| 10 | $^1$H NMR (200 MHz, CHLOROFORM-d) d ppm 0.36 - 0.51 (m, 2 H) 0.60 - 0.74 (m, 2 H) 1.29 - 1.53 (m, 1 H) 4.17 (d, J=7.47 Hz, 2 H) 6.58 - 6.74 (m, 1 H) 7.16 - 7.41 (m, 2 H) 7.55 - 7.82 (m, 3 H) 8.33 - 8.45 (m, 1 H) | ESI(Pos) 322 $(M+H)^+$ | 53.5-54.5 |
| 11 | $^1$H NMR (300 MHz, CHLOROFORM-d) d ppm 0.35 - 0.75 (m, 4 H) 1.18 - 1.42 (m, 1 H) 4.12 (d, J=7.31 Hz, 2 H) 6.61 - 6.71 (m, 1 H) 7.19 - 7.42 (m, 2 H) 7.58 - 7.85 (m. 3 H) 8.30 - 8.39 (m. 1 H) | ESI(Pos)339 $(M+H)^+$ | 88-89 |
| 12 | $^1$H NMR (600 MHz, CHLOROFORM-d) d ppm 0.44 - 0.72 (m, 4 H) 0.74 - 0.90 (m, 2 H) 1.84 - 2.05 (m, 1 H) 3.57 - 3.85 (m, 2 H) 6.43 - 6.57 (m, 1 H) 7.22 - 7.38 (m, 1 H) 7.64 - 7.85 (m, 3 H) 8.42 - 8.55 (m, 1 H) 8.70 - 8.83 (m, 10 H) | ESI(Pos) 353 $(M+H)^+$ | 93-94 |
| 13 | $^1$H NMR (200 MHz, CHLOROFORM-d) d ppm -0.00 - 0.53 (m, 4 H) 0.54 - 0.77 (m, 1 H) 1.81 (q, J=7.0 Hz. 2 H) 4.43 (t, J=7.0 Hz, 2 H) 6.58 - 6.69 (m, 1 H) 7.18 - 7.31 (m, 1 H) 7.55 - 7.71 (m, 3 H) 8.14 - 8.27 (m, 1 H) 8.40 - 8.51 (m, 1 H) | ESI(Pos) 353 $(M+H)^+$ | 56-58 |

(continued)

| Compound No. | $^1$H-NMR | Mass | m.p. (˚C) |
|---|---|---|---|
| 14 | $^1$H NMR (200 MHz, CHLOROFORM-d) d ppm 1.12 (t. 3=7.03 Hz, 3 H) 3.44 (q, J=7.03 Hz, 2 H) 3.84 (t, J=4.83 Hz, 2 H) 4.54 (t, J=4.83 Hz, 2 H) 6.56 - 6.69 (m, 1 H) 7.18 - 7.31 (m, 1 H) 7.57 - 7.77 (m, 3 H) 8.13 - 8.26 (m, 1 H) 8.41 - 8.50 (m, 1 H) | ESI(Pos) 357 $(M+H)^+$ | 104.5-105.5 |
| 15 | $^1$H NMR (200 MHz, CHLOROFORM-d) d ppm 0.41 - 0.77 (m, 4 H) 1.32 - 1.51 (m, 1 H) 4.19 (d, J=7.5 Hz, 2 H) 7.20- 7.31 (m, 1 H) 7.57 - 7.71 (m, 2 H) 7.81 - 7.87 (m, 1 H) 8.14 - 8.26 (m, 1 H) 8.35 - 8.43 (m, 1 H) | ESI(Pos) 439 $(M+Na)^+$ | 124-126 |
| 16 | $^1$H NMR (200 MHz, CHLOROFORM-d) d ppm 321 (t, J=7.3 Hz, 2 H) 4.54 (t, J=7.3 Hz, 2 H) 6.43 - 6.55 (m, 1 H) 7.10 - 7.36 (m, 7 H) 7.54 - 7.73 (m, 2 H) 8.19 - 8.32 (m, 1 H) 8.45 - 8.57 (m, 1 H) | ESI(Pos) 389 $(M+H)^+$ | 117-118 |
| 17 | 1HNMR(200MHz,CHLOROFORM-d)dppm0.59-0.73(m,2H)1.26-1.46(m, 1H)4.13(d.J=7.47Hz,2H) 6.62-6.74(m,1H)7.02-7.17(m,1H)7.39-7.50 (m,1H)7.58-7.77(m,3H)8.30-8.41(m,1H) | ESI(Pos)339 $(M+H)^+$ | 88.5-89 |
| 18 | $^1$HNMR(200MHz,CHLOROFORM-d)dppm0.40-0.56(m,2H)0.56-0.75(m, 2H)1.34-1.56(m,1H)4.22(d, J=7.03Hz.2H)6.59-6.74(m,1H)7,10-7.28 (m,1H)7.53-7.79(m,3H)8.29-8.53(m,2H) | ESI(Pos)339 $(M+H)^+$ | 127-127.5 |
| 19 | $^1$HNMR(200MHz,CHLOROFORM-d)dppm0.39-0.51(m,2H)0.60-0.72(m, 2H)1.33-1.55(m,1H)3.94(s,3H) 4.16(d.J=7.03Hz,2H)6.55-6.66(m.1H) 6.95-7.05(m,1H)7.52-7.72(m.3H)8.09-8.14(m,1H)8.36-8.46(m,1H) | ESI(Pos)351 $(M+H)^+$ | - |
| 20 | $^1$HNMR(200MHz,CHLOROFORM-d)dppm0.36-0.72(m,4H)1.34-1.58(m, 1H)3.80(s,3H)3.85(s,3H)4.15(d, J=7.5Hz.2H)6.48-6.61(m,1H)6.84-6.93(m,2H)7.40-7.46(m,1H)7.48-7.59(m,1H)7.60-7.69(m,1H)8.27-8.38 (m,1H) | ESI(Pos)313 $(M+H)^+$ | - |
| 21 | $^1$HNMR(200MHz,CHLOROFORM-d)dppm0.38-0.74(m,4H)1.34-1.54(m, 1H)4.20(d,J=7.0Hz,2H)6.65-6.77 (m,1H)7.51-7.62(m,11H)7.63-7.80 (m,3H)7.84-7.91(m,1H)8.09-8.17(m,1H)8.49-8.59(m,1H)-8.81-8.90 (m, 1H)8.94-9.01(m,1H) | ESt(Pos)304 $(M+H)^+$ | - |
| 22 | $^1$HNMR(200MHz,CHLOROFORM-d)dppm0.40-0.77(m,4H)1.30-1.48(m, 1H)2.38(s,3H)4.23(d,J=7.0Hz,2H) 6.79-6.91(m,1H)7.19-7.31(m,1H) 7.55-7.69(m,2H)7.75-7.84(m,1H)8.05-8.18(m,1H) | ESI(Pos)353 $(M+H)^+$ | - |
| 23 | $^1$HNMR(200MHz,CHLOROFORM-D)dppm0.36-0.51(m,2H)0.56-0.71(m, 2H)1.32-1.52(m,1H)2.36(s,3H) 4.18(d,J=7.0Hz,2H)6.48-6.56(m,1H) 7.18-7.30(m,1H)7.56-7.67(m,2H)8.14-8.31(m.2H) | ESI(Pos)353 $(M+H)^+$ | 81-83 |
| 24 | $^1$HNMR(200MHz,CHLOROFORM-d)dppm0.37-0.73(m,4H)1.30-1.55(m, 1H)2.25(s,3H)4.21(d,J=7.5Hz,2H) 7.17-7.31(m,1H)7.44-7.69(m,2H) 8.10-8.27(m,1H)8.35-8.48(m,1H) | ESI(Pos)353 $(M+H)^+$ | 118-120 |
| 25 | $^1$HNMR(200MHz,CHLOROFORM-d)dppm0.49-0.62(m,4H)1.20-1.40(m, 1H)2.61(s,3H)4.50(d,J=7.0Hz,2H) 6.54(d,J=7.0Hz,1H)7.19-7.30(m,1 H)7.45-7.67(m,2H)8.11-8.31(m,2H) | ESI(Pos)353 $(M+H)^+$ | 96-98 |

(continued)

| Compound No. | $^1$H-NMR | Mass | m.p. (˚C) |
|---|---|---|---|
| 26 | $^1$HNMR(600MHz,CHLOROFORM-d)dppm0.39-0.45(m,2H)0.67-0.73(m, 2H)1.31-1.39(m,1H)4.10(d, J=7.34Hz,2H)7.47(t,J=7.79Hz,1H)7.57(t, J=7.57Hz,1H)7.65(dd,J=9.40,2.06Hz,1H)7.68(d,J=7.79Hz.1H) 7.76(d. J=7.79Hz,1H)8.02(s,1H)8.27(d,J=9.17Hz,1H) | APCI(Pos)389 (M+H)$^+$ | 93-93.5 |
| 27 | $^1$HNMR(600MHz,CHLOROFORM-d)dppm0.46-0.54(m,2H)0.72-0.80(m, 2H)1.44-1.51(m.1H)4.22(d, J=7.34Hz,2H)7.56(t,J=7.79Hz.1H)7.64 (dd,J=9.63,2.29Hz.1H)7.73(d,J=7.79Hz.1H)8.02(s,1H)8.33(d, J=9.63Hz. 1H)8.41(d.J=7.79Hz,1H)8.51(s.1H) | APCI(Pos)389 (M+H)$^+$ | 84-85 |
| 28 | $^1$HNMR(600MHz,CHLOROFORM-d)dppm-1.19--1.12(m,2H)-0.78--0.72 (m,2H)-0.14--0.04(m,1H)3.46(d, J=9.2Hz,2H)6.92-6.97(m,1H)7.29-7.34(m,1H)7.75-7.82(m,1H)8.35-8.43(m.2H)8.60-8.64(m,1H) | ESI(Pos)407 (M+H)$^+$ | - |
| 29 | $^1$HNMR(200MHz.CHLOROFORM-d)dppm0.40-0.77(m,4H)1.37-1.52(m, 1H)4.22(d,J=7.5Hz,2H)6.68-6.77 (m,1H)7.21-7.33(m,1H)7.61-7.74 (m,1H)7.78-7.88(m,1H)8.16-8.30(m,1H)8.65-8.73(m,1H) | ESI(Pos)407 (M+H)$^+$ | 85-86 |
| 30 | $^1$HNMR(200MHz,CHLOROFORM-d)dppm0.40-0.77(m,4H)1.30-1.48(m, 1H)2.38(s,3H)4.23(d,J=7.0Hz,2H) 6.79-6.91(m,1H)7.19-7.31(m,1H) 7.55-7.69(m,2H)7.75-7.84(m,1H)8.05-8.18(m,1H) | ESI(Pos)353 (M+H)$^+$ | - |
| 31 | $^1$H NMR (600 MHz, CHLOROFORM-d) d ppm.0.42 - 0.53 (m, 2 H) 0.66 - 0.79 (m, 2 H) 1.37 - 1.48 (m, 1 H) 4.20 (d, J=7.34 Hz, 2 H) 7.18 - 7.23 (m, 1 H) 7.65 - 7.70 (m, 2 H) 8.03 (s, 1 H) 8.36 (dd, J=6.65, 2.52 Hz, 1 H) 8.39 (d, J=9.63 Hz, 1 H) | APCI(Pos) 407 (M+H)$^+$ | 109-110 |
| 32 | $^1$H NMR (500 MHz, CHLOROFORM-d) d ppm 0-36 - 0.70 (m, 4 H) 1.26 - 1.37 (m, 1 H) 4.30 (d, J=6.9 Hz, 2 H) 6.97 - 7.03 (m, 1 H) 7.17 - 7.22 (m, 1 H) 7.22 - 7.26 (m, 1 H) 7.54 - 7.59 (m, 1 H) 7.59 - 7.64 (m. 1 H) 7.66 - 7.72 (m. 1 H) | ESI(Pos) 407 (M+H)$^+$ | - |
| 33 | $^1$H NMR (200 MHz, CHLOROFORM-d) d ppm 0.38 - 0.74 (m, 4 H) 1.30 - 1.49 (m, 1 H) 4.17 (d, J=7.0 Hz, 2 H) 6.61 - 6.68 (m, 1 H) 7.22 - 7.32 (m, 1 H) 7.59 - 7.72 (m. 2 H) 8.15 - 8.26 (m, 1 H) 8.51 - 8.56 (m, 1 H) | ESI(Pos) 395 (M+Na)$^+$ | 91-93 |
| 34 | $^1$H NMR (200 MHz, CHLOROFORM-d) d ppm 0.40 - 0.79 (m, 4 H) 1.33 - 1.52 (m, 1H) 4.20 (d, J=7.5 Hz, 2 H) 7.18 - 7.30 (m, 1 H) 7.51 - 7.71 (m, 2 H) 7.72 - 7.79 (m, 1 H) 8.13 - 8.26 (m, 1 H) 8.39 - 8.51 (m, 1 H) | ESI(Pos) 373 (M+H)$^+$ | 117-119 |
| 35 | $^1$H NMR (200 MHz, CHLOROFORM-d) d ppm 0.48 - 0.67 (m, 4 H) 1.35 - 1.55 (m, 1 H) 4.67 (d, J=7.5 Hz, 2 H) 6.70 - 6.76 (m, 1 H) 7.20 - 7.31 (m, 1 H) 7.42 - 7.54 (m, 1H) 7.59 - 7.70 (m, 1 H) 8.12 - 8.23 (m, 1 H) 8.25 - 8.35 (m, 1 H) | ESI(Pos) 373 (M+H)$^+$ | 81-83 |
| 36 | $^1$H NMR (200 MHz, CHLOROFORM-d) d ppm 0.38 - 0.83 (m, 4 H) 1.32 - 1.51 (m, 1 H) 4.19 (d, J=7.5 Hz, 2 H) 7.21 - 7.33 (m, 1 H) 7.59 - 7.74 (m, 2 H) 7.99 - 8.07 (m, 1 H) 8.15 - 8.28 (m, 1 H) 8.30 - 8.40 (m, 1 H) | ESI(Pos) 407 (M+H)$^+$ | 93-94 |

(continued)

| Compound No. | $^1$H-NMR | Mass | m.p. (˚C) |
|---|---|---|---|
| 37 | $^1$H NMR (200 MHz, CHLOROFORM-d) d ppm 0.41 - 0.54 (m, 2H) 0.64 - 0.80 (m, 2 H) 1.31 - 1.51 (m, 1 H) 4.23 (d, J=7.5 Hz, 2 H) 7.48 - 7.78 (m, 3 H) 8.13 - 8.27 (m, 1 H) 8.49 - 8.63 (m, 1 H) | ESI(Pos) 357 $(M+H)^+$ | 89-91 |
| 38 | $^1$H NMR (200 MHz, CHLOROFORM-D) d ppm 0.43 - 0.57 (m, 2 H) 0.60 - 0.77 (m, 2 H) 1.39 - 1.58 (m, 1 H) 4.31 (d, J=7.0 Hz. 2 H) 7.19 - 7.32 (m, 1 H) 7.35 - 7.55 (m, 5 H) 7.57 - 7.71 (m, 1 H) 7.83 - 7.98 (m, 2 H) 8.15 - 8.30 (m, 1 H) 8.46 - 8.61 (m. 1 H) | ESI(Pos) 415 $(M+H)^+$ | 115-116 |
| 39 | $^1$HNMR(200MHz,CHLOROFORM-d)dppm0.44-0.54(m,4H)1.28-1.51(m, 1H)4.02(s,3H)4.46(d,J=7.5Hz,2H) 6.03-6.09(m,1H)7.17-7.29(m,1H) 7.52-7.66(m,2H)7.99-8.09(m,1H)8.12-8.25(m,1H) | ESI(Pos)369 $(M+H)^+$ | 126-128 |
| 40 | $^1$HNMR(200MHz,CHLOROFORM-d)dppm0.32-0.54(m,4H)1.41-1.58(m, 1H)2.73(s,6H)4.49(d,J=6.6Hz,2H) 6.33-6.42(m,1H)7.18-7.29(m,1H) 7.49-7.67(m,2H)8.06-825(m,2H) | ESI(Pos)382 $(M+H)^+$ | - |
| 41 | $^1$HNMR(200MHz,CHLOROFORM-d)dppm0.53-0.63(m,4H)1.12-1.37(m, 1H)2.78(s,3H)4.61(d,J=7.0Hz,2H) 7.25(d.1H)7.56-7.73(m,2H)8.05-8.26(m.2H) | ESI(Pos)431 $(M+H)^+$ | 135-137 |
| 42 | $^1$HNMR(200MHz,CHLOROFORM-D)dppm0.44-0.83(m,4H)1.27-1.45(m, 1H)4.16(d,J=7.0Hz,2H)7.19-7.42 (m.2H)7.61-7.71(m,2H)8.13-8:24 (m,1H) | ESI(Pos)397 $(M+Na)^+$ | 69-71 |
| 43 | $^1$HNMR(200MHz,CHLOROFORM-d)dppm2.23(s.3H)2.43(s,3H)3.81(s,3 H)3.83(s,3H)3.89(s.3H)622-6.27 (m,1H)6.40-6.53(m,2H)7.93-8.01 (m.2H) | ESI(Pos)301 $(M+H)^+$ | 117.5-118 |
| 44 | $^1$HNMR(200MHz,CHLOROFORM-d)dppm2.27(s,3H)2.46(s,3H)2.63(s,3 H)4.97-5.30(m,4H)5.89-6.12(m, 1H)6.27(s,1H)7.11-7.32(m,3H)7.86 -7.95(m,1H)8.02(s,1H) | ESI(Pos)281 $(M+H)^+$ | - |
| 45 | $^1$HNMR(200MHz.CHLOROFORM-d)dppm0.50-0.60(m,4H)1.15-1.37(m, 1H)2.30(s,3H)2.56(s,3H)4.46(d, J=7.0Hz,2H)6.34-6.44(m,1H)7.16-7.28(m,1H)7.54-7.67(m,1H)8.09-8.24(m,2H) | ESI(Pos)367 $(M+H)^+$ | 114-115 |
| 46 | $^1$HNMR(200MHz,CHLOROFORM-d)dppm0.52-0.62(m,4H)1.14-1.30(m, 1H)2.43(s,3H)2.83(s,3H)4.63(d, J=6.6Hz,2H)7.18-1.29(m,1H)7.56-7.68(m,1H)8.09-8.22(m.1H)8.28(s,1H) | ESI(Pos)445 $(M+H)^+$ | 121-122 |
| 47 | $^1$HNMR(200MHz,CHLOROFORM-d)dppm0.48-0.73(m,4H)1.23-1.49(m, 1H)4.71(d,J=7.0Hz,2H)7.20-7.45 (m,2H)7.62-7.90(m,5H)8.03-8.26 (m,2H) | ESI(Pos)411 $(M+Na)^+$ | 125-126 |
| 48 | $^1$HNMR(200MHz,CHLOROFORM-d)dppm0.42-0.76(m,4H)1.33-1.52(m, 1H)4.23(d,J=7.0Hz,2H)7.06-7.13 (m,1H)7.26-7.37(m,1H)7.55-7.82 (m,5H)8.21-8.39(m,2H) | ESI(Pos)389 $(M+H)^+$ | - |
| 49 | $^1$HNMR(200MHz,CHLOROFORM-d)dppm0.27-0.55(m,4H)1.22-1.44(m, 1H)4.19(d,J=7.0Hz,2H)6.91-6.99 (m,1H)7.18-7.32(m,1H)7.51-7.82 (m,2H)8.34-8.43(m,2H) | ESI(Pos)340 $(M+H)^+$ | - |
| 50 | $^1$HNMR(200MHz,CHLOROFORM-d)dppm0.28-0.40(2H,m).0.57-0.69(2 H,m),1.21-1.39(1H,m),3.98(2H,d, J=7.47Hz),6.54-6.64(2H,m),7.45-7.73(4H,m),7.76-7.84(1H,m),8.38-8.46(1H,m) | ESI(Pos)356 $(M+H)^+$ | - |

(continued)

| Compound No. | $^1$H-NMR | Mass | m.p. (˚C) |
|---|---|---|---|
| 51 | $^1$HNMR(200MHz,CHLOROFORM-d)dppm0.42-0.69(m,4H)1.19-1.33(m,1 H)1.37(s,9H)4.12(d,J=1.0Hz,2H) 7.17(s,1H)7.18-7.28(m,1H)7.57-7.68(m,1H)8.12-8.23(m,1H) | ESI(Pos)342 (M+H)+ | - |
| 52 | $^1$HNMR(200MHz,CHLOROFORM-d)dppm0.56-0.69(m,4H)1.20-1.37(m, 1H)1.42(d,J=6.6Hz.6H)3.13-3.31 (m,1H)4.24(d.J=7.0Hz.2H)7.26-7.39(m,1H)7.67-7.80(m,1H)8.27-8.40(m,1H) | ESI(Pos)388 $(M+H)^+$ | - |
| 53 | $^1$HNMR(200MHz,CHLOROFORM-d)dppm0.41-0.73(m,4H)1.17-1.41(m, 1H)1.36(s,9H)4.17(d.J=7.0Hz,2H) 7.17(s,1H)7.46-7.57(m,1H)7.65-7.73(m,1H)8.38-8.46(m,1H)8.48-8.57(m,1H) | ESI(Pos)367 $(M+H)^+$ | - |
| 54 | $^1$HNMR(200MHz,CHLOROFORM-d)dppm0.42-0.70(m,4H)1.21-1.32(m, 1H)1.36(s,9H)4.13(d.J=7.0Hz,2H) 7.11-7.23(m,1H)7.19(s,1H)7.56-7.67(m,1H)8.29-8.37(m,1H) | ESI(Pos)385 $(M+H)^+$ | - |
| 55 | $^1$H NMR (200 MHz, CHLOROFORM-d) d ppm 0.38 - 0.69 (m, 4 H) 1.20 - 1.34 (m, 1 H) 1.35 (s, 9 H) 3.93 (s, 3 H) 4.09 (d, J=7.5 Hz, 2 H) 6.95 - 7.03 (m, 1 H) 7.23 (s, 1 H) 7.54 - 7.62 (m, 1 H) 8.05 - 8.12 (m, 1 H) | ESI(Pos) 397 $(M+H)^+$ | 64-65 |
| 56 | $^1$H NMR (200 MHz, CHLOROFORM-d) d ppm 0.41 - 0.55 (m, 2 H) 0.67 - 0.81 (m, 2 H) 1.20 - 1.35 (m, 1 H) 1.43 (s, 9 H) 4.01 (d, J=7.0 Hz, 2 H) 7.16 - 7.32 (m, 1 H) 7.56 - 7.70 (m, 1 H) 7.85 (s, 1 H) 8.16 - 8.29 (m, 1 H) | ESI(Pos) 401 $(M+H)^+$ | 85-87 |
| 57 | $^1$H NMR (200 MHz, CHLOROFORM-d) d ppm 0.44 - 0.58 (m, 2 H) 0.68 - 0.83 (m, 2 H) 1.21 - 1.38 (m, 1 H) 1.43 (s, 9 H) 4.05 (d, J=7.5 Hz. 2 H) 7.52 (t, J=7.5 Hz, 1 H) 7.69 (d, J=7.5 Hz, 1 H) 7.86 (s, 1 H) 8.46 (d, J=7.5 Hz, 1 H) 8.57 (s, 1 H) | ESI(Pos) 383 $(M+H)^+$ | 54-55 |
| 58 | $^1$H NMR (200 MHz, CHLOROFORM-d) d ppm 0.67 - 0.80 (m, 2 H) 1.19 - 1.38 (m, 1 H) 1.43 (s, 9 H) 4.02 (d, J=7.0 Hz, 2 H) 7.09 - 7.28 (m, 1 H) 7.55 - 7.68 (m, 1 H) 7.88 (s, 1 H) 8.33 - 8.43 (m, 1 H) | ESI(Pos) 401 $(M+H)^+$ | 63-65 |
| 59 | $^1$H NMR (200 MHz, CHLOROFORM-d) d ppm 0.40 - 0.53 (m, 52 H) 0.64 - 0.80 (m, 2 H) 1.18 - 1.36 (m, 1 H) 1.42 (s, 9 H) 3.94 (s, 3 H) 3.97 (d, J=7.5 Hz, 2 H) 6.95 -7.05 (m, 1 H) 7.52 - 7.63 (m, 1 H) 7.91 (s, 1 H) 8.11 - 8.17 (m, 1 H) | ESI(Pos) 413 $(M+H)^+$ | 95-97 |

Table 9

| Compound No. | H-NMR | m.p. (˚C) |
|---|---|---|
| 60 | 1H NMR (200 MHz, CHLOROFORM-D) d ppm 0.33 - 0.84 (m, 4 H) 1.14 - 1.50 (m, 1 H) 4.15 (d, J=7.5 Hz, 2 H) 6.76 (d, J=4.8 Hz. 1 H) 7.12 - 7.39 (m. 2 H) 7.63 -7.75 (m, 1 H) 8. 26 - 8. 39 (m. 1 H) | 96-97 |
| 61 | 1H NMR (200 MHz, CHLOROFORM-D) d ppm 1.16 (t, J=7. 0 Hz, 3 H), 3. 49 (q. J=7.0 Hz, 2 H), 3. 81 (t, J=4. 8 Hz, 2 H), 4.47 (t, J=4. 8 Hz, 2 H). 6. 70 (d, J=4.4 Hz, 1 H), 7.17 - 7.36 (m, 2 H). 7.62 - 7.76 (m, 1 H). 8.25 - 8.37 (m, 1 H) | 199-201 |
| 62 | 1H NMR (200 MHz, CHLOROFORM-D) d ppm 1.41 (t, J=7.0 Hz, 3 H). 2. 38 (s, 3 H). 4.32 (q, J=7.0 Hz, 2 H), 6.36 (s, 1 H). 7.27 (t, J=7. 7 Hz, 1 H), 7.68 (t, J=7.7 Hz, 1 H), 8.33 (t, J=7.7 Hz. 1 H) | 48-50 |
| 63 | 1H NMR (200 MHz. CHLOROFORM-D) d ppm 0.38 - 0. 73 (m. 4 H) 1.13 - 1.42 (m, 1 H) 2. 41 (s, 3 H) 4. 20 (d. J=7.0 Hz, 2 H) 6. 38 (s, 1 H) 7. 21 - 7.33 (m. 1 H) 7. 63 - 7.74 (m. 1 H) 8. 26 - 8. 36 (m. 1 H) | 109-111 |
| 64 | 1 H NMR (200 MHz. CHLOROFORM-D) d ppm 2.33 (s, 3 H) . 3.81 (s, 3 H) , 6. 71 (s, 1 H) , 7. 32 - 7. 45 (m, 1 H), 8. 51 - 8. 59 (m, 1 H), 8. 65 - 8. 74 (m. 1 H). 9.51 -9. 59 (m. 4 H) | 173-174 |
| 65 | 1H NMR (200 MHz, CHLOROFORM-D) d ppm 1.47 (t, J=7.0 Hz. 3 H), 2. 35 (s, 3 H). 4. 35 (q, J=7.0 Hz, 2 H) , 6.78 (s, 1 H) , 7.29 (t, J=7. 7 Hz. 1 H) , 7.69 (t, J=7.7 Hz, 1 H), 8. 30 (t, J=7.7 Hz, 1 H) | 90-92 |
| 66 | 1H NMR (200 MHz, CHLOROFORM-D) d ppm 0.38 - 0. 77 (m, 4 H) . 1.18 - 1.43 (m, 1 H), 2. 35 (s, 3 H), 4.11 (d, J=7.5 Hz, 2 H). 6. 84 (s, 1 H), 7.31 - 7.42 (m, 1 H), 8.47 - 8.56 (m, 1 H), 8.62 - 8. 74 (m, 1 H), 9.48 - 9.54 (m, 1 H) | 115-117 |
| 67 | 1H NMR (200 MHz, CHLOROFORM-D) d ppm 0.39 - 0.76 (m, 4 H) 1.18 - 1. 42 (m, 1 H) 2.32 - 2. 39 (m, 3 H) 4, 08 (d, J=7. 0 Hz, 2 H) 6. 83 - 6. 89 (m. 1 H) 7.19 - 7. 34 (m, 1 H) 7.60 - 7. 75 (m, 1 H) 8.22 - 8.37 (m, 1 H) | 129-130 |
| 68 | 3. 82 (s, 3 H) 7. 55 (t, 1H NMR (200 MHz, CHLOROFORM-D) d ppm 2. 26 (m, 6 H) 3.82 (s, 3 H) 7.55 (t, J=7.9 Hz, 1 H) 7.71 (d, J=7. 9 Hz, 1 H) 8.50 (d, J=7.9 Hz, 1 H) 8.61 (s, 1 H) | 110-111 |
| 69 | 1H NMR (200 MHz, CHLOROFORM-D) d ppm 2.26 (s, 6 H) 3.78 (s, 3 H) 7.21 - 7.33 (m, 1 H) 7.62 - 7. 72 (m, 1 H) 8.28 - 8. 39 (m, 1 H) | 182-183 |
| 70 | 1H NMR (200 MHz, CHLOROFORM-D) d ppm 1.39 (t, J=7.0 Hz, 3 H) 2.26 (s, 3 H) 2.26 (s, 3 H) 4. 30 (q, J=7.0 Hz. 2 H) 7. 16 - 7. 35 (m, 1 H) 7.55 - 7. 76 (m, 1 H) 8.14 - 8.47 (m, 1 H) 1H | 198-200 |
| 71 | 1H NMR (300 MHz, CHLOROFORM-D) d ppm 0.47 - 0.61 (m. 4 H) 1.22 - 1. 33 (m, 1 H) 1.26 (t. J=7.5 Hz. 3 H) 2.24 (s, 3 H) 2. 27 (s, 3 H) 3.11 (q, J=7.5 Hz, 2 H) 4.14 (d. J=6.8 Hz. 2 H) 7.19 - 7.27 (m, 2 H) 7.30 - 1. 37 (m, 1 H) 7. 97 - 8.02 | 150-152 |

(continued)

| Compound No. | H-NMR | m.p. (˚C) |
|---|---|---|
| 72 | 1H NMR (200 MHz, CHLOROFORM-D) d ppm 0.53 - 0.65 (m. 4 H), 1.18 - 1. 41 (m, 1 H), 2.26 (s, 3H). 2.29 (s, 3 H), 4.21 (d, J=7. 0 Hz, 2 H), 7. 54 (t, J=7.9 Hz, 1H). 7. 70 (d. J=7.9 Hz, 1 H) 8.44 (d, J=7.9 Hz. 1 H) . 8. 56 (s, 1 H) | 197-199 |
| 73 | 1H NMR (300 MHz, CHLOROFORM-D) d ppm 0.46 - 0. 60 (m. 4 H) 1. 21 - 1.37 (m, 1H) 2.23 (s, 3H) 2.26 (s, 3 H) 2.34 (s, 3 H) 2.69 (s, 3 H) 4.15 (d, J=7.0 Hz, 2 H) 7.02 - 7.08 (m. 2 H) 8.03 - 8.08 (m, 1 H) | 146-148 |
| 74 | 1H NMR (300 MHz, CHLOROFORM-D) d ppm 0.48 - 0.60 (m, 4 H). 1.22 - 1. 33 (m, 1 H), 2.26 (s. 3 H), 2.28 (s, 3 H), 4.16 (d, J=7.0 Hz, 2 H), 7.18 - 7.25 (m, 1 H), 7.29 - 7.38 (m, 1 H). 7.59 - 7.66 (m, 1H), 7.87 - 7.94 (m, 1 H) | 125-127 |
| 75 | 1H NMR (300 MHz, CHLOROFORM-D) d ppm 0.46 - 0.62 (m, 4 H). 1.20 - 1.35 (m, 1 H). 2.25 (s, 3 H). 2.28 (s, 3 H), 4. 15 (d, J=6.8 Hz, 2 H), 7.23 - 7.34 (m, 2 H). 7.37 - 7.44 (m, 1 H). 7.90 - 8. 00 (m.1H) | 125-127 |
| 76 | 1H NMR (300 MHz, CHLOROFORM-D) d ppm 0.43 - 0. 58 (m, 4 H). 1.18-1.29 (m, 1 H),2.25(s,3H).2.28(s,3H),4.12(d,J=7.0Hz, 2H),7.43-7.52(m, 1 H),7.52-7.61(m,1H),7.67-7.74(m,1H).7.81-7.87(m,1H) | 114-116 |
| 77 | 1H NMR (300 MHz, CHLOROFORM-D) d ppm 0.52 - 0.64 (m, 4 H), 1.21 - 1.36 (m, 1 H) . 2.26 (s, 3 H), 2. 29 (s, 3 H), 4.20 (d, J=7.0 Hz, 2 H), 7.26 - 7.35 (m, 1 H), 7.55 - 7. 61 (m, 1 H). 8.17 - 8.22 (m, 1 H). 8.40 - 8.43 (m, 1 H) | 159-160 |
| 78 | 1H NMR (300 MHz, CHLOROFORM-D) d ppm 0.47 - 0.62 (m, 4 H). 1.21 - 1.35 (m, 1 H) , 2.25 (s, 3 H), 2.28 (s, 3 H), 4.18 (d, J=6.8 Hz, 2 H) , 7.69 - 7.06 (m, 1 H), 7.33 - 7. 40 (m, 1H). 7.88 - 7. 99 (m, 2 H) | 130-132 |
| 79 | 1H NMR (300 MHz, CHLOROFORM-D) d ppm 0.53 - 0. 61 (m, 4 H), 1. 22 - 1. 32 (m, 1 H),2.26(s,3H),2.29(s,3H),4.18(d,J=7.1Hz, 2H),7.06-7.16(m,1 H).7.41-7.50(m,1H),7.94-8.03(m,1H) | 109-111 |
| 80 | 1H NMR(300MHz,CHLOROFORM-D)dppm0.46-0.62(m,4H),1.21-1.32(m,1 H),2.26(s,3H),2.28(s,3H),4.16(d,J=7.0Hz,2H), 6.97-7.07(m,1 H).7.32-7.41(m.1H),7.63-7.72(m,1H) | 139-140 |
| 81 | 1H MMR(300MHz,CHLOROFORM-D)dppm0.46-0.61(m,4H),1.21-1.31(m,1 H),2.26(s,3H),2.29(s,3H),4.15(d,J=7.0Hz,2H), 7.24-7.31(m,1 H),7.41-7.46(m,1H),7.90-7.97(m,1H) | 57-58 |
| 82 | 1H NMR(200MHz,CHLOROFORM-D)dppm0.47-0.61(m,4H)1.14-1.38(m.1H) 2.26(s,3H)2.29(s,3H)4.15(d,J=7.0Hz,2H)7.22-7.37 (m.2H)7.90 -7.94(m.1H) | 113-115 |
| 83 | 1H NMR(300MHz,CHLOROFORM-D)dppm0.52-0.59(m,4H),1.18-1.32(m,1 H),2.26(s,3H),2.29(s,3H),4.17(d,J=6.8Hz,2H), 7.26-7.35(m,2 H),7,96-8.06(m,1H) | 154-155 |

(continued)

| Compound No. | H-NMR | m.p. (˚C) |
|---|---|---|
| 84 | 1H NMR(200MHz.CHLOROFORM-D)dppm0.48-0.60(m,4H),1.19-1.34(m,1 H),2.25(s,3H),2.28(s,3H),4.16(d.J=7.0Hz,2H). 6.98-7.11(m,1 H)7.32-7.41(m,1H)7.94-8.05(m,1H) | 144-146 |
| 85 | 1H NMR(300MHz,CHLOROFORM-D)dppm0.46-0.63(m,4H)1.19-1.33(m,1H) 2.26(s,3H)2.29(s,3H)4.16(d.J=7.0Hz.2H)7.21-7.29 (m,1H)7.85 -7.94(m,1H) | 161-163 |
| 86 | 1HNMR(300MHz,CHLOROFORM-D)dppm0.46-0.62(m,4H),1.26(s,1H), 2.26(s,3H),2.29(s,3H),4.15(d,J=7.0Hz,2H),7.44-7.51(m, 1H), 7.78-7.85(m,1H) | 59-60 |
| 87 | 1H NMR(200MHz,CHLOROFORM-D)dppm0.44-0.58(m,4H).1.14-1.33(m,1H).2.25(s,3H),2.28(s,3H),4.15(d.J=7.0Hz.2H), 7.28-7.48(m,3 H),8.06(dd,J=7.5.1.8Hz.1H) | |
| 88 | 1H NMR(300MHz,CHLOROFORM-D)dppm0.52-0.60(m,4H).1.20-1.35(m,1 H),2.24(s,3H),2.27(s,3H),3.81(s,3H),3.87(s,3H),4.14 (d,J=7.0 Hz, 2 H), 6.88- 6.97 (m, 2 H). 7.56- 7.61 (m, 1 H) | 124-126 |
| 89 | 1H NMR(300MHz,CHLOROFORM-D)dppm0.47-0.62(m,4H),1.20-1.36(m,1 H),2.25(s,3H),2.28(s,3H),2.64-2.67(m,3H),4.16(d, J=6.9Hz,2 H),6.95-7.05(m,1H),7.11-7.20(m,1H),7.77-7.86(m,1H) | 82-84 |
| 90 | 1H NMR(300MHz,CHLOROFORM-D)dppm0.46-0.61(m,4H),1.20-1.34(m,I H),2.24(s,3H),2.28(s,3H),2.69(s,3H),4.14(d,J=6.8Hz, 2H),7.18 -7.23(m.2H).8.03-8.10(m,1H) | 124-125 |
| 91 | 1H NMR(300MHz,CHLOROFORM-D)dppm0.47-0.64(m,4H)1.20-1.34(m,1H) 2.24(s,3H)2.27(s,3H)2.35(s,3H)4.16(d,J=7.0Hz,2H) 7.11-7.17 (m,1H)7.45-7.47(m,1H)7.85-7.90(m,1H) | 90-92 |
| 92 | 1H NMR(300MHz,CHLOROFORM-D)dppm0.54-0.61(m,4H),1.20-1.32(m,1 H),2.27(s,3H),2.30(s,3H),4.19(d,J=7.1Hz,2H), 7.16-7.25(m,1 H),7.62-7.69(m,1H),8.41-8.48(m,1H) | 122-123 |
| 93 | 1H NMR(300MHz,CHLOROFORM-D)dppm0.42-0.59(m,4H),1.17-1.28(m,1 H),2.26(s,3H),2.29(s,3H),4.12(d,J=7.0Hz,2H). 7.10-7.19(m,1 H),7.51-7.59(m,1H),7.66-7.74(m,1H) | 131-133 |
| 94 | 1H NMR(300MHz,CHLOROFORM-D)dppm0.40-0.55(m,4H),1.13-1.24(m,1 H),2.26(s,3H),2.28(s,3H),4.07(d,J=7.0Hz,2H), 7.23-7.31(m,1 H),7.35-7.48(m,2H) | 111-113 |
| 95 | 1H NMR(300MHz,CHLOROFORM-D)dppm0.43-0.59(m,4H),1.15-1.30(m, H),2.26(s,3H),2.28(s,3H),4.12(d,J=7.1Hz,2H), 7.20-7.30(m,1 H),7.37-7.45(m,1H),7.88-7.97(m,1H) | 143-144 |
| 96 | 1H NMR(300MHz,CHLOROFORM-D)dppm0.51-0.61(m,4H),1.20-1.34(m,1 H),2.24(s,3H),2.27(s,3H).3.89(s,3H),4.14(d,J=7.0Hz, 2H),6.86 -6.94(m,1H),7.02-7.11(m,1H),7.66-7.74(m,1H) | 107-109 |
| 97 | 1H NMR(300MHz,CHLOROFORM-D)dppm0.52-0.59(m,4H),1.19-1.34(m,1 H),2.24(s,3H),2.27(s,3H),3.89(s,3H),4.14(d,J=7.0Hz, 2H),6.86 -6.93(m,1H),7.27-7.35(m,1H),7.9,1-7.96(m,1H) | 121-123 |

(continued)

| Compound No. | H-NMR | m.p. (˚C) |
|---|---|---|
| 98 | 1H NMR(300MHz,CHLOROFORM-D)dppm0.46-0.54(m,4H),0.87(t,J=7.5Hz, 6H),1.11-1.22(m,1H),1.46-1.78(m,4H),2.19(s,3H), 2.23(s,3 H),2.25-2.35(m,1H),4.06(d,J=7.0Hz,2H) | 67-69 |
| 99 | 1H NMR(300MHz,CHLOROFORM-D)dppm0.48-0.56(m.4H)0.91(s,9H)0.97 (d.J=6.4Hz,3H)1.07-1.37(m.4H)2.19(s,3H)2.21-2.55 (m,2H) 2.23(s.3H)4.05(d,J=7.2Hz.2H) | 42-43 |
| 100 | 1H NMR(300MHz,CHLOROFORM-D)dppm0.45-0.57(m,4H)1.09-1.29(m,4H) 1.44-1.68(m,3H)1.74-1.89(m,2H)2.19(s,3H)2.23(s. 3H)2.27-2.44(m,1H)2.45-2.52(m,2H)4.04(d.J=7.0Hz.2H) | 108-109 |
| 101 | 1H NMR(300MHz,CHLOROFORM-D)dppm0.51-0.58(m,4H),1.11-1.22(m,1 H),2.29(s,3H).2.32(s.3H).4.13(d,J=7.0Hz.2H) | 92-94 |
| 102 | 1H NMR(300MHz,CHLOROFORM-D)dppm0.35-0.51(m,4H)0.90(t.J=7.3Hz,3 H)1.01-1.16(m,1H)1.79-2.31(m,2H)2.17(s.3H)2.20 (s,3H)3.59 (t,J=7.7Hz,1H)4.00(d,J=7.2Hz,2H)7.13-7.20(m,1H)7.22-7.30 (m,2H)7.33-7.44(m,2H) | 108-110 |
| 103 | 1H NMR(200MHz,CHLOROFORM-D)dppm2.25(s,3H)2.28(s,3H)3.33(s,3H) 3.81(t,J=5.3Hz,2H)4.42(t,J=5.3Hz,2H)7.54(t, J=7.5Hz,1H)7.71 (d.J=7.5Hz,1H)8.46(d,J=7.5Hz,1H)8.56(s,1H) | 64-66 |
| 104 | 1H NMR(200 MHz,CHLOROFORM-D)dppm2.25(s,3H)2.28(s,3H)3.31(s,3H) 3.79(t,J=5.3Hz,2H)4.37(t,J=5.3Hz,2H)7.09-7.38(m, 1H)7.53-7.82(m,1H)8.16-8.39(m,1H) | 182-183 |
| 105 | 1H NMR(200MHz,CHLOROFORM-D)dppm2.01-2.19(m,2H),2.26(s,6H), 3.33(s,3H),3.43(t,J=5.7Hz,2H),4.31(t,J=5.7Hz,2H), 7.20-7.33 (m,1H).7.60-7.75(m.1H),8.25-8.40(m.1H) | 104-106 |
| 106 | 1H NMR(200MHz,CHLOROFORM-D)dppm1.15(t,J=7;0Hz,3H)2.26(s,3H) 2.29(s,3H)3.48(q.J=7.0Hz,2H)3.84(t,J=5.5Hz,2H)4.42 (t,J=5.5 Hz,2H)7.54(t.J=7.5Hz,1H)7.71(d,J=7.5Hz,1H)8.46(d.J=7.5Hz,1 | 138-140 |
| 107 | 1H NMR(200MHz,CHLOROFORM-D)dppm1.13(t,J=6.7Hz,3H)2.26(s,3H) 2.29(s,3H)3.45(q,J=6.7Hz,2H)3.82(t,J=5.3Hz,2H)4.37 (t,J=5.3 Hz,2H)7.10-7.31(m,1H)7.51-7.81(m,1H)8.06-8.42(m,1H) | 147-148 |
| 108 | 1H NMR(300MHz,CHLOROFORM-D)dppm1.25(t,J=7.6Hz,3H).1.40(t,J=7.1 Hz,3H).2.27(s,3H).2.67(q,J=7.6,0.5Hz,211):4.30(q, J=7.1Hz,2 H).7.22-7,30(m,1H),7.63-7.70(m,1H).8.27-8.35(m,1H) | 115-117 |
| 109 | 1H NMR(300MHz,CHLOROFORM-D)dppm0.50-0.60(m,4H),1.18-1.36(m,1 H),1.27(t,J=7.5Hz,3H),2.30(s,3H).2.68(q,J=7.5Hz, 2H).4.19(d. J=7.1Hz,2H).7.27(t,J=7.0Hz,1H),7.67(t,J=7.0Hz,1H),8.29(t | 164-166 |
| 110 | 1H NMR(200MHz,CHLOROFORM-D)dppm1.29(d,J=6.6Hz,6H)2.27(s,3H) 3.08-3.28(m,1H)3.78(s,3H)7.21-7.33(m,1H)7.62-7.72 (m.1H) 7.21-7.33(m.1H) | 89-91 |
| 111 | 1H NMR(200MHz,CHLOROFORM-D)dppm1.29(d.J=7.0Hz,6H)1.40(t,J=7.0 Hz,3H)2.28(s,3H)3.02-3.31(m,1H)4.30(q,J=7.0Hz, 2H)7.20-7.32(m,1H)7.61-7.71(m.1H)8.26-8.36(m,1H) | 64-66 |

(continued)

| Compound No. | H-NMR | m.p. (˚C) |
|---|---|---|
| 112 | 1H NMR(200MHz,CHLOROFORM-D)dppm0.52-0.69(m,4H)1.09-1.42(m,1H) 1.30(d,J=7.0Hz,6H)2.32(s,3H)3.06-3.31(m,1H)4.22 (d,J=7.0Hz, 2H)7.55(t.J=7.5Hz,1H)7.70(d.J=7.5Hz,1H)8.44(d.J=7.5Hz,1H) | 89-91 |
| 113 | 1H NMR(200MHz,CHLOROFORM-D)dppm0.52-0.63(m,4H)1.15-1.38(m,1H) 1.30(d,J=7.0Hz,6H)2.32(s,3H)3.09-3.27(m,1H)4.19 (d,J=7.0Hz, 2H)7.19-7.34(m,1H)7.58-7.73(m.1H)8.20-8.36(m,1H) | 64-66 |
| 114 | 1H NMR(200MHz,CHLOROFORM-D)dppm1.29(d,J=7.0Hz.6H)2.30(s,3H) 3.07-3.26(m,1H)3.32(s,3H)3.79(t,J=5.3Hz,2H)4.37(t, J=5.3Hz, 2H)7.20-7.32(m.1H)7.61-7.72(m.1H)8.23-8.34(m,1H) | 132-134 |
| 115 | 1H NMR(200MHz,CHLOROFORM-D)dppm1.12(dd,J=7.0Hz,3H)1.29(d,J=6.6 Hz.6H)2.31(s,3H)3.09-3.26(m.1H)3.45(q.J=7.0Hz. 2H)382(t, J=5.3Hz,2H)4.38(t,J=5.3Hz.2H)7.20-7.32(m,1H)7.62-7.72(m,1 H)8.23-8.34(m.1H) | 96-97 |
| 116 | 1H NMR(200MHz,CHLOROFORM-D)dppm1.42(s,9H),2.38(s,3H),2.71(s,3 H),3.75(s,3H),7.17-7.35(m,3H),8.08-8.19(m,1H) | 173-175 |
| 117 | 1HNMR(200MHz,CHLOROFORM-D)dppm1.43(s,9H)2.39(s,3H)3.71(s,3H) 7.40-7.62(m,2H)7.67-7.75(m,1H)7.84-7.92(m,1H) | 74-76 |
| 118 | 1H NMR(200MHz,CHLOROFORM-D)dppm1.43(s,9H),2.40(s,3H),3.81(s,3 H),7.43-7.80(m.2H).8.38-8.68(m,2H) | 140-142 |
| 119 | 1H NMR(200MHz,CHLOROFORM-D)dppm1.43(s,9H)2.39(s,3H)3.76(s,3H) 7.20-7.36(m,2H)7.37-7.48(m,1H)7.91-8.04(m,1H) | 119-121 |
| 120 | 1HNMR(200MHz,CHLOROFORM-D)dppm1.43(s.9H)2.39(s,3H)3.76(s,3H) 7.15-7.39(m,1H)7.58-7.67(m.1H)7.90-7.99(m,1H) | 182-184 |
| 121 | 1H NMR(200MHz,CHLOROFORM-D)dppm1.42(s,9H),2.39(s,3H),3.79(s,3 H),7.20-7.43(m,1H).7.54-7.63(m,1H),8.17-8.29(m, 1H),8.42-8,52(m,1H) | 173-175 |
| 122 | 1H NMR(200MHz,CHLOROFORM-D)dppm1.43(s,9H)2.39(s,3H)3.79(s,3H) 6.98-7.10(m,1H)7.31-7.43(m,1H)7.91-7.99(m,2H) | 83-85 |
| 123 | 1H NMR(200MHz,CHLOROFORM-D)dppm1.43(s,9H),2.39(s,3H).3.74(s,3 H),7.22-7.51(m.3H).8.06-8.17(m,1H) | |
| 124 | 1H NMR(200MHz,CHLOROFORM-D)dppm1.42(s,9H),2.38(s,3H),3.00(s,6 H).3.79(s.3H),6.81-6.91(m,1H),7.24-7.35(m,1H), 7.70-7.80(m, | 189-190 |
| 125 | 1H NMR(200MHz,CHLOROFORM-D)dppm1.44(s,9H),2.42(s,3H),3.82(s,3 H),7.47-7.59(m,1H),7.67-7.78(m,1H),8.46-8.55(m, 1H).8.62-8.68(m.1H) | 131-133 |

(continued)

| Compound No. | H-NMR | m.p. (˚C) |
|---|---|---|
| 126 | 1H68NMR(200MHz.CHLOROFORM-D)dppm1.43(s,9H)2.40(s,3H)3.83(s,3H) 3.94(s,3H)7.50(t,J=7.9Hz,1H)8.07-8.21(m,1H) 8.44-8.58(m,1 H)8.99(t,J=1,5Hz,1H) | 192-193 |
| 127 | 1H NMR(200MHz,CHLOROFORM-D)dppm1.44(s,9H)2.41(s,3H)3.84(s,3H) 7.55(t,J=7.9Hz,1H)8.20(d,J=7.9Hz,1H)8.55(d, J=7.9Hz,1H)9.07 | 250-252 |
| 128 | 1HNMR(200MHz,CHLOROFORM-D)dppm1.42(s,9H),2.40(s.3H),3.77(s,3 H).7.04-7.28(m.2H),7.88(t.J=6.8Hz,1H) | 176-178 |
| 129 | 1H NMR(200MHz,CHLOROFORM-D)dppm1.43(s,9H)2.40(s,3H)3.78(s,3H) 7.05-7.17(m,1H)7.39-7.52(m,1H)7.95-8.07(m.1H) | 203-205 |
| 130 | 1H NMR(200MHz,CHLOROFORM-D)dppm1.43(s,9H)2.40(s,3H)3.76(s,3H) 6.95-7.09.(m,1H)7.31-7.43(m,1H)7.68-7.78(m,1H) | 125-126 |
| 131 | 1H NMR(200MHz,CHLOROFORM-D)dppm1.43(s,9H)2.40(s,3H)3.76(s,3H) 6.88-7.02(m,1H)7.52-7.63(m.1H)7.65-7.75(m,1H) | 161-162 |
| 132 | 1H NMR(200MHz,CHLOROFORM-D)dppm1.42(s,9H)2.40(s,3H)3.77(s,3H) 6.93-7.06(m,1H)7.43-7.54(m.1H)8.23-8.32(m,1H) | 144-145 |
| 133 | 1H NMR(200MHz.CHLOROFORM-D)dppm1.43(s,9H)2.40(s,3H)3.76(s,3H) 7.21-7.39(m.2H)7.96-8.01(m.1H) | 171-173 |
| 134 | 1H NMR(200MHz,CHLOROFORM-D)dppm1.43(s,9H)2.39(s,3H)3.72(s,3H) 6.72-6.94(m.1H)6.98-7.21(m,1H) | 143-145 |
| 135 | 1H NMR(200MHz,CHLOROFORM-D)dppm1.43(s,9H)2.40(s,3H)3.76(s,3H) 7.19-7.31(m,1H)7.88-8.02(m,1H) | 145-146 |
| 136 | 1H NMR(200MHz,CHLOROFORM-D)dppm1.43(s,9H)2.40(s,3H)3.76(s,3H) 7.44-7.51(m,1H),7.83-7.92(m,1H) | 205-207 |
| 137 | 1H NMR(200MHz.CHLOROFORM-D)dppm1.44(s,9H).2.45(s,3H),3.46(s,3 H) 3.80(s.3H),3.85(s,3H).6.46-6.60(m,2H).7.30(s,1H) | 138-140 |
| 138 | 1H NMR(200MHz,CHLOROFORMS)dppm1.43(s,9H)2.40(s.3H)2.G6(s, 3 H) 3.76(s.3H)6.92-7.06(m,1H)7.10-7.21(m.1H) 7.81-7.92(m.1H) | 145--146 |
| 139 | 1H NMR(200MHz,CHLOROFORM-D)dppm1.43(s,9H)2.39(s,3H)2.66(s,3H) 3.73(s,3H)7.09-7.20(m,1H)7.35-7.44(m,1H) 7.77-7.85(m,1H) | 161-162 |
| 140 | 1H NMR(200MHz,CHLOROFORM-D)dppm1.43(s,9H)2.39(s,3H)2.68(s,3H) 3.73(s.3H)7.01-7.12(m,1H7.54-7.62(m,1H)7.79-7.87 (m,1H) | 143-144 |
| 141 | 1HNMR(200MHz,CHLOROFORM-Ddppm1.43(s,9H).2.40(s,3H),3.78(s,3 H).7.21-7.32(m,1H).7.66(t,J=6.4Hz,1H),8.31 (t.J=6.6Hz.1H) | 176-178 |
| 142 | 1HNMR(200MHz,CHLOROFORM-D)dppm1.43(s,9H),2.41(s,3H),3.78(s,3 H).7.15-7.29(m,1H).7.60-7.70(m,1H).8.46(dd, J=6.6,1.8Hz.1H) | 140-142 |
| 143 | 1HNMR(200MHz.CHLOROFORM-D)dppm1.43(s,9H)2.40(s,3H)3.72(s,3H) 7.05-7.23(m.1H)7.55-7.65(m.1H)7.65-7.76(m,1H) | 83-85 |

(continued)

| Compound No. | H-NMR | m.p. (˚C) |
|---|---|---|
| 144 | 1HNMR(200MHz.CHLOROFORM-D)dppm1.43(s,9H)2.24(s,3H)2.38(s,3H) 3.71(s.3H)6.73-6.86(m.1H)6.98-7.20(m1H) | 140-142 |
| 145 | 1H NMR(200MHz,CHLOROFORM-D)dppn1.44(s,9H)2.39(s,3H)3.67(s,3H) 7.31-7.64(m.2H) | 129-130 |
| 146 | 1H NMR(200MHz,CHLOROFORM-D)dppm1.41(s.9H)2.38(s,3H)3.74(s,3H) 3.90(s,3H)6.85-6.94(m,1H)7.26-7,36(m,1H) 7.96-8.02m,1H) | 177-179 |
| 147 | 1H NMR(200MHz.CHLOROFORM-D)dppm1.41(s,9H)2.38(s,3H)3.74(s,3H) 3.91(s,3H)6.89-7.02(m,2H)7.96-8.05(m,1H) | 117-119 |
| 148 | 1H NMR(200MHz,CHLOROFORM-D)dppm1.46(s,9H),2.46(s,3H),3.85(s,3 H),7.92-8.03(m,1H),8.75(d,J=5.7Hz,1H),9.18(d, J=7.9Hz,1H), | 202-203 |
| 149 | 1H NMR(200MHz,CHLOROFORM-D)dppm1.42(s,9H)2.41(s,3H)3.74(s,3H) | 180-182 |
| 150 | 1H NMR(200MHz,CHLOROFORM-D)dppm1.28(t,J=7.7Hz,3H),1.37(t,J=7.3 Hz,3H),1.43(s,9H),2.40(s,3H),3.14(q.J=7.7Hz,2H), 4.28(q. J=7.3Hz,2H),7.17-7.38(m,3H).7.98-8.08(m.1H) | |
| 151 | 1H NMR(200MHz,CHLOROFORM-D)dppm1.34(t,J=7.0Hz,3H)1.43(s,9H) 2.40(s,3H)4.26(q,J=7.0Hz,2H)7.40-7.62(m,2H) 7.67-7.75(m,1H) 7.84-7.92(m,1H) | 74-76 |
| 152 | 1H NMR(200MHz,CHLOROFORM-D)dppm1.36-1.52(m.12H),2.42(s.3H). 4.35(q,J=7.0Hz.2H).7.38-7.85(m,**2**H).8.32-8.70(m,2H) | 158-160 |
| 153 | 1H NMR(200MHz,CHLOROFORM-D)dppm1.35(t,J=7.3Hz.3H)1.42(s,9H) 2.40(s.3H)4.30(q,J=7.3Hz,2H)7.23-7.48(m,3H) 8.06-8.15(m,1 | 104-105 |
| 154 | 1H NMR(200MHz,CHLOROFORM-D)dppm1.39(t,J=7.0Hz,3H)1.42(s,9H) 2.41(s,3H)4.31(q,J=7.0Hz,2H)6.92-7.06(m,1H) 7.42-7.54(m,1 H)8.20-8.29(m,1H) | 105-106 |
| 155 | 1H NMR(200MHz,CHLOROFORM-D)dppm1.38(t,J=7.2Hz,3H)1.43(s,9H) 2.41(s,3H)4.30(q.J=7.2Hz,2H)6.92-7.02(m,1H) 7.52-7.63(m,1 H)7.63-7,74(m,1H) | 115-116 |
| 156 | 1H NMR(200MHz,CHLOROFORM-D)dppm1.38(t,J=7.0Hz.3H)1.43(s,9H) 2.41(s,3H)4.30(q,J=7.0Hz,2H)6.98-7.11(m.1H) 7.32-7.42(m,1 H)7.97-8.09(m,1H) | 99-100 |
| 157 | 1H MR(200MHz,CHLOROFORM-D)dppm1.39(t,J=7.2Hz,3H)1.42(s.9H) 2.41(s,3H)4.30(q,J=7.2Hz,2H)6.80-6.94(m,1H)7.60-7.72 (m,1 H)8.38-8.47(m,1H) | 87-89 |
| 158 | 1H NMR(300MHz,CHLOROFORM-D)dppm1.38(t,J=7.2Hz,3H)1.43(s,9H) 2.42 (s,3H)4.30(q,J=7.2Hz,2H)7.23-7.31(m,1H) 7.31-7.38(m,1 H)7.94-7.99(m,1H) | 127-128 |
| 159 | 1H NMR(200MHz,CHLOROFORM-D)dppm1.38(t,J=7.2Hz,3H)1.43(s,9H) 2.42(s,3H)4.29(q,J=7.2Hz.2H)7.16-7.33(m.1H) 7.86-8.00(m,1 | 107-108 |

(continued)

| Compound No. | H-NMR | m.p. (˚C) |
|---|---|---|
| 160 | 1H NMR(300MHz.CHLOROFORM-D)dppm1.38(t,J=7.1Hz,3H).1.43(s,9H); 2.42(s,3H).4.29(q,J=7.1Hz.2H).7.45-7.50(m.1H). 7.83-7.89(m | 112-114 |
| 161 | 1H NMR(200MHz.CHLOROFORM-D)dppm1.39(t,J=7.0Hz,3H)1.43(s.9H) 2.41(s.3H)2.67(s,3H)4.30(q,1=7.0Hz.2H)6.92-7.06(m. 1H)7.10-7.21(m.1H)7.80-7.91(m,1H) | 108-109 |
| 162 | 1H NMR(200MHz,CHLOROFORM-D)dppm1.38(t,J=7.0Hz,3H),1.42(s,9H), 2.40(s.3H).2.70(s.3H),4.29(q,J=7.0Hz,2H),7.15-7.24 (m.2H), 8.06-8.13(m,1H) | 125-126 |
| 163 | 1H NMR(200MHz,CHLOROFORM-D)dppm1.37(t,J=7.3Hz,3H).1.42(s,9H), 2.34(s,3H),2.40(s,3H),4.30(q,J=7.3Hz,2H),7.09-7.17 (m,1H), 7.44-7.49(m,1H),7.86-7.93(m,1H) | 100-102 |
| 164 | 1H MR(200MHz,CHLOROFORM-D)dppm1.34(t,J=7.2Hz,3H)1.43(s,9H) 2.41(s,3H)4.27(q,J=7.2Hz,2H)7.07-7.21(m,1H)7.54-7.64 (m,1 H)7.65-7.76(m,1H) | 103-104 |
| 165 | 1H NMR(200MHz,CHLOROFORM-D)dppm1.39(t,J=7.0Hz,3H)1.43(s,9H) 2.42(s,3H)4.32(q,J=7.0Hz,2H)7.20-7.32(m,1H) 7.61-7.72(m,1 H)8.25-8.36(m,1H) | 97-99 |
| 166 | 1H NMR(200MHz,CHLOROFORM-D)dppm1.39(t,J=7.2Hz,3H)1.42(s,9H) 2.41(s,3H)4.30(q,J=7.2Hz,2H)6.80-6.94(m,1H) 7.60-7.72(m,1 H)8.38-8.47(m,1H) | 113-115 |
| 167 | 1H NMR(200MHz,CHLOROFORM-D)dppm1.37(t,J=7.3Hz.3H)1.41(s.9H) 2.40(s,3H)3.90(s,3H)4.28(q,J=7.3Hz,2H)6.85-6.94(m, 1H)7.26 -7.36(m,1H)7.94-7.99(m,1H) | 108-109 |
| 168 | 1H NMR(200MHz,CHLOROFORM-D)dppm1.37(t,J=7.0Hz,3H),1.41(s,9H), 2.39(s,3H),3.91(s,3H),4.27(q,J=7.0Hz,2H),6.91-6.99 (m,2H), 7.96-8.03(m,1H) | |
| 169 | 1H NMR(200MHz,CHLOROFORM-D)dppm1.42(t,J=7.2Hz,3H)1.43(s,9H) 2.42(s, 3H)4.35(q,J=7.2Hz,2H)7.37(dd,J=7.9.5.3Hz, 1H)8.52(d, J=7.9Hz,1H)8.67(d.J=5.3Hz,1H)9.53(s,1H) | |
| 170 | 1H NMR(300MHz,CHLOROFORM-D)dppm1.36(t,J=7.2Hz,3H)1.44(s,9H) 2.42(s,3H)4.28(q,J=7.2Hz,2H)7.59(d,J=5.3Hz,1H)8.78 (d,J=5.3 Hz,1H)9.25(s,1H) | 110-112 |
| 171 | 1H NMR(300MHz,CHLOROFORM-D)dppm1.14-1.35(m,2H)1.30(t,J=7.1Hz,3 H)1.38(s,9H)1.47-1.69(m,4H)1.77-1.90(m,2H) 2.31-2.45(m.1 H)2.35(s,3H)2.48-2.53(m,2H)4.18(q,J=7.1Hz,2H) | 149-150 |
| 172 | 1H NMR(300MHz,CHLOROFORM-D)dppm1.34(t,J=7,1Hz,3H),1.42(s,9H), 2.42(s,3H),4.27(q,J=7,1Hz,2H) | 113-115 |

(continued)

| Compound No. | H-NMR | m.p. (˚C) |
|---|---|---|
| 173 | 1H NMR(200MHz.CHLOROFORM-d)dppm1.32-1.46(m,2H)2.42(s,3H)2.76 (s,3H)4.30(q,J=7.3Hz,2H)7.26-7.35(m,1H)7.47-7.57 (m,1H) 8.37-8.43(m,1H) | 63-65 |
| 174 | 1H NMR(200MHz,CHLOROFORM-d)dppm1.37(t.J=7.3Hz,3H)1.43(s,9H) 2.41(s,3H)4.29(q,J=7.3Hz,2H)6.33-7.16(m,2H) 7.46-7.56(m,1 H)8.17-8.21(m,1H) | 95-97 |
| 175 | 1H NNR(200MHz,CHLOROFORM-0)dppm1,06(t,J=7,5Hz,3H),1.43(s,9H), 1.73-1.96(m,2H),2.41(s,3H),4.24(t.J=7.9Hz,2H).7.54(t, J=7.7 Hz,1H),7.65-7.77(d.J=7,7Hz,1H),8.46(d,J=7.7Hz,1H),8.60(s.1 | 172-174 |
| 176 | 1H NMR(200MHz,CHLOROFORM-D)dppm1.43(s,9H),1.78(d,J=6.6Hz,6H), 2.41(s,3H),4.48-4.87(m,1H),7.55(t,J=7.7Hz,1H),7.71 (d,J=7.7 Hz,1H),8.46(d,J=1.7Hz,1H),8.57(s,1H) | 135-137 |
| 177 | 1HN MR(200MHz,CHLOROFORM-D)dppm1.00(d,J=7.0Hz,6H)1.44(s,9H) 2.23-2.50(m,1H)2.39(s,3H)4.11(d,J=7.5Hz,2H)7.37 (dd,J=7.9. 5.3Hz,1H)8.51(d,J=7.9Hz,1H)8.67(d,J=5.3Hz,1H)9.51(s,1H) | |
| 178 | 1H NMR(200MHz,CHLOROFORM-D)dppm0.52-0.67(m,4H),1.17-1.36(m,1 H),1.44(s,9H),2.44(s,3H),4.24(d,J=7.0Hz,2H), 7.35-7.49(m,3 H),8.23-8.35(m,2H) | 106-107 |
| 179 | 1H NMR(300MHz,CHLOROFORM-D)dppm0.56-0.63(m,4H),1.22-1.29(m,1 H),1.43(s,9H),2.42(s,3H),2.44(s,3H),4.24(d,J=6.8Hz, 2H),7.27 -7.35(m.2H),8.06-8.13(m.2H) | 132-134 |
| 180 | 1H NMR(300MHz,CHLOROFORM-D)dppm0.48-0.62(m,4H),1.19-1.31(m,1 H),1.44(s,9H),2.43(s,3H),2.71(s,3H),4.19(d,J=6.8Hz, 2H),7.18 -7.34(m,3H),8.05-8.13(m,1H) | |
| 181 | 1H NMR(200MHz,CHLOROFORM-D)dppm0.46-0.62(m,4H)1.10-1.36(m,1H) 1.27(t,J=7,5Hz.3H)1.44(s,9H)2.43(s,3H)3.12(q, J=7.5Hz,2H) 4.17(d,J=6.6Hz,2H)7.14-7.39(m,3H)7.93-8.01(m,1H) | 120-122 |
| 182 | 1H NMR(200MHz,CHLOROFORM-D)dppm0.25-0.69(m,4H)1.06-1.34(m,1H) 1.27(d,J=7.0Hz.6H)1.43(s,9H)2.42(s,3H)3.86-4.09 (m,1H)4.15 (d,J=7.0Hz.2H)7.08-7.49(m,3H)7.69-7.90(m,1H) | 168-169 |
| 183 | 1H NMR(200MHz.CHLOROFORM-D)dppm0.39-0.62(m,4H)1.07-1.31(m,1H) 1.43(s,9H)2.42(s,3H)4.13(d,J=7.0Hz.2H)7.39-7.62 (m,2H)7.68 (d,J=7.0Hz,1H)7.82(d,J=7.0Hz,1H) | 90-91 |
| 184 | 1H NMR(200MHz.CHLOROFORM-D)dppm0.54-0.64(m,4H)1.16-1.35(m,1H) 1.45(s,9H)2.46(s,3H)4.25(d,J=7.0Hz.2H)7.54 (t.J=7.7Hz,1H) 7.70(d,J=7.7Hz,1H)8.44(d,J=7.7Hz.1H)8.56(s.1H) | 93-94 |

(continued)

| Compound No. | H-NMR | m.p. (˚C) |
|---|---|---|
| 185 | 1H NMR(300MHz,CHLOROFORM-D)dppm0.53-0.62(m,4H),1.18-1.29(m,1 H),1.43(s,9H),2.44(s,3H),4.21(d,J=7.0Hz,2H), 7.04-7.22(m,2 H).7.35-7.43(m,1H),8.06-8.16(m,J=7.8.1H) | 182-183 |
| 186 | 1H NMR(300MHz,CHLOROFORM-D)dppm0.51-0,59(m,4H),1.17-1.30(m,1 H),1.44(s,9H),2.44(s,3H),4.19(d,J=6.8Hz.2H). 7.26-7.31(m,2 H),7.38-7.45(m.1H)7.92-7.96(m,1H) | |
| 187 | 1H NMR(200MHz,CHLOROFORM-D)dppm0.55-0.63(m,4H).1.18-1.34(m,1 H),1.44(s,9H),2.45(s,3H),4.23(d,J=7.0Hz,2H), 7.30-7.46(m,2 H),8.10-8.27(m,2H) | |
| 188 | 1H NMR(300MHz,CHLOROFORM-0)dppm0.50-0.63(m,4H)1.16-1.35(m,1H) 1.44(s,9H)2.44(s,3H)4.22(d,J=7.0Hz,2H)7.39(d, J=8.9Hz,2H) 8.21(d,J=8.9Hz,2H) | 94-95 |
| 189 | 1H NMR(300MHz,CHLOROFORM-D)dppm0.51-0.58(m,4H),1.16-1.28(m,1 H.),1.44(s,9H),2.44(s,3H),4.19(d,J=7.0Hz.2H).7.21(t, J=7.3Hz, 1H).7.33(t,J=7.3Hz,1H).7.62(d,J=7.3Hz.1H).7.89(d,J=7.3Hz,1 | |
| 190 | 1H NMR(200MHz,CHLOROFORM-D)dppm0.50-0.63(m.4H),1.17-1.34(m,1 H),1.44(s,9H).2.44(s.3H),4.19(d,J=7.0Hz,2H), 7.14-7.38(m,2 H),7.58-7.66(m.1H),7.85-7.93(m.1H) | |
| 191 | 1H NMR(300MHz,CHLOROFORM-D)dppm0.48-0.61(m,4H),1.15-1.33(m,1 H),1.44(s,9H),2.44(s,3H).4.21(d,J=7.0Hz,2H), 7.01-7.08(m,1 H)7.24-7.42(m.1H),7.87-7.99(m.2H) | |
| 192 | 1H NMR(200MHz,CHLOROFORM-D)dppm0.53-0.65(m,4H)1.15-1.36(m,1H) 1.44(s,9H)2.45(s,3H)4.23(d,J=7.0Hz,2H)7.16(t, J=7.9Hz,1H) 7.73-7.81(m,1H)8.18-8.26(m,1H)8.62(t.J=1.5Hz,1H) | 114-115 |
| 193 | 1H NMR(300MHz,CHLOROFORM-D)dppm0.54-0.62(m,4H),1.20-1.32(m,1 H),1.43(s,9H),2.43(s,3H),3.86(s,3H),4.21(d,J=6.8Hz, 2H),6.93 (d,J=9.0Hz,2H).8.24(d.J=9.0Hz,2H) | |
| 194 | 1H NMR(300MHz,CHLOROFORM-D)dppm0.53-0.67(m,4H),1.17-1.34(m,1 H),1.44(s,9H),2.44(s,3H),3.88(s,3H),4.23(d,J=7.0Hz, 2H).6.99 -7.05(m,1H),7.30-7.37(m.1H),7.84-7.92(m,2H) | 108-109 |
| 195 | 1H NMR(300MHz,CHLOROFORM-D)dppm0.53-0.61(m,4H),1.17-1.30(m,1 H),1.42(s,9H),2.42(s,3H),3.91(s,3H),4.17(d,J=7.0Hz, 2H),6.93 -7.01(m,2H),7.33-7.42(m,1H),7.96(dd,J=7,8,1.8Hz,1H) | |
| 196 | 1H NMR(300MHz,CHLOROFORM-D)dppm0.55-0.63(m,4H),1.17-1.31(m,1 H),1.44(s,9H),2.45(is,3H),4.23(d,J=7.0Hz,2H), 7.24-7.32(m,1 H),7.45(t,J=7.9Hz,1H),8.12-8.22(m.2H) | 106-108 |
| 197 | 1H NMR(200MHz,CHLOROFORM-D)dppm0.48-0.59(m,4H),1.11-1.33(m,1 H),1.44(s,9H),2.44(s,3H),4.18(d,J=6.6Hz,2H), 7.28-7.49(m,3 H).8.01-8.10(m,1H) | |
| 198 | 1H NMR(300MHz,CHLOROFORM-D)dppm0.56-0.63(m,4H),1.20-1.30(m,1 H),1.44(s,9H),2.43-2.48(m,3H),4.24(d.J=7.0Hz,2H), 5.94(tt, J=53.2,3,0Hz,1H),7.26-7.32(m,1H),7.39-7.48(m,1H),8.13-8.21 | 109-111 |

(continued)

| Compound No. | H-NMR | m.p. (˚C) |
|---|---|---|
| 199 | 1H NMR(200MHz,CHLOROFORM-D)dppm0.36-0.74(m,4H)1.18-1.38(m,1H) 1991(s,9H)2.45(s,3H)4.14(d,J=6.6Hz,2H)6.66-7.05 (m,2H)7.24 -7.51(m,1H)8.10-8.19(m,1H)13.16(s,1H) | 178-180 |
| 200 | 1H NMR(300MHz,CHLOROFORM-D)dppm0.52-0.63(m,4H)1.20-1.35(m,1H) 1.43(s,9H)2.42(s,3H)2.93(s,3H)4.18(d,J=6.8Hz,2H) 6.57-6.69 (m.2H)7.28-7.36(m,1H)8.34(dd,J=7.9,1.7Hz.1H)8,53-8.70(m,1 | 158-159 |
| 201 | 1H NMR(200MHz,CHLOROFORM-D)dppm0.55-0.70(m,4H),1.17-1.35(m,1 H),1.46(s,9H),2.48(s,3H),3.22(s,6H),4.36(d,J=7.0Hz, 2H),7.57 (t,J=7.9Hz,1H),8.13(d,J=7.9Hz,1H),8.35(d,J=7.9Hz,1H),8.54(s, | 128-130 |
| 202 | 1H NMR(300MHz,CHLOROFORM-D)dppm0.51-0.67(m,4H)1.14-1.31(m,1H) 1.19(t,J=7.1Hz,6H)1.43(s,9H)2.43(s,3H)3.41(q, J=7.1Nz,4H) 4.22(d,J=7.0Hz,2H)6.79(dd.J=8.2,2.8Hz,)1H)7.26(t,J=7.6Hz,1H) 7.59(d,J=7.6Hz,1H)7.67-7.73(m,1H) | 89-90 |
| 203 | 1H NMR(200MHz,CHLOROFORM-D)dppm0.51-0.64(m,4H),1.20(t,J=7.0Hz, 6H),1.14-1.34(m,1H),1.42(s,9H),2.41(s,3H),3.41(q, J=7.0Hz,4 H),4.19(d,J=7.0Hz,2H),6.66(d,J=9.2Hz,2H),8.15(d,J=9.2Hz,2H) | 134-136 |
| 204 | 1H NMR(200MHz,CHLOROFORM-D)dppm0.51-0.67(m,4H)1.19-1.36(m,1H) 1.43(s,9H)1.96-2.09(m,4H)2.43(s,3H)3.29-3.41(m, 4H)4.23(d, J=7.0Hz,2H)6.67(dd,J=8,1.2.4Hz,1H)7.27(t,J=8.1Hz,1H)7.52-7.57(m,1H)7.61(d,J=7.5Hz,1H) | 118-120 |
| 205 | 1H NMR(300MHz,CHLOROFORM-D)dppm0.53-0.67(m,4H)1.19-1.36(m,1H) 1.44(s,9H)1.50-1.80(m,5H)2.45(s,3H)3.20-3.29(m, 4H)4.24(d, J=7.0Hz,2H)7.07(dd,J=8.2,2.4Hz,1H)7.31(t,J=8.2Hz,1H)7.77(d, J=8.2Hz,1H)7.90-7.96(m,1H) | 89-90 |
| 206 | 1H NMR(300MHz,CHLOROFORM-D)dppm0.48-0.60(m,4H)1.15-1.33(m.1H) 1,44(s,9H)2.43(s,3H)2.99-3.12(m,4H)3.76-3.86(m, 4H)4.15(d, J=6.8Hz,2H)6.92-7.03(m,2H)7.27-7.35(m,1H)7.72(d.J=7.6Hz,1 | |
| 207 | 1H NMR(200MHz.CHLOROFORM-D)dppm0.52-0.68(m,4H)1.17-1.36(m.1H) 1.44(s,9H)2.44(s,3H)3.17-3.30(m,4H),3.83-3.95(m, 4H)4.23(d, J=7.0Hz,2H)7.03(dd,J=7.9,2.2Hz,1H)7.33(t,J=7.9Hz,1H)7.82(d. J-=’1,5Hz.1.H)7.86-7.94(m,1H) | 114-115 |
| 208 | 1H NMR(300MHz.CHLOROFORM-D)dppm0.50-0.69-(m,4H)1.15-1.36(m,1H) 1.43(s,9H)2.38(s.3H)2.44(s.3H)2.55-2.71(m.4H) 3,21-3.39(m, 4H)4.23(d.J=6.8Hz,2H)7.06(d.J=7.3Hz.1H)7.32(t.J=7.3Hz,1H) 7.79(d.J=7.3Hz.1H)7.91(s.1H) | 126-128 |
| 209 | 1H NMR(200MHz,CHLOROFORM-D)dppm0.56-0.69(m,4H)1.14-1.38(m,1H) 1.45(s,9H)2.47(s.3H)4.28(d.J=7.0Hz,2H)7.60(t, J=7.5Hz.1H) 8.29(d,J=75Hz,1H)8.57(d,J=7.5Hz,1H)9.10(s,1H) | 87-89 |
| 210 | 1H NMR(200MHz,CHLOROFORM-D)dppm0.55-0.66(m.4H)1.09-1.37(m,1H) 1.45(s,9H)2.46(s,3H)4.24(d,J=7.0Hz,2H)7.71(d, J=8.8Hz,2H) 8.35(d,J=8.8Hz,2H) | 125-126 |
| 211 | 1H NMR(200MHz,CHLOROFORM-D)dppm0.39-0.83(m,4H)1.09-1.38(m,1H) 1.45(s,9H)2.46(s,3H)4.25(d,J=7.0Hz.2H)7.54(t, J=7.7Hz,1H) 7,65-7.86(m,1H)8.43-8.59(m,2H) | |

(continued)

| Compound No. | H-NMR | m.p. (˚C) |
|---|---|---|
| 212 | 1H NMR(200MHz,CHLOROFORM-D)dppm0.47-0.80(m,4H)1.13-1.39(m,1H) 1.45(s,9H)2.46(s,3H)4.26(d,J=6.6Hz,2H)7.53(t, J=7.7Hz,1H) 8.03(d,J=7.7Hz,1H)8.44(d,J=7.7Hz,1H)8.63(s,1H) | 76-78 |
| 213 | 1H NMR(200MHz,CHLOROFORM-Ddppm0.55-0.64(m,4H),1.16-1.34(m,1 H),1.44(s,9H),2.46(s,3H),3.94(s,3H),4.25(d,J=7.0Hz, 2H),8.09 (d,J=8,8Hz,2H).8.32(d,J=8.8Hz.2H) | 122-123, |
| 214 | 1H NMR(200MHz,CHLOROFORM-D)dppm0.57-0.65(m,4H),1.19-1.33(m.1 H),1.45(s,9H),2.46(s,3H).4.26(d,J=7.0Hz,2H),8.16(d, J=8.8Hz, 2H),8.36(d,J=8.8Hz,2H) | 196-197 |
| 215 | 1H NMR(300MHz,CHLOROFORM-D)dppm0.51-0.60(m,4H).1.18-1.28(m, H),1.44(s,9H).2.32(s,3H).2.43(s,3H),2.51(s,3H),4.16 (d,J=7.0 Hz,2H),7.07-7.15(m,1H),7.16-7.21(m,1H).7.67-7.74(m,1H) | 97-98 |
| 216 | 1H NMR(300MHz,CHLOROFORM-D)dppm0.54(s,4H),119-1.30(m,1H), 1,43(s,9H),2.34(s,3H),2.42(s,3H),2.69(s,1H),4.18(d, J=6.8Hz, 2H),7.00-7.08(m,2H),8.02-8.06(m,1H) | 100-101 |
| 217 | 1H NMR(300MHz,CHLOROFORM-D)dppm0.51-0.60(m,4H),1.17-1.32(m,1 H),1.43(s,9H),2.35(s,3H),2.43(s,3H),2.65(s,3H),4.19 (d,J=6.8 Hz,2H),7.07-7.14(m,2H),7.89(s,1H) | 106-107 |
| 218 | 1H NMR(200MHz,CHLOROFORM-D)dppm0.38-0.57(m,4H)1.07-1.32(m,1H) 1.45(s,9H)2.29(s.6H)2.43(s,3H)4.11(d,J=6.6Hz,2H) 6.95-7.18 | 98-100 |
| 219 | 1H NMR(200MHz,CHLOROFORM-D)dppm0.35-0.75(m,4H)1.02-1.37(m,1H) 1.44(s,9H)2.45(s,3H)2.16(s,3H)4.20(d,J=7.0Hz,2H) 7.31(d, J=7.9Hz,1H)7.52(d.J=7.9Hz,1H)8.41(s,1H) | 91-92 |
| 220 | 1H NMR(200MHz,CHLOROFORM-D)dppm0.30-0.73(m.4H)0.99-1.35(m,1H) 1.45(s,9H)2.46(s.3H)4.16(d.J=7.0Hz.2H)7.72(d, J=8.4Hz,1H) 7.84(dJ=8.4Hz.1H)8.15(s,1H) | 159-161 |
| 221 | 1H NMR(200MHz,CHLOROFORM-Ddppm0.50-0.67(m,4H)1.08-1.34(m,1H) 1.44(s.9H)2.45(s.3H)4.20(d.J=7.0Hz,2H)6.98-7.12 (m,2H)7.71 -7.87(m,1H) | 170-172 |
| 222 | 1H NMR(200MHz,CHLOROFORM-D)dppm0.47-0.69(m,4H)1.08-1.36(m,1H) 1.44(s,9H)2.45(s,3H)4.21(d,J=7.0Hz,2H)6.98-7.36 (m,2H)7.76 -7.93(m,1H) | |
| 223 | 1H NMR(300MHz,CHLOROFORM-D)dppm0.44-0.62(m,4H),1.13-1.29(m,1 H),1.44(s,9H),2.44(s,3H),4.13(d,J=6.8Hz,2H),6.90(t, J=7.8Hz, 2H),7.20-7.32(m,1H) | 123-124 |

(continued)

| Compound No. | H-NMR | m.p. (˚C) |
|---|---|---|
| 224 | 1HN MR(300MHz,CHLOROFORM-D)dppm0.53-0.61(m,4H),1.18-1.27(m,1 H),1.43(s,9H),2.44(s,3H),4.20(d,J=7.1Hz,2H), 6.19-6,93(m,2 H),8.09-8.21(m,1H) | 111-112 |
| 225 | 1H NMR(300MHz,CHLOROFORM-D)dppm0.53-0.63(m,4H),1.19-1.30(m,1 H),1.44(s,9H),2.45(s,3H),4.21(d,J=7.1Hz,2H), 7.06-7.16(m,1 H),7.40-7.50(m,1H),7.93-8.02(m.1H) | |
| 226 | 1H NMR(300MHz.CHLOROFORM-D)dppm0.53-0.61(m,4H),1.16-1.29(m,1 H),1.43(s,9H),2.45(s,3H),4.20(d,J=7.0Hz,2H), 7.09-7.21(m,2 H),8.02-8.12(m,1H) | 108-110 |
| 227 | 1H NMR(300MHz,CHLOROFORM-D)dppm0.52-0.62(m,4H),1.15-1.30(m,1 H),1.43(s,9H),2.45(s,3H),4.20(d,J=7.1Hz,2H). 7.24-7.35(m,2 H).8.00(t,J=8.0Hz,1H) | 121-131 |
| 228 | 1H NMR(300MHz,CHLOROFORM-D)dppm0.48-0.62(m,4H),1.17-1.29(m.1 H),1.44(s,9H),2.45(s,3H),4.19(d.J=6.8Hz,2H), 6.89-7.00(m,1 H),7.53-7.67(m,2H) | 92-94 |
| 229 | 1H NMR(300MHz,CHLOROFORM-D)dppm0.49-0.61(m,4H),1.18-1.29(m,1 H).1.44(s.9H).2.44(s,3H),4.19(d,J=7.0Hz,2H), 6.09-7.10(m.1 H)7.33-7.40(m.1H)7.94-8.03(m,1H) | 126-128 |
| 230 | 1H NMR(300MHz.CHLOROFORM-D)dppm0.54-0.61(m.4H).1.17-1.28(m,1 H),1.44(s.9H).2.45(s,3H).4.20(d,J=7.0Hz,2H), 6.93-7.04(m.1 H),7.43-7.52(m,1H).8.19-8.26(m1H) | 113-114 |
| 231 | 1H NMR(200MHz,CHLOROFORM-D)dppm0.52-0.65(m,4H)1.06-1.35(m.1H) 1.44(s,9H)2.45(,s.3H)4.20(d.J=7.0Hz,2H)6.79-6.93 (m.1H)7.59 -7.74(m,1H)8,36-8.45(m,1H) | 95-96 |
| 232 | 1H NMR(300MHz,CHLOROFORM-D)dppm0.48-0.63(m,4H),1.16-1.29(m,1 H),1.44(s,9H),2.45(s.3H).4.19(d.J=7.0Hz,2H), 7.23-7.28(m,1 H).7.30-7.37(m,1H).7.90-7.95(m,1H) | 129-131 |
| 233 | 1H NMR(300MHz,CHLOROFORM-D)dppm0.55-0.63(m,4H),1.30(s,1H), 1.44(s,9H),2.46(s,3H),4.23(d,J=7.0Hz,2H),7.43(t, J=1.9Hz,1 H).8.12(d.J=1.9Hz,2H) | |
| 234 | 1H NMR(200MHz,CHLOROFORM-D)dppm0.52-0.64(m,4H)1.14-1.34(m,1H) 1.43(s,9H)2.43(s,3H)3.80(s,3H)3.87(s,3H)4.19(d, J=6.6Hz,2 H)6.86-6.98(m,2H)7.52-7.59(m,1H) | 83-85 |
| 235 | 1H NMR(200MHz,CHLOROFORM-D)dppm0.49-0.59(m,4H)1.15-1.32(m,1H) 1.43(s,9H)2.43(s,3H)3.88(s,3H)3.95(s,1H)4.17(d, J=6.6Hz,2 H)6.96(d,J=7.5Hz,1H)7.07(t,J=7.5Hz.1H)7.46(d,J=7.5Hz,1H) | 93-94 |
| 236 | 1H NMR(200MHz,CHLOROFORM-D)dppm0.49-0.62(m,4H),1.19.-1.32Cm,1 H),1.41(s.9H).2.41(s,3H),3.85(s,3H),3.90(s,3H), 4.16(d,J=7.9 Hz.2H),6.46-6.56(m.2H).8.06-8.14(m.1H) | |

(continued)

| Compound No. | H-NMR | m.p. (˚C) |
|---|---|---|
| 237 | 1HNMR(300MHz,CHLOROFORM-D)dppm0.51-0.59(m,4H)1.17-1.33(m,1H) 1.42(s,9H)1.42(t,J=7.0Hz,3H)1.46(t,J=7.0Hz,3H) 2.40(s,3H) 4.07(q,J=7.0Hz,2H)4.11-4.20(m,4H)6.46-6.51(m,2H)8.02-8.07 | 116-118 |
| 238 | 1HNMR(300MHz,CHLOROFORM-D)dppm0.51-0.57(m,4H)1.17-1.31(m,1H) 1.39(t,J=7.0Hz,6H)1.43(s,9H)2.42(s,3H)4.03(q, J=7.0Hz,2H) 4.11(q,J=7.0Hz,2H)4.17(d,J=6.8Hz,2H)6.88-6.91(m,2H)7.47- | 97-99 |
| 239 | 1HNMR(300MHz,CHLOROFORM-D)dppm0.50-0.65(m,4H),1.17-1.29(m,1 H),1.43(s,9H),2.35(s,3H),2.44(s,3H),4.21(d,J=7.0Hz, 2H),6.92 -7.02(m,1H).7.13-7.22(m,1H),7.85-7.92(m,1H) | 117-119 |
| 240 | 1HNMR(200MHz,CHLOROFORM-D)dppm0.48-0.62(m,4H)1.16-1.35(m,1H) 1.44(s,9H)2.44(s,3H)2.57(d.J=2.2Hz,3H)4.17 (d.J=7.0Hz,2H) 6.98-7.13(m,1H)7.09-7.24(m.1H)7.79(d.J=7,5Nz,1H) | 94-96 |
| 241 | 1HNMR(200MHz,CHLOROFORM-D)dppm0.48-0.64(m,4H)1.18-1.37(m,1H) 1.44(s.9H)2.44(s.3H)2.66(s,3H)4.19(d.J=6.6Hz,2H) 6.92-7.06 (m.1H)7.09-7.21(m,1H)7.75-7.85(m,H) | 91-93 |
| 242 | 1HNMR(300MHz,CHLOROFORM-D)dppm0.47-0.62(m,4H).1.19-1.30(m,1 H).1.44(s,9H),2.43(s,3H).2.69(s,3H),4.18(d.J=6.8Hz, 2H),7.17 -7.23(m.2H).8.06(d,J=8.9Hz.1H) | 108-109 |
| 243 | 1HNMR(200MHz,CHLOROFORM-D)dppm0.47-0.62(m,4H)1.11-1.34(m.1H) 1.44(s,9H)2.44(s.3N)2.65(s.3H)4.16(d,J=6.6Hz.2H) 7.14(t, J=7.5Hz,1H)7.39(d,J=7.5Hz,1H)7.76(d,J=7.5Hz,1H) | 88-90 |
| 244 | 1HNMR(300MHz,CHLOROFORM-D)dppm0.46-0.54(m,4H),1.08-1.22(m.1 H).1.45(s,9H).2.31(s.3H),2.44(s,3H).4.11(d.J=6.8Hz, 2H).7.04 -7.24(m,3H) | 159-160 |
| 245 | 1HNMR(300MHz,CHLOROFORM-D)dppm0.47-0.62(m,4H).1.16-1.28(m,1 H).1.44(s,9H),2.44(s.3H),2.67(s,3H),4.15(d,J=6.8Hz, 2H).7.02 -7.11(m,1H),7.54-7.61(m,1H).7.74-7.81(m,1H) | 89-90 |
| 246 | 1HNMR(300MHz,CHLOROFORM-D)dppm0.51-0.58(m,4H),1.18-1.29(m,1 H),1.43(s.9H).2.34(s,3H),2.43(s,3H).4.19(d,J=7.0Hz. 2H),7.10 7.16(m,1H).7.44-7.48(m,1H),7.83-7.90(m,1H) | 82-84 |
| 247 | 1H NMR(300MHz,CHLOROFORM-D)dppm0.50-0.59(m,4H),1.17-1.27(m,1 H),1.44(s,9H),2.43(s.3H),2.46(s,3H),4.15(d,J=6.8Hz, 2H),7.17 -7.27(m.2H),7.46-7.53(m,1H) | 142-144 |
| 248 | 1HNMR(300MHz,CHLOROFORM-D)dppm0.46-0,54(m,4H),1.10-1.23(m,1 H),1.45(s.9H),2.31(s,3H),2.44(s,3H),4.12(d,J=6.8Hz, 2H),7.00 -7.15(m,2H),7.34-7.41(m,1H) | 89-90 |

(continued)

| Compound No. | H-NMR | m.p. (˚C) |
|---|---|---|
| 249 | 1H NMR(300MHz,CHLOROFORM-D)dppm0.48-0.59(m.4H),1.15-1.29(m,1 H),1.44(s,9H),2.43(s,3H),2.51(s,3H),4.15(d,J=6.8Hz, 2H),7.20 -7.29(m.2H).7.35-7.42(m,1H) | 149-151 |
| 250 | 1H NMR(200MHz,CHLOROFORM-D)dppm0.51-0.64(m,4H)1.11-1.33(m,1H) 1.44(s,9H)2.46(s,3H)4.21(d,J=7.0Hz,2H)7.26(t, J=7.0Hz,1H) 7.58-7.72(m,1H)8.20-8.34(m.1H) | |
| 251 | 1H NMR(200MHz,CHLOROFORM-D)dppm0.47-0.67(m,4H)1.08-1.39(m,1H) 1.44(s,9H)2.46(s.3H)4.22(d,J=7.0Hz,2H)7.20(t, J=8.8Hz,1H) 7.56-7.72(m.1H)8.43(dd,J=6.8,2.4Hz,1H) | 113-115 |
| 252 | 1H NMR(300MHz,CHLOROFORM-D)dppm0.55-0.63(m,4H).1.17-1.31(m,1 H),1.44(s,9H),2.46(s,3H),4.23(d,J=7.0Hz.2H), 7.18-7.28(m,1 H),8.40-8.47(m,1H),8.57(dd,J=7.3,1.9Hz.1H) | 123-124 |
| 253 | 1H NMR(300MHz,CHLOROFORM-D)dppm0.49-0.69(m,4H),1.17-1.34(m,1 H),1.45(s,9H),2.47(s,3H),4.24(d,J=7.0Hz,2H), 7.36-7.43(m,1 H),8.08-8.15(m,1H),8.35(s,1H) | 100-102 |
| 254 | 1H MMR(200MHz,CHLOROFORM-D)dppm0.37-0.70(m,4H)0.99-1.33(m,1H) 1.44(s,9H)2.44(s,3H)4.15(d,J=6.6Hz,2H)7.07-7.20 (m,1H)7.48 -7.58(m,1H)7.64-7.75(m,1H) | 110-112 |
| 255 | 1H NMR(200MHz,CHLOROFORM-D)dppm0.33-0.78(m,4H)0.99-1.35(m,1H) 1.42(s,9H)2.42(s,3H)4.13(d,J=7.0Hz,2H)7.08-7.57 (m,2H)7.76 -8.02(m,1H) | |
| 256 | 1H NMR (200MHz,CHLOROFORM-D)dppm0.42-0.55(m,4H)1.03-1.30(m,1H) 1.44(s.9H)2.43(s,3H)4.10(d.J=7.0Hz,2H)7.19-7.51 (m,3H) | 128-130 |
| 257 | 1H NMR(300MHz,CHLOROFORM-D)dppm0.50-0.64(m,4H),1.16-1.30(m,1 H),1.45(s,9H),2.46(s,3H),4.19(d,J=7.0Hz,2H), 7.49-7.57(m,2 H),8.24-8.27(m,1H) | 100-102 |
| 258 | 1H NMR(200MHz,CHLOROFORM-D)dppm0.24-0.80(m,4H)1.04-1.34(m, 1H) 1.45(s,9H)2.45(s,3H)4.15(d,J=1.0Hz,2H)7.37(t, J=7.9Hz,1H) 7.70(d,J=7.9Hz,1H)7.88(d,J=7.9Hz,1H) | 93-94 |
| 259 | 1H NMR(200MHz,CHLOROFORM-D)dppm0.51-0.68(m,4H)1.14-1.37(m,1H) 1.45(s,9H)2.46(s,3H)4.23(d,J=7.0Hz,2H)7.51-7.59 (m,1H)8.29-8.38(m,1H)8.60-8.65(m,1H) | 122-124 |
| 260 | 1H NMR(200MHz,CHLOROFORM-D)dppm0.47-0.63(m,4H),0.88(s,9H), 1.14-1.45(m,1H),1.49(s,6H).1.78(s,2H),2.45(s,3H).4.23 (d, J=7.0Hz,2H).7.20(t,J=9.2Hz,1H).7.60-7.71(m,1H).8.39-8.51(m. | 88-89 |

(continued)

| Compound No. | H-NMR | m.p. (˚C) |
|---|---|---|
| 261 | 1H MR(200NHz,CHLOROFORM-D)dppm0.49-0.62(m,4H)1.14-1.38(m,1H) 1.43(s,9H)2.42(s,3H)2.74(s,3H)3.84(s,3H)4.18(d, J=7.0Hz,2 H)6.70-6.82(m.2H)8.16-8.24(m,1H) | 95-96 |
| 262 | 1H NMR(300MHz,CHLOROFORM-D)dppm0.53-0.60(m,4H),1.18-1.29(m,1 H),1.43(s,9H),2.43(s,3H).3.89(s,3H),4.17(d,J=6.8Hz, 2H),6.85 -6,94(m,1H)7.00-7.11(m,1H)7.63-7.71(m.1H) | 87-89 |
| 263 | 1H NMR(300MHz,CHLOROFORM-D)dppm0.46-0,61(m,4H),1.11-1.24(m,1 H),1.43(s.9H).2.42(s,3H),3.82(s,3H),4.11(d, J=7.11Hz,2H).6.66 -6.74(m,2H).7.16-7.28(m.1H) | 119-121 |
| 264 | 1H NMR(200MHz,CHLOROFORM-D)dppm0.51-0.62(m,4H)1.13-1.33(m,1H) 1.42(s,9H)2.42(s,3H)3.90(s,3H)4.16(d,J=7.0Hz,2H) 6.89-7.01 (m.2H)7.88-7.97(m,1H) | 163-165 |
| 265 | 1H NMR(200MHz,CHLOROFORM-D)dppm0.52-0.63(m,4H)1.09-1.34(m,1H) 1.43(s,9H)2.43(s,3H)3.89(s,3H)4.17(d,J=6.6Hz,2H) 6.84-8.93 (m,1H)7.25-7.37(m,1H)7.88-7,94(m,1H) | 98-100 |
| 266 | 1H NMR(200MHz,CHLOROFORM-D)dppm0.28-0.71(m,4H)1.07-1.34(m,1H) 1.44(s.9H)2.44(s,3H)4.17(d,J=6.6Hz,2H)6.74(t, J=76.0Hz,1H) 7.06-7.14(m,1H)7.46-7.55(m,1H)8.14-8.19(m,1H) | 169-170 |
| 267 | 1H NMR(200MHz,CHLOROFORM-D)dppm0.48-0.66(m,4H)1.07-1.34(m,1H) 1.43(s.9H)2.44(s,3H)3.95(s,3H)4.18(d,J=7.0Hz,2H) 7.02(d, J=9.2Hz,1H)7.60(d,J=9.2Hz,1H)8.27(s,1H) | 175-177 |
| 268 | 1H NMR(200MHz,CHLOROFORM-D)dppm0.50-0.65(m,4H)1.10-1.35(m,1H) 1.40-1.53(m,3H)1.44(s,9H)2.44(s,3H)4.11-4.30(m, 2H)4.21(d, J=7.0Hz,2H)7.00(d.J=8.8Hz,1H)7.56(d.J=8.8Hz,1H)8.20(s,1H) | 140-142 |
| 269 | 1H NMR(200MHz,CHLOROFORM-D)dppm0.48-0.64(m,4H)1.12-1.34(m,1H) 1.39(d,J=6.2Hz,6H)1.43(s.9H)2.43(s,3H)4.15(d, J=6.6Hz.2H) 4.55-4.77(m,1H)7.04(d,J=8..8Hz,1H)7.55(d,J=8.8Hz.1H)8.12(s, | 106-108 |
| 270 | 1H NMR(200MHz.CHLOROFORM-D)dppm0.46-0.66(m,4H)1.06-1.34(m,1H) 1.44(s,9H)2.44(s,3H)3.42(s.3H3.76-3.86(m,2H) 4.18(d.J=6.6 Hz.2H)4.22-4.35(m,2H)7.08(d,J=8.8Hz.1H)7.58(d.J=8.8Hz,1H) | 108-110 |
| 271 | 1H NMR(200MHz,CHLOROFORM-D)dppm0.50-0.66(m,4H)1.13-1.34(m,1H) 1.44(s.9H)2.44(s.3H)3.41(s.3H)3.49(t,J=5.5Hz.2H) 3.65(t. J=5.5Hz,2H)4.20(d.J=7.0Hz.2H)6.73(d.J=8.8Hz.1H)7.47(d,J=8.8 Hz,1H)8.61(s.1H)9.09-9.27(m.1H) | 131-132 |
| 272 | 1H NMR(200MHz.CHLOROFORM-D)dppm0.52-0.65(m,4H)1.16-1.36(m,1H) 1.44(s,9H)2.44(s,3H)3.49(t,J=5.5Hz,2H)3.88(t, J=5.5Hz,2H) 4.21(d,J=6.6Hz,2H)6.77(d,J=8.8Hz,1H)7.42-7.53(m,1H)8.64(s, | 131-133 |
| 273 | 1H NMR(200MHz,CHLOROFORM-D)dppm0.50-0.66(m,4H)1.13-1.34(m,1H) 1.44(s,9H)2.44(s,3H)3,41(s,3H)3.49(t,J=5.5Hz,2H) 3.65(t, J=5,5Hz,2H)4.24(d,J=7.0Hz,2H)6.73(d,J=8.8Hz,1H)7.47(d,J=8.8 Hz.1H)8.61(s.1H)9.09-9.27(m.1H) | 131-132 |

(continued)

| Compound No. | H-NMR | m.p. (˚C) |
|---|---|---|
| 274 | 1H NMR(200MHz,CHLOROFORM-D)dppm0,49-0.64(m,4H)1.12(t,J=7.2Hz,3 H)1.18-1.37(m,1H)1.43(s,9H)2.44(s,3H)2.87(s,3H) 3.29(q, J=7.2Hz,2H)4.15(d,J=7.0Hz,2H)6.90(d,J=8.4Hz,1H)7.43(d,J=8.4 Hz,1H)7.92(s.1H) | 101-103 |
| 275 | 1H NMR(200MHz,CHLORFORM-D)dppm0.52-0.66(m,4H)1.08-1.33(m,1H) 1.43(s,9H)2.29(qn,J=7.5Hz,2H)2.43(s,3H)3.96(t, J=7.5Hz,4H) 4.16(d,J=6.6Hz,2H)6.48(d,J=9.2Hz.1H)7.43(d.J=9.2Hz,1H)7.98 | 100-102 |
| 276 | 1HNMK(200MHz,CHLOROFORM-D)dppm0.48-0.62(m,4H)1,09(t,J=7.0Hz,6 H)1.15-1.33(m,1H)1.44(s,9H)2.43(s,3H)3.27(q, J=7.0Hz,4H) 4.14(d,J=7.0Hz,2H)6.94(d.J=8.4Hz,1H)7.43(d,J=8.4Hz,1H)7.89 | 131-133 |
| 277 | 1HNMR(200MHz,CHLOROFORM-D)dppm0.51-0.64(m,4H)1.11-1.31(m,1H) 1.44(s,9H)2.44(s,3H)2.91(s,6H)4.16(d,J=7.0Hz.2H) 6.90(d, J=8.8Hz.1H)7.45(dd.J=8.8.2.0Hz,1H)8.00(d,J=2.0Hz,1H) | 126-128 |
| 278 | 1HNMR(200MHz,CHLOROFORM-D)dppm0.51-0.64(m,4H),1.10-1,32(m.1 H),1.43(s,9H),1.83-1.97(m,4H),2.43(s,3H),3.22-3.38 (m,4H). 4.15(d,J=6.6Hz,2H),6.75(d,J=8.8Hz,1H),1.41(dd,J=8.8.2.6Hz,1 H),7.91(d.J=2.6Hz.1H) | |
| 279 | 1HNMR(200MHz,CHLOROFORM-D)dppm0.48-0.62(m,4H),1.15-1.34(m,1 H),1.44(s,9H),1.49-1.73(m,6H),2.43(s,3H),3.07-3.19 (m,4H), 4.15(d,J=7.0Hz,2H),6.97(d,J=8.8Hz,1H),7.45(dd,J=8.8,2.2Hz,1 H),7.96(d,J=2.2Hz,1H) | 138-140 |
| 280 | 1HNMR(200MHz,CHLOROFORM-D)dppm0.48-0.65(m,4H)1.09-1.35(m,1H) 1.45(s,9H)2.44(s,3H)3.05-3.22(m,4H)3.75-3.91(m, 4H)4.16(d, J=6.6Hz,2H)6.98(d.J=8.8Hz,1H)7.51(dd,J=8.8,1.8Hz,1H)8.03(d, | 86-87 |
| 281 | 1HNMR(200MHz,CHLOROFORM-D)dppm0.43-0.88(m,4H)1.08-1.39(m,1H) 1.45(s,9H)2.46(s,3H)4.23(d,J=7.0Hz,2H)7.03-7.55 (m,1H)8.27 | 195-197 |
| 282 | 1HNMR(300MHz,CHLOROFORM-D)dppm0.51-0.64(m,4H),1.16-1.31(m,1 H),1.44(s,9H),2.45(s,3H).4.21(d,J=7.0Hz,2H), 6.93-7.03(m,1 H),7.83-7.93(m,1H) | 82-84 |
| 283 | 1HNMR(300MHz,CHLOROFORM-D)dppm0.54-0.60(m,4H),1.17-1.29(m, H),1,43(s,9H),2.46(s,3H),4.20(d,J=7.0Hz,2H), 6.88-7.01(m,1 H)7.94-8.03(m,1H) | 78-79 |
| 284 | 1HNMR(300MHz,CHLOROFORM-D)dppm0.47-0.63(m,4H),1.16-1.30(m, H),1.44(s,9H),2.45(s,3H),4.18(d,J=7.0Hz.2H), 7.20-7.29(m,1 H),7.83-7.94(m,2H) | 1112-114 |
| 285 | 1HNMR(200MHz,CHLOROFORM-D)dppm0.49-0.67(m,4H)1.08.-1.35(m,1H) 1.44(s,9H)2.45(s,3H)4.21(d,J=7.0Hz,2H)6.93-7.07 (m,1H)7.94 -8.12(m,1H) | 76-77 |
| 286 | 1HNMR(300MHz.CHLOROFORM-D)dppm0.52-0.60(m,4H),1.18-1.34(m,1 H),1.43(s,9H),2.42(s,3H),3.89(s,3H).3.90(s,3H),3.99 (s,3H), 4.18(d,J=6.8Hz,2H),6.71(d,J=8,9Hz,1H),7.78(d,J=8.9Hz,1H) | 88-89 |

(continued)

| Compound No. | H-NMR | m.p. (˚C) |
|---|---|---|
| 287 | 1HNMR(300MHz,CHLOROFORM-D)dppm0.50-0.63(m,4H),1.17-1.28(m,1 H),1.43(s,9H),2.33(d,J=2.0Hz,3H),2.44(s,3H),4.21(d, J=7.0Hz, 2H),6.91-6.99(m,1H),7.11-7.81(m,1H) | 122-124 |
| 288 | 1HNMR(200MHz,CHLOROFORM-D)dppm0.42-0.69(m,4H)0.94-1.34(m,1H) 1.45(s,9H)2.45(s,3H)4.12(d.J=7.0Hz,2H)6.92-7.05 (m,1H)7.40 -7.68(m,1H) | 109-111 |
| 289 | 1HNMR(200MHz,CHLOROFORM-D)dppm0.37-0.58(m,4H)1.06-1.26(m,1H) 1.45(s,9H)2.44(s,3H)4.09(d,J=7.0Hz,2H)7.347.52 (m,2H) | 182-183 |
| 290 | 1HMMR(200MHz,CHLOROFORM-D)dppm0.51-0.67(m,4H),1.15-1.32(m,1 H),1.45(s,9H),2.45(s,3H),4.28(d,J=7.0Hz,2H). 7.28-7.39(m,1 H).7.72-7.84(m.1H),8.30(d.J=7.7Hz,1H),8.75(d.J=7.7Hz,1H) | |
| 291 | 1HNMR(200MHz,CHLOROFORM-D)dppm0.59(d,J=6.6Hz,4H),1.17-1.38(m, 1H),1.45(s,9H).2.46(s,3H),4.24(d,J=7.0Hz.2H), 7.30-7.43(m,1 H).8.43-8.55(m,1H),8.63-8.71(m,1H),9.46-9.52(m,1H) | |
| 292 | 1HNMR(200MHz,CHLOROFORM-D)dppm0.53-0.64(m,4H),1.18-1.31(m,1 H),1.46(s,9H).2.47(s,3H),4.26(d.J=7.0Hz.2H), 8.58-8.64(m,1 H),8.68-8.74(m.1H),9.49-9.54(m,1H) | |
| 293 | 1HNMR(300MHz,CHLOROFORM-D)dppm0.48-0.56(m,4H),1.10-1.28(m,1 H),1.44(s,9H),2.43(s,3H),2.54(s,3H),4.19(d,J=6.8Hz, 2H),7.16 -7.22(m,1H),7.51-7.55(m,1H),8.46-8.51(m,1H) | 88-89 |
| 294 | 1HNMR(200MHz,CHLOROFORM-D)dppm0.52-0.64(d,4H),1.08-1.38(m,1 H),1.44(s,9H),2.42(s,3H),2.46(s,3H),4.24(d,J=7.0Hz. 2H),7.31 -7.47(m.1H)7.67-7.94(m,1H),8.35-8.50(m,1H) | |
| 295 | 1HNMR(200MHz,CHLOROFORM-D)dppm0.42-0.66(m,4H),1.14-1.34(m,1 H),1.44(s,9H),2.45(s,3H),2.97(s,3H),4.18(d,J=6.8Hz, 2H),7.24 -7.46(m,1H),7.64-7.93(m,1H),8.41-8.55(m,1H) | |
| 296 | 1HNMR(300MHz,CHLOROFORM-D)dppm0.52-0.62(m,4H)1.16-1.31(m,1H) 1.44(s,9H)2.45(s,3H)2.62(s,3H)4.22(d,J=7.0Hz,2H) 7.22(d, J=7.9Hz,1H)8.38(dd,J=7.9,2.2Hz,1H)9.38(d,J=2.2Hz,1H) | 228-230 |
| 297 | 1HNMR(300MHz,CHLOROFORM-D)dppm0.49-0.67(m,4H),1.15-1.33(m,1 H),1.29(t,J=7.6Hz,3H),1.44(s,9H),2.45(s,3H),2.73(q, J=7.6Hz, 2H),4.29(d,J=7.0Hz,2H),7.18(d,J=5.0Hz,1H),8.15(s,1H),8.62 | 104-106 |
| 298 | 1HNMR(200MHz,CHLOROFORM-D)dppm0.43-0.62(m,4H)1.05-1.35(m,1H) 1.45(s,9H)2.45(s,3H)4.16(d,J=7.0Hz,2H)7.58 (d.J=4.8Hz,1H) 8.77(d.J=4.8Hz.1H)9.20(s,1H) | 74-76 |

(continued)

| Compound No. | H-NMR | m.p. (˚C) |
|---|---|---|
| 299 | 1HHMR(300MHz,CHLOROFORM-D)dppm0.40-0.58(m,4H),1.07-1.29(m,1 H).1.46(s,9H),2.29(s,3H),2.45(s,3H),2.52(s,3H),4.11 (d,J=6.8 Hz,2H),6.96(d,J=5.1Hz,1H).8.31(d,J=5.1Hz.1H) | 156-157 |
| 300 | 1HNMR(200MHz,CHLOROFORM-D)dppm0.50-0,66(m,4H),1.09-1.38(m,1 H),1.44(s,9H),2.46(s,3H),4.24(d,J=22.0Hz,2H). 7.24-7.34(m,1 H).8.21-8.32(m,1H).8.45-8.59(m,1H) | 83-84 |
| 301 | 1HNMR(200MHz,CHLOROFORM-D)dppm0.49-0.63(m,4H)1.10-1.34(m,1H) 1.45(s,9H)2.45(s,3H)4.20(d,J=7.0Hz,2H)7.28(dd, J=7.5,4.8Hz,1 H)8.25(dd,J=7.5.2.2Hz,1H)8.41(dd,J=4.8,2.2Hz,1H) | 124-125 |
| 302 | 1HNMR(200MHz,CHLOROFORM-D)dppm0.46-0.65(m,4H)1.14-1.35(m,1H) 1.44 (s,9H)2.45(s,3H)4.20(d,J=7.0Hz,2H)7.36(d, J=5.3Hz,1H) 8.47(d.J=5.3Hz,1H)9.21(s.1H) | |
| 303 | 1HNMR(200MHz,CHLOROFORM-D)dppm0.52-0.67(m,4H)1.13-1.37(m,1H) 1.45(s,9H)2.46(s,3H)4.23(d,J=6.6Hz,2H)8.60 (dd.J=2.6,1.3Hz,1 H)8.73(d,J=2.6Hz,1H)9.36(d,J=1.3Hz,1H) | 152-154 |
| 304 | 1HNMR(200MHz,CHLOROFORM-D)dppm0.46-0.58(m,4H)1.08-1.34(m,1H) 1.45(s,9H)2,44(s,3H)4.19(d,J=7.0Hz,2H)7.17(dd, J=7.9.4.8Hz,1 H)7.93(d.J=7.9Hz.1H)8.58(d.J=4.8Hz.1H) | 141-142 |
| 305 | 1HNMR(200MHz.CHLOROFORM-D)dppm0.47-0.61(m,4H)1.09-1.34(m,1H) 1.45(s,9H)2.46(s,3H)4.17(d,J=7.0Hz,2H)8.37(d, J=2.6Hz,1H) 8.52(d.J=2.6Hz,1H) | 115-116 |
| 306 | 1HNMR(200MHz,CHLOROFORM-D)dppm0.48-0.61(m.4H)1.06-1.31(m,1H) 1.44(s,9H)2.44(s,3H)4.15(d,J=7.0Hz,2H)7.26(d, J=8.4Hz,1H) 7.67(d.J=8.4Hz,1H) | 143-145 |
| 307 | 1H NMR(200MHz,CHLOROFORM-D)dppm0.44-0.66(m,H)1.07-1.37(m,1H) 1.45(s,9H)2.47(s,3H)4.19(d,J=7.0Hz,2H)7.77(s,1H) 8.41(s,1 | |
| 308 | 1HNMR(200MHz,CHLOROFORM-D)dppm0.40-0.64(m,4H)0.98-1.32(m,1H) 1.46(s,9H)2.46(s.3H)4.1(d.J=7.0Hz,2H)8.48(s,2H) | 260-261 |
| 309 | 1HNMR(300MHz,CHLOROFORM-D)dppm0.48-0.64(m,4H),1.17-1.29(m,1 H),1.45(s,9H),2.47(s,3H),4.20(d,J=6.8Hz,2H),8.23(d, J=2.6Hz, 1H),8.36(d,J=2.6Hz,1H) | 113-134 |
| 310 | 1HNMR(200MHz,CHLOROFORM-D)dppm0.49-0.63(m,4H)1.15-1.34(m,1H) 1.43(s,9H)1.44(t,J=7.0Hz,3H)2.43(s,3H)4.18(d, J=7.0Hz,2H) 4.53(q,J=7.0Hz,2H)6.90(dd,J=7.5,4.8Hz,1H)8.19(dd,J=4.8,2.2Hz, 1H)8.28(dd,J=7.5,2.2Hz,1H) | 102-104 |
| 311 | 1HNMR(200MHz,CHLOROFORM-D)dppm0.49-0.60(m,4H),1.22-1.35(m,1 H),1.40(d,J=6.2Hz,6H),1.43(s,9H),2.42(s,3H).4.17(d, J=7.0Hz, 2H),5.35-5.55(m,1H),6.80-6.92(m.1H).8.13-8.26(m,2H) | 151-153 |
| 312 | 1HNMR(300MHz,CHLOROFORM-D)dppm0.53-0.61(m,4H),1.20-1.31(m,1 H).1.43(s,9H),2.45(s,3H),2.52(s,3H),4.29(d.J=6.8Hz. 2H),7.01 -7.10(m,1H).8.46-8.53(m.2H) | 138-139 |

(continued)

| Compound No. | H-NMR | m.p. (˚C) |
|---|---|---|
| 313 | 1HNMR(200MHz,CHLOROFORM-D)dppm5.11-5.22(m,4H)5,82-6.07(m,1H) 6.16(s,9H)7.15(s,3H)8.85(d,J=7.0Hz,2H)11.79(dd, J=7.5,4.8Hz, 1H)11.83-11.96(m,3H)12.04-12.19(m,2H)12.92(dd,J=4.8,2.2Hz,1 H)13.14(dd.J=7.5,2.2Hz,1H) | 111-114 |
| 314 | 1HNMR(200MHz.CHLOROFORM-D)dppm0,48-0.66(m,4H)1.07-1.33(m,1H) 1.43(s,9H)2.45(s,3H)4.08(s,3H)4.14(s,3H)4.16(d, J=6.6Hz,2 H)7.39(s,1H) | 124-126 |
| 315 | 1HNMR(200MHz,CHLOROFORM-D)dppm0.49-0.63(m,4H),1.14-1.31(m,1 H),1.43(s,9H),1.82-1.94(m,4H),2.43(s,3H),3.38-3.51 (m,4H), 4.13(d.J=6.6Hz,2H),6.58(dd,J=7.5,4.8Hz,1H).7.89(dd.J=7,5.2.2 Hz.1H),8.17(dd,J=4.82.2Hz,2H) | 97-98 |
| 316 | 1HNMR(200MHz,CHLOROFORM-D)dppm0.34-0.78(m,4H)1.03-1.38(m.1H) 1.44(s.9H)2.45(s,3H)2.89(s,3H)4.18(d,J=7.0Hz,2H) 6.73-6.81 (m.1H)8.45-8.57(m,1H) | 67-69 |
| 317 | 1HNMR(200MHz,CHLOROFORM-D)dppm0.52-0.66(m,4H),1.12-1.34(m,1 H).1.44(s,9H),2.46(s,3H).3.98(s,3H).4.22(d,J=7.0Hz, 2H).7.43 (d,J=0.9Hz,1H).7.65(d,J=0,9Hz,1H) | 127-128 |
| 318 | 1HNMR(300MHz,CHLOROFORM-D)dppm.0.53-4.60(m,4H),0.85(t,J=7.3Hz, 3H),1.20-1.31(m,1H),1.43(s.9H),1.52-1,70(m,2H). 2.42(s,3 H),3.07-3.16(m.2H),4.17(d,J=6.8Hz,2H),7.39-7.54(m,5H),8.18 | 104-105 |
| 319 | 1HNMR(200MHz,CHLOROFOFM-D)dppm0.54-0.70(m,4H)1.07-1.31(m,1H) 1.44(s,9H)2.47(s,3H)4.21(d,J=7.0Hz,2H)7.19(d, J=4.0Hz,1H) 7.33(d,J=4.0Hz,1H) | 129-130 |
| 320 | 1HNMR(200MHz,CHLOROFORM-D)dppm0.55-0.68(m,4H)1.16-1.39(m,1H) 1.46(s,9H)2.48(s,3H)4.27(d,J=7.0Hz,2H)7.84(d, J=9.7Hz.1H) 8.33(d.J=9.7Hz,1H)8.77(s,1H) | 139-141 |
| 321 | 1HNMR(200MHz,CHLOROFORM-D)dppm0.49-0.71(m.4H)1.17-1.40(m,1H) 1.47(s,9H)2.47(s,3H)4.25(d,J=7.0Hz,2H)7.65 (dd.J=8.1,7.3Hz,1 H)8.05(d,J=8.1Hz,1H)8.56(d,J=6.2Hz,1H)8.62(dd,J=7.3.1.5Hz,1 H)9.14(d.J=6.2Hz.1H)9.26(s,1H) | 109-110 |
| 322 | 1HNMR(200MHz,CHLOROFORM-D)dppm046-0.67(m,4H)1.03-1.38(m,1H) 1.45(s.9H)2.44(s,3H)4.15(d,J=7.0Hz,2H)7.38(dd, J=8.1,4.2Hz,1 H)7.56(dd.J=8.1,7.0Hz,1H)7.84(dd,J=8.1,1.8Hz,1H)8.06(dd, J=7.0.1.8Hz.1H)8.15(dd,J=8.1,2.0Hz,1H)9.04(dd, J=4.2,2.0Hz,1 | 193-195 |
| 323 | 1HNMR(200MHz,CHLOROFORM-D)dppm0.47-0.68(m,4H)1.12-1.39(m,1H) 1.47(s,9H)2.48(s,3H)4.23(d,J=7.0Hz,2H)7.57(ddd, J=8.4,6.8,1.5 Hz,1H)7.71(ddd.J=8.4,6.8,1.5Hz.1,H)8.01(d.J=4.4Hz,1H)8.13(d. J=7.5Hz,1H)9.00(d,J=4.4Hz.1H)905 (dd.J=7.5.1.1Hz.1H) | 124-126 |

(continued)

| Compound No. | H-NMR | m.p. (˚C) |
|---|---|---|
| 324 | 1HNMR(300MHz.CHLOROFORM-D)dppm0.47-0.54(m.4H),1.12-1.24(m.1 H),1.48(s.9H).2.46(s,3H).4.22(d,J=7.9Hz,2H), 7.52-7.87(m.4 H).8.56-8.72(m,2H) | |
| 325 | 1HNMR(200MHz,CHLOROFORM-D)dppm0,43-0.75(m,4H),1.15-1.41(m.1 H),1.50(s,9H),2.54(s,3H).4.13-4.50(m,2H),8.02-8.27 (m,2H), 8.72-8.92(m,1H).9.54-9.75(m,1H).9.90-10.21.(m,.1H) | 140-141 |
| 326 | 1HNMR(200MHz.CHLOROFORM-D)dppm0.46-0.72(m.4H)1.12-1.38(m.1H) 1.48(s,9H)2.49(s.3H)4.23(d.J=7..0Hz,2H)7.57 (t.J=8.4Hz.1H) 7.72(t,J=8.4.Hz.1H)7.98(s,1H)8.04(d.J=8.4Hz,1H)9.00(d,J=8.4 | 201-203 |
| 327 | 1HNMR(200MHz.CHLOROFORM-D)dppm0.38-0.59(m,4H)1.01-1.28(m,1H) 1.46 (s,9H)2.44(s,3H)4.10(d,J=7.0Hz,2H)7.56(d, J=9.2Hz,1H) 7.65(d,J=9.2Hz,1H)8.09(d,J=2.6Hz,1H)8.80(d,J=2.6Hz.1H) | 172-174 |
| 328 | 1HNMR(200MHz.CHLOROFORM-D)dppm0.36-0.65(m,4H)1.06-1.36(m.1H) 1.46(s,9H)2.46(s,3H)4,18(d,J=1.0Hz,2H)7.69 (d.J=5.3Hz,1H) 7.92(d.J=5.3Hz,1H)8.69(s,1H) | 147-149 |
| 329 | 1HNMR(200MHz,CHLOROFORM-D)dppm1.38(s,9H)2.07(s,3H)3.15(t, J=7.0Hz,2H)4.46(t,J=7.0Hz.2H)7.10-7.19(m,2H) 7.22-7.36(m,3 H)7.40(dd,J=7.9.4.8Hz.1H)8.56(d.J=7,9Hz,1H)8.69(d,J=4.8Hz.1 | |
| 330 | 1HNMR(200MHz,CHLOROFORM-D)dppm0.45-0.61(m,4H)1.02-1.22(m,1H) 1,24(s.9H)1.39(s,9H)2.39(s,3H)4.10(d,J=7.0Hz,2H) | 102-104 |
| 331 | 1HNMR(300MHz,CHLOROFORM-D)dppm0.49-0.60(m,4H),1.09-1.20(m,1 H)1.39(s,9H).1.83-2.02(m,2H),2.12-2.42(m,4H),2.39 (s,1H). 3.22-3.35(m,1H),4,08(d,J=6.8Hz,2H) | 189-191 |
| 332 | 1HMMR(200MHz,CHLOROFORM-D)dppm0.39-0.65(m,4H)1.02-2.07(m,12 H)1.39(s,9H)2.38(s,3H)4.08(d,J=7.0Hz,2H) | 141-142 |
| 333 | 1HNMR(200MHz,CHLOROFORM-D)dppm0.52-0.64(m,4H)1.07-1.36(m,1 H),1.44(s,9H),2.47(s,3H),4.15(d,J=7.0Hz,2H) | |
| 334 | 1HNMR(200MHz,CHLOROFORM-D)dppm0.49-0.63(m,4H)1.05-1.29(m,1H) 1.43(s,9H)2.46(s,3H)4.14(d,J=7.0Hz,2H)6.21(tt, J=53.2.5.7Hz, | 93-94 |
| 335 | 1HNMR(300MHz,CHLOROFORM-D)dppm0.46-0.60(m,4H).1.08-1.22(m,1 H),1.41(s,9H),2.41(s,3H).2.47-2.64(m,2H).2.68-2.76 (m,2H), 4.08(m,J=6.8Hz.2H) | 102-103 |
| 336 | 1HNMR(300MHz,CHLOROFORM-D)dppm0.53-0.59(m,4H),1.08-1.20(m,1 H),1.43(s,9H).2.47(s.3H),4.15(d.J=1.0Hz;2H) | 147-148 |
| 337 | 1HNMR(200MHz,CHLOROFORM-D)dppm0.46-0.59(m.4H)1.05-1.36(m,2H) 1.37(s.9H)1.60-1.74(m,1H).2.07-2.20(m,1H)2.38(s, 3H)2.49-2.65(m.1H)4.07(d.J=6.6Hz.2H)7.08-7.32(m.5H) | 110-112 |

(continued)

| Compound No. | H-NMR | m.p. (˚C) |
|---|---|---|
| 338 | 1HMMR(300MHz.CHLOROFORM-D)dppm1.42(s,9H).2.44(s.3H),3.29(s,3 H).3.70(t.J=5,2Hz,2H),4.34.(t,J=5.1Hz.2H) | 69-71 |
| 339 | 1HNMR(300MHz.CHLOROFORM-D)dppm0.35-0.51(m,4H),1.01-1.16(m.1 H).1.37(s.9H).2.36(s,3H).3.76(s.2H).4.01(d.J=7.0Hz. 2H),7.14 -7.22(m.1H).7.22-7.38(m.4H) | 92-94 |
| 340 | 1HNMR(300MHz,CHLOROFORM-0)dppm0.43-0.54(m,4H).1.04-1.18(m,1 H),1.38(s,9H).2.38(s,3H),3.97(s,2H),4.07(d,J=7.0Hz. 2H).6.91 -6.94(m,2H),7.13-7.17(m.1H) | 157-159 |
| 341 | 1HNMR(300MHz,CHLOROFORM-D)dppm0.21-0.54(m.4H),0.97-1.08(m,1 H).1.36(s.9H),2.22(s,3H),2.35(s.3H),3.92(dd, J=14.2.6.5Hz,1 H).4.08(dd,J=14.2,7.5Hz,1H),6.14(s.1H),7.23-7.36(m,3H),7.50 | 147-148 |
| 342 | 1HNMR(300MHz,CHLOROFORM-D)dppm0.32-0.47(m,4H),0.99-1.10(m,1 H),1.37(s,9H),2.36(s,3H),3.75(s,6H),3.78(s.2H),3.96 (d,J=7.0 Hz,2H),6.69-6,88(m,3H) | 89-91 |
| 343 | 1HNMR(200MHz,CHLOROFORM-D)dppm1.42(s,9H),2.37(s,3H),4.92-5.00(m,2H),5.02-5.30(m,2H),5.89-6.11(m,1H),7.35-7.47 (m,3 H).8.26-8.35(m,2H) | |
| 344 | 1HNMR(200MHz,CHLOROFORM-D)dppm1.44(s,9H)2.44(s,3H)3.34(s,3H) 3,81(t,J=5.5Hz,2H)4.44(t,J=5.5Hz,2H)7.54 (t.J=7.5Hz,3H)7.71 (d.J=7.5Hz,1H)8.45(d.J=7.5Hz.1H)8.56(s,1H) | 172-173 |
| 345 | 1HNMR(300MHz,CHLOROFORM-D)dppm1.43(s,9H),2.44(s,3H),3.34(s,3 H),3.81(t,J=5.4Hz,2H),4.42(t,J=5.4Hz.2H),7.26-7.33 (m,1H). 7.55-7.61(m,1H).8.18-8.23(m,1H),8.40-8.44(m,1H) | 89-91 |
| 346 | 1HNMR(200MHz.CHLOROFORM-D)dppm1.43(s.9H)2.43(s,3H)3.34(s.3H) 3.80(t,J=5.5Hz.2H)4.41(t,J=5.5Hz,2H)7.16(t, J=7.9Hz,1H)7.78 (d,J=7,9Hz,1H)8.23(d,J=7.9Hz,1H)8.62(s,1H) | 119-121 |
| 347 | 1HNMR(200MHz,CHLOROFORM-D)dppm1.42(s,9H).2.42(s,3H),3.31(s,3 H),3,77(t,J=5.5Hz,2H),4.37(t,J=5.5Hz,2H),7.07-7.21 (m,2H). 8.02-8.14(m,1H) | 68-69 |
| 348 | 1HNMR(200MHz.CHLOROFORM-D)dppm1.43(s,9H).2.43(s,3H),3.32(s,3 H),3.78(t,J=5.3Hz,2H).4.38(t,J=5.3Hz,2H),6.92-7.04 (m,1H). 7.43-7.53(m,1H),8.18-8.26(m,1H) | 95-97 |
| 349 | 1HNMR(200MHz,CHLOROFORM-D)dppm1.43(s,9H)2.44(s,3H)3.31(s,3H) 3.75(t,J=5.3Hz,2H)4.37(t.J=5.3Hz,2H)7.43-7.51(m, 1H)7.78- | 161-163 |

(continued)

| Compound No. | H-NMR | m.p. (˚C) |
|---|---|---|
| 350 | 1HNMR(200MHz,CHLOROFORM-D)dppm1.43(s,9H)2.43(s,3H)3.34(s,3H) 3.80(t,J=5.5Hz,2H)4.42(t,J=5.5Hz,2H)5.39-6.34(m, 1H)7.18-7.36(m,1H)7.38-7.50(m,1H)8.01-8.32(m,2H) | 114-116 |
| 351 | 1HNMR(200MHz,CHLOROFORM-D)dppm1.43(s.9H)2.43(s,3H)2.66(s,3H) 3.32(s,3H)3.77(t,J=5.5Hz,2H)4.37(t,J=5.5Hz.2H) 6.91-7,07(m, 1H)7.08-7.23.(m,1H)7.72-7.90(m,1H) | 106-107 |
| 352 | 1H NMR(200MHz,CHLOROFORM-D)dppm1.14(t,J=7.0Hz.3H)1.43(s,9H) 2.46(s,3H)3.47(q,J=7.4Hz,2H)3,84(t,J=5.5Hz,2H)4.44 (t,J=5.5 Hz.2H)7.54(t.J=7.7Hz,1H)1.71(d,J=7.7Hz,1H)8.46(d.J=7.7Hz,1 | 91-92 |
| 353 | 1H NMR(200MHz,CHOROFORM-D)dppm1.43(s,9H)2.42(s,3H)3.93(s.1H) 4.08(t,J=5.3Hz,2H)4.48(t,J=5.3Hz,2H)T.55(t,J=7.9Hz, 1H)7.72 (d,J=7.9Hz,1H)8.42(d.J=7.9Hz.1H)8.51(s,1H) | 147-149 |
| 354 | 1H NMR(200MHz,CHLOROFORM-D)dppm1.44(s,9H)2.45(s,3H)3.19(t.76-78 J=6.4Hz,2H)4.37(t,J=6.4Hz,2H)7.55(t,J=7.7Hz,1H) 7.71(d,J=7.7 Hz,1H)8.46(d,J=7.7Hz.1H)8.56(s,1H) | |
| 355 | 1H NMR(200MHz,CHLOROFORM-d)dppm1.42(s,9H)2.16-2.33(m,2H)3.82-129-130 3.93(m,2H)4.32-4.44(m,2H)7.37-7.50(m,1H) 7.63-7.77(m,3H) 7.79-7.88(m,2H)8.35-8.44(m,1H)8.51-8.55(m,1H) | |
| 356 | 1H NMR(200MHz,CHLOROFORM-D)dppm0.53-0.71(m,4H)1.16-1.45(m,1H) 1.37(t,J=7.0Hz,3H)2.79(s,3H)4.34(q,J=7.0Hz,2H) 4.30(d,J=7.9 Hz,2H)7.58(t,J=7.7Hz,1H)7.75(d,J=7.7Hz,1H)8.45(d,J=7.7Hz,1 (m, | 115-116 |
| 357 | 1H NMR(200MHz,CHLOROFORM-D)dppm0.63-1.01(m,4H)1.16-1.411H) 2.34-2.73(m,3H)3.53-3.87(m,2H)7.01-7.24(m.1H) 7.30-7.59(m, 1H)7.91-8.29(m,2H) | 291-293 |
| 358 | 1H NMR(200MHz,CHLOROFORM-D)dppm0.52-0.71(m,4H)1.17-1.43(m,1H) 1.25(d,J=6.6Hz,6H)2.78(s,3H)4.10-4.35(m,1H)4.29 (d,J=7.0Hz, 2H)5.47-5.65(m,1H)7.58(t,J=7.7Hz.1H)7.16(d,J=7.7Hz,1H)8.44 (d,J=7.7Hz,1H)8.55(s.1H) | 212-214 |
| 359 | 1H NMR(200MHz,CHLOROFORM-D)dppm0.54-0.70(m,4H)1.17-1.45(m,1H) 2.49(s,3H)3.13(s,6H)4.25(d,J=7.0Hz,2H)7.57(t, J=7.7Hz,1H) 7.74(d,J=7.7Hz,1H)8.44(d.J=7.7Hz,1H)8.56(s,1H) | 138-140 |
| 360 | 1HNMR(200MHz,CHLOROFORM-D)dppm0.54-0.71(m,4H)1.19-1.45(m,1H) 1.89-2.06(m,4H)2.59(s,3H)3.53-3.74(m,4H)4.27(d, J=7.0Hz,2 H)7.57(t,J=7.7Hz,1H)7.74(d,J=7.7Hz,1H)8.44(d,J=7.7Hz,1H) | 125-127 |
| 361 | 1HNMR(200MHz,CHLOROFORM-D)dppm0.54-0.71(m,4H)1.14-1.46(m, 1H) 2.43-2.57(m,4H)2.58(t,J=6.2HZ,2H)2.78(s,3H)3.51 (q,J=6.2Hz, 2H)3.67-3.83(m,4H)4.30(d,J=7.0Hz.2H)6.31-6.49(m,H)7.59 (t,J=7.7Hz.1H)7.76(d,J=7.7Hz,1H)8.45(d,J=7.7Hz,1H) 8.55 (s, 1 | 156-158 |
| 362 | 1HNMR(20dMHz,CHLOROFORM-d)dppm1.35(6H,d.J=5.27Hz).3.47-3.72(1 H,m),3.67(3H,s),7.84-7.95(1H,m),8.52-8.61(1H,m), 8.79-8.86 (1H,m),9.49-9.58(1H,m) | |
| 363 | 1HNMR(200MHz,CHLOROFORMS)dppm0.52-0.63(m,2H).0.81-0.96(m.2 H).1.21-1.51(m,1H),1.36(d.J=7.0Hz,6H),3.45-3.67(m, 1H),4.41 (d.J=7.5Hz,2H).8.25(s1H,9.18(s,1H).9.43(s.1H) | 139-141 |

(continued)

| Compound No. | H-NMR | m.p. (˚C) |
|---|---|---|
| 364 | 1HNMR(300MHz,CHlOROFORM-D)dppm1.42(t.J=7.1Hz.3H),2.36(s,3H), 4.33(q,J=7.0Hz.2H).7.24-7.33(m,1H),7.66-7.74(m,1H), 8.27- | 158-160 |
| 365 | 1HNMR(300MHz,CHLOROFORM-D)dppm0.49-0.67(m,4H)1.20-1.38(m,1 H)2.39(s,3H)4.21(d,J=7.0Hz,2H)7.29(t,J=7.7Hz,1H) 7.70(t, J=7.7Hz.1H)8.29(t.J=7-7Hz.1H) | 130-132 |
| 366 | 1HNMR(200MHz,CHLOROFORM-D)dppm0.13-0.48(m.4H).0.98-1.18(m.1 H),2.15(s,3H).4.07(d,J=7.0Hz,2H).7.27-7.41(m.2H). 7.47-7.63 (m,4H)7,72(d,J=7.5Hz.1H),8.48(d.J=7.5Hz.,1H).8.59(s,1H) | 161-162 |
| 367 | 1HNMR(200MHz.CHLOROFORM-D)dppm2.04(s.3H).2.22(s,3H),2.65(s3 H),4.39-4.47(m,2H),5.10-5.30(m,2H),5.79-6.01(m,1H), 7.11-7.33(m,3H),7.96-8.05(m,1H) | |
| 368 | 1HNMR(200MHz,CHLOROFORM-D)dppm4.66-4.83(m,2H),4.89-5.26(m,2-H),5.86-6.07(m,1H),7.10-7.56(m,13H),8.31-8.42(m, 2H) | 141-143 |
| 369 | 1MNMR(600MHz,CHLOROFORM-d)dppm0.43-0.48(m,2H)0.56-0.61(m,2 H)1.13-1.20(m,1H)1.26(d,J=7.3Hz,6H)2.13(s,3H) 2.90-2.96(m, 1H)3.70(d,J=7.3Hz,2H)7.21-7.25(m,1H)7.61-7.65(m,1H)8.17- | |
| 370 | 1HNMR(200MHz.CHLOROFORM-d)dppm0.42-0.68(m,4H)1.14-1.36(m,1H) 1.31(d,J=7.0Hz,6H)2.14(s,3H)2.86-3.05(m,1H)3.74 (d,J=7.0Hz. 2H)7.44-7.57(m,1H)7.64-7.73(m.1H)8.37-8.44(m,1H)8.47- | 65-67 |
| 371 | 1HNMR(300MHz,CHLOROFORM-d)dppm0.43-0.64(m,4H)1.08-1.24(m,1H) 1.29(d,J=7.0Hz,6H)2.14(s,3H)2.86-3.00(m,1H)3.71 (d,J=7.1Hz, 2H)7.10-7.22(m,1H)7.57-7.68(m,1H)8.30-8.39(m,1H) | 76-78 |
| 372 | 1HNMR(200MHz,CHLOROFORM-d)dppm0.41-0.65(m,4H)1.09-1.31(m,1 H)1.20(d,J=7.0Hz,6H)2.11(s,3H)2.80-2.97(m,1H)3.68 (d,J=7.0 Hz,2H)3.90(s,3H)6.93-7.01(m,1H)7.53-7.61(m,1H)8.03-8.07 | 118-120 |
| 373 | 1HNMR(200MHz,CHLOROFORM-D)dppm0.50-0.65(m,4H)1.35-1.54(m,1H) 2.75(s,3H)4.42(d,J=7.0Hz,2H)7.20-7.54(m,6H)7.70 (d,J=7.0Hz, 1H)8.21(d,J=7.9Hz,1H) | 115-117 |
| 374 | 1HNMR(200MHz,CHLOROFORM-D)dppm0.52-0.74(m,4H),1.33-1.55(m,1 H),3.03(s,6H),4.45(d,J=7.0Hz,2H),6.86-6.97(m,1H). 7.25-7.51 (m.4H),7.67-7.80(m,3H) | 156-158 |
| 375 | 1HNMR(200MHz,CHLOROFORM-D)dppm0.52-0.66,(m,4H),1.34-1.53(m,1 H),2.98(s,3H),4.43(d,J=7.5Hz,2H),7.18-7.57(m,4H), 7.73(d. J=7.0Hz,1H).8.42-8.51(m,1H),8.54-8.62(m,1H) | 181-183 |
| 376 | 1HNMR(200MHz,CHLOROFORM-D)dppm0.53-0.65(m,4H)1.34-1.52(m,1H) 2.36(s,3H)2.41(s,3H)2.74(s,3H)4.38(d,J=7.0Hz,2H) 7.17-7.42 (m,3H)7.19(s,1H)7.45(s,1H)8.19(d.J=7.9Hz,1H) | 160-161 |
| 377 | 1HNMR(200MHz,CHLOROFORM-D)dppm0.55-0.65(m,4H),1.32-1.50(m,1 H),2.74(s,3H),4.39(d,J=7.0Hz,2H),7.12-7.46(m,6H), 8.17-8.25 | 143-145 |

(continued)

| Compound No. | H-NMR | m.p. (˚C) |
|---|---|---|
| 378 | 1HNMR(300MHz,CHLOROFORM-D)dppm0.50-059(m,2H),0.62-0.72(m,2 H),1.45-1.61(m,1H),2.75(s,3H),4.98(d,J=7.1Hz,2H), 7.17-7.48 (m,5H),7.60d,J=7.6Hz,1H),8.22(d,J=7.8Hz,1H) | 146-148 |
| 379 | HNMR(300MHz,CHLOROFORM-D)dppm0.49-0.69(m,4H),1.19-1.35(m,1 H),2.74(s,3H),2.86(s,3H),4.78(d,J=6.5Hz,2H), 7.15-7.40(m,5 H),7.51-7.59(m.1H).8.19(d,J=6.2Hz,1H) | 133-134 |
| 380 | 1HNMR(300MHz,CHLOROFORM-D)dppm0.50-0.65(m,4H),1.40-1.52(m,1H), 2.74(s,3H),4.57(d,J=8.5Hz,2H),6.92-7.03(m,1H), 7.19-70.43 (m,4H),8.22(d,J=7.5Hz.1H) | 146-148 |
| 381 | 1H NMR(200MHz,CHLOROFORM-D)dppm0.52-0.65(m,4H)1.18-1.42(m,1H) 1.79-2.04(m,4H)2.56-2.70(m,4H)4.15(d,J=7.0Hz, 2H)7.55(t, J=7.7Hz.1H)7.71(d,J=7.7Hz.1H)8.46(d,J=7.7Hz.1H)8.57(s,1H) | 159-160 |
| 382 | 1H NMR(200MHz,CHLOROFORM-d)dppm0.56-0.69(m,4H)1.28-1.50(m,1H) 4.42(d,J=7.5Hz.2H)7.24-7.47(m,2H)7.62-7.82(m, 2H)8.29-8.42 (m,1H)8.49-8.57(m,1H) | 156-158 |

Table 10

| Compound No. | m.p. (˚C) or NMR |
|---|---|
| 383 | 178.5-180.5 |
| 384 | 141.5-142.5 |
| 385 | 1HNMR(200MHz,CHLOROFORM-D)dppm1.35(s,9H),2.25(s,3H),3.43(s,3H),7.38-7.52(m,3H),7.94-8.02(m,2H) |
| 386 | 173-174.5 |
| 387 | 190-192 |
| 388 | 1H NMR(200MHz,CHLOROFORM-D)dppm0.29-0.51(m,4H),0.89-1.12(m,1H),1.36(s. 9H).2.29(s,3H),3.84(d,J=7.0Hz,2H).7.37-7.54(m,3H),7.91-8.01(m, 2H) |
| 389 | 198-199.5 |
| 390 | 121-124 |
| 391 | 122-123 |
| 392 | 125-127 |
| 393 | 1H NMR(200MHz,CHLOROPORM-D)dppm0.29-0.54(m,4H),0.99-1.19(m,1H),1.36(s, 9H),2.31(s,3H).3.85(d,J=6.6Hz,2H),7.29-7.50(m.3H),8.15-8.26(m, 1H) |
| 394 | 126-128 |
| 395 | 159-160 |
| 396 | 140.5-141.5 |
| 397 | 132-134 |
| 398 | 100-102 |
| 399 | 104-105 |
| 400 | 130-131 |
| 401 | 149-151 |
| 402 | 132-135 |
| 403 | 110-112 |

(continued)

| Compound No. | m.p. (˚C) or NMR |
|---|---|
| 404 | 150-152 |
| 405 | 69-72 |
| 406 | 127-129 |
| 407 | 124-126 |
| 408 | 126.5-128. |
| 409 | 136-138 |
| 410 | 136-138 |
| 411 | 129-131 |
| 412 | 1HNMR(200MHz,CHLOROFORM-D)dppm0.31-0.53(m,4H),0.94-1.11(m.1H).,1.36(s, 9H).2.30(s.3H).3.84(d,J=7.0Hz,2H).7.11-7.23(m,1H).7.31-7.44(m, 2H), 7.87-7.95(m.2H) |
| 413 | 132.5-134 |
| 414 | 1HNMR(200MHz,CHLOROFORM-D)dppm0.29-0.58(m,4H),0.96-1.13(m,1H).1.36(s, 9H).2.30(s.3H).2.67(s.3H),7.00-7.12(m.1H),7.16-7.27(m.1H), 7.74-7.84 |
| 415 | |
| 416 | |
| 417 | 124-125.5 |
| 418 | 1H NMR (200 MHz, CHLOROFORM-D) d ppm 0.30 - 0.44 (m, 4 H), 0.90 - 1.11 (m, 1 H), 1.38 (s, 9 H), 2.37 (s, 3 H), 3.80 (s, 3 H), 3.84 (d. J=7.0 Hz. 2 H), 6.70 - 6.84 (m, 2 H), 7.87 - |
| 419 | 158-160 |
| 420 | 97-99 |
| 421 | 86-88 |
| 422 | 145.5-147 |
| 423 | 146-147.5 |
| 424 | 85-87 |

(continued)

| Compound No. | m.p. (˚C) or NMR |
|---|---|
| 425 | 145-146 |
| 426 | 156-158.5 |
| 427 | 207.5-208.5 |
| 428 | 1H NMR (200 MHz, CHLOROFORM-D) d ppm 1.35 (s, 9 H), 2.15 (s, 3H), 5. 21 (s, 2 H), 7. 10 -7.18 (m, 1H), 7. 23 - 7. 57 (m, 6H), 7.84 - 7. 92 (m, 2 H) |
| 429 | 94-95 |
| 430 | 186.5-188 |
| 431 | 273-274.5 |
| 432 | 139-142 |
| 433 | 171-173 |
| 434 | 1H NMR (200 MHz, CHLOROFORM-D) d ppm 1.20 (t. J=7.3 Hz, 3 H), 1.36 (s. 9H). 2.18 (s, 3 H), 4.13 (g, J=7.0 Hz, 2 H), 4. 68 (s, 2 H), 7. 36 - 7. 53 (m, 3 H), 7. 88 - 7. 95 (m, 2 H) |
| 435 | 163.5-165 |
| 436 | 103-104 |
| 437 | 75-79 |
| 438 | 194-195 |
| 439 | 68-69 |
| 440 | 111-112 |
| 441 | 74-78 |
| 442 | 1H NMR (200 MHz, CHLOROFORM-D) d ppm 0. 19 - 0. 43 (m, 4 H), 0. 91 1. 12 (m, 1 H), 2. 14 (s, 3 H), 2.74 (t, J=6.2 Hz, 4 H), 3.77 (t, J=6. 2 Hz, 2 H), 3. 80 (d, J=7.0 Hz, 2 H), 7. 45 -7. 66 (m, 3 H), 7. 82 - 8.01 (m, 2 H), 8.28 - 8.38 (m, 2 H), 8.85 - 8.93 (m, 1 H) |
| 443 | 135-136.5 |
| 444 | 144-146 |
| 445 | 170-172 |
| 446 | 1H NMR (200 MHz, CHLOROFORM-D) d ppm 4.63 - 4.75 (m, 2 H), 5.05 - 5.27 (m, 2 H).5.65 -5.96 (m. 1 H). 7.10 - 7.64 (m. 8 H), 7.88 - 8.06 (m. 2 H) |
| 447 | 174-175.5 |

Table 11

| Compound No. | 1H-NMR | MASS | m.p. (˚C) |
|---|---|---|---|
| 448 | 1H NMR (200 MHz. CHLOROFORM-d) δ ppm 1.75 - 2.17 (m, 8 H) 2.31 448 (s, 3 H) 3.59 (s, 3 H) 4.38 (d, =7.0 Hz, 2 H) 6.99 (s, 1H) 7.13 - 7.29 (m, 17.29 (m, 1 H) 7.51 - 7.63 (m, 1H) 8.11 - 8.24 (m, 1H) | ESI(Pos) 370 $(M+H)^+$ | 137-133 |
| 449 | 1H NMR (200 MHz, CHLOROFORM-d) δ ppm -0.03 - 0.12 (m, 2 H) 0.38 - 0.55 (m, 2 H) 0.56 - 0.75 (m, 1 H) 1.63 (q, J=7.0 Hz. 2 H) 2.32 (s, 3 H) 3.64 (s, 3 H) 4.37 (t, J=7.0 Hz, 2 H) 6.98 (s, 1 H) 7.12 -7.25 (m. 1H) 8.09 -8.26 (m, 1H) | ESI(Pos) 370 $(M+H))^+$ | 97-98 |
| 450 | 1H NMR (200 MHz. CLOROFORM-d) δ ppm 1.88 - 2.31 (m, 4 H). 2.35 (s, 3 H), 3.63 (s, 3 H), 4.39 (t, J=7.0 Hz, 2 H), 7.00 (s, 1 H) 7.13 - 7.29 (m, 1 H), 7.52 - 7.64 (m, 5 H), 8.09 - 8.23 (m, 1 H) | ESI(Pos) 412 $(M+H)^+$ | amorphous |
| 451 | 1H NMR (200 MHz, CHLOROFORM-d) δ ppm 2.33 (s, 3 H) 3.30 (s, 3 H) 3.63 -3.74 (m, 5 H) 4.45 (t, J=4.6 Hz, 2 H) 6.95 (s, 1 H) 7.15 - 7.25 (m, 1 H) 7.51 - 7.62 (m, 1 H) 8.11 - 8.22 (m, 1 H) | FAB(Pos) 360 $(M+H)^+$ | 144-145 |
| 452 | 1H NMR (200 MHz, CHLOROFORM-d) δ ppm 1.05 - 1.22 (m, 3 H) 2.33 (s, 3 H) 3.33 - 3.53 (m, 2 H) 3.72 (s, 3 H) 3.63 - 3.81 (m, 2 H) 4.35 - 4.53 (m, 2 H) 6.96 (s, 1 H) 7.10 - 7.25 (m, 1 H) 7.49 - 7.62 (m, 1 H) 8.07 - 8.25 (m, 1 H) | FAB(Pos) 374 $(M+H)^+$ | 136-137 |
| 453 | 1H NMR (200 MHz, CHLOROFORM-d) δ ppm 1.89-2.17 (m, 2 H) 2.33 (s, 3 H) 3.32 (s, 3 H) 3.38 (t, J=5.7 Hz, 2 H) 3.65 (s, 3 H) 4.38 (t. J=6.8 Hz 2 H) 6.98 (s, 1 H) 7.13 - 7.25 (m, 1 H) 7.51 - 7.62 (m, 1 H) 8.12 - 8.23 (m, 1H) | ESI(Pos) 374 $(M+H)^+$ | 88-90 |
| 454 | 1H NMR (200 MHz, CHLOROFORM-d) δ ppm 1.08 (d, J=6.2 Hz, 6 H) 2.33 (s, 3H) 3.43-3.59 (m, 1 H) 3.73 (t, J=4.4 Hz, 2H) 3.73 (s, 3H 4.45 (t, J=4.4 Hz. 2 H) 6.96 (s, 1 H) 7.14 - 7.25 (m, 1 H) 7.50 - 7.61 (m, 1 H) 8.11 - 8.22 (m, 1 H) | ESI(Pos) 388 $(M+H)^+$ | 88-90 |
| 455 | 1H NMR (200 MHz, CHLOROFORM-d) δ ppm 2.34 (s, 3H) 2.51 2.71 (m, 1 H) 3.02 - 3.32 (m, 3 H) 3.62 (m, 1 H) 3.65 (s, 3H) 4.39-4.54 (m, 1 H) 5.04 5.27 (m, 1 H) 7.04 (s, 1 H) 7.25 (m, 1H) 7.55-7.67 (m, 1 H) 8.04 -8.16 (m, H) | ESI(Pos) 420 $(M+H)^+$ | amorphous |
| 456 | 1 H NMR (600 MHz, CHLOROFORM-d) δ ppm 2.58-2.81 (m, 3 H). 5.15 (d, 1 H, J=10.55 Hz), 5.53 - 5.55 (m, 2 H), 5.65 - 5.74 (m, 1H), 5.74-5.78 (m, 2 H), 7.07-7.10 (m, 1 H). 7.16-7.18 (m, 1 H), 7.32-7.36 (m, 2 H), 7.38-7.43 (m, 1 H), 7.71-7.76 (m, 1 H), 8.15-8.19 (m, 1 H) | ESI(Pos) 470 $(M+H)^+$ | amorphous |
| 457 | 1H NMR (200 MHz CHLOROFORM-*d*) δ ppm 0.64 (m, 4H), 0.96-1.17 (m, 1H), 1.42 (s, 9 H), 3.31 (s, 3 H), 4.12 (d, J=7.0 Hz, 2 H), 6.60 (s, 1H), 8.78 (s, 7H) | ESI(Pos) 236 $(M+H)^+$ | 122-124 |
| 458 | 1H NMR (200 MHz, CHLOROFORM-d) δ ppm 0.51 - 0.63 (m, 4 H) 0.93 -1.14 (m, 1 H) 1.43 (s, 9 H) 3.90 (s. 3 H) 4.21 (d, J=7.0 Hz. 2 H) 6.94 (s. 1 | ESI(Pos) 304 $(M+H)^+$ | 122-124 |
| 459 | 1 H NMR (200 MHz, CHLOROFORM-d) δ ppm 0.41 - 0.83 (m, 4H) 1.00 - 1.18 (m, 1 H) 1.26 (d, J=7.0 Hz, 6 H) 1.44 (s, 9 H) 3.79-3.96 (m, 1 H) 4.20 (d, J=6.6 Hz, 2 H) 7.06 (s, 1 H) 7.08 - 7.35 (m, 4 H) 7.57 - 7.65 (m, 1 H) | ESI(Pos) 354 $(M+H)^+$ | 113-114 |

(continued)

| Compound No. | 1H-NMR | MASS | m.p. (˚C) |
|---|---|---|---|
| 460 | 1H MMR (200 MHz, CHLOROFORM-d) δ ppm 0.39 - 0.62 (m, 4 H) 0.97 459 - 1.18 (m, 1 H) 1.43 (s, 3H) 3.81 (s, 3H) 4.18 (d, J=7.0 Hz, 2 H) 7.02 (s, 1 H) 7.24 - 7.79 (m, 4 H) | ESI(Pos) 380 $(M+H)^+$ | 83-85 |
| 461 | 1 H NMR (200 MHz, CHLOROFORM-d) δ ppm 0.51 - 0.68 (m, 4 H) 1.00 1.29 (m, 1H) 1.45 (s, 9H) 3.85 (s. 3H) 4.30 (d, J=7.0 Hz, 2H) 7.10 (s,1) H) 740-7.55 7.55 (m, 1H) 7.58-7.68 (m, 1H) 8.35 - 8.47 (m, 1H) 8.48 -8.58 (m, 1H) | ESI(Pos) 380 $(M+H)^+$ | 97-99 |
| 462 | 1H NMR (200 MHz, CHLOROFORM-d) δ ppm 0.52 - 0.65 (m, 4 H) 1.02 -1.23 (m, 1H) 1.44 (s, 9H) 3.64H (s, 3H) 4.29 (d, J=6.6 Hz, 2H) 5.81 -6.22 (m, H) 7.10 (s, 1 H) 7.16- 7.29 (m, 1H) 7.32 - 7.44 (m, 1 H) 8.07 -8.20 (m, 2 H) | ESI(Pos) 428 $(M+H)^+$ | 110-111 |
| 463 | 1H NMR (200 MHz, CHLOROFORM-d) δ ppm 0.49 - 0.61 (m, 4H) 1.01 - 1.17 (m, 1 H) 1.45 (s, 9 H) 3.85 (s, 3 H) 4.22 (d, J=6.6 Hz, 2 H) 7.08 (s, H) 7.10 - 7.22 (m, 1H) 7.33-7.41 (m, 1H) 7.47-7.55 (m, 1 H) | ESI(Pos) 380 $(M+H)^+$ | 158-159 |
| 464 | 1H NMR (200 MHz, CHLOROFORM-d) δ ppm 0.54 - 0.61 (m, 4H) 1.02-1.18 (m, 1H) 1.44 (s, 3H) 4.26 (d, J=7.0 Hz. 2H) 6.94 7.05 (m, 1 H) 7.07 (s, 1H) 7.46 - 7.56 (m, 1H) 7.83 - 7.93 (m, 1 H) | ESI(Pos) 408 $(M+H)^+$ | 127-128 |
| 465 | 1H NMR (200 MHz, CHLOROFORM-d) δ ppm 0.47 - 0.62 (m, 4 H) 1.00 -1.19 (m, 1 H) 1.44 (s, 9 H) 2.43 (s, 3 H) 3.82 (s, 3 H) 4.20 (d, J=7.0 Hz. 2 7.08 (s, 3 H) 7.11 - 7.19 (m, 2 H) 7.29 - 7.40 (m, 1 H) | ESI(Pos) 404 $(M+H)^+$ | 153-155 |
| 466 | 1H NMR (200 MHz. CHLOROFORM-d) δ ppm 0.49-0.60 (m, 4 H) 1,01 - 1.17 (m, 1H) 1.45 (s, 9 H) 2.48 (s, 3 H) 3.82 (s, 3 H) 4.22 (d, J=7.0 Hz, 2 H) 7.06 (s, 1H) 7.08 - 7,30 (m, 3H) | ESI(Pos) 452 $(M+H)^+$ | 147-148 |
| 467 | 1H NMR (200 MHz, CHLOROFORM-d) δ ppm 0.43-0.65 (m, 4 H) 0.98 - 467 1.21 (m, 1 H) 1.45 (s, 9 H) 3.82 (s, 3 H) 4.19 (d, J=6.6 Hz, 2 H) 7.03-7.15 (m, 2 H) 7.24-7.33 (m, 1 H) 7.49-7.57 (m, 1 H) | ESI(Pos) 380 $(M+H)^+$ | 127-128 |
| 468 | 1H NMR (200 MHz CHLOROFORM-*d*) δ ppm 0.46 - 0.65 (m, 4H) 1.00 1.17(m,1H)1.45(6.9H)2.60(s.3H)3.83(s. 3H)4.21(d,J=6.2Hz,2H) 6.96-7.09(m,1H)7.06(s,1H)7.45-7.60(m,2H) | ESI(Pos) 404 $(M+H)^+$ | 138-138 |
| 469 | 1H NMR (200 MHz, CHLOROFORM-d) δ ppm 0.43-0.64 (m, 4 H) 0.98-1,17 (m, 1 H) 1,45 (s, 9 H) 2.63 (s, 3 H) 3.82 (s, 3 H) 4.20 (d, J=7.0 Hz, 2 H) 6.78 - 6.90 (m, 1H) 7.05 (s, 1H) 7.53-761 (m, 1H) 7.73 - 7.80 (m, 1H) | ESI(Pos) 452 $(M+H)^+$ | 139-140 |
| 470 | 1H NMR (200 MHz, CHLOROFORM-d) δ ppm 0.43-0.65 (m, 4H) 0.98 -1.16 (m, 1H) 1,45 (s, 9H) 2.59-2.66 (m, 3H) 3.83 (s, 3H) 4.20 3H) 4.20 (d, J=7.0 Hz. 2 H) 7.06 (s, 1H) 7.17 - 7.28 (m, 1H) 7.51 - 7.58 (m, 1H) 1.68 - 7.77 (m, 1 H) | ESI(Pos) 394 $(M+H)^+$ | 90-91 |
| 471 | 1H MMR (200 MHz, CHLOROFORM-d) δ ppm 0.50 - 0.60 (m, 4 H) 1.02 1.19 (m, 1H) 1.43 (s, 9H) 2.31 (s. 3H) 3.81 (s. 3H) 4.24 (d, J= 7.0 Hz, 2 H) 7.03 - 7.11 (m,1 H) 7.09 (s, 1H) 7.36-7.40 (m, 1H) 7.59 - 7.67 (m, 1 | ESI(Pos) 404 $(M+H)^+$ | 153-154 |
| 472 | 1H NMR(200MHz,CHLOROFORM-d) δ pm 0.50-0.62(m,4H)0.97 -1.20 (m, 1 H) 1.43 (s, 8 H) 3.80 (s, 3 H) 3.85 (s, 3 H) 4.19 (d, J=6.6 Hz, 2 H) 6.84 - 6.93(m, 2 H) 7.13 (s,1 H) 7.60 - 7.69 (m, 1 H) | ESI(Pos)376 $(M+H)^+$ | 128-129 |

(continued)

| Compound No. | 1H-NMR | MASS | m.p. (˚C) |
|---|---|---|---|
| 473 | 1H NMR (200 MHz, CHLOROFORM-d) δ ppm 0.51 - 0.63 (m, 4 H) 1.02. - 1.18 (m, 1 H) 1.45 (s, 9 H)3.85 (s, 3H) 4.24 (d, J=7.03 Hz,2 2H) 7.08 (s, 1 H) 7.13 - 7.21 (m, 1H) 7.24 - 7.32 (m, 1H) 7.66 - 7.75 (m, J=2.64 Hz, 1H) | ESI(Pos) 380 $(M+H)^+$ | 164-165 |
| 474 | 1H NMR (200 MHz, CHLOROFORM-d) d ppm 0.51-0.65 (m, 4 H) 1.01 - 1.19 (m, 1 H) 1.45 (s, 9 H) 3.88 (s, 3 H) 4.26 (d, J=7.0 Hz, 2 H) 7.07 (s, 1 H) 7.13-7.25 (m, 1 H) 7.49 - 7.83 (m, 1 H) 8.07 - 8.22 (m, 1 H) | ESI(Pos) 398 $(M+H)^+$ | 104-106 |
| 475 | 1H NMR (200 MHz, CHLOROFORM-d) δ ppm 0.53 - 0.65 (m, 4 H) 1.03 - 1.18 (m, 1 H) 1.45 (s, 9 H) 3.86 (s, 3 H) 4.27 (d, J=7.0 Hz, 2 H) 7.06-7.19 (m, 1 H) 7.10 (s, 1 H) 7.50 - 7.60 (m, 1 H) 8.27 - 8.35 (m, 1 H) | ESI(Pos) 388 $(M+H)^+$ | 124-126 |
| 476 | 1H NMR (200 MHz, CHLOROFORM-d) d ppm 0.47 - 0.62 (m, 4H) 1.00 - 1.16 (m, 1H) 1.45 (s, 8, H) 3,85 (s, 3 H) 4.21 (d, J=7.0 Hz, 2 H) 7.07 (s, 1 H) 7.27 -7.37 (m, 1H) 7.57 - 7.64 (m, 1 H) 7.69-7.77 (m, 1H) | ESI(Pos) 414 $(M+H)^+$ | 145-146 |
| 477 | 1H NMR (200 MHz, CHLOROFORM-d) δ ppm 0.50-0.66 (m, 4 H) 1.02 -1.17 (m, 1H) 1.45 (s, 8H)2.68 (s, 3H) 3.84 (s,3H) 4.24 (d, J=66 Hz, 2 H) 7.06 (s, 1 H) 7.21 -7.29 (m, 1H) 7.40 - 7.47 (m, 1 H) 8.13 8.17 (m, 1 H) | ESI(Pos) 393 $(M+H)^+$ | 156-157 |
| 478 | 1H NMR (200 MHz, CHLOROFORM-d) δ ppm 0.51 - 0.65 (m, 1H) 0.98 -119 (m, 1 H), 1.43 (s, 9 H) 3.82 (s, 3 H) 3.60 (s, 3 H) 4.20 (d, J=7.0 Hz, 2 H) 8.91 - 701 (m, 1 H) 7.12 (s, 1 H) 7.46 - 7.55 (m, 1 H) 7.94 - 8.01 (m, 1 H) | ESI(Pos) 410 $(M+H)^+$ | 99-101 |
| 479 | 1H NMR (200 MHz, CHLOROFORM-d) δ ppm 0.45-0.66 (m. 4H) 1.00- 1.20 (m, 1H) 1.44 (s, 8H) 2.58 (s, 3 H) 3.82 (s, 3 H) 4.22 (d, J=7.0 Hz, 2H) 6.82 6.96 (m, H) 7.03-7.15 (m, 2H) 7.50-7.61 (m, 1H) | ESI(Pos) 344 $(M+H)^+$ | 161-162 |
| 480 | 1H NMR (200 MHz, CHLOFORM-d) δ ppm 0.53-0.63 (m, 4 H) 1.03 -1.25 (m, 1 H) 1.43 (s, 9 H) 2.40 (s, 3 H) 3.82 (s, 3 H) 4.28 (d, J=7.0 Hz, 2 H) 7.17 - 7.29 (m, 1 H) 7.97-8.06 (m, 1 H) 8.19-8.24 (m, 1 H) | ESI(Pos) 360 $(M+H)^+$ | 135-136 |
| 481 | 1H NMR (200 MHz, CHLOROFORM-d) δ ppm 1.43 (s, 9 H) 1.82 - 2.08 (m, 6 H) 2.56-2.75 (m, 1 H) 3.73 (s, 3 H) 4.34 (d, J=7.03 Hz, 2 H) 7.08 (s, 1H) 7.17 (t, J= 7.91) Hz, 1H) 7.38 (dd, J=7.91, 1.76 Hz, 1H) 7.55 (dd. J=7.47, 1.76 Hz. 1 H) | ESI(Pos) 394 $(M+H)^+$ | 152.5-154 |
| 482 | 1H NMR (200 MHz, CHLOROFORM-d) δ ppm 1.42 (s. 9 H) 1.83-2.11 (m, 6 H) 2.55-2.77 (m, 1 H) 3.74 (s, 3 H) 4.38 (d, J=7.0 Hz, 2 H) 7.07 (s, 1) H) 7.15-7.26 (m, 1 H) 7.52-7.62 (m, 1 H) 8.12-8.25 (m, 1 H), | ESI(Pos) 412 $(M+H)^+$ | amorphous |
| 483 | 1H NMR (600 MHz, CHLOROFORM-d) δ ppm 1.39 - 1.46 (m, 9 H) 1.82 - 1.92 (m, 4 H) 1.97 - 206 (m, 2 H) 2.59 - 2.68 (m, 1 H) 2.64 (s, 3 H) 3.72 (s, 3 H) 4.31 (s, 2 H) 7.04 (s, 1H) 7.20 - 7.24 (m, J=8.25 Hz, 1 H) 7.52 -7.59 (m, 1H) 7.72 - 180 (m, 1H) | ESI(Pos) 408 $(M+H)^+$ | 133-134 |
| 484 | 1H HMR (200 MHz, CHLOROFORM-d) δ ppm 1.44 (s, 8 H) 1.48 - 1.80 (m, J=11.43 Hz, 8 H) 2.13 - 2.43 (m, 1 H) 3.76 (s, 3H) 4.23 (d, J=7.91 Hz, 2 H) 7.09 (s, 1 H) 7.16 (t, J=7.91 Hz, 1 H) 7.34 - 7.41 (m, 1 H) 7.53 (dd. J=7.69 1.54 Hz, 1 H) | ESI(Pos) 408 $(M+H)^+$ | 168-169 |
| 485 | 1H NMR (200 MHz, CHLOROFORM-d) δ ppm 1.37 - 1.80 (m, 8 H) 1.39 -149 (m, 9H) 2.18 - 2.40 (m, 1H) 3.77 (s, 3 H) 4.27 (d, J=7.47 Hz, 2 H) 7.08 (s, 1 H) 7.20 (t J=7.69 Hz, 1H) 7.50 - 7.63 (m, 1 H) 8.06 - 8.24 (m, 1 H) | ESI(Pos) 426 $(M+H)^+$ | 140.5-141.5 |

(continued)

| Compound No. | 1H-NMR | MASS | m.p. (˚C) |
|---|---|---|---|
| 486 | 1H NMR (200 MHz, CHLOFOFORM-d) δ ppm 1,11 (t, J=7.0 Hz, 3H) 1.44 (s, 9 H) 3.41 (, J=7.0 Hz, 2 H) 3.69 (t, J=4.7 Hz; 2 H) 3.88 (s, 3 H) 4.43 (t, J=4.7 Hz. 2 H) 7.03 (s, 1 H) 7.11 - 7.21 (m, 1H) 7.33 - 7.41 (m, 1 H) 7.51 - 7.58 (m, 1 H) | ESI(Pos) 398 $(M+H)^+$ | 145-146 |
| 487 | 1H NMR (600 Hz, CHOROFORM-d) δ ppm 1.10 (t, J=8.9 Hz, 3 H) 1.42 (s, 9 H) 2.42 (s, 3 H) 3.99 (q, J=6.9 Hz, 2 H) 3.69 (t, J=4.6 Hz, 2 H) 3.85 (s, 3 H) 4.42 (br. s, 2 H) 7.02 (s, 1 H) 7.11 - 7.17 (m, 2 H) 7.35 - 739 (m, 1H) | ESI(Pos) 422 $(M+H)^+$ | 168-167 |
| 488 | 1H NMR (200 MHz, CHLOROFORM-d) δ ppm 1.12 (t, J=7.0 Hz, 3H) ESI(Pos) 416 $(M+H)^+$ 1.43 (s,9 H) 3.43 (q, J=7.0 Hz.2 H) 3.73 (t, J=4.8 Hz. 2H) 3.89 (s, 3 H) 4.47 (t J=4.8 Hz, 2 H)7.02 (s, H) 7.14 - 7.25 (m, 1H) 7.51 - 7.61 - 7.61 (m,1 H) 8.10 - 8.23 (m, 1 H) | ESI(Pos) 416 $(M+H)^+$ | 95-96 |
| 489 | 1H NMR(600MHz,CHLOROFORM-d) δ ppm 1.11 (t,J=6.88 Hz,3H) 1.43 (s,9H)2.63(s,3H)3.41(q,J=6.88Hz,2H) 3.87(t,J=4.81Hz,2H) 3.87 (s,3H)4.40-4.47(m.2H)7.01(s,1H)7.22(dd,J=7.79Hz,1H) 7.54(d,J=7.79Hz,1H)7.75(d, J=7.79Hz,1H) | ESI(Pos)412$(M+H)^+$ | 132-133 |
| 490 | 1HNMR(200MHz,CHLOROFORM-d)δppm1.12(t,J=7.0Hz,3H) 143(s,9H)2.59(s,3H)3.42(q,J=7.0Hz,2H)3.68(t, J=4.6Hz,2H) 3.86(s,3H)4.42(t,J=4.6Hz,2H)7.02(s,1H)7.04-7.14(m,1H)7.25 -7.33(m,1H)7.54-7.60(m,1H) | ESI(Pos)378$(M+H)^+$ | 127-128 |
| 491 | 1HNMR(200MHz,CHLOROFORM-d)δppm1,12(t,J=6.8Hz,3H)1.43(s,9H)2.57-2.68(m,3H)3,41(q,J=6.8Hz.2H) 3.68(t,J=4.8Hz, 2H)4.42(t,J=4.8Hz.2H)6.95-7.06(m,1H)7.02(s,1H)7.43-7.53 (m,1H)7.55-764(m,1H) | ESI(Pos)422 $(M+H)^+$ | 125-126 |
| 492 | 1HNMR(600MHz,CHLOROFORM-d)δppm1.05(d,J=8.0Hz.6H), 1.42(s,9H).3.41-3.50(m,1H).3.64-3.71(m,2H), 388(s,3H),437 -4.45(m,2H),7.02(s,1H),7.13-7.18(m.1H),7.34-7.38(m.1H), 7.51-7.56(m,1H) | ESI(Pos)412$(M+H)^+$ | amorphous |
| 493 | 1HNMR(200MHz,CHLOROFORM-d)δppm1,06(d,J=6.2Hz,6H) 1.43(s,9H)3.42-3.57(m,1H)3.73(t,J=4.8Hz.2H) 3.90(s,3H,4.46 (t,J=4.8Hz,2H)7.03(s,1H)7.13-7.25(m,1H)7.51-7.82(m,1H) 812-8.24(m,1H) | ESI(Pos)430$(M+H)^+$ | amorphous |
| 494 | 1HNMR(600MHz.CHLOROFORM-d)δppm1.06(d,J=6.4Hz,6H), 1.43(s,9H),2.63(s,3H),3.44-3.50(m,1H).3.66(t, J=4.8Hz,2H) 3.88(s,3H),4.40(br,s,2H),7.02(s.H).7.19-7.25(m,1H)7.53-7.56(m,1H),7.74-7.79(m,1H) | ESI(Pos)426$(M+H)^+$ | amorphous |
| 495 | 1HNMR(200MHz,CHLOROFORM-d)δppm0.48-0.62(m,4H)1.03 -1.22(m,1H)1.29(tJ=6.8Hz,3H)1.48(s,9H)4.28 (q,J=6.8Hz,H) 4.25(d,J=7.0Hz,2H)7.14(s,1H)7.17-7.29(m,1H)7.54-7.67(m,1 H)8.12-8.24(m,1H) | ESI(Pos)412$(M+H)^+$ | amorphous |
| 496 | 1HNMR(200$MH_2$CHLOROFORM-d)δppm0.43-0.69(m,4H)0.99 -1,21(m,1H)1.52(s.9H)2.15(s,3H)3.85(s,3H) 4.14(d.J=7.0Hz.2 H)7.14-7.25(m,1H)7.49-7.60(m,1H)7.96-8.09(m,1H) | ESI(Pos)412$(M+H)^+$ | 149-150 |
| 497 | 1HHMR(200MHz,CHLOROFORM-d)dppm0.96-1.07(m,2H)1.12-1.24(m,2H)1.70-1.86(m,1H)3.52(s,3H)6.92(s, 1H)7.02-7.13(m. 1H)7.40-7.63(m,6H)7.91-8.05(m,1H) | ESI(Pos)404$(M+H)^+$ | 168.5-169. 6 |

(continued)

| Compound No. | 1H-NMR | MASS | m.p. (˚C) |
|---|---|---|---|
| 498 | 1HNMR(600MHz,CHLOROFORM-d)dppm3.93(3H,s),7.06-7.10(1 H,m)7.30-7.37(5H,m).7.48-7.50(1H,m), 7.71-7.75(1H,m) | ESI(Pos)446$(M+H)^+$ | 16.9.5-170 |
| 499 | 1HNMR(600MHz,CHLOROFORM-d)dppm3.39(3H,s),3.78(2H,s). 4.47(2H,s),7.32-7.35(1H,m).7.48-7.51(2H, m),7.79-7.83(1H,m),8.13-8.17(1H,m),8.18-8.22(1H,m) | ESI(Pos)361$(M+H)^+$ | 128.5-130 |
| 500 | 1HNMR(600MHz,CHLOROFORM-*d*)dppm0.52-0.56(2H,m)0.83-0.87(2H,m),0.98-1.06(1H,m),1.43(9H,s),3.87 (3H,s),4.18(2H,d, J=6.88Hz),6.92-6.96(1H,m),6.99(1H,s),7.59-7.64(2H,m) | ESI(Pos)375$(M+H)^+$ | amorphous |
| 501 | 1HNMR(600MHz.CHLOROFORM-d)dppm1.42(9H,s),3.89(3H,s), 4.22(2H,d,J=6.88Hz).6.77(1H.s),6.93(1H,s). 754(1H,dd,J=8.94. | ESI(Pos)428$(M+H)^+$ | amorphous |
| 502 | 1HNMR(600MHz.CHLOROFORM-d)dppm0.28-D.37(2H.m),0.51-0.58(2H,m)1.01-1.09(1H,m),1.43(9H,s).3.83 (3H,s),4.04(2H,s), 6.916.95(2H,m),7.36(2H,s),7.36-7.40(2H,m),10.47(1H,s) | ESI(Pos)361$(M+H)^+$ | 147.5-149. 5 |
| 503 | 1HNMR(200MHz,CHLOROFORM-d)δppm0.34-0.57(m,4H)0.89 -1.07(m,1H)1.37s,8H)3.74(s,3H)4.03(d, J=7.0Hz,2H)6.18(s,1 H)7.48-7.60(m,1H)7.62-7.72(m,1H)8.10-8.19(m,1H)8.19-8.26(m,1H) | ESI(Pos)416$(M+H)^+$ | 121-122 |
| 504 | 1HNMR(200MHz,CHLOROFORM-d)dppm0.47-0.68(m,4H)1.35-1.44(m;1H)1.43(s,9H)4.28(d,J=7.0Hz.2H) 7.24-7.35(m.1H)7.65 -7.77,(m,1H)8.25-8.37(m,1H) | ESI(Pos)402$(M+H)^+$ | amorphous |
| 505 | 1HNMR(200MHz,CHLOROFORM-d)dppm0.50-0.68(m,4H)1.36-1.52(m,1H)1.44,(s,9H)4.32(d.J=7.5+Hz.2H) 7.57(t,J=7.7Hz,1H) 7.75(d,J=7.7Hz,1H)8.47(d,J=7.7Hz,1H)8.57(s,1H) | ESI(pos)384$(M+H)^+$ | amorphous |
| 506 | 1HNMR(200MHzCHLOROFORM-d)dppm0.430.68(m,4H)1.30-1.52(m,10H)2.35(s,3H)4.28(d,J=7.5Hz,2H) 7.29-7.41(m,1H) 7.49-1.65(m,1H)8.37-8.47(m,1H) | ESI(Pos)398$(M+H)^+$ | 63-65 |
| 507 | 1HNMR(200MHz,CHLOROFORM-d)dppm0.41-0.65(m,4H)1.29-1.50(m,1H)1.43(s,9H)4.23(d,J=7.5Hz.2H) 7.20-7.33(m,1H)7.40 -7,50(m,1H)7.94-8.05(m,1H) | ESI(Pos)402$(M+H)^+$ | 76-78 |
| 508 | 1HNMR(200MHz,CHLOROFORM-d)ppm0.47-0.67(m,4H)1.33-1.56(m,1H)1.44(s,9H)4.29(d,J=1.0Hz.2H) 7.167.31(m,1H)7.64 -7.78(m,1H)8.39-8.49(m,1H) | ESI(Pos)402$(M+H)^+$ | 47-49 |
| 509 | 1H NMR(200MHz,CHLOROFORM-d)dppm0.44-0.66(m,4H)1.30-1.52(m,1H)1.42(s,9H)3.97(s,3H)4.28(d, J=7.5Hz,2H)7.01-7.10 (m,1H)7.61-7.70(m,1H)8.27-8.33(m,1H) | ESI(Pos)414$(M+H)^+$ | 82-83 |
| 510 | 1HNMR(200MHz,CHLOROFORMS-d)dppm0.41-0.62(m,4H)1.29-1.5(m,1H)1.47(s,9H)4.30(d,J-7.5Hz.2H) 7.52-7.89(m,4H)8.59 -8.88(m,2H) | ESI(Pos)367$(M+H)^+$ | 89-90 |
| 511 | 1HNMR(200MHz,CHLOROFORM-d)dppm0.43-0.67(m,4H)1.28-1.51(m,4H)1.44(s,9H)4.27(d,J=7.5Hz,2H) 7.33-7.43(m,1H)8.46 -8.57(m,1H)9.20-9.33(m,1H) | ESI(Pos)351$(M+H)^+$ | 62-63 |

(continued)

| Compound No. | 1H-NMR | MASS | m.p. (˚C) |
|---|---|---|---|
| 512 | 1HNMR,(200MHz,CHLOROFORM-d)dppm0.44-0.65(m,4H)1.31-1.50(m,1H)1.42(s,9H)3.98(s,3H)4.26(d, J=7.0Hz,2H)6.90(d. J=5.7Hz,1H)8.54(d,J=5.7Hz,1H)9.22(s,1H) | ESI(Pos)347$(M+H)^+$ | 94-95 |
| 513 | 1HNMR(200MHz,CHLOROFORM-d)dppm0.40-0.66(m,4H)1.29-1.50(m,1H)1.42(s,9H)1.50(t,J=7.0Hz,3H) 4.24(q,J=7.0Hz,2H) 4.25(d,J=7.0Hz,2H)6.87(d,J=6.2Hz,H)8.49(d,J=6.2Hz,1H)9.17 (s,1H) | ESI(Pos)361$(M+H)^+$ | amorphous |
| 514 | 1HNMR(200MHz,CHLOROFORM-d)dppm0.41-0.67(m,4H)1.23-1.35(m,1H)1.41(s,9H)3.82(d,J=7.5Hz,2H) 7.20-7.31(m,1H)7.63 -7.74(m,1H)8.16-8.27(m,1H) | ESI(Pos)386$(M+H)^+$ | 108-110 |
| 515 | 1HNMR(200MHz,CHLOROFORM-d)dppm0.38-0.70(m,4H)1.18- 1.38(m,1H)1.41(s.9H)3.83(d,J=7.5Hz,2H) 7.13-7.30(m,1H)7.59 -7.75(m,1H)8.29-8.43(m,1H) | ESI(Pos) 386$(M+H)^+$ | 61-63 |

Table 12

| Compound No. | MASS | Compound No. | MASS | Compound No. | MASS |
|---|---|---|---|---|---|
| 1001 | APCI(Pos) 247 (M+H) | 1026 | APCI(Pos) 303 (M+H) | 1051 | APCI(Pos) 283 (M+H) |
| 1002 | APCI(Pos) 233 (M+H) | 1027 | APCI(Pos) 303 (M+H) | 1052 | APCI(Pos) 297 (M+H) |
| 1003 | APCI(Pos) 247 (M+H) | 1028 | APCI(Pos) 341 (M+H) | 1053 | APCI(Pos) 287 (M+H) |
| 1004 | APCI(Pos) 288 (M+H) | 1029 | APCI(Pos) 267 (M+H) | 1054 | APCI(Pos) 337 (M+H) |
| 1005 | APCI(Pos) 259 (M+H) | 1030 | APCI(Pos) 267 (M+H) | 1056 | APCI(Pos) 337 (M+H) |
| 1006 | APCI(Pos) 273 (M+H) | 1031 | APCI(Pos) 267 (M+H) | 1056 | APCI(Pos) 345 (M+H) |
| 1007 | APCI(Pos) 287 (M+H) | 1032 | APCI(Pos) 281 (M+H) | 1057 | APCI(Pos) 345 (M+H) |
| 1008 | APCI(Pos) 281 (M+H) | 1033 | APCI(Pos) 281 (M+H) | 1058 | APCI(Pos) 345 (M+H) |
| 1009 | APCI(Pos) 281 (M+H) | 1034 | APCI(Pos) 295 (M+H) | 1059 | APCI(Pos) 369 (M+H) |
| 1010 | APCI(Pos) 295 (M+H) | 1035 | APCI(Pos) 295 (M+H) | 1060 | APCI(Pos) 365 (M+H) |
| 1011 | APCI(Pos) 343 (M+H) | 1036 | APCI(Pos) 321 (M+H) | 1061 | APCI(Pos) 368 (M+H) |
| 1012 | APCI(Pos) 353 (M+H) | 1037 | APCI(Pos) 335 (M+H) | 1062 | APCI(Pos) 343 (M+H) |
| 1013 | APCI(Pos) 273 (M+M) | 1038 | APCI(Pos) 271 (M+H) | 1063 | APCI(Pos) 328 (M+H) |
| 1014 | APCI(Pos) 285 (M+H) | 1039 | APCI(Pos) 271 (M+H) | 1064 | APCI(Pos) 329 (M+H) |
| 1016 | APCI(Pos) 285 (M+H) | 1040 | APCI(Pos) 271 (M+H) | 1065 | APCI(Pos) 359 (M+H) |
| 1016 | APCI(Pos) 273 (M+H) | 1041 | APCI(Pos) 287 (M+H) | 1065 | APCI(Pos) 352 (M+H) |
| 1017 | APCI(Pos) 279 (M+H) | 1042 | APCI(Pos) 287 (M+H) | 1067 | APCI(Pos) 344 (M+H) |
| 1018 | APCI(Pos) 245 (M+H) | 1043 | APCI(Pos) 287 (M+H) | 1068 | APCI(Pos) 338 (M+H) |
| 1019 | APCI(Pos) 259 (M+H) | 1044 | APCI(Pos) 33 (M+H) | 1069 | APCI(Pos) 278 (M+H) |
| 1020 | APCI(Pos) 307 (M+H) | 1045 | APCO(Pos) 331 (M+H) | 1070 | APCI(Pos) 278 (M+H) |
| 1021 | APCI(Pos) 293 (M+H) | 1046 | APCI(Pos) 331 (M+H) | 1071 | APCI(Pos) 278 (M+H) |
| 1022 | APCI(Pos) 293 (M+H) | 1047 | APCI(Pos) 373 (M+H) | 1072 | APCI(Pos) 349 (M+H) |
| 1023 | APCI(Pos) 289 (M+H) | 1048 | APCI(Pos) 379 (M+H) | 1073 | APCI(Pos) 331 (M+H) |
| 1024 | APCI(Pos) 289 (M+H) | 1049 | APCI(Pos) 283 (M+H) | 1074 | APCI(Pos) 331 (M+H) |
| 1025 | APCI(Pos) 253 (M+H) | 1050 | APCI(Pos) 283 (M+H) | 1075 | APCI(Pos) 281 (M+H) |

(continued)

| Compound No. | MASS | Compound No. | MASS | Compound No. | MASS |
|---|---|---|---|---|---|
| 1076 | APCI:(Pos) 281 (M+H) | 1101 | APCI(Pos) 349 (M+H) | 1126 | APCI(Pos) 285 (M+H) |
| 1077 | APCI(Pos) 281 (M+H) | 1102 | APCI(Pos) 349 (M+H) | 1127 | APCI(Pos) 301 (M+H) |
| 1078 | APCI(Pos) 281 (M+H) | 1103 | APCI(Pos) 349 (M+H) | 1128 | APCI(Pos) 301 (M+H) |
| 1079 | APCI(Pos) 335 (M+H) | 1104 | APCI(Pos) 349 (M+H) | 1128 | APCI(Pos) 301 (M+H) |
| 1080 | APCI(Pos) 335 (M+H) | 1146 | APCI(Pos) 349 (M+H) | 1130 | APCI(Pos) 301 (M+H) |
| 1081 | APCI(Pos) 365 (M+H) | 1106 | APCI(Pos) 349 (M+H) | 1151 | APCI(Pos) 345 (M+H) |
| 1082 | APCI(Pos) 399 (M+H) | 1107 | APCI(Pos) 349 (M+H) | 1132 | APCI(Pos) 345 (M+H) |
| 1083 | APCI(Pos) 398 (M+H) | 1108 | APCI(Pos) 365 (M+H) | 1133 | APCI(Pos) 345 (M+H) |
| 1084 | APCI(Pos) 399 (M+H) | 1109 | APCI(Pos) 365 (M+H) | 1134 | APCI(Pos) 345 (M+H) |
| 1085 | APCI(Pos) 289 (M+H) | 1110 | APCI(Pos) 365 (M+H) | 1135 | APCI(Pos) 345 (M+H) |
| 1086 | APCI(Pos) 289 (M+H) | 1111 | APCI(Pos) 385 (M+H) | 1136 | APCI(Pos) 345 (M+H) |
| 1087 | APCI(Pos) 289 (M+H) | 1112 | APCI(Pos) 397 (M+H) | 1137 | APCI(Pos) 393 (M+H) |
| 1088 | APCI(Pos) 288 (M+H) | 1113 | APCI(Pos) 397 (M+H) | 1138 | APCI(Pos) 393 (M+H) |
| 1089 | APCI(Pos) 289 (M+H) | 1114 | APCI(Pos) 313 (M+H) | 1139 | APCI(Pos) 393 (M+H) |
| 1090 | APCI(Pos) 289 (M+H) | 1115 | APCI(Pos) 313 (M+H) | 1140 | APCI(Pos) 339 (M+H) |
| 1091 | APCI(Pos) 305 (M+H) | 1116 | APCI(Pos) 313 (M+H) | 1141 | APCI(Pos) 339 (M+H) |
| 1092 | APCI(Pos) 305 (M+H) | 1117 | APCI(Pos) 313 (M+H) | 1142 | APCI(Pos) 339 (M+H) |
| 1093 | APCI(Pos) 305 (M+H) | 1118 | APCI(Pos) 313 (M+H) | 1143 | APCI(Pos) 407 (M+H) |
| 1094 | APCI(Pos) 305 (M+H) | 1119 | APCI(Pos) 341 (M+H) | 1144 | APCI(Pos) 339 (M+H) |
| 1095 | APCI(Pos) 285 (M+H) | 1120 | APCI(Pos) 341 (M+H) | 1145 | APCI(Pos) 339 (M+H) |
| 1096 | APCI(Pos) 305 (M+H) | 1121 | APCI(Pos) 341 (M+H) | 1146 | APCI(Pos) 355 (M+H) |
| 1097 | APCI(Pos) 321 (M+H) | 1122 | APCI(Pos) 341 (M+H) | 1147 | APCI(Pos) 355 (M+H) |
| 1098 | APCI(Pos) 321 (M+H) | 1123 | APCI(Pos) 285 (M+H) | 1148 | APCI(Pos) 355 (M+H) |
| 1099 | APCI(Pos) 321 (M+H) | 1124 | APCI(Pos) 285 (M+H) | 1149 | APCI(Pos) 339 (M+H) |
| 1100 | APCI(Pos) 321 (M+H) | 1125 | APCI(Pos) 285 (M+H) | 1150 | APCI(Pos) 297 (M+H) |

(continued)

| Compound No. | MASS | Compound No. | MASS | Compound No. | MASS |
|---|---|---|---|---|---|
| 1151 | APCI(Pos) 297 (M+H) | 1178 | APCI(Pos) 323 (M+H) | 1201 | APCI(Pos) 333 (M+H) |
| 1152 | APCI(Pos) 297 (M+H) | 1177 | APCI(Pos) 323 (M+H) | 1202 | APCI(Pos) 311 (M+H) |
| 1153 | APCI(Pos) 339 (M+H) | 1178 | APCI(Pos) 323 (M+H) | 1203 | APCI(Pos) 311 (M+H) |
| 1154 | APCI(Pos) 351 (M+H) | 1179 | APCI(Pos) 339 (M+H) | 1204 | APCI(Pos) 325 (M+H) |
| 1155 | APCI(Pos) 301 (M+H) | 1180 | APCI(Pos) 355 (M+H) | 1205 | APCI(Pos) 295 (M+H) |
| 1156 | APCI(Pos) 301 (M+H) | 1181 | APCI(Pos) 355 (M+H) | 1208 | APCI(Pos) 311 (M+H) |
| 1157 | APCI(Pos) 301 (M+H) | 1182 | APCI(Pos) 387 (M+H) | 1207 | APCI(Pos) 268 (M+H) |
| 1158 | APCI(Pos) 317 (M+H) | 1183 | APCR (Pos) 343 (M+H) | 1208 | APCI(Pos) 264 (M+H) |
| 1159 | APCI(Pos) 317 (M+H) | 1184 | APCI(Pos) 343 (M+H) | 1209 | APCI(Pos) 279 (M+H) |
| 1160 | APCI(Pos) 317 (M+H) | 1185 | ESI(Pos) 343 (M+H) | 1210 | APCI(Pos) 322 (M+H) |
| 1161 | APCI(Pos) 361 (MH) | 1186 | APCR (Pos) 311 (M+H) | 1211 | APCI(Pos) 322 (M+H) |
| 1162 | APCI(Pos) 387 (M+H) | 1187 | APCI(Pos) 303 (M+H) | 1212 | APCI(Pos) 322 (M+H) |
| 1163 | APCI(Pos) 468 (M+H) | 1188 | APCI(Pos) 303 (M+H) | 1213 | APCI(Pos) 289 (M+H) |
| 1164 | APCI(Pos) 286 (M+H) | 1189 | APCI(Pos) 319 (M+H) | 1214 | APCI(Pos) 304 (M+H) |
| 1165 | APCI(Pos) 286 (M+H) | 1190 | APCI(Pos319 (M+H) | 1215 | APCI(Pos) 304 (M+H) |
| 1165 | APCI(Pos) 308 (M+H) | 1191 | APCI(Pos) 3(M+H) | 1216 | APCI(Pos) 304 (M+H) |
| 1167 | APCI(Pos) 285 (M+H) | 1192 | APCI(Pos) 367 (M+H) | 1217 | APCI(Pos) 304 (M+H) |
| 1168 | APCI(Pos) 285 (M+H) | 1193 | APCI(Pos) 373 (M+H) | 1218 | APCI(Pos) 304 (M+H) |
| 1168 | APCI(Pos) 307 (M+H) | 1194 | APCI(Pos) 373 (M+H) | 1218 | APCI(Pos) 338 (M+H) |
| 1170 | APCI(Pos) 307 (M+H) | 1196 | APCI(Pos) 327 (M+H) | 1220 | APCI(Pos) 378 (M+H) |
| 1171 | APCI(Pos) 307 (M+H) | 1196 | APCI(Pos) 337 (M+H) | | |
| 1172 | APCI(Pos) 307 (M+H) | 1197 | APCI(Pos) 339 (M+H) | | |
| 1173 | APCI(Pos) 307 (M+H) | 1198 | APCI(Pos) 247 (M+H) | | |
| 1174 | APCI(Pos) 323 (M+H) | 1199 | APCI(Pos) 311 (M+H) | | |
| 1175 | APCI(Pos) 323 (M+H) | 1200 | APCI(Pos) 323 (M+H) | | |

(continued)

| Compound No. | MASS | Compound No. | MASS | Compound No. | MASS |
|---|---|---|---|---|---|
| 1221 | APCI (Pos) 310 (M+H) | 1245 | APCI(Pos)396(M+H) | 1269 | APCI (Pos) 389 (M+H) |
| 1222 | APCI(Pos) 310 (M+H) | 1246 | APCI(Pos) 408 (M+H) | 1270 | APCI:(Pos) 405 (M+H) |
| 1223 | APCI(Pos) 311 (M+H) | 1247 | APCI(Pos) 408 (M+H) | 1271 | APCI(Pos) 360 (M+H) |
| 1224 | APCI(Pos) 311 (M+H) | 1248 | APCI(Po) 410 (M+H) | 1272 | APCI(Pos) 361 (M+H) |
| 1225 | APCI(Pos) 327 (M+H) | 1249 | APCI(Pos)410(M+H) | 1273 | APCI(Pos) 405 (M+H) |
| 1226 | APCI(Pos) 327 (M+H) | 1250 | APCI (Pos) 368 (M+H) | 1274 | APCI(Pos) 405 (M+H) |
| 1227 | APCI(Pos) 211(M+H) | 1251 | APIC(Pos) 387 (M+H) | 1275 | APCI(Pos) 405 (M+H) |
| 1228 | APCI(Pos) 311 (M+H) | 1252 | APCI(Pos) 401 (M+H) | 1276 | APCI(Pos) 372 (M+H) |
| 1229 | APCI(Pos) 311 (M+H) | 1253 | APCI(Pos) 417 (M+H) | 1277 | APCI(Pos) 384 (M+H) |
| 1230 | APCI(Pos) 311 (M+H) | 1254 | APCI(Pos) 417 (M+H) | 1276 | AFCI(Pos) 371 (M+H) |
| 1231 | APCI(Pos) 312 (M+H) | 1255 | APCI(Pos) 421 (M+H) | 1279 | APCI(Pos) 357 (M+H) |
| 1232 | APCI(Pos) 328 (M+H) | 1256 | APCI(Pos) 432 (M+H) | 1280 | APCI(Pos) 405 (M+H) |
| 1233 | APCI(Pos) 329 (M+H) | 1257 | APCI(Pos) 432 (M+H) | 1281 | APCI(Pos) 325 (M+H) |
| 1234 | ESI(Neg) 322 (M-H) | 1258 | APCI(Pos) 446 (M+H) | 1282 | APCI(Pos) 325 (M+H) |
| 1235 | APCI(Pos) 355 (M+H) | 1259 | APCI(Pos) 341 (M+H) | 1283 | ACPI(Pos) 325 (M+H) |
| 1236 | APCI(Pos) 389 (M+H) | 1260 | APCI(Pos) 341 (M+H) | 1284 | APCI(Pos) 325 (M+H) |
| 1237 | ACPI(Pos) 437 (M+H) | 1261 | APCI(Pos) 341 (M+H) | 1285 | APCI(Pos) 325 (M+H) |
| 1238 | APCI(Pos) 356 (M+H) | 1262 | APCI(Pos) 355 (M+H) | 1286 | APCI(Pos) 339 (M+H) |
| 1239 | APCI(Pos) 471 (M+H) | 1263 | APCI(Pos) 333 (M+H) | 1287 | APCI(Pos) 339 (M+H) |
| 1240 | APCI(Pos) 325 (M+H) | 1264 | APCI(Pos) 392 (M+H) | 1288 | APCI(Pos) 339 (M+H) |
| 1241 | APCI(Pos) 325 (M+H) | 1265 | APCI(Pos) 403 (M+H) | 1289 | APCI(Pos) 387 (M+H) |
| 1242 | APCI(Pos) 325 (M+H) | 1266 | APCI(Pos) 404 (M+H) | 1290 | APCI(Pos) 387 (M+H) |
| 1243 | APCI(Pos) 388 (M+H) | 1267 | APCI(Pos) 409 (M+H) | 1291 | APCI(Pos) 405 (M+H) |
| 1244 | APCI(Pos) 394 (M+H) | 1268 | APCI(Pos) 424 (M+H) | 1292 | APCI(Pos) 417 (M+H) |
| 1293 | APCI(Pos) 345 (M+H) | 1317 | APCI(Pos)339 (M+H) | 1341 | APCI(Pos) 381 (M+H) |

(continued)

| Compound No. | MASS | Compound No. | MASS | Compound No. | MASS |
|---|---|---|---|---|---|
| 1294 | APCI(Pos) 356 (M+H) | 1318 | APCI(Pos) 381 (M+H) | 1342 | APCI(Pos) 393 (M+H) |
| 1295 | APCI(Pos)356 (M+H) | 1319 | APCI(Pos) 393 (M+H) | 1343 | APCI(Pos) 393 (M+H) |
| 1296 | APCI(Pos) 388 (M+H) | 1320 | APCI(Pos) 424(M+H) | 1344 | APCI(Pos) 393 (M+H) |
| 1297 | APCI(Pos) 388 (M+H) | 1321 | APCI(Pos) 419 (M+H) | 1345 | APCI(Pos) 457 (M+H) |
| 1298 | APCI(Pos) 388 (M+H) | 1322 | APCI(Pos) 515 (M+H) | 1346 | APCI(Pos) 525 (M+H) |
| 1299 | APCI(Pos) 326 (M+H) | 1323 | APCI(Pos) 391 (M+H) | 1347 | APCI(Pos) 415 (M+H) |
| 1300 | APCI(Pos) 326 (M+H) | 1324 | APCI(Pos) 435 (M+H) | 1348 | APCI(Pos) 401 (M+H) |
| 1301 | APCI(Poz) 326 (M+H) | 1325 | APCI(Pos) 439 (M+H) | 1349 | APCI(Pos) 401 (M+H) |
| 1302 | APCI(Pos) 342 (M+H) | 1326 | APCI(Pos) 448 (M+H) | 1350 | APCI(Pos) 415 (M+H) |
| 1303 | APCI(Pos) 342 (M+H) | 1327 | APCI(Pos) 463 (M+H) | 1351 | APCI(Pos) 419 (M+H) |
| 1304 | APCI(Pos) 405 (M+H) | 1328 | APCI(Pos) 489 (M+H) | 1352 | APCI(Pos) 429 (M+H) |
| 1305 | APCI(Pos) 405 (M+H) | 1329 | APCI(Pos) 500 (M+H) | 1353 | APCI(Pos) 455 (M+H) |
| 1306 | APCI(Pos) 410 (M+H) | 1330 | APCI(Pos) 339 (M+H) | 1354 | APCI(Pos) 339 (M+H) |
| 1307 | APCI(Pas) 418 (M+H) | 133 | APCI(Pos) 339 (M+H) | 1355 | APCI(Pos) 359 (M+H) |
| 1308 | APCI(Pos) 438 (M+H) | 1332 | APCI(Pos) 339 (M+H) | 1356 | APCI(Pos) 359 (M+H) |
| 1309 | APCI(Pos) 404 (M+H) | 1333 | APCI(Pos) 353 (M+H) | 1357 | APCI(Pos) 359 (M+H) |
| 1310 | APCI(Pos) 343 (M+H) | 1334 | APCI(Pos) 353 (M+H) | 1358 | APCI(Pos) 373 (M+H) |
| 1311 | APCI(Pos) 405 (M+H) | 1335 | APCI(Pos) 353 (M+H) | 1359 | APCI(Pos) 373 (M+H) |
| 1312 | APC(Pos) 419 (M+H) | 1336 | APCI(Pos) 353 (M+H) | 1360 | APCI(Pos) 403 (M+H) |
| 1313 | APCI(Pos) 415 (M+H) | 1337 | APCI(Pos) 353 (M+H) | 1361 | APCI(Pos) 403 (M+H) |
| 1314 | APCI(Pos) 431 (M+H) | 1338 | APCI(Pos) 367 (M+H) | 1362 | APCI(Pos) 417 (M+H) |
| 1315 | APCI(Pos) 489 (M+H) | 1339 | APCI(Pos) 367 (M+H) | 1363 | APCI(Pos) 340 (M+H) |
| 1316 | APCI(Pos)339 (M+H) | 1340 | APCI(Pos) 367 (M+H) | 1364 | APCI(Pos) 340 (M+H) |
| 1365 | APCI(Pos)384 (M+H) | 1389 | APCI(Pos) 353 (M+H) | 1413 | APCI(Pos) 396 (M+H) |
| 1366 | APCI(Pos) 402 (M+H) | 1390 | APCI(Pos) 333 (M+H) | 1414 | APCI(Pos) 390 (M+H) |

(continued)

| Compound No. | MASS | Compound No. | MASS | Compound No. | MASS |
|---|---|---|---|---|---|
| 1367 | APCI(Pos) 402 (M+H) | 1391 | APCI(Pos) 387 (M+H) | 1415 | APCI(Pos) 444 (M+H) |
| 1268 | APCI(Pos) 420 (M+H) | 1392 | APCI(Pos) 417 (M+H) | 1418 | APCI(Pos) 427 (M+H) |
| 1369 | APCI(Pos) 436 (M+H) | 1393 | APCI(Pos) 417 (M+H) | 1417 | APCI(Pos) 38 (M+H) |
| 1370 | APCI(Pos) 454 (M+H) | 1394 | APCI(Pos) 427 (M+H) | 1418 | APCI(Pos) 361 (M+H) |
| 1371 | APCI(Pos) 470 (M+H) | 1395 | APCI(Pos) 431 (M+H) | 1419 | APCI(Pos) 375 (M+H) |
| 1372 | APCI(Pos) 419 (M+H) | 1396 | APCI(Pos) 373 (M+H) | 1420 | APCI(Pos) 391 (M+H) |
| 1373 | APCI(Pos) 419 (M+H) | 1397 | APCI(Pos) 374 (M+H) | 1421 | APCI(Pos) 405 (M+H) |
| 1374 | APCI(Pos) 420 (M+H) | 1398 | APCI(Pos) 374 (M+H) | 1422 | APCI(Pos) 379 (M+H) |
| 1375 | APCI(Pos) 460 (M+H) | 1399 | APCI(Pos) 374 (M+H) | 1423 | APCI(Pos) 367 (M+H) |
| 1376 | APCI (Pos) 356 (M+H) | 1400 | APCI(Pos) 388 (M+H) | 1424 | APCI(Pos) 361 (M+H) |
| 1377 | APCI(Pos) 410 (M+H) | 1401 | APCI(Pos) 390 (M+H) | 1425 | APCI(Pos) 377 (M+H) |
| 1378 | APCI(Pos) 396 (M+H) | 1402 | APCI(Pos) 376 (M+H) | 1426 | APCI(Pos) 377 (M+H) |
| 1378 | AFCI(Pos) 402 (M+H) | 1403 | APCI(Pos) 378 (M+H) | 1427 | APCI(Pos) 391 (M+H) |
| 1380 | APCI(Pos) 340 (M+H) | 1404 | APCI (Pos) 390 (M+H) | 1428 | APCI(Pos) 411 (M+H) |
| 1381 | APCI(Pos) 429 (M+H) | 1405 | APCI(Pos) 444 (M+H) | 1429 | APCI(Pos) 425 (M+H) |
| 1382 | APCI(Pos) 378 (M+H) | 1406 | APCI(Pos) 405 (M+H) | 1430 | APCI(Pos) 383 (M+H) |
| 1383 | APCI(Pos) 434 (M+H) | 1407 | APCI(Pos) 420 (M+H) | 1431 | APCI(Pos) 362 (M+H) |
| 1384 | APCI(Pos) 443 (M+H) | 1408 | APCI(Pos) 361 (M+H) | | |
| 1385 | APCI(Pos) 380 (M+H) | 1409 | APCI(Pos) 361 (M+H) | | |
| 1386 | APCI(Pos) 413 (M+H) | 1410 | APCI(Pos) 361 (M+H) | | |
| 1387 | APCI(Pos) 467 (M+H) | 1411 | APCI(Pos) 375 (M+H) | | |
| 1388 | APCI(Pos) 429 (M+H) | 1412 | APCI(Pos) 395 (M+H) | | |

Table 13

| Compound | MASS | Compound No. | MASS |
|---|---|---|---|
| 2001 | APCI: 267 (M+H)+ | 2041 | APCI: 338 (M+H)+ |
| 2002 | APCI: 207 (M+H)+ | 2042 | APCI: 338 (M+H)+ |
| 2003 | APCI: 293 (M+H)+ | 2043 | APCI: 339 (M+H)+ |
| 2004 | APCI: 297 (M+H)+ | 2044 | APCI: 339 (M+H)+ |
| 2005 | APCI: 301 (M+H)+ | 2045 | APCI: 339 (M+H)+ |
| 2006 | APCI: 301 (M+H)+ | 2046 | APCI: 339 (M+H)+ |
| 2007 | APCI: 302 (M+H)+ | 2047 | APCI: 341 (M+H)+ |
| 2008 | APCI: 302 (M+H)+ | 2048 | APCI: 341 (M+H)+ |
| 2009 | APCI: 305 (M+H)+ | 2049 | APCI: 341 (M+H)+ |
| 2010 | APCI: 300 (M+H)+ | 2050 | APCI: 347 (M+H)+ |
| 2011 | APCI: 307 (M+H)+ | 2051 | APCI: 341 (M+H)+ |
| 2012 | APCI: 307 (M+H)+ | 2052 | APCI: 351 (M+H)+ |
| 2013 | APCI: 312 (M+H)+ | 2053 | APCI: 351 (M+H)+ |
| 2014 | APCI: 315 (M+H)+ | 2054 | APCI: 353 (M+H)+ |
| 2015 | APCI: 315 (M+H)+ | 2055 | APCI: 355 (M+H)+ |
| 2016 | APCI: 315 (M+H)+ | 2056 | APCI: 365 (M+H)+ |
| 2017 | APCI: 316 (M+H)+ | 2057 | APCI: 356(M+H)+ |
| 2018 | APCI: 317 (M+H)+ | 2058 | APCI: 358 (M+H)+ |
| 2019 | APCI: 319 (M+H)+ | 2059 | APCI: 358 (M+H)+ |
| 2020 | APCI: 319 (M+H)+ | 2060 | APCI: 356 (M+H)+ |
| 2021 | APCI: 321 (M+H)+ | 2081 | APCI: 356 (M+H)+ |
| 2022 | APCI: 322 (M+H)+ | 2062 | APCI: 357 (M+H)+ |
| 2023 | APCI: 323 (M+H)+ | 2063 | APCI: 366 (M+H)+ |
| 2024 | APCI: 323 (M+H)+ | 2064 | APCI: 366 (M+H)+ |
| 2025 | APCI: 323 (M+H)+ | 2065 | APCI: 369 (M+H)+ |
| 2026 | APCI: 323 (M+H)+ | 2066 | APCI: 371 (M+H)+ |
| 2027 | APCI: 323 (M+H)+ | 2087 | APCI: 372 (M+H)+ |
| 2028 | APCI: 327 (M+H)+ | 2068 | APCI: 372 (M+H)+ |
| 2029 | APCI: 330 (M+H)+ | 2069 | APCI: 373 (M+H)+ |
| 2030 | APCI: 331 (M+H)+ | 2010 | APCI: 375 (M+H)+ |
| 2031 | APCI: 331 (M+H)+ | 2071 | APCI: 375 (M+H)+ |
| 2032 | APCI: 332 (M+H)+ | 2072 | APCI: 379 (M+H)+ |
| 2033 | APCI: 333 (M+H)+ | 2073 | APCI: 379 (M+H)+ |
| 2034 | APCI: 335 (M+H)+ | 2074 | APCI: 379 (M+H)+ |
| 2035 | APCI: 335 (M+H)+ | 2075 | APCI: 383 (M+H)+ |
| 2036 | APCI: 337 (M+H)+ | 2076 | APCI: 383 (M+H)+ |
| 2037 | APCI: 337 (M+H)+ | 2077 | APCI: 389 (M+H)+ |

(continued)

| Compound | MASS | Compound No. | MASS |
|---|---|---|---|
| 2038 | APCI: 337 (M+H)+ | 2078 | APCI: 395 (M+H)+ |
| 2039 | APCI: 338 (M+H)+ | 2079 | APCI: 403 (M+H)+ |
| 2040 | APCI: 338 (M+H)+ | 2080 | APCI: 406 (M+H)+ |
| 2081 | APCI: 407 (M+H)+ | 2121 | APCI: 337 (M+H)+ |
| 2082 | APCI: 412 (M+H)+ | 2122 | APCI: 337 (M+H)+ |
| 2083 | APCI: 413 (M+H)+ | 2123 | APCI: 337 (M+H)+ |
| 2084 | APCI: 427 (M+H)+ | 2124 | APCI: 339 (M+H)+ |
| 2085 | APCI: 431 (M+H)+ | 2125 | APCI: 339 (M+H)+ |
| 2086 | APCI: 269 (M+H)+ | 2126 | APCI: 340 (M+H)+ |
| 2087 | APCI: 269 (M+H)+ | 2127 | APCI: 340 (M+H)+ |
| 2088 | APCI: 283 (M+H)+ | 2128 | APCI: 340 (M+H)+ |
| 2089 | APCI: 295 (M+H)+ | 2129 | APCI: 340 (M+H)+ |
| 2090 | APCI: 295 (M+H)+ | 2130 | APCI: 341 (M+H)+ |
| 2091 | APCI: 299 (M+H)+ | 2131 | APCI: 341 (M+H)+ |
| 2092 | APCI: 304 (M+H)+ | 2132 | APCI: 341 (M +H)+ |
| 2093 | APCI: 304 (M+H)+ | 2133 | APCI: 341 (M+H)+ |
| 2094 | APCI: 304 (M+H)+ | 2134 | APCI: 343 (M+H)+ |
| 2095 | APCI: 307 (M+H)+ | 2135 | APCI: 343 (M+H)+ |
| 2096 | APCI 308 (M+H)+ | 2136 | APCI: 349 (M+H)+ |
| 2097 | APCI: 309 (M+H)+ | 2137 | APCI: 349 (M+H)+ |
| 2098 | APCI: 309 (M+H)+ | 2138 | APCI: 355 (M+H)+ |
| 2099 | APCI: 317 (M+H)+ | 2139 | APCI: 355 (M+H)+ |
| 2100 | APCI: 317 (M+H)+ | 2140 | APCI: 357 (M+H)+ |
| 2101 | APCI: 313 (M+H). | 2141 | APCI: 357 (M+H)+ |
| 2102 | APCI: 317 (M+H)+ | 2142 | APCI: 357 (M+H)+ |
| 2103 | APCI: 317 (M+H)+ | 2143 | APCI: 358 (M+H)+ |
| 2104 | APCI: 319 (M+H)+ | 2144 | APCI: 358 (M+H)+ |
| 2105 | APCI: 319 (M+H)+ | 2145 | APCI: 358 (M+H)+ |
| 2106 | APCI:321 (M+H)+ | 2146 | APCI: 358 (M+H)+ |
| 2107 | APCI: 321 (M+H)+ | 2147 | APCI: 358 (M+H)+ |
| 2108 | APCI: 323 (M+H)+ | 2148 | APCI: 359 (M+H) |
| 2109 | APCI: 324 (M+H)+ | 2148 | APCI: 368 (M+H)+ |
| 2110 | APCI: 325 (M+H)+ | 2150 | APCI: 368 (M+H)+ |
| 2111 | APCI: 325 (M+H)+ | 2151 | APCI: 371 (M+H)+ |
| 2112 | APCI: 325 (M+H)+ | 2152 | APCI: 373 (M+H)+ |
| 2113 | APCI: 325(M+H)+ | 2153 | APCI: 374 (M+H)+ |
| 2114 | APCI: 325 (M+H)+ | 2154 | APCI: 375 (M+H)+ |
| 2115 | APCI: 329 (M+H)+ | 2155 | APCI:375 (M+H)+ |

(continued)

| Compound | MASS | Compound No. | MASS |
|---|---|---|---|
| 2116 | APCI 331 (M+H)+ | 2156 | APCI: 377 (M+H)+ |
| 2117 | APCI: 333 (M+H)+ | 2157 | APCI: 377 (M+H)+ |
| 2118 | APCI: 333 (M+H)+ | 2158 | APCI: 379 (M+H)+ |
| 2119 | APCI: 334 (M+H)+ | 2159 | APCI: 381 (M+H)+ |
| 2120 | APCI: 335 (M+H)+ | 2160 | APCI: 381 (M+H)+ |
| 2161 | APCI: 381 (M+H)+ | 2201 | APCI: 339 (M+H)+ |
| 2182 | APCI: 385 (M+H)+ | 2202 | APCI: 338 (M+H)+ |
| 2163 | APCI: 385 (M+H)+ | 2203 | APCI: 343 (M+H)+ |
| 2154 | APCI: 391 (M+H)+ | 2204 | APCI: 345 (M+H)+ |
| 2185 | APCI: 397 (M+H)+ | 2205 | APCI: 348 (M+H)+ |
| 2166 | APCI: 405 (M+H)+ | 2206 | APCI: 347 (M+H)+ |
| 2157 | APCI: 408 (M+H)+ | 2207 | APCI: 347 (M+H)+ |
| 2168 | APCI: 414 (M+H)+ | 2208 | APCI: 348 (M+H)+ |
| 2169 | APCI: 415 (M+H)+ | 2209 | APCI: 349 (M+H)+ |
| 2170 | APCI: 429 (M+H)+ | 2210 | APCI: 351 (M+H)+ |
| 2171 | APCI: 433 (M+H)+ | 2211 | APCI: 351 (M+H)+ |
| 2172 | APCI: 283 (M+H)+ | 2212 | APCI: 353 (M+H)+ |
| 2173 | APCI: 283 (M+H)+ | 2213 | APCI: 353 (M+H)+ |
| 2174 | APCI: 297 (M+H)+ | 2214 | APCI: 353 (M+H)+ |
| 2175 | APCI: 309 (M+H)+ | 2215 | APCI: 354 (M+H)+ |
| 2176 | APCI: 313 (M+H)+ | 2216 | APCI: 354 (M+H)+ |
| 2177 | APCI: 317 (M+H)+ | 2217 | APCI: 354 (M+H)+ |
| 2178 | APCI: 317 (M+H)+ | 2218 | APCI: 354 (M+H)+ |
| 2179 | APCI: 318 (M+H)+ | 2219 | APCI: 355 (M+H)+ |
| 2180 | APCI: 318 (M+H)+ | 2220 | APCI 355 (M+H)+ |
| 2181 | APCI: 321 (M+H)+ | 2221 | APCI: 355 (M+H)+ |
| 2182 | APCI: 322 (M+H)+ | 2222 | APCI: 355 (M+H)+ |
| 2183 | APCI: 323 (M+H)+ | 2223 | APCI: 355 (M+H)+ |
| 2184 | APCI: 323 (M+H)+ | 2224 | APCI: 355 (M+H)+ |
| 2185 | APCI: 328 (M+H)+ | 2225 | APCI: 357 (M+H)+ |
| 2188 | APCI: 331 (M+H)+ | 2226 | APCI: 357 (M+H)+ |
| 2187 | APCI: 331 (M+H)+ | 2227 | APCI: 357 (M+H)+ |
| 2188 | APCI: 331 (M+H)+ | 2228 | APCI: 363 (M+H)+ |
| 2199 | APCI: 331 (M+H)+ | 2229 | APCI: 363 (M+H)+ |
| 2190 | APCI: 332 (M+H)+ | 2230 | APCI: 363 (M+H)+ |
| 2191 | APCI: 333 (M+H)+ | 2231 | APCI: 369 (M+H)+ |
| 2192 | APCI: 335 (M+H)+ | 2232 | APCI: 369 (M+H)+ |
| 2193 | APCI: 335 (M+H)+ | 2233 | APCI: 371 (M+H)+ |

(continued)

| Compound | MASS | Compound No. | MASS |
|---|---|---|---|
| 2194 | APCI: 337(M+H)+ | 2234 | APCI: 371 (M+H)+ |
| 2195 | APCI: 337 (M+H)+ | 2235 | APCI: 371 (M+H)+ |
| 2196 | APCI: 338 (M+H)+ | 2236 | APCI: 372(M+H)+ |
| 2197 | APCI: 339 (M+H)+ | 2237 | APCI: 372 (M+H)+ |
| 2198 | APCI: 339 (M+H)+ | 2238 | APCI: 372 (M+H)+ |
| 2199 | APCI: 339 (M+H)+ | 2239 | APCI: 372 (M+H)+ |
| 2200 | APCI: 339 (M+H)+ | 2240 | APCI: 373 (M+H)+ |
| 2241 | APCI: 381 (M+H)+ | 2281 | APCI: 358 (M+H)+ |
| 2242 | APCI: 381 (M+H)+ | 2282 | APCI: 361 (M+H)+ |
| 2243 | APCI: 382 (M+H)+ | 2283 | APCI: 361 (M+H)+ |
| 2244 | APCI: 382 (M+H)+ | 2284 | APCI: 361 (M+H)+ |
| 2245 | APCI: 385 (M+H)+ | 2285 | APCI: 361 (M+H)+ |
| 2246 | APCI: 387 (M+H)+ | 2286 | APCI 361 (M+H)+ |
| 2247 | APCI: 388 (M+H)+ | 2287 | APCI: 362 (M+H)+ |
| 2248 | APCI: 388 (M+H)+ | 2288 | APCI: 363 (M+H)+ |
| 2249 | APCI: 389 (M+H)+ | 2289 | APCI: 365 (M+H)+ |
| 2250 | APCI: 389 (M+H)+ | 2287 | APCI: 365 (M+H)+ |
| 2251 | APCI: 389 (M+H)+ | 2291 | APCI: 367 (M+H)+ |
| 2252 | APCI: 391 (M+H)+ | 2292 | APCI:367 (M+H)+ |
| 2253 | APCI: 391 (M+H) | 2293 | APCI: 368 (M+H)+ |
| 2254 | APCI: 393 (M+H)+ | 2294 | APCI: 369 (M+H)+ |
| 2255 | APCI: 395 (M+H)+ | 2295 | APCI: 369 (M+H)+ |
| 2256 | APCI: 395 (M+H)+ | 2296 | APCI: 369 (M+H)+ |
| 2257 | APCI: 395 (M+H)+ | 2297 | APCI: 369 (M+H)+ |
| 2258 | APCI: 399(M+H)+ | 2298 | APCI: 369 (M+H)+ |
| 2259 | APCI: 405 (M+H)+ | 2299 | APCI: 369 (M+H)+ |
| 2260 | APCI: 411 (M+H)+ | 2300 | APCI: 373 (M+H)+ |
| 2261 | APCI: 419 (M+H)+ | 2301 | APCI: 375 (M+H)+ |
| 2262 | APCI: 423 (M+H)+ | 2302 | APCI: 376 (M+H)+ |
| 2263 | APCI: 428 (M+H)+ | 2303 | APCI: 377 (M+H)+ |
| 2264 | APCI: 429 (M+H)+ | 2304 | APCI: 377 (M+H)+ |
| 2265 | APCI: 429 (M+H)+ | 2305 | APCI: 378 (M+H)+ |
| 2266 | APCI: 443 (M+H)+ | 2306 | APCI: 379 (M+H)+ |
| 2267 | APCI: 313 (M+H)+ | 2307 | APCI: 381 (M+H)+ |
| 2268 | APCI: 313 (M+H)+ | 2308 | APCI: 381 (M+H)+ |
| 2269 | APCI: 327 (M+H)+ | 2309 | APCI: 381 (M+H)+ |
| 2270 | APCI: 339 (M+H)+ | 2310 | APCI: 381 (M+H)+ |
| 2271 | APGI: 339 (M+H)+ | 2311 | APCI: 383 (M+H)+ |

(continued)

| Compound | MASS | Compound No. | MASS |
|---|---|---|---|
| 2272 | APCI: 343 (M+H)+ | 2312 | APCI: 383 (M+H)+ |
| 2273 | APCI: 347 (M+H)+ | 2313 | APCI: 383 (M+H)+ |
| 2274 | APCI: 347 (M+H)+ | 2314 | APCI: 384 (M+H)+ |
| 2275 | APCI: 348 (M+H)+ | 2315 | APCI: 384 (M+H)+ |
| 2276 | APCI: 348 (M+H)+ | 2316 | APCI: 384 (M+H)+ |
| 2277 | APCI: 351 (M+H)+ | 2317 | APCI: 384 (M+H)+ |
| 2278 | APCI: 352 (M+H)+ | 2318 | APCI: 385 (M+H)+ |
| 2279 | APCI: 353 (M+H)+ | 2319 | APCI: 385 (M+H)+ |
| 2280 | APCI: 353 (M+H)+ | 2320 | APCI: 385 (M+H)+ |
| 2321 | APCI: 385 (M+H)+ | 2361 | APCI: 425 (M+H)+ |
| 2322 | APCI: 385 (M+H)+ | 2362 | APCI: 425 (M+H)+ |
| 2323 | APCI: 387 (M+H)+ | 2363 | APCI: 425 (M+H)+ |
| 2324 | APCI: 387 (M+H)+ | 2364 | ESI: 429 (M+H)+ |
| 2325 | APCI: 387 (M+H)+ | 2365 | APCI: 429 (M+H)+ |
| 2326 | APCI: 393 (M+H)+ | 2366 | APCI: 429 (M+H)+ |
| 2327 | APCI: 393 (M+H)+ | 2367 | APCI: 435 (M+H)+ |
| 2328 | APCI: 393 (M+H)+ | 2368 | APCI: 441 (M+H)+ |
| 2329 | APCI: 397 (M+H)+ | 2369 | APCI: 452 (M+H)+ |
| 2330 | APCI: 397 (M+H)+ | 2370 | APCI: 453 (M+H)+ |
| 2331 | APCI: 399 (M+H)+ | 2371 | APCI: 458 (M+H)+ |
| 2332 | APCI: 399 (M+H)+ | 2372 | APCI: 459 (M+H)+ |
| 2333 | APCI: 401 (M+H)+ | 2373 | APCI: 473 (M+H)+ |
| 2334 | APCI: 401 (M+H)+ | 2374 | APCI: 477 (M+H)+ |
| 2335 | APCI: 401 (M+H)+ | 2375 | APCI: 267 (M+H)+ |
| 2336 | APCI: 401 (M+H)+ | 2376 | APCI: 281 (M+H)+ |
| 2337 | APCI: 401 (M+H)+ | 2377 | APCI: 293 (M+H)+ |
| 2338 | APCI: 402 (M+H)+ | 2378 | APCI: 295 (M+H)+ |
| 2339 | APCI: 402 (M+H)+ | 2379 | APCI: 295 (M+H)+ |
| 2340 | APCI: 402 (M+H)+ | 2380 | APCI: 297 (M+H)+ |
| 2341 | APCI: 402 (M+H)+ | 2381 | APCI: 307 (M+H)+ |
| 2342 | APCI: 402 (M+H)+ | 2382 | APCI: 309 (M+H)+ |
| 2343 | APCI: 401 (M+H)+ | 2383 | APCI: 309 (M+H)+ |
| 2344 | APCI: 403 (M+H)+ | 2384 | APCI: 309 (M+H)+ |
| 2345 | APCI: 411 (M+H)+ | 2385 | APCI: 310 (M+H)+ |
| 2346 | APCI: 412 (M+M)+ | 2386 | APCI: 319 (M+H)+ |
| 2347 | APCI: 412 (M+H)+ | 2387 | APCI: 319 (M+H)+ |
| 2348 | APCI: 417 (M+H)+ | 2388 | APCI: 320 (M+H)+ |
| 2349 | APCI: 417 (M+H)+ | 2389 | APCI: 321 (M+H)+ |

(continued)

| Compound | MASS | Compound No. | MASS |
|---|---|---|---|
| 2350 | APCI: 418 (M+H)+ | 2390 | APCI: 323 (M+H)+ |
| 2351 | APCI: 418-(M+H)+ | 2391 | APCI: 323 (M+H)+ |
| 2352 | APCI: 419 (M+H)+ | 2392 | APCI: 323 (M+H)+ |
| 2353 | APCI: 419 (M+H)+ | 2393 | APCI: 323 (M+H)+ |
| 2354 | APCI: 419 (M+H)+ | 2304 | APCI: 325 (M+H)+ |
| 2355 | APCI: 419 (M+H)+ | 2395 | APCI: 327 (M+H)+ |
| 2356 | APCI: 419 (M+H)+ | 2396 | APCI: 330 (M+H)+ |
| 2357 | APCI: 421 (M+H)+ | 2397 | APCI: 331 (M+H)+ |
| 2358 | APCI: 421 (M+H)+ | 2398 | APCI: 331 (M+H)+ |
| 2359 | APCI: 423 (M+H)+ | 2399 | APCI: 331 (M+H)+ |
| 2360 | APCI: 425 (M+H)+ | 2400 | APCI: 332 (M+H)+ |
| 2401 | APCI: 333 (M+H)+ | 2441 | APCI: 358 (M+H)+ |
| 2402 | APCI: 333 (M+H)+ | 2442 | APCI: 359 (M+H)+ |
| 2403 | APCI: 334 (M+H)+ | 2443 | APCI: 359 (M+H)+ |
| 2404 | APCI: 335 (M+H)+ | 2444 | APCI: 359 (M+H)+ |
| 2405 | APCI: 335 (M+H)+ | 2445 | APCI: 360 (M+H)+ |
| 2400 | APCI: 336 (M+H)+ | 2446 | ESI: 360 (M+H)+ |
| 2407 | APCI: 337 (M+H)+ | 2447 | APCI: 361 (M+H)+ |
| 2408 | APCI: 338 (M+H)+ | 2448 | APCI: 361 (M+H)+ |
| 2409 | APCI: 339 (M+H)+ | 2449 | APCI: 361 (M+H)+ |
| 4210 | APCI: 339 (M+H)+ | 2450 | APCI: 361(M+H)+ |
| 2411 | APCI: 344 (M+H)+ | 2451 | APCI: 361 (M+H)+ |
| 2412 | APCI: 344 (M+H)+ | 2452 | APCI: 364 (M+H)+ |
| 2413 | APCI: 344 (M+H)+ | 2453 | APCI: 364 (M+H)+ |
| 2414 | APCI: 345 (M+H)+ | 2454 | APCI: 364 (M+H)+ |
| 2415 | APCI: 345. (M+H)+ | 2455 | APCI: 364 (M+H)+ |
| 2416 | APCI: 346 (M+H)+ | 2456 | APCI: 365 (M+H)+ |
| 2411 | APCI: 346 (M+H)+ | 2457 | APCI: 367 (M+H)+ |
| 2418 | APCI: 346 (M+H)+ | 2458 | APCI: 368 (M+H)+ |
| 2419 | APCI: 346 (M+H)+ | 2459 | APCI: 369 (M+H)+ |
| 2420 | APCI: 347 (M+H)+ | 2460 | APCI: 369 (M+H)+ |
| 2421 | APCI: 347 (M+H)+ | 2461 | APCI: 369 (M+H)+ |
| 2422 | APCI: 347 (M+H)+ | 2462 | APCI: 369 (M+H)+ |
| 2423 | APCI: 347 (M+H)+ | 2463 | APCI: 369 (M+H)+ |
| 2424 | APCI: 347 (M+H)+ | 2464 | APCI: 369 (M+H)+ |
| 2425 | APCI: 347 (M+H)+ | 2465 | APCI: 369 (M+H)+ |
| 2426 | APCI: 348 (M+H)+ | 2466 | APCI: 371 (M+H)+ |
| 2427 | APCI: 348 (M+H)+ | 2467 | APCI: 371 (M+H)+ |

(continued)

| Compound | MASS | Compound No. | MASS |
|---|---|---|---|
| 2428 | APCI: 349 (M+H)+ | 2468 | APCI: 371 (M+H)+ |
| 2429 | APCI: 350 (M+H)+ | 2469 | APCI: 371 (M+H)+ |
| 2430 | APCI: 351 (M+H)+ | 2470 | APCI: 372 (M+H)+ |
| 2431 | APCI: 351 (M+H)+ | 2471 | APCI: 373 (M+H)+ |
| 2432 | APCI: 351 (M+H)+ | 2472 | APCI: 373 (M+H)+ |
| 2433 | APCI: 353(M+H)+ | 2473 | APCI:373 (M+H)+ |
| 2434 | APCI: 354 (M+H)+ | 2474 | APCI: 373 (M+H)+ |
| 2435 | APCI: 355:(M+H)+ | 2475 | APCI: 373 (M+H)+ |
| 2438 | APCI: 355 (M+H)+ | 2476 | APCI: 373 (M+H)+ |
| 2437 | APCI: 355 (M+H)+ | 2477 | APCI: 374 (M+H)+ |
| 2438 | APCI: 357 (M+H)+ | 2478 | APCI: 374 (M+H)+ |
| 2439 | APCI: 357 (M+H)+ | 2479 | AFCI: 374 (M+H)+ |
| 2440 | APCI: 357 (M+H)+ | 2480 | APCI: 375 (M+H)+ |
| 2481 | APCI: 375 (M+H)+ | 2521 | APCI: 389 (M+H)+ |
| 2482 | APCI: 376 (M+H)+ | 2522 | APCI: 389 (M+H)+ |
| 2483 | APCI: 376 (M+H)+ | 2523 | APCI: 389 (M+H)+ |
| 2484 | APCI: 377 (M+H)+ | 2524 | APCI: 389 (M+H)+ |
| 2485 | APCI: 378 (M+H)+ | 2525 | APCI: 389 (M+H)+ |
| 2486 | APCI: 378 (M+H)+ | 2528 | APCI: 389 (M+H)+ |
| 2487 | APCI: 379 (M+H)+ | 2527 | APCI: 390 (M+H)+ |
| 2488 | APCI: 379 (M+H)+ | 2528 | APCI: 390 (M+H)+ |
| 2489 | APCI:379 (M+H)+ | 2529 | APCI: 390 (M+H)+ |
| 2490 | APCI: 380 (M+H)+ | 2530 | APCI: 393 (M+H)+ |
| 2491 | APCI: 381 (M+H)+ | 2531 | APCI: 393 (M+H)+ |
| 2492 | APCI: 381 (M+H)+ | 2532 | APCI: 395 (M+H)+ |
| 2493 | APCI: 381 (M+H)+ | 2533 | APCI: 395 (M+H)+ |
| 2494 | APCI: 361 (M+H)+ | 2534 | APCI: 395 (M+H)+ |
| 2495 | APCI: 381 (M+H)+ | 2535 | APCI: 395 (M+H)+ |
| 2496 | APCI: 381 (M+H)+ | 2536 | APCI: 395 (M+H)+ |
| 2497 | APCI: 381 (M+H)+ | 2537 | APCI: 395 (M+H)+ |
| 2498 | APCI: 382 (M+H)+ | 2538 | APCI: 398 (M+H)+ |
| 2499 | APCI: 382 (M+H)+ | 2539 | APCI: 396 (M+H)+ |
| 2500 | APCI: 382 (M+H)+ | 2540 | APCI: 397 (M+H)+ |
| 2501 | APCI: 383 (M+H)+ | 2541 | APCI: 397 (M+H)+ |
| 2502 | APCI: 383 (M+H)+ | 2542 | APCI: 397 (M+H)+ |
| 2503 | APCI: 383 (M+H)+ | 2543 | APCI: 397 (M+H)+ |
| 2504 | APCI: 383 (M+H)+ | 2544 | APCI: 397 (M+H)+ |
| 2505 | APCI: 384 (M+H)+ | 2545 | APCI: 397 (M+H)+ |

(continued)

| Compound | MASS | Compound No. | MASS |
|---|---|---|---|
| 2506 | APCI: 385 (M+H)+ | 2546 | APCI: 397 (M+H)+ |
| 2507 | APCI: 385 (M+H)+ | 2547 | APCI: 398 (M+H)+ |
| 2508 | APCI: 385 (M+H)+ | 2548 | APCI: 398 (M+H)+ |
| 2509 | APCI: 385 (M+H)+ | 2549 | APCI: 398 (M+H)+ |
| 2510 | APCI: 386 (M+H)+ | 2550 | APCI: 398 (M+H)+ |
| 2511 | APCI: 387 (M+H)+ | 2551 | APCI: 398 (M+H)+ |
| 2512 | APCI: 387 (M+H)+ | 2552 | APCI: 398 (M+H)+ |
| 2513 | APCI: 387 (M+H)+ | 2553 | ESI: 399 (M+H)+ |
| 2514 | APCI: 387 (M+H)+ | 2554 | APCI: 399 (M+H)+ |
| 2515 | APCI: 387 (M+H)+ | 2555 | APCI: 399 (M+H)+ |
| 2516 | APCI: 387 (M+H)+ | 2556 | APCI: 399 (M+H)+ |
| 2517 | APCI: 387 (M+H)+ | 2557 | APCI: 400 (M+H)+ |
| 2518 | APCI: 388 (M+H)+ | 2558 | APCI: 401(M+H)+ |
| 2519 | APCI: 389 (M+H)+ | 2559 | APCI: 401 (M+H)+ |
| 2520 | APCI: 389 (M+H)+ | 2560 | APCI: 401 (M+H)+ |
| 2561 | APCI: 401 (M+H)+ | 2601 | APCI: 418 (M+H)+ |
| 2582 | APCl: 403 (M+H)+ | 2602 | APCI: 419 (M+H)+ |
| 2563 | APCl: 403 (M+H)+ | 2603 | APCI: 419 (M+H)+ |
| 2564 | APCI: 403 (M+H)+ | 2604 | APCI: 419 (M+H)+ |
| 2565 | APCI: 404 (M+H)+ | 2605 | APCI: 419 (M+H)+ |
| 2566 | APCI: 404 (M+H)+ | 2606 | APCI: 419 (M+H)+ |
| 2587 | APCI: 404 (M+H)+ | 2607 | APCI: 421 (M+H)+ |
| 2568 | APCI: 404 (M+H)+ | 2608 | APCI: 421 (M+H)+ |
| 2569 | APCI: 405 (M+H)+ | 2609 | APCI: 421 (M+H)+ |
| 2570 | APCI: 405 (M+H)+ | 2610 | APCI: 421 (M+H)+ |
| 2571 | APCI: 406 (M+H)+ | 2611 | APCI: 422 (M+H)+ |
| 2572 | APCI: 408 (M+H)+ | 2612 | APCI: 422 (M+H)+ |
| 2573 | APCI: 408 (M+H)+ | 2613 | APCI: 423 (M+H)+ |
| 2574 | ESI: 409 (M+H)+ | 2614 | APCI: 425 (M+H)+ |
| 2575 | APCI: 410 (M+H)+ | 2615 | APCI: 427 (M+H)+ |
| 2576 | APCI: 410 (M+H)+ | 2616 | APCI: 427 (M+H)+ |
| 2577 | APCI: 410 (M+H)+ | 2617 | APCI: 427 (M+H)+ |
| 2578 | APCI: 411 (M+H)+ | 2618 | APCI: 427 (M+H)+ |
| 2570 | APCI: 412 (M+H)+ | 2619 | APCI: 427 (M+H)+ |
| 2580 | APCI: 412 (M+H)+ | 2620 | APCI: 427 (M+H)+ |
| 2581 | APCI: 412 (M+H)+ | 2621 | AFCI: 428 (M+H)+ |
| 2582 | APCI: 412 (M+H)+ | 2622 | APCI: 428 (M+H)+ |
| 2583 | APCI: 412 (M+H)+ | 2623 | APCI: 428 (M+H)+ |

(continued)

| Compound | MASS | Compound No. | MASS |
|---|---|---|---|
| 2584 | APCI: 413 (M+H)+ | 2624 | APCI: 429 (M+H)+ |
| 2585 | APCI: 413 (M+H)+ | 2625 | APCI: 429 (M+H)+ |
| 2586 | APCI: 413(M+H)+ | 2626 | APCI: 429 (M+H)+ |
| 2587 | APCI: 413 (M+H)+ | 2627 | APCI: 431 (M+H)+ |
| 2588 | APCI: 413(M+H)+ | 2628 | APCI 431(M+H)+ |
| 2589 | APCI: 414 (M+H)+ | 2629 | APCI: 431 (M+H)+ |
| 2590 | APCI: 415 (M+H)+ | 2630 | APCI: 432 (M+H)+ |
| 2591 | APCI: 415 (M+H)+ | 2631 | APCI: 432 (M+H)+ |
| 2592 | APCI: 415 (M+H)+ | 2632 | APCI: 435 (M+H)+ |
| 2593 | APCI: 415 (M+H)+ | 2633 | APCI: 437 (M+H)+ |
| 2594 | APCI: 415 (M+H) | 2634 | APCI: 437 (M+H)+ |
| 2595 | APCI: 415 (M+H)+ | 2635 | APCI: 438 (M+H)+ |
| 2596 | APCI: 415 (M+H)+ | 2636 | APCI: 439 (M+H)+ |
| 2597 | APCI: 416 (M+H)+ | 2637 | APCI: 439 (M+H)+ |
| 2598 | APCI: 417 (M+H)+ | 2638 | APCI: 439 (M+H)+ |
| 2599 | APCI: 417 (M+H)+ | 2639 | APCI: 440 (M+H)+ |
| 2600 | APCI: 418 (M+H)+ | 2640 | APCI: 441 (M+H)+ |
| 2641 | APCI: 443 (M+H)+ | | |
| 2642 | APCI: 444 (M+H)+ | | |
| 2643 | APCI: 444 (M+H)+ | | |
| 2644 | APCI: 448 (M+H)+ | | |
| 2645 | APCI: 449 (M+H)+ | | |
| 2646 | APCI: 449 (M+H)+ | | |
| 2647 | APCI: 449 (M+H)+ | | |
| 2648 | APCI: 453 (M+H)+ | | |
| 2649 | APCI: 454 (M+H)+ | | |
| 2650 | APCI: 455 (M+H)+ | | |
| 2651 | APCI: 455 (M+H)+ | | |
| 2652 | APCI: 456 (M+H)+ | | |
| 2653 | APCI: 457 (M+H)+ | | |
| 2654 | APCI: 459 (M+H)+ | | |
| 2655 | APCI: 462 (M+H)+ | | |
| 2656 | APCI: 462 (M+H)+ | | |
| 2657 | APCI: 462 (M+H)+ | | |
| 2658 | APCI: 463 (M+H)+ | | |
| 2659 | APCI: 465 (M+H)+ | | |
| 2660 | APCI: 465 (M+H)+ | | |
| 2661 | APCI: 465 (M+H)+ | | |

(continued)

| Compound | MASS | Compound No. | MASS |
|---|---|---|---|
| 2862 | APCI: 471 (M+H)+ | | |
| 2663 | APCI: 475 (M+H)+ | | |
| 2664 | APCI: 476 (M+H)+ | | |
| 2665 | APCI: 477 (M+H)+ | | |
| 2866 | APCI: 478 (M+H)+ | | |
| 2667 | APCI: 482 (M+H)+ | | |
| 2668 | APCI: 483 (M+H)+ | | |
| 2669 | APCI: 492 (M+H)+ | | |
| 2670 | APCI: 484 (M+H)+ | | |
| 2671 | APCI: 495 (M+H)+ | | |
| 2672 | APCI: 497 (M+H)+ | | |
| 2673 | APCI: 497 (M+H)+ | | |
| 2674 | APCI: 498 (M+H)+ | | |
| 2675 | APCI: 508 (M+H)+ | | |
| 2676 | APCI: 523 (M+H)+ | | |
| 2677 | APCI: 531 (M+H)+ | | |
| 2678 | APCI: 533 (M+H)+ | | |

Table 14

| No. | MASS | No. | MASS |
|---|---|---|---|
| 3001 | ESI(Pos) 250 (M+H) | 3041 | ESI(Pos) 312 (M+H) |
| 3002 | ESI(Pos) 264 (M+H) | 3042 | ESI(Pos) 326 (M+H) |
| 3003 | ESI(Pos) 278 (M+H) | 3043 | ESI(Pos) 326 (M+H) |
| 3004 | ESI(Pos) 278 (M+H) | 3044 | ESI(Pos) 326 (M+H) |
| 3005 | ESI(Pos) 292 (M+H) | 3045 | ESI(Pos) 330 (M+H) |
| 3006 | ESI(Pos) 292 (M+H) | 3046 | ESI(Pos) 330 (M+H) |
| 3007 | ESI(Pos) 292 (M+H) | 3047 | ESI(Pos) 330 (M+H) |
| 3003 | ESI(Pos) 306 (M+H) | 3048 | ESI(Pos) 337 (M+H) |
| 3009 | ESI(Pos) 306 (M+H) | 3049 | ESI(Pos) 337 (M+H) |
| 3010 | ESI(Pos) 306 (M+H) | 3050 | ESI(Pos) 340 (M+H) |
| 3011 | ESI(Pos) 290 (M+H) | 3051 | ESI(Pos) 340 (M+H) |
| 3012 | ESI(Pos) 318 (M+H) | 3052 | ESI(Pos) 340 (M+H) |
| 3013 | ESI(Pos) 332 (M+H) | 3053 | ESI(Pos) 342 (M+H) |
| 3014 | ESI(Pos) 326 (M+H) | 3054 | ESI(Pos) 342 (M+H) |
| 3015 | ESI(Pos) 340 (M+H) | 3055 | ESI(Pos) 344 (M+H) |
| 3016 | ESI(Pos) 354 (M+H) | 3056 | ESI(Pos) 344 (M+H) |
| 3017 | ESI(Pos) 402 (M+H). | 3057 | ESI(Pos) 346 (M+H) |

(continued)

| No. | MASS | No. | MASS |
|---|---|---|---|
| 3018 | ESI(Neg) 330 (M-H) | 3058 | ESI(Pos) 346 (M+H) |
| 3019 | ESI(Pos) 342 (M+H) | 3059 | ESI(Pos) 346 (M+H) |
| 3020 | ESI(Pos) 356 (M+H) | 3060 | ESI(Pos) 348 (M+H) |
| 3021 | ESI (Pos) 280 (M+H) | 3061 | ESI(Pos) 348 (M+H) |
| 3022 | ESI(Pos) 340 (M+H) | 3062 | ESI(Pos) 348 (M+H) |
| 3023 | ESI(Pos) 356 (M+H) | 3063 | ESI(Pos) 348 (M+H) |
| 3024 | ESI(Pos) 356 (M+H) | 3064 | ESI(Pos) 348 (M+H) |
| 3025 | ESI(Pos) 310 (M+H) | 3065 | ESI(Pos) 348 (M+H) |
| 3026 | ESI(Pos) 308 (M+H) | 3066 | ESI(Pos) 354 (M+H) |
| 3027 | ESI(Pos) 384 (M+H) | 3067 | ESI(Pos) 354 (M+H) |
| 3028 | ESI(Pos) 404 (M+H) | 3068 | ESI(Pos) 355 (M+H) |
| 3029 | ESI(Pos) 336 (M+H) | 3069 | ESI(Pos) 357 (M+H) |
| 3030 | ESI(Pos) 280 (M+H) | 3070 | ESI(Pos) 362(M+H) |
| 3031 | ESI(Pos) 310 (M+H) | 3071 | ESI(Pos) 362 (M+H) |
| 3032 | ESI(Pos) 321 (M+H) | 3072 | ESI(Pos) 362 (M+H) |
| 3033 | ESI(Pos) 322 (M+H) | 3073 | ESI(Pos) 382 (M+H) |
| 3034 | ESI(Pos) 276 (M+H) | 3074 | ESI(Pos) 364 (M+H) |
| 3035 | ESI(Pos) 290 (M+H) | 3075 | ESI(Pos) 364 (M+H) |
| 3036 | ESI(Pos) 304 (M+H) | 3076 | ESI(Pos) 364 (M+H) |
| 3037 | ESI(Pos) 318 (M+H) | 3077 | ESI(Pos) 364 (M+H) |
| 3038 | ESI(Pos) 370 (M+H) | 3078 | ESI(Pos) 364 (M+H) |
| 3039 | ESI(Pos) 352 (M+H) | 3079 | ESI(Pos) 366 (M+H) |
| 3040 | ESI(Pos) 414 (M+H) | 3080 | ESI(Pos) 366(M+H) |
| 3081 | ESI(Pos) 366 (M+H) | 3121 | ESI(Pos) 438 (M+H) |
| 3082 | ESI(Pos) 366 (M+H) | 3122 | ESI(Pos) 438 (M+H) |
| 3083 | ESI(Pos) 368 (M+H) | 3123 | ESI(Pos) 442 (M+H) |
| 3084 | ESI(Pos) 392 (M+H) | 3124 | ESI(Pos) 448 (M+H) |
| 3085 | ESI(Pos) 372 (M+H) | 3125 | ESI(Pos) 448 (M+H) |
| 3086 | ESI(Pos) 378 (M+H) | 3126 | ESI(Pos) 355 (M+H) |
| 3087 | ESI(Pos) 378 (M+H) | 3127 | ESI(Pos) 396 (M+H) |
| 3088 | ESI(Pos) 380 (M+H) | 3128 | ESI(Pos) 313 (M+H) |
| 3089 | ESI(Pos) 380 (M+H) | 3129 | ESI(Pos) 313 (M+H) |
| 3090 | ESI(Neg) 378 (M+H) | 3130 | ESI(Pos) 384 (M+H) |
| 3091 | ESI(Pos) 380 (M+H) | 3131 | ESI(Pos) 377 (M+H) |
| 3092 | ESI(Pos) 380 (M+H) | 3132 | ESI(Pos) 395 (M+H) |
| 3093 | ESI(Pos) 382 (M+H) | 3133 | ESI(Pos) 421 (M+H) |
| 3094 | ESI(Pos) 382 (M+H) | 3134 | ESI(Pos) 439 (M+H) |
| 3095 | ESI(Pos) 382 (M+H) | 3135 | ESI(Pos) 302 (M+H) |

(continued)

| No. | MASS | No. | MASS |
|---|---|---|---|
| 3096 | ESI(Pos) 384 (M+H) | 3136 | ESI(Pos) 303 (M+H) |
| 3097 | ESI(Pos) 388 (M+H) | 3137 | ESI(Pos) 317 (M+H) |
| 3098 | ESI(Pos) 390 (M+H) | 3138 | ESI(Pos) 318 (M+H) |
| 3099 | ESI(Pos) 390 (M+H) | 3139 | ESI(Pos) 347 (M+H) |
| 3100 | ESI(Pos) 390 (M+H) | 3140 | ESI(Pos) 330(M+H) |
| 3101 | ESI(Pos) 396 (M+H) | 3141 | ESI(Pos) 331 (M+H) |
| 3102 | ESI(Pos) 396 (M+H) | 3142 | ESI(Pos) 344 (M+H) |
| 3103 | ESI(Pos) 396 (M+H) | 3143 | ESI(Pos) 372 (M+H) |
| 3104 | ESI(Pos) 398 (M+H) | 3144 | ESI(Pos) 372 (M+H) |
| 3105 | ESI(Pos) 398 (M+H) | 3145 | ESI(Pos) 384 (M+H) |
| 3106 | ESI(Pos) 398 (M+H) | 3146 | ESI(Pos) 368 (M+H) |
| 3107 | ESI(Pos) 398 (M+H) | 3147 | ESI(Pos) 368 (M+H) |
| 3108 | ESI(Pos) 398 (M+H) | 3148 | ESI(Pos) 368 (M+H) |
| 3109 | ESI(Pos) 398 (M+H) | 3149 | ESI(Pos) 398 (M+H) |
| 3110 | ESI(Pos) 398 (M+H) | 3150 | ESI(Pos) 400 (M+H) |
| 3111 | ESI(Pos) 400 (M+H) | 3151 | ESI(Pos) 403 (M+H) |
| 3112 | ESI(Pos) 402 (M+H) | 3152 | ESI(Pos) 420 (M+H) |
| 3113 | ESI(Pos) 402 (M+H) | 3153 | ESI(Pos) 427 (M+H) |
| 3114 | ESI(Pos) 404 (M+H) | 3154 | ESI(Pos) 446 (M+H) |
| 3115 | ESI(Pos) 412 (M+H) | 3155 | ESI(Pos) 448 (M+H) |
| 3116 | ESI(Pos) 414 (M+H) | 3156 | ESI(Pos) 454 (M+H) |
| 3117 | ESI(Pos) 414 (M+H) | 3157 | ESI(Pos) 462 (M+H) |
| 3118 | ESI(Neg) 418 (M-H) | 3158 | ESI(Pos) 480 (M+H) |
| 3119 | ESI(Pos) 432 (M+H) | | |
| 3120 | ESI(Pos) 438 (M+H) | | |
| 3159 | APCI: 317 (M+H)+ | 3199 | APCI: 361 (M+H)+ |
| 3160 | APCI: 317 (M+H)+ | 3200 | APCI: 361 (M+H)+ |
| 3161 | APCI: 317 (M+H)+ | 3201 | APCI: 383 (M+H)+ |
| 3162 | APCI: 318 (M+H)+ | 3202 | APCI: 365 (M+H)+ |
| 3163 | APCI: 318 (M+H)+ | 3203 | APCI: 365 (M+H)+ |
| 3164 | APCI: 318 (M+H)+ | 3204 | APCI: 369 (M+H)+ |
| 3165 | APCI: 318 (M+H)+ | 3205 | APCI: 371 (M+H)+ |
| 3166 | APCI: 331 (M+H)+ | 3206 | APCI: 374 (M+H)+ |
| 3167 | APCI: 331 (M+H)+ | 3207 | APCI: 377 (M+H)+ |
| 3168 | APCI: 331 (M+H)+ | 3208 | APCI: 378 (M+H)+ |
| 3169 | APCI 331 (M+H)+ | 3209 | APCI: 378 (M+H)+ |
| 3170 | APCI: 331 (M+H)+ | 3210 | APCI 381 (M+H)+ |
| 3171 | APCI: 331 (M+H)+ | 3211 | APCI: 383 (M+H)+ |

(continued)

| No. | MASS | No. | MASS |
|---|---|---|---|
| 3172 | APCI: 331 (M+H)+ | 3212 | APCI: 383 (M+H)+ |
| 3173 | APCI: 332 (M+H)+ | 3213 | APCI: 384 (M+H)+ |
| 3174 | APCI: 333 (M+H)+ | 3214 | APCI: 385 (M+H)+ |
| 3175 | APCI: 333 (M+H)+ | 3215 | APCI: 385 (M+H)+ |
| 3176 | APCI: 333 (M+H)+ | 3216 | APCI: 385 (M+H)+ |
| 3177 | APCI: 333 (M+H)+ | 3217 | APCI: 385 (M+H)+ |
| 3178 | APCI: 335 (M+H)+ | 3218 | APCI: 385 (M+H)+ |
| 3179 | APCI: 335 (M+H)+ | 3219 | APCI: 385 (M+H)+ |
| 3180 | APCI: 335 (M+H)+ | 3220 | APCI: 385 (M+H)+ |
| 3181 | APCI: 335 (M+H)+ | 3221 | APCI: 385 (M+H)+ |
| 3182 | APCI: 342 (M+H)+ | 3222 | APCI: 385 (M+H)+ |
| 3183 | APCI: 345 (M+H)+ | 3223 | APCI: 385 (M+H)+ |
| 3184 | APCI: 345 (M+H)+ | 3224 | APCI: 386 (M+H)+ |
| 3185 | APCI: 345 (M+H)+ | 3225 | APCI: 386 (M+H )+ |
| 3186 | APCI: 345 (M+H)+ | 3226 | APCI: 386 (M+H)+ |
| 3187 | APCI: 347 (M+H)+ | 3227 | APCI: 386 (M+H)+ |
| 3188 | APCI: 347 (M+H)+ | 3228 | APCI: 389 (M+H)+ |
| 3189 | APCI:348 (M+H)+ | 3229 | APCI: 391 (M+H)+ |
| 3190 | APCI: 349 (M+H)+ | 3230 | APCI: 393 (M+H)+ |
| 3191 | APCI: 351 (M+H)+ | 3231 | APCI: 395 (M+H)+ |
| 3192 | APCI: 351 (M+H)+ | 3232 | APCI: 395 (M+H)+ |
| 3193 | APCI: 351 (M+H)+ | 3233 | APCI: 395 (M+H)+ |
| 3194 | APCI: 351 (M+H)+ | 3234 | APCI: 395 (M+H)+ |
| 3195 | APCI: 351 (M+H)+ | 3235 | APCI: 395 (M+H)+ |
| 3196 | APCI: 352 (M+H)+ | 3236 | APCI: 399 (M+H)+ |
| 3197 | APCI: 353 (M+H)+ | 3237 | APCI: 399 (M+H)+ |
| 3198 | APCI: 360 (M+H)+ | 3233 | APCI: 399 (M+H)+ |
| 3239 | APCI: 400 (M+H)+ | 3279 | APCI: 382 (M+H)+ |
| 3240 | APCI: 403 (M+H)+ | 3280 | APCI: 382 (M+H)+ |
| 3241 | APCI: 405 (M+H)+ | 3281 | APCI: 383 (M+H)+ |
| 3242 | APCI: 409 (M+H)+ | 3282 | APCI: 383 (M+H)+ |
| 3243 | APCI: 410 (M+H)+ | 3283 | APCI: 387(M+H)+ |
| 3244 | APCI: 415 (M+H)+ | 3284 | APCI: 387 (M+H)+ |
| 3245 | APCI: 414 (M+H)+ | 3285 | APCI: 399 (M+H)+ |
| 3246 | APCI: 415 (M+H)+ | 3286 | APCI: 401 (M+H)+ |
| 3247 | APCI: 418 (M+H)+ | 3287 | APCI: 409 (M+H)+ |
| 3248 | APCI: 418 (M+H)+ | 3288 | APCI: 414 (M+H)+ |
| 3249 | APCI: 419 (M+H)+ | 3289 | APCI: 419 (M+H)+ |

(continued)

| No. | MASS | No. | MASS |
|---|---|---|---|
| 3250 | ESI: 423 (M+H)+ | 3290 | APCI: 419 (M+H)+ |
| 3251 | APCI: 425 (M+H)+ | 3291 | APCI: 421 (M+H)+ |
| 3252 | APCI: 427(M+H)+ | 3292 | ACPI: 435 (M+H)+ |
| 3253 | APCI: 440 (M+H)+ | 3293 | APCI: 441 (M+H)+ |
| 3254 | APCI: 443 (M+H)+ | 3294 | APCI: 443 (M+H)+ |
| 3255 | APCI: 355 (M+H)+ | 3295 | APCI: 444 (M+H)+ |
| 3256 | APCI: 355 (M+H)+ | 3296 | APCI: 456 (M+H)+ |
| 3257 | APCI: 355 (M+H)+ | 3297 | APCI: 477 M+H)+ |
| 3258 | APCI: 355 (M+H)+ | 3298 | APCI: 359(M+H)+ |
| 3259 | APCI: 356 (M+H)+ | 3299 | APCI: 359 (M+H)+ |
| 3260 | APCI: 356 (M+H)+ | 3300 | APCI: 381 (M+H)+ |
| 3261 | APCI: 367 (M+H)+ | 3301 | APCI: 381 (M+H)+ |
| 3262 | APCI: 367 (M+H)+ | 3302 | APCI: 381 (M+H)+ |
| 3263 | APCI: 367 (M+H)+ | 3303 | APCI: 382 (M+H)+ |
| 3264 | APCI: 387 (M+H)+ | 3304 | APCI: 382 (M+H)+ |
| 3265 | APCI: 367 (M-H)+ | 3305 | APCI: 385 (M+H)+ |
| 3266 | APCI: 368 (M+H)+ | 3306 | APCI: 387 (M+H)+ |
| 3267 | APCI: 368 (M+H)+ | 3307 | APCI: 398 (M+H)+ |
| 3268 | APCI: 368 (M+H)+ | 3308 | APCI: 398 (M+H)+ |
| 3269 | APCI: 368 (M+H)+ | 3309 | APCI: 399 (M+H)+ |
| 3270 | APCI: 368 (M+H)+ | 3310 | APCI: 400 (M+H)+ |
| 3271 | APCI: 369 (M+H)+ | 3311 | APCI: 401 (M+H)+ |
| 3272 | APCI: 370 (M+H)+ | 3312 | APCI: 402 (M+H)+ |
| 3273 | APCI: 371 (M+H)+ | 3313 | APCI: 410 (M+H)+ |
| 3274 | APCI: 372 (M+H)+ | 3314 | APCI: 413 (M+H)+ |
| 3275 | APCI: 373 (M+H)+ | 3315 | APCI: 414 (M+H)+ |
| 3276 | APCI: 374 (M+H)+ | 3316 | APCI: 415 (M+H)+ |
| 3277 | APCI 381 (M+H)+ | 3317 | APCI: 419 (M+H)+ |
| 3278 | APCI: 381 (M+H)+ | 3318 | APCI: 427 (M+H)+ |
| 3319 | APCI: 431 (M+H)+ | | |
| 3320 | APCI: 435 (M+H)+ | | |
| 3321 | APCI: 444 (M+H)+ | | |
| 3322 | APCI: 449 (M+H)+ | | |
| 3323 | APCI: 449 (M+H)+ | | |
| 3324 | APCI: 451 (M+H)+ | | |
| 3325 | APCI: 451 (M+H)+ | | |
| 3326 | APCI 459 (M+H)+ | | |
| 3327 | APCI: 463 (M+H)+ | | |

Experimental Example 1

Human CB2 receptor binding inhibition test

First, cDNA sequence (Munro et al., Nature, 1993, 365, 61-65) encoding a human CB2 receptor was inserted in the forward direction in an animal-cell expression vector, pTARGET Vector (manufactured by Promega) at a region downstream of a CMV promoter. Host cells CHO-DHFR(-) were transfected with the obtained expression vector with the aid of Lipofectamine (manufactured by Invitrogen) to obtain cells capable of stably expressing the CB2 receptor.

The membrane fractions prepared from CHO cells capable of stably expressing the CB2 receptor were incubated together with a test compound and [$^3$H]CP-55,940 (final concentration: 0.57 nM, manufactured by Perkin Elmer) in an assay buffer (50 mM Tris-HCl buffer (pH 7.4), 2.5 mM EDTA, 5 mM $MgCl_2$) containing 0.2% bovine serum albumin at 25°C for 2 hours, and thereafter, filtrated by a glass filter GF/C treated with 0.1% poly-L-lysine (manufactured by SIGMA). After the filtrate was washed with an assay buffer containing 0.1% bovine serum albumin, the radioactivity on the glass filter was measured by a liquid scintillation counter. Nonspecific binding was measured in the presence of 2.0 μM CP-55,940 (manufactured by Tocris). 50% inhibitory concentration ($IC_{50}$ value) of the test compound for the specific binding was obtained. The test results are shown in Table 16. As shown in the table, the tested compounds exhibited affinity for the CB2 receptor.

Table 16 Inhibition of binding to human C82 receptor

| Compound No. | CB2 IC50 (nM) | Compound No. | CB2 IC50 (nM) | Compound No. | CB2 IC50 (nM) | Compound No. | CB2 IC50 (nM) |
|---|---|---|---|---|---|---|---|
| 9 | 11.3 | 67 | 3.0 | 99 | 6.5 | 138 | 12.0 |
| 12 | 13.3 | 70 | 14.9 | 100 | 7.0 | 139 | 7.4 |
| 23 | 8.9 | 71 | 5.9 | 104 | 1.7 | 140 | 6.1 |
| 24 | 7.5 | 73 | 10.2 | 105 | 1.7 | 141 | 13.9 |
| 25 | 17.4 | 74 | 4.6 | 106 | 6.5 | 143 | 74 |
| 29 | 8.6 | 76 | 14.4 | 107 | 0.5 | 144 | 4.3 |
| 30 | 6.6 | 77 | 19.3 | 109 | 1.3 | 145 | 3.9 |
| 33 | 8.2 | 78 | 9.8 | 113 | 1.1 | 146 | 6.6 |
| 34 | 6.6 | 79 | 8.4 | 114 | 1.6 | 147 | 9.6 |
| 35 | 3.4 | 80 | 17.9 | 115 | 0.5 | 149 | 12.2 |
| 36 | 2.2 | 81 | 12.3 | 117 | 5.6 | 150 | 15.0 |
| 37 | 11.3 | 85 | 18.9 | 119 | 18.5 | 151 | 3.2 |
| 41 | 1.4 | 86 | 6.5 | 120 | 74 | 153 | 17.8 |
| 45 | 16.5 | 88 | 17.8 | 122 | 8.9 | 154 | 5.9 |
| 46 | 1.2 | 89 | 9.8 | 129 | 7.1 | 155 | 4.3 |
| 51 | 2.6 | 90 | 6.5 | 130 | 8.5 | 156 | 11.4 |
| 54 | 187 | 91 | 7.9 | 131 | 5.6 | 157 | 12.1 |
| 56 | 0.8 | 92 | 3.9 | 132 | 157 | 158 | 7.1 |
| 57 | 3.8 | 93 | 17.6 | 133 | 7.3 | 159 | 8.4 |
| 58 | 1.8 | 94 | 2.9 | 134 | 6.5 | 160 | 8.5 |
| 59 | 7.2 | 96 | 7.7 | 135 | 11.9 | 161 | 9.7 |
| 63 | 4.9 | 97 | 33 | 136 | 11.6 | 162 | 12.0 |
| 163 | 14.9 | 195 | 3.2 | 227 | 82 | 250 | 1.1 |
| 164 | 4.1 | 196 | 7.6 | 228 | 0.5 | 251 | 3.4 |
| 165 | 4.3 | 197 | 20 | 229 | 1.0 | 254 | 1.4 |

(continued)

| Compound No. | CB2 IC50 (nM) | Compound No. | CB2 IC50 (nM) | Compound No. | CB2 IC50 (nM) | Compound No. | CB2 IC50 (nM) |
|---|---|---|---|---|---|---|---|
| 166 | 7.2 | 198 | 2.7 | 230 | 1.5 | 255 | 0.6 |
| 167 | 4.6 | 200 | 15.1 | 231 | 3.0 | 256 | 1.1 |
| 168 | 5.4 | 201 | 19.1 | 232 | 0.5 | 257 | 1.3 |
| 170 | 138 | 206 | 5.7 | 234 | 0.7 | 258 | 0.6 |
| 174 | 11.7 | 209 | 1.4 | 235 | 3.4 | 261 | 1.0 |
| 179 | 178 | 210 | 15.7 | 236 | 2.0 | 262 | 0.6 |
| 180 | 1.0 | 211 | 12.0 | 237 | 13.1 | 263 | 0.6 |
| 181 | 0.8 | 215 | 1.1 | 238 | 4.4 | 264 | 1.0 |
| 182 | 0.3 | 216 | 1.7 | 239 | 2.4 | 265 | 1.0 |
| 183 | 0.5 | 217 | 2.6 | 240 | 3.4 | 266 | 0.8 |
| 184 | 12.0 | 218 | 5.7 | 241 | 1.0 | 267 | 1.1 |
| 185 | 11.0 | 219 | 2.1 | 242 | 4.0 | 268 | 1.1 |
| 186 | 1.7 | 220 | 3.9 | 243 | 0.4 | 269 | 3.6 |
| 187 | 17.0 | 221 | 156 | 244 | 3.2 | 270 | 0.7 |
| 189 | 15 | 222 | 11.3 | 245 | 0.8 | 272 | 5.0 |
| 190 | 1.0 | 223 | 2.0 | 246 | 0.8 | 274 | 13.2 |
| 191 | 0.7 | 224 | 13.0 | 247 | 0.3 | 277 | 9.0 |
| 192 | 13.2 | 225 | 3.9 | 248 | 5.6 | 282 | 14.5 |
| 194 | 19.1 | 226 | 7.7 | 249 | 0.4 | 283 | 5.9 |
| 284 | 1.2 | 320 | 6.2 | 351 | 2.2 | 488 | 4.0 |
| 285 | 14.2 | 321 | 2.3 | 352 | 2.3 | 489 | 6.0 |
| 286 | 12.7 | 322 | 5.6 | 365 | 3.4 | 490 | 10.0 |
| 287 | 13.5 | 323 | 6.2 | 369 | 1.4 | 491 | 9.3 |
| 288 | 0.2 | 324 | 2.0 | 382 | 17.0 | 492 | 5.0 |
| 289 | 0.5 | 325 | 13.0 | 387 | 13.7 | 493 | 1.9 |

(continued)

| Compound No. | CB2 IC50 (nM) | Compound No. | CB2 IC50 (nM) | Compound No. | CB2 IC50 (nM) | Compound No. | CB2 IC50 (nM) |
|---|---|---|---|---|---|---|---|
| 293 | 7.0 | 326 | 2.9 | 399 | 6.8 | 4.94 | 2.1 |
| 295 | 12.3 | 327 | 3.6 | 402 | 15.4 | 495 | 6.3 |
| 298 | 1.5 | 328 | 11.8 | 410 | 7.1 | 504 | 15.4 |
| 299 | 2.8 | 330 | 13.8 | 411 | 8.7 | 509 | 5.9 |
| 300 | 15.0 | 332 | 3.3 | 427 | 5.9 | 510 | 13.2 |
| 301 | 2.0 | 335 | 5.5 | 470 | 10.6 | 513 | 16.9 |
| 304 | 2.3 | 336 | 2.9 | 470 | 10.6 | 514 | 2.6 |
| 305 | 3.7 | 337 | 2.8 | 474 | 4.9 | | |
| 306 | 0.5 | 338 | 9.1 | 476 | 3.9 | | |
| 307 | 1.0 | 341 | 5.3 | 481 | 3.4 | | |
| 308 | 3.6 | 344 | 12.2 | 482 | 1.2 | | |
| 309 | 2.1 | 345 | 12.7 | 483 | 8.3 | | |
| 310 | 17.3 | 348 | 13.5 | 484 | 2.7 | | |
| 312 | 14.0 | 348 | 3.8 | 485 | 1.1 | | |
| 316 | 5.3 | 349 | 1.9 | 486 | 5.1 | | |
| 319 | 18.6 | 350 | 5.2 | 487 | 9.9 | | |

Experimental Example 2

Human CB1 receptor binding inhibition test

The CHO-DHFR(-) cells capable of stably expressing a CB1 receptor were prepared in the same manner as in Experimental Example 1. The binding test to the human CB1 receptor was performed to obtain 50% inhibitory concentration ($IC_{50}$ value) of a test compound. The test results are shown in Table 17. As shown in the table, the tested compounds exhibited affinity for the CB1 receptor.

Table 17 Inhibition of binding to human CB1 receptor

| Compound No. | CB1 IC50 (nM) | Compound No. | CB1 IC50 (nM) | Compound No. | CB1 IC50 (nM) |
|---|---|---|---|---|---|
| 56 | 294 | 236 | 215 | 299 | 28 |
| 104 | 327 | 237 | 200 | 301 | 191 |
| 107 | 18 | 240 | 168 | 304 | 372 |
| 115 | 25 | 243 | 73 | 306 | 68 |
| 180 | 395 | 245 | 32 | 308 | 336 |
| 181 | 115 | 246 | 319 | 309 | 405 |
| 182 | 22 | 247 | 3 | 323 | 362 |
| 183 | 138 | 249 | 2 | 324 | 353 |
| 186 | 271 | 256 | 102 | 327 | 55 |
| 189 | 212 | 258 | 19 | 332 | 98 |
| 190 | 53 | 261 | 375 | 486 | 211 |
| 191 | 193 | 262 | 435 | 487 | 248 |
| 206 | 460 | 263 | 56 | 490 | 291 |
| 215 | 61 | 264 | 181 | 491 | 172 |
| 223 | 168 | 265 | 100 | 492 | 265 |
| 228 | 228 | 286 | 435 | 494 | 279 |
| 229 | 263 | 288 | 49 | | |
| 232 | 211 | 289 | 136 | | |
| 235 | 315 | 295 | 497 | | |

Experimental Example 3

Test for GTPγS binding via human CB1 receptor

CHO cells capable of stably expressing the human CB1 receptor were prepared in the same manner as in Experimental Example 2. The membrane fractions thereof were incubated together with a test compound in an assay buffer [50 mM Tris-HCl (pH 7.4), 2.5 mM EDTA, 5 mM $MgCl_2$, 3 μM GDP (manufactured by SIGMA), 30 μg/ml Saponin (manufactured by SIGMA)] containing 0.2% bovine serum albumin at 30°C for 30 minutes. Thereafter, 0.1 nM [$^{35}S$] GTPγS (manufactured by Perkin Elmer) was added to the buffer, incubation was performed at 30°C for 30 minutes. The resultant solution was filtrated by a glass filter GF/C and washed. Thereafter, radioactivity on the glass filter was measured by a liquid scintillation counter. The nonspecific binding was measured in the absence of the test compound. On the condition that the maximum activity value for each of the tested compounds was regarded as 100%, an effective concentration exhibiting 50% activity ($EC_{50}$ value) was calculated.

The $EC_{50}$ values for test compounds Nos. 247 and 249 were 33 nM and 19 nM, respectively. In this way, the compounds according to the present invention showed an agonist effect on the CB1 receptor.

Experimental Example 4

Test for GTPγS binding via human CB2 receptor

The CHO cells capable of stably expressing the human CB2 receptor were prepared in the same manner as in Experimental Example 1. The GTPγS binding test was performed in the same manner as in Experimental Example 3 to obtain an effective concentration of the test compound exhibiting 50% activity ($EC_{50}$ value), on the condition that the maximum activity value for each of the test compounds was regarded as 50%.

The $EC_{50}$ values of test compounds Nos. 9, 184, 267 and 474 were 23.7 nM, 9.8 nM, 0.4 nM and 2.3 nM, respectively. In this way, the compounds according to the present invention exhibited an agonist effect on the CB2 receptor.

Experimental Example 5

Writhing test with acetic acid in mouse

This test was performed in accordance with the method of Futaki N et al. (Gen Pharmacol. 24(1):105-110 (1993). A test compound suspended in a 5% gum Arabic solution was orally administered to Jcl: ICR-series male mice (5 weeks old). One hour after the administration of the test compound, a 0.9% aqueous acetic acid solution was intraperitoneally administered. Five minutes after the administration, the number of abdomen stretch movements (pain-related behavior) were counted for 10 minutes. Only the 5% gum Arabic solution was orally administered to the control group. A pain-related behavior inhibition rate (%) was obtained based on the following equation.

$$\text{Pain-related behavior inhibition (\%)} = \frac{\text{The number of pain-related behaviors counted for control group} - \text{The number of pain-related behaviors counted for test compound group}}{\text{The number of pain-related behaviors counted for control group}} \times 100$$

The inhibitory rates of test compounds Nos. 59, 247, 267, 411, 474 and 510 when they were orally administered in a dose of 30 mg/kg were 44.8%, 93.0%, 59.9%, 49.0%, 61.9% and 19.6%, respectively. The compounds according to the present invention exhibited an analgesic effect.

Experimental Example 6

Neuropathic pain test in rat

Using SD:IGS-series male rats (5 weeks old), neuropathic pain model were prepared by partially clipping the sciatic nerve of the femoral region in accordance with the method of Seltzer et al. (SeltzerZ; Pain. 43(2):205-218(1990) ). When the plantar surface of the paw on the affected side was stimulated by touching with a von Frey filament(s) (nylon fiber for use in a touch test: North Coast Medical, Inc.), the pain threshold (load (g) applied to the filament when an animal responds to touch stimulation) was measured. The test compound was suspended in a 5% gum Arabic solution and administered in a dose of 0 mg/kg, 3 mg/kg, 10 mg/kg and 30 mg/kg. One hour after the administration, pain threshold (g) was measured.

The oral administration of test compound No. 184 increased pain threshold in a dose depending manner and demonstrated improvement of pain sensitivity (Figure 1).

Experimental Example 7

Test for edema on ear in mouse

Using Balb/c male mice (5 weeks old), a test compound dissolved in acetone was applied in an amount of 20 μL to the inside the ear. Ten minutes after the application of the test compound, an acetone solution (20 μL) containing

0.8 μg of PMA (phorbol 12-myristate 13-acetate) was applied. Five hours later, the thickness (hyperplasia) of the ear was measured by a dial sickness gauge. Acetone alone was applied to the control group. The inhibition rate (%) of edema on the ear was calculated based on the following equation.

$$\text{Ear edema inhibition (\%)} = \frac{\text{Ear thickness of PMA-treated control group} - \text{Ear thickness of PMA-treated test compound group}}{\text{Ear thickness of PMA-treated control group} - \text{Ear thickness of PMA-untreated control group}} \times 100$$

The inhibition rates of test compounds No. 184, 267 and 474 at a dose of 1 mg per murine ear, were 65%, 84% and 37%, respectively. The compounds according to the present invention exhibited an anti-edema effect.

Industrial Applicability

In the present invention, there is provided an imine compound having a cannabinoid receptor agonist effect. The imine compound of the present invention has a cannabinoid receptor agonist effect, and is useful as a therapeutic drug or prophylactic drug for pain and autoimmune disease.

Brief Description of the Drawing

Figure 1 shows the results of the neuropathic pain test in rat of Experimental Example 6.

## Claims

**1.** A cannabinoid-receptor agonist comprising an imine compound represented by Formula (I)

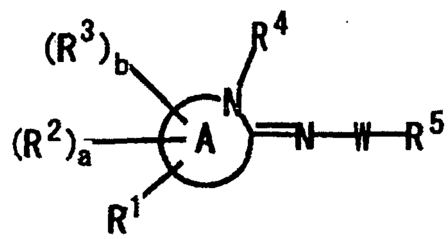

(I)

[where A represents any one of the rings represented by the following formulas (where X represents an oxygen atom or a sulfur atom, and X' represents CH or a nitrogen atom):

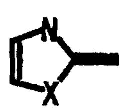 , 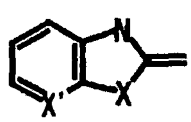 , 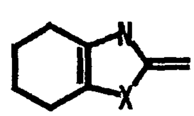 ,

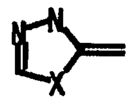 , 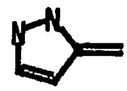 , 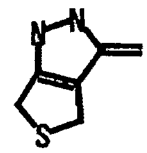 ,

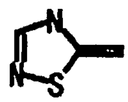 , 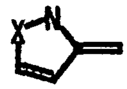 , 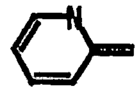 ,

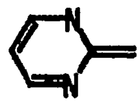 , 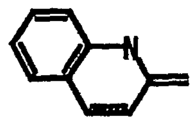 , 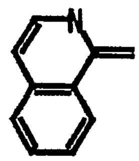

$R^1$ represents
a hydrogen atom;
a halogen atom;
a $C_{1-10}$ alkyl group that may be substituted with an aryl group(s) substituted with a halogen atom(s);
a $C_{3-10}$ cycloalkyl group;
a $C_{2-6}$ alkenyl group;
a $C_{1-6}$ haloalkyl group ;
a $C_{1-10}$ alkoxy group;
a carboxyl group;
a $C_{2-6}$ alkoxycarbonyl group;
a hydroxy-$C_{1-6}$ alkyl group;
a group represented by Formula -N($R^6$)$R^7$ (where $R^6$ and $R^7$ each represent a hydrogen atom or a $C_{1-6}$ alkyl group, or $R^6$ and $R^7$, in combination with the adjacent nitrogen atom, form a cyclic amino group);
a group represented by Formula -CON ($R^{61}$)$R^{71}$ (where $R^{61}$ and $R^{71}$ each represent a hydrogen atom or a $C_{1-6}$ alkyl group that may be substituted with a cyclic amino group(s), or $R^{61}$ and $R^{71}$, in combination with the adjacent nitrogen atom, form a cyclic amino group); or
an aryl group that may be substituted with 1 to 3 groups selected from the group consisting of: a $C_{1-6}$ alkyl group, a $C_{1-6}$ haloalkyl group and a halogen atom,
$R^2$ and $R^3$ each represent
a hydrogen atom;
a halogen atom;
a $C_{1-6}$ alkyl group;
a $C_{1-6}$ haloalkyl group; or
an aryl group that may be substituted with 1 to 3 groups selected from the group consisting of: a $C_{1-6}$ alkyl group, a $C_{1-6}$ haloalkyl group and a halogen atom,
$R^4$ represents
a $C_{1-10}$ alkyl group;
a $C_{1-6}$ haloalkyl group;
a $C_{1-10}$ alkyl group or $C_{1-6}$ alkenyl group substituted with: a $C_{3-10}$ cycloalkyl group (s) , a $C_{1-6}$ alkoxy group(s), a hydroxyl group(s), an amino group(s), a phthalimide group(s), a cyano group(s), an arylthio group(s), a $C_{2-6}$ alkoxycarbonyl group(s), a carboxyl group(s), a group(s) represented by Formula -CON($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group), or an aryl group(s) that may be substituted with a $C_{1-6}$ haloalkyl group(s), a $C_{2-6}$ alkoxycarbonyl group(s), a carboxyl group(s), or an N-piperidinocarbamoyl group(s);
a $C_{2-6}$ haloalkenyl group;
a $C_{2-6}$ alkynyl group;
a 1,1-dioxothiolanyl group; or
an aryl group,

$R^5$ represents
a hydrogen atom;
a $C_{1-10}$ alkoxy group;
a $C_{1-6}$ alkoxy-$C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, a $C_{2-6}$ alkoxycarbonyl group, a $C_{1-6}$ haloalkyl group, a $C_{1-6}$ alkoxy group that may be substituted with a $C_{1-6}$ alkoxy group(s) or an aryl group(s), a $C_{3-10}$ cycloalkoxy group that may be substituted with 1 to 2 $C_{1-6}$ alkyl groups, an aryl or aryloxy group that may be substituted with 1 to 5 groups selected from the group consisting of a $C_{1-6}$ alkyl group, a $C_{1-6}$ alkoxy group, a $C_{1-6}$ haloalkyl group, a $C_{1-6}$ haloalkoxy group, an aralkyloxy group, a nitro group and a halogen atom, a heterocyclic group, a phthalimide group, a $C_{1-6}$ alkanoyloxy group, an aralkyloxy group, a $C_{1-6}$ alkylthio group, an arylthio group, and a group represented by Formula $-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with a $C_{1-6}$ alkyl group(s), a $C_{1-6}$ alkoxy group (s), a $C_{2-6}$ alkoxycarbonyl group(s), a $C_{1-6}$ haloalkyl group (s) or a $C_{1-6}$ haloalkoxy group (s);
an aralkyloxy group; or
a group represented by Formula (II)

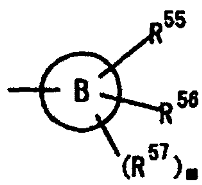

(II)

{where
B represents
a $C_{3-10}$ cycloalkyl group;
an aryl group;
a heterocyclic group;
a $C_{2-6}$ cyclic amino group;
a fluorenyl group;
a phthalimide group;
a 2-oxopyrrolidinyl group;
a group represented by Formula (III)

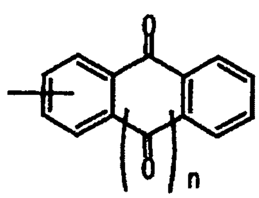

(III)

(where n represents 0 or 1); or
a group represented by Formula (IV)

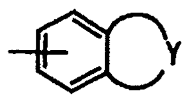

(IV)

(where Y represents $-(CH_2)p-$, $-CO-CH_2-CH_2-$, $-CO-CH_2-CH_2-CH_2-$, $-O-CH_2-CH_2-$, $-O-CH_2-CH=CH-$, or $-O-(CH_2)q-O-$, in which p represents an integer of 2 to 4 and q represents an integer of 1 to 3);
$R^{55}$ represents
a hydrogen atom;
a halogen atom;
a $C_{1-10}$ alkyl group that may be substituted with an aryl group(s), a heterocyclic group(s) or an aryloxy group(s), each of which may be substituted with a halogen atom(s); or with a group(s) represented by Formula $-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ alkylsulfonyl group;
an arylsulfonyl group that may be substituted with a $C_{1-6}$ alkyl group(s) or a halogen atom(s);
a $C_{1-6}$ haloalkoxy group;
a $C_{1-6}$ haloalkylthio group;
a $C_{3-10}$ cycloalkyl group;
a $C_{2-6}$ alkenyl group;
a $C_{2-6}$ alkenyloxy group;
a $C_{2-6}$ alkenylthio group;
a $C_{1-6}$ alkoxy-$C_{1-6}$alkoxy group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group, a cyano group and a nitro group;
a heterocyclic group that may be substituted with a $C_{1-6}$ alkyl group(s) or a $C_{1-6}$ haloalkyl group(s);
an aryloxy group or arylthio group that may be substituted with a halogen atom(s) or a $C_{1-6}$ alkyl group(s);
a group represented by Formula $-N(R^{63})R^{73}$ (where $R^{63}$ and $R^{73}$ each represent a hydrogen atom, a $C_{1-6}$ alkyl group, a $C_{1-6}$ hydroxyalkyl group, a $C_{1-6}$ alkoxy-$C_{1-6}$ alkyl group, an aryl group, a $C_{1-6}$ alkanoyl group, a di-$C_{1-6}$ alkylamino-$C_{2-6}$ alkanoyl group, a benzoyl group, or a heterocyclic group that may be substituted with a $C_{1-6}$ alkyl group (s), or $R^{63}$ and $R^{73}$, in combination with the adjacent nitrogen atom, form a cyclic amino group);
a hydroxyl group;
a cyano group;
a nitro group;
a $C_{1-6}$ alkanoyl group;
a $C_{1-6}$ alkanoyloxy group;
a $C_{1-6}$ alkanoyloxy-$C_{1-6}$ alkyl group;
a $C_{2-6}$ haloalkanoyl group;
a carboxyl group;
a $C_{2-6}$ alkoxycarbonyl group;
a $C_{2-6}$ cyclic amino group that may be substituted with an aryl group(s);
a group represented by Formula $-CON(R^{64})R^{74}$ (where $R^{64}$ and$R^{74}$ each represent a hydrogen atom, a $C_{1-6}$ alkyl group, a $C_{1-6}$ alkoxy-$C_{1-6}$ alkyl group, or a heterocyclic group that may be substituted with a $C_{1-6}$ alkyl group(s), or $R^{64}$ and $R^{74}$, in combination with the adjacent nitrogen atom, form a cyclic amino group);
a group represented by Formula $-SO_2N(R^{62})R^{62}$ (where $R^{62}$ and $R^{72}$ each represent a hydrogen atom, or a $C_{1-6}$ alkyl group, or $R^{62}$ and $R^{72}$, in combination with the adjacent nitrogen atom, form a cyclic amino group);
a $C_{1-6}$ alkylsulfenyl group;
a $C_{1-6}$ alkylsulfonyl group that may be substituted with a halogen atom(s); or
an arylsulfonyl group that may be substituted with a halogen atom(s),
$R^{56}$ represents
a hydrogen atom;
a halogen atom;

a $C_{1-10}$ alkyl group that may be substituted with: an aryl group(s), a pyridyl group(s), a thienyl group(s) or a heterocyclic group(s), each of which may be substituted with a $C_{1-6}$ alkyl group(s) or a halogen atom(s);
a $C_{1-6}$ haloalkyl group;
a $C_{3-10}$ cycloalkyl group;
a $C_{1-10}$ alkoxy group;
a $C_{2-6}$ alkenyl group;
an aryl group that may be substituted with 1 to 3 groups selected from the group consisting of: a $C_{1-6}$ alkyl group, a halogen atom, a $C_{1-6}$ alkoxy group and a nitro group;
a heterocyclic group that may be substituted with a $C_{1-6}$ alkyl group(s);
a $C_{1-6}$ alkanoyl group;
a $C_{1-6}$ alkylsulfenyl group;
a $C_{1-6}$ alkylsulfonyl group;
an arylsulfonyl group that may be substituted with a halogen atom(s);
a hydroxyl group;
a cyano group; or
a nitro group,
$R^{57}$ represents
a hydrogen atom;
a $C_{1-10}$ alkyl group that may be substituted with a pyridyl group(s) or a thienyl group(s);
a $C_{1-6}$ haloalkyl group;
a $C_{3-10}$ cycloalkyl group;
a halogen atom;
a $C_{2-6}$ alkenyl group;
an aryl group that may be substituted with a halogen atom(s); a $C_{1-10}$ alkoxy group;
a $C_{1-6}$ alkanoyl group; or
a $C_{1-6}$ alkylsulfenyl group, and
m represents an integer of 1 to 3),
a and b each represent 0 or 1, and
W represents -CO-, -CO-CO-, -CO-NH-, -CS-NH-, or $-SO_2-$] or a pharmaceutically acceptable salt thereof, as an active ingredient.

**2.** The cannabinoid-receptor agonist according to claim 1 comprising the imine compound or the pharmaceutically acceptable salt thereof, as an active ingredient,
wherein
$R^1$ represents
a hydrogen atom;
a halogen atom;
a $C_{1-10}$ alkyl group that may be substituted with an aryl group(s) substituted with a halogen atom(s);
a $C_{3-10}$ cycloalkyl group;
a $C_{1-6}$ haloalkyl group;
a $C_{1-10}$ alkoxy group;
a carboxyl group;
a $C_{2-6}$ alkoxycarbonyl group;
a hydroxy-$C_{1-6}$ alkyl group;
a group represented by Formula $-N(R^6)R^7$ (where $R^6$ and $R^7$ each represent a hydrogen atom or a $C_{1-6}$ alkyl group, or $R^6$ and $R^7$,
in combination with the adjacent nitrogen atom, form a cyclic amino group);
a group represented by Formula $-CON(R^{61})R^{71}$ (where $R^{61}$ and $R^{71}$ each represent a hydrogen atom or a $C_{1-6}$ alkyl group that may be substituted with a cyclic amino group(s), or $R^{61}$ and $R^{71}$, in combination with the adjacent nitrogen atom, form a cyclic amino group); or
an aryl group that may be substituted with 1 to 3 groups selected from the group consisting of: a $C_{1-6}$ alkyl group, a $C_{1-6}$ haloalkyl group and a halogen atom,
$R^4$ represents
a $C_{1-10}$ alkyl group;
a $C_{1-6}$ haloalkyl group;
a $C_{1-10}$ alkyl group substituted with a $C_{3-10}$ cycloalkyl group(s), a $C_{1-6}$ alkoxy group(s), a hydroxyl group(s), an amino group(s), a phthalimide group(s), a cyano group(s), an arylthio group(s), a $C_{2-6}$ alkoxycarbonyl group(s), a carboxyl group(s), a group(s) represented by Formula $-CON(R^{62})R^{72}$ (where $R^{62}$ and $R^{72}$ each represent a hydrogen atom,

or a $C_{1-6}$ alkyl group, or $R^{62}$ and $R^{72}$, in combination with the adjacent nitrogen atom, form a cyclic amino group), or an aryl group(s) that may be substituted with a $C_{1-6}$ haloalkyl group(s), a $C_{2-6}$ alkoxycarbonyl group(s), a carboxyl group(s) or an N-piperidinocarbamoyl group(s);
a $C_{2-6}$ alkenyl group that may be substituted with an aryl group(s);
a $C_{2-6}$ haloalkenyl group;
a $C_{2-6}$ alkynyl group;
a 1,1-dioxothiolanyl group; or
an aryl group,
$R^5$ represents
a $C_{1-10}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, a $C_{2-6}$ alkoxycarbonyl group, a $C_{1-6}$ haloalkyl group, a $C_{1-6}$ alkoxy group that may be substituted with a $C_{1-6}$ alkoxy group(s) or an aryl group(s), a $C_{3-10}$ cycloalkoxy group that may be substituted with 1 to 2 $C_{1-6}$ alkyl groups, an aryl group or aryloxy group that may be substituted with 1 to 5 groups selected from the group consisting of a $C_{1-6}$ alkyl group, a $C_{1-6}$ alkoxy group, a $C_{1-6}$ haloalkyl group, a $C_{1-6}$ haloalkoxy group,
an aralkyloxy group, a nitro group and a halogen atom, a heterocyclic group, a phthalimide group, a $C_{1-6}$ alkanoyloxy group, an aralkyloxy group, a $C_{1-6}$ alkylthio group, an arylthio group, and a group represented by Formula -N($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with a $C_{1-6}$ alkyl group(s), a $C_{1-6}$ alkoxy group(s), a $C_{2-6}$ alkoxycarbonyl group(s), a $C_{1-6}$ haloalkyl group (s) or a $C_{1-6}$ haloalkoxy group (s) ; or
a group represented by Formula (II), where
B represents
a $C_{3-10}$ cycloalkyl group, an aryl group, or a heterocyclic group,
$R^{55}$ and $R^{56}$ each represent
a hydrogen atom;
a halogen atom;
a $C_{1-10}$ alkyl group that may be substituted with: an aryl group(s), a heterocyclic group(s) or an aryloxy group(s), each of which may be substituted with a halogen atom(s); or with a group(s) represented by Formula -N($R^{62}$)$R^{72}$ ($R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ alkylsulfonyl group;
an arylsulfonyl group that may be substituted with a $C_{1-6}$ alkyl group(s) or a halogen atom(s);
a $C_{1-6}$ haloalkoxy group
a $C_{1-6}$ haloalkylthio group;
a $C_{3-10}$ cycloalkyl group;
a $C_{2-6}$ alkenyl group;
a $C_{2-6}$ alkenyloxy group;
a $C_{2-6}$ alkenylthio group;
a $C_{1-6}$ alkoxy-$C_{1-6}$alkoxy group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group, a cyano group and a nitro group;
a heterocyclic group that may be substituted with a $C_{1-6}$ alkyl group(s) or a $C_{1-6}$ haloalkyl group(s);
an aryloxy group or arylthio group that may be substituted with a halogen atom(s) or a $C_{1-6}$ alkyl group(s);
a group represented by Formula-N($R^{63}$)$R^{73}$(where $R^{63}$ and $R^{73}$ each represent a hydrogen atom, a $C_{1-6}$ alkyl group, a $C_{1-6}$ hydroxyalkyl group, a $C_{1-6}$ alkoxy-$C_{1-6}$ alkyl group, an aryl group, a $C_{1-6}$ alkanoyl group, a di-$C_{1-6}$ alkylamino-$C_{2-6}$ alkanoyl group, a benzoyl group or a heterocyclic group that may be substituted with a $C_{1-6}$ alkyl group(s), or $R^{63}$ and $R^{73}$, in combination with the adjacent nitrogen atom, form a cyclic amino group);
a hydroxyl group;
a cyano group;
a nitro group;
a $C_{1-6}$ alkanoyl group;
a $C_{1-6}$ alkanoyloxy group;

a $C_{1-6}$ alkanoyloxy-$C_{1-6}$ alkyl group;
a $C_{2-6}$ haloalkanoyl group;
a carboxyl group;
a $C_{2-6}$ alkoxycarbonyl group; or a group represented by Formula -CON($R^{64}$)$R^{74}$(where $R^{64}$ and $R^{74}$ each represent a hydrogen atom, a $C_{1-6}$ alkyl group, a $C_{1-6}$ alkoxy-$C_{1-6}$ alkyl group, or a heterocyclic group that may be substituted with a $C_{1-6}$ alkyl group(s), or $R^{64}$ and $R^{74}$, in combination with the adjacent nitrogen atom, form a cyclic amino group),
$R^{57}$ represents
a hydrogen atom;
a $C_{1-10}$ alkyl group;
a $C_{1-6}$ haloalkyl group;
a halogen atom; or
a $C_{1-10}$ alkoxy group.

**3.** A cannabinoid-receptor agonist comprising an imine compound represented by Formula (I-1)

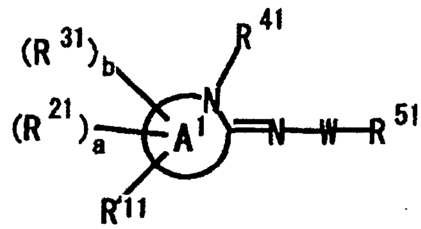

(I-1)

[where $A^1$ represents any one of the rings represented by the following formulas (where X represents an oxygen atom or a sulfur atom):

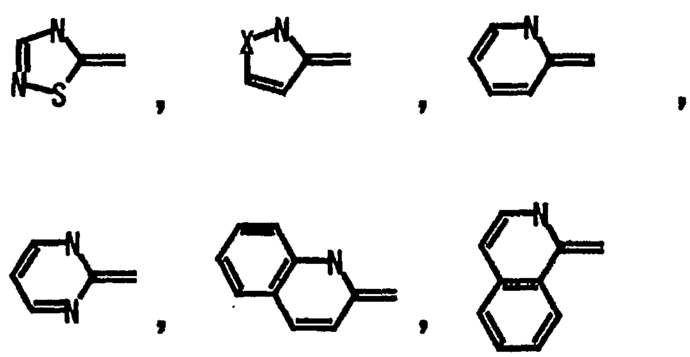

$R^{11}$ represents
a hydrogen atom;
a halogen atom;
a $C_{1-6}$ alkyl group
a $C_{2-6}$ alkenyl group;
a $C_{1-6}$ haloalkyl group;
a $C_{1-6}$ alkoxy group; or a group represented by Formula -N($R^6$)$R^7$ (where $R^6$ and $R^7$ each represent a hydrogen atom or a $C_{1-6}$ alkyl group, or $R^6$ and $R^7$ in combination with the adjacent nitrogen atom, form a cyclic amino group),
$R^{21}$ and $R^{31}$ each represent
a hydrogen atom;
a halogen atom; or
a $C_{1-6}$ alkyl group,
$R^{41}$ represents

a $C_{1-10}$ alkyl group or $C_{2-6}$ alkenyl group that may be substituted with a halogen atom(s), a $C_{3-10}$ cycloalkyl group(s), an aryl group (s) or a $C_{1-6}$ alkoxy group(s),
$R^{51}$ represents
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, an aryl group or aryloxy group that may be substituted with 1 to 5 groups selected from the group consisting of a $C_{1-6}$ alkyl group, a $C_{1-6}$ alkoxy group, a $C_{1-6}$ haloalkyl group and a halogen atom, and a heterocyclic group;
a group represented by Formula (II-1)

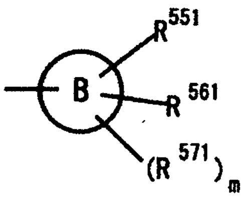

(II-1)

{where
B represents
a $C_{3-10}$ cycloalkyl group;
an aryl group;
a heterocyclic group;
a fluorenyl group; or
a group represented by Formula (IV-1)

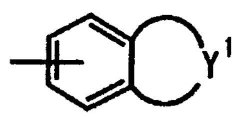

(IV-1)

(where $Y^1$ represents $-(CH_2)p-$, or $-O-(CH_2)q-O-$, in which p represents an integer of 2 to 4, and q represents an integer of 1 to 3),
$R^{551}$ and $R^{561}$ each represent
a hydrogen atom;
a halogen atom;
a $C_{1-10}$ alkyl group that may be substituted with: an aryl group(s), a heterocyclic group(s) or an aryloxy group(s), each of which may be substituted with a halogen atom(s);
or with a group(s) represented by Formula $-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ haloalkoxy group;
a $C_{3-10}$ cycloalkyl group;
a $C_{2-6}$ alkenyl group;
an aryl group that may be substituted with 1 to 3 groups selected from the group consisting of: a $C_{1-6}$ alkyl group, a $C_1$-6 haloalkyl group, a halogen atom, a $C_{1-6}$ alkoxy group, and a nitro group;
a heterocyclic group that may be substituted with a $C_{1-6}$ alkyl group(s);
an aryloxy group that may be substituted with a halogen atom(s);

a group represented by Formula -N ($R^{634}$) $R^{734}$ (where $R^{634}$ and $R^{734}$ each represent a hydrogen atom, a $C_{1-6}$ alkyl group, an aryl group, or a $C_{1-6}$ alkanoyl group, or $R^{634}$ and $R^{734}$, in combination with the adjacent nitrogen atom, form a cyclic amino group);
a hydroxyl group;
a cyano group;
a nitro group;
a $C_{1-6}$ alkanoyl group;
a $C_{2-6}$ haloalkanoyl group;
a $C_{1-6}$ alkylsulfonyl group; or
an arylsulfonyl group that may be substituted with a halogen atom(s),
$R^{571}$ represents
a hydrogen atom;
$C_{1-10}$ alkyl group ;
a $C_{1-10}$ alkoxy group; or
a halogen atom, and
m represents an integer of 1 to 3}
a and b each represent 0 or 1, and
W represents CO or $SO_2$]
or a pharmaceutically acceptable salt thereof, as an active ingredient.

**4.** A cannabinoid-receptor agonist comprising the imine compound or the pharmaceutically acceptable salt thereof according to claim 3, as an active ingredient, wherein
$R^{51}$ represents a $C_{1-10}$ alkyl group or $C_{2-6}$ alkenyl group that may be substituted with 1 to 3 groups selected from the group consisting of: a halogen atom, a $C_{3-10}$ cycloalkyl group, and an aryl group, a thienyl group and an aryloxy group, each of which may be substituted with a $C_{1-6}$ haloalkyl group(s) or a halogen atom(s); or
represents a group represented by Formula (II-1) where
$R^{551}$ represents
a hydrogen atom;
a halogen atom;
a $C_{1-10}$ alkyl group that may be substituted with an aryl group(s) that may be substituted with a halogen atom(s), a heterocyclic group(s) or an aryloxy group(s);
a $C_{1-6}$ haloalkyl group;
a $C_{1-10}$ alkoxy group;
a $C_{1-6}$ haloalkoxy group;
a $C_{3-10}$ cycloalkyl group;
an aryl group that may be substituted with 1 to 3 groups selected from the group consisting of: a $C_{1-6}$ alkyl group, a halogen atom, a $C_{1-6}$ alkoxy group and a nitro group;
a heterocyclic group that may be substituted with a $C_{1-6}$ alkyl group(s); an aryloxy group that may be substituted with a halogen atom(s);
a group represented by Formula -N($R^{631}$)$R^{731}$(where $R^{631}$ and $R^{731}$ each represent a hydrogen atom, a $C_{1-6}$ alkyl group, an aryl group or' a $C_{1-6}$ alkanoyl group, or $R^{631}$ and $R^{731}$, in combination with the adjacent nitrogen atom, form a cyclic amino group); a cyano group;
a $C_{1-6}$ alkanoyl group;
a $C_{2-6}$ haloalkanoyl group; or
a $C_{1-6}$ alkylsulfonyl group,
$R^{561}$ represents
a hydrogen atom;
a halogen atom;
a $C_{1-10}$ alkyl group;
a $C_{1-6}$ haloalkyl group; or
a $C_{1-10}$ alkoxy group,
$R^{571}$ represents
a hydrogen atom;
a halogen atom;
$C_{1-10}$ alkyl group; or
a $C_{1-10}$ alkoxy group.

**5.** A cannabinoid-receptor agonist comprising the imine compound or the pharmaceutically acceptable salt thereof

according to claim 3 or 4, as an active ingredient, wherein
$A^1$ is a ring represented by Formula below:

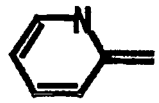

.

**6.** A cannabinoid-receptor agonist comprising the imine compound or the pharmaceutically acceptable salt thereof according to claim 5, as an active ingredient, wherein
$R^{51}$ represents
a $C_{1-10}$ alkyl group that may be substituted with: a $C_{3-10}$ cycloalkyl group(s), or an aryl group(s), a thienyl group(s) or an aryloxy group(s), each of which may be substituted with a $C_{1-6}$ haloalkyl group(s) or a halogen atom(s); or
a group(s) represented by Formula (II-1), where
B represents
a $C_{3-10}$ cycloalkyl group, an aryl group, or a heterocyclic group,
$R^{551}$ represents
a halogen atom;
a $C_{1-10}$ alkyl group;
a $C_{1-6}$ haloalkyl group;
a $C_{1-10}$ alkoxy group;
a $C_{1-6}$ haloalkoxy group;
a $C_{3-10}$ cycloalkyl group;
an aryl group that may be substituted with 1 to 3 groups selected from the group consisting of a $C_{1-6}$ alkyl group and a halogen atom;
a heterocyclic group that may be substituted with a $C_{1-6}$ alkyl group(s);
an aryloxy group;
a morpholino group;
an arylamino group;
a cyano group;
a $C_{1-6}$ alkanoyl group;
a $C_{2-6}$ haloalkanoyl group; or
a $C_{1-6}$ alkylsulfonyl group,
$R^{561}$ represents
a hydrogen atom;
a halogen atom;
a $C_{1-10}$ alkyl group;
a $C_{1-6}$ haloalkyl group; or
a $C_{1-10}$ alkoxy group,
$R^{571}$ represents
a hydrogen atom;
a halogen atom;
a $C_{1-10}$ alkyl group; or
a $C_{1-10}$ alkoxy group.

**7.** A cannabinoid-receptor agonist comprising the imine compound or the pharmaceutically acceptable salt thereof according to claim 6, as an active ingredient, wherein
$R^{51}$ represents a group represented by Formula (II-1),
B represents a phenyl group,
$R^{551}$ represents
a halogen atom;
a $C_{1-10}$ alkyl group
a $C_{1-6}$ haloalkyl group;
a $C_{1-6}$ alkoxy group;
a $C_{1-6}$ haloalkoxy group;
a $C_{3-10}$ cycloalkyl group;

an aryl group
an aryloxy group;
a morpholino group;
an arylamino group
a cyano group
a $C_{1-6}$ alkanoyl group;
a $C_{2-6}$ haloalkanoyl group; or
a $C_{1-6}$ alkylsulfonyl group,
$R^{561}$ represents
a hydrogen atom;
a halogen atom;
a $C_{1-6}$ haloalkyl group; or
a $C_{1-6}$ alkoxy group,
$R^{571}$ represents
a hydrogen atom;
a halogen atom;
a $C_{1-10}$ alkyl group; or
a $C_{1-10}$ alkoxy group (provided that when $R^{551}$, $R^{571}$ and $R^{41}$ each are an alkyl group, the number of carbon atoms of $R^{551}$ and $R^{571}$ are 1 to 6, and the number of carbon atoms of $R^{41}$ is 2 to 10), and
m represents 1.

**8.** The cannabinoid-receptor agonist according to any one of claims 1 to 7, being a cannabinoid type-1 receptor agonist or a cannabinoid type-2 receptor agonist.

**9.** The cannabinoid-receptor agonist according to any one of claims 1 to 7, being a therapeutic drug or prophylactic drug for pain.

**10.** The cannabinoid-receptor agonist according to any one of claims 1 to 7, being a therapeutic drug or prophylactic drug for autoimmune disease.

**11.** An imine compound represented by Formula (I-1)

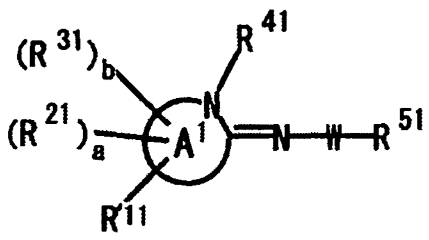

(I-1)

[where $A^1$ represents any one of the rings represented by the following formulas (where X represents an oxygen atom or a sulfur atom):

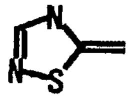 , 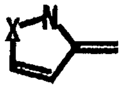 , 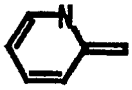 ,

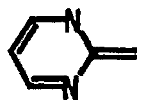 , 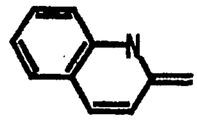 , 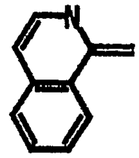

$R^{11}$ represents
a hydrogen atom;
a halogen atom;
a $C_{1-6}$ alkyl group
a $C_{2-6}$ alkenyl group;
a $C_{1-6}$ haloalkyl group;
a $C_{1-6}$ alkoxy group; or a group represented by Formula-$N(R^6)R^7$ (where $R^6$ and $R^7$ each represent a hydrogen atom or a $C_{1-6}$ alkyl group, or $R^6$ and $R^7$, in combination with the adjacent nitrogen atom, form a cyclic amino group),
$R^{21}$ and $R^{31}$ each represent
a hydrogen atom;
a halogen atom; or
a $C_{1-6}$ alkyl group,
$R^{41}$ represents
a $C_{1-10}$ alkyl group or $C_{2-6}$ alkenyl group that may be substituted with a halogen atom(s), a $C_{3-10}$ cycloalkyl group (s), an aryl group(s) or a $C_{1-6}$ alkoxy group(s),
$R^{51}$ represents
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, an aryl group or aryloxy group that may be substituted with 1 to 5 groups selected from the group consisting of a $C_{1-6}$ alkyl group, a $C_{1-6}$ alkoxy group, a $C_{1-6}$ haloalkyl group and a halogen atom, and a heterocyclic group;
a group represented by Formula (II-1)

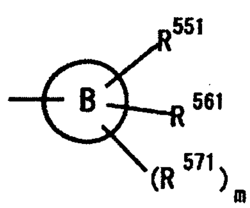

(II-1)

{where
B represents
a $C_{3-10}$ cycloalkyl group;
an aryl group;
a heterocyclic group;
a fluorenyl group; or
a group represented by Formula (IV-1)

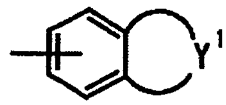

(IV-1)

(where $Y^1$ represents $-(CH_2)p-$, or $-O-(CH_2)q-O-$, in which p represents an integer of 2 to 4, and q represents an integer of 1 to 3),
$R^{551}$ and $R^{561}$ each represent
a hydrogen atom;
a halogen atom;
a $C_{1-10}$ alkyl group that may be substituted with: an aryl group(s), a heterocyclic group(s) or an aryloxy group(s), each of which may be substituted with a halogen atom(s); or with a group(s) represented by Formula $-N(R^{62})R^{72}$ (where $R^{62}$ and $R^{71}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ haloalkoxy group
a $C_{3-10}$ cycloalkyl group;
a $C_{2-6}$ alkenyl group;
an aryl group that may be substituted with 1 to 3 groups selected from the group consisting of: a $C_{1-6}$ alkyl group, a $C_{1-6}$ haloalkyl group, a halogen atom, a $C_{1-6}$ alkoxy group and a nitro group;
a heterocyclic group that may be substituted with a $C_{1-6}$ alkyl group(s);
an aryloxy group that may be substituted with a halogen atom(s);
a group represented by Formula $-N(R^{634})R^{734}$(where $R^{634}$ and $R^{734}$ each represent a hydrogen atom, a $C_{1-6}$ alkyl group, an aryl group, or a $C_{1-6}$ alkanoyl group, or $R^{634}$ and $R^{734}$, in combination with the adjacent nitrogen atom, form a cyclic amino group);
a hydroxyl group;
a cyano group;
a nitro group;
a $C_{1-6}$ alkanoyl group;
a $C_{2-6}$ haloalkanoyl group;
a $C_{1-6}$ alkylsulfonyl group; or
an arylsulfonyl group that may be substituted with a halogen atom(s),
$R^{571}$ represents
a hydrogen atom;
a halogen atom;
a $C_{1-10}$ alkyl group; or
a $C_{1-10}$ alkoxy group, and
m represents an integer of 1 to 3}
a and b each represent 0 or 1, and
W represents CO or $SO_2$],
or a pharmaceutically acceptable salt thereof.

**12.** The imine compound or the pharmaceutically acceptable salt thereof according to claim 11, wherein
$R^{51}$ represents
a $C_{1-10}$ alkyl group or $C_{2-6}$ alkenyl group that may be substituted with 1 to 3 groups selected from the group consisting of: a halogen atom, a $C_{3-10}$ cycloalkyl group, and an aryl group, a thienyl group and an aryloxy group, each of which may be substituted with a $C_{1-6}$ haloalkyl group(s) or a halogen atom(s); or represents a group represented by Formula (II-1) where
$R^{551}$ represents
a hydrogen atom;
a halogen atom;
a $C_{1-10}$ alkyl group that may be substituted with an aryl group(s), a heterocyclic group(s) or an aryloxy group(s) that

may be substituted with a halogen atom(s);
a $C_{1-6}$ haloalkyl group;
a $C_{1-10}$ alkoxy group;
a $C_{1-6}$ haloalkoxy group;
a $C_{3-10}$ cycloalkyl group;
an aryl group that may be substituted with 1 to 3 groups selected from the group consisting of: a $C_{1-6}$ alkyl group, a halogen atom, a $C_{1-6}$ alkoxy group and a nitro group;
a heterocyclic group that may be substituted with a $C_{1-6}$ alkyl group(s);
an aryloxy group that may be substituted with a halogen atom(s);
a group represented by Formula $-N(R^{631})R^{731}$(where $R^{631}$ and $R^{731}$ each represent a hydrogen atom, a $C_{1-6}$ alkyl group, an aryl group or a $C_{1-6}$ alkanoyl group, or $R^{631}$ and $R^{731}$, in combination with the adjacent nitrogen atom, form a cyclic amino group); a cyano group;
a $C_{1-6}$ alkanoyl group;
a $C_{2-6}$ haloalkanoyl group; or
a $C_{1-6}$ alkylsulfonyl group,
$R^{561}$ represents
a hydrogen atom;
a halogen atom;
a $C_{1-10}$ alkyl group;
a $C_{1-6}$ haloalkyl group; or
a $C_{1-10}$ alkoxy group,
$R^{571}$ represents
a hydrogen atom;
a halogen atom;
a $C_{1-10}$ alkyl group; or
a $C_{1-10}$ alkoxy group.

**13.** The imine compound or the pharmaceutically acceptable salt thereof according to claim 11 or 12, wherein W is -CO-.

**14.** The imine compound or the pharmaceutically acceptable salt thereof according to claim 13, wherein
$R^{41}$ represents a $C_{1-10}$ alkyl group substituted with a $C_{1-6}$ alkoxy group(s), or an aryl group(s); $R^{551}$ is a $C_{1-6}$ haloalkyl group; and $R^{564}$ is a halogen atom.

**15.** The imine compound or the pharmaceutically acceptable salt thereof according to claim 13, wherein $R^{41}$ represents a $C_{1-10}$ alkyl group substituted with a $C_{3-10}$ cycloalkyl group(s) or a $C_{1-6}$ alkoxy group(s).

**16.** The imine compound or the pharmaceutically acceptable salt thereof according to claim 14 or 15, wherein $A^1$ is a 1,2-dihydropyridine ring.

**17.** The imine compound or the pharmaceutically acceptable salt thereof according to claim 16, wherein
$R^{51}$ represents a $C_{1-10}$ alkyl group that may be substituted with: a $C_{3-10}$ cycloalkyl group(s), or an aryl group, a thienyl group(s) or an aryloxy group(s), each of which may be substituted with a $C_{1-6}$ haloalkyl group(s) or a halogen atom(s); or
a group represented by Formula (II-1),
B represents a $C_{3-10}$ cycloalkyl group, an aryl group, or a heterocyclic group;
$R^{551}$ represents
a halogen atom;
a $C_{1-10}$ alkyl group
a $C_{1-6}$ haloalkyl group;
a $C_{1-10}$ alkoxy group;
a $C_{1-6}$ haloalkoxy group;
a $C_{3-10}$ cycloalkyl group;
an aryl group that may be substituted with 1 to 3 groups selected from the group consisting of a $C_{1-6}$ alkyl group and a halogen atom;
a heterocyclic group that may be substituted with a $C_{1-6}$ alkyl group(s);
an aryloxy group;
a morpholino group;
an arylamino group

a cyano group
a $C_{1-6}$ alkanoyl group;
a $C_{2-6}$ haloalkanoyl group; or
a $C_{1-6}$ alkylsulfonyl group,
$R^{561}$ represents
a hydrogen atom;
a halogen atom;
a $C_{1-10}$ alkyl group;
a $C_{1-6}$ haloalkyl group; or
a $C_{1-10}$ alkoxy group,
$R^{571}$ represents
a hydrogen atom;
a halogen atom;
a $C_{1-10}$ alkyl group; or
a $C_{1-10}$ alkoxy group.

**18.** The imine compound or the pharmaceutically acceptable salt thereof according to claim 17, wherein
$R^{51}$ represents a group represented by Formula (II-1),
B represents a phenyl group,
$R^{551}$ represents
a halogen atom;
a $C_{1-10}$ alkyl group
a $C_{1-6}$ haloalkyl group;
a $C_{1-6}$ alkoxy group;
a $C_{1-6}$ haloalkoxy group;
a $C_{3-10}$ cycloalkyl group;
an aryl group
an aryloxy group;
a morpholino group;
an arylamino group
a cyano group
a $C_{1-6}$ alkanoyl group;
a $C_{2-6}$ haloalkanoyl group; or
a $C_{1-6}$ alkylsulfonyl group,
$R^{561}$ represents
a hydrogen atom;
a halogen atom;
a $C_{1-6}$ haloalkyl group; or
a $C_{1-6}$ alkoxy group,
$R^{571}$ represents
a hydrogen atom;
a halogen atom;
$C_{1-10}$ alkyl group; or
a $C_{1-10}$ alkoxy group, and
m represents 1.

**19.** The imine compound or the pharmaceutically acceptable salt thereof according to claim 18, wherein $R^{41}$ is a $C_{1-10}$ alkyl group substituted with a $C_{3-10}$ cycloalkyl group(s).

**20.** The imine compound or the pharmaceutically acceptable salt thereof according to claim 19, wherein $R^{51}$ represents a phenyl group substituted with 1 to 3 groups selected from the group consisting of: a halogen atom, a $C_{1-10}$ alkyl group, a $C_{1-6}$ haloalkyl group, a $C_{1-10}$ alkoxy group, a cyano group, and a $C_{1-6}$ haloalkoxy group.

**21.** The imine compound or the pharmaceutically acceptable salt thereof according to any one of claims 13 to 20, wherein the double bond made of the carbon atom and the nitrogen atom contained in the group represented by >C=N-CO- in Formula (I-1) is in (Z) configuration.

**22.** A cannabinoid-receptor agonist comprising an imine compound represented by Formula (1-2)

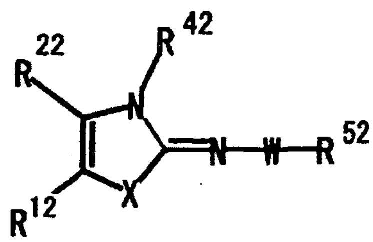

(I-2)

[where $R^{12}$ and $R^{22}$ each represent
a hydrogen atom;
a halogen atom;
a $C_{1-10}$ alkyl group;
a $C_{1-6}$ haloalkyl group;
a $C_{1-6}$ alkoxy group;
a carboxyl group;
a $C_{2-6}$ alkoxycarbonyl group;
a hydroxy-$C_{1-6}$ alkyl group;
an aryl group that may be substituted with 1 to 3 halogen atoms; or a group represented by Formula -CON($R^{61}$)$R^{71}$ (where $R^{61}$ and $R^{71}$ each represent a hydrogen atom or a $C_{1-6}$ alkyl group that may be substituted with a cyclic amino group(s), or $R^{61}$ and $R^{71}$, in combination with the adjacent nitrogen atom, form a cyclic amino group), or, $R^{11}$ and $R^{22}$, in combination with the adjacent carbon atom, form a benzene ring, a pyridine ring or a cyclohexenyl ring that may be substituted with a $C_{1-6}$ alkyl group(s) or a halogen atom(s);
$R^{42}$ represents
a $C_{1-10}$ alkyl group or $C_{2-6}$ alkenyl group that may be substituted with: a halogen atom(s), a cyano group(s), a carboxyl group (s), a $C_{2-6}$ alkoxycarbonyl group (s), a $C_{3-10}$ cycloalkyl group(s), an aryl group(s) that may be substituted with a $C_{1-6}$ haloalkyl group(s), a $C_{1-6}$ haloalkoxy group(s), a $C_{1-6}$ haloalkylthio group(s), a carboxyl group(s), a $C_{2-6}$ alkoxycarbonyl group(s) or a piperidinocarbamoyl group(s), an arylthio group(s), a $C_{1-6}$ alkoxy group(s), or a group(s) represented by Formula -CON($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group); or
a $C_{2-6}$ alkynyl group,
$R^{52}$ represents
a hydrogen atom;
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a halogen atom, a $C_{3-10}$ cycloalkyl group, a $C_{2-6}$ alkoxycarbonyl group, a $C_{1-6}$ alkoxy group that may be substituted with a $C_{1-6}$ alkoxy group(s) or an aryl group(s), a $C_{3-10}$ cycloalkoxy group that may be substituted with a $C_{1-6}$ alkyl group(s), an aryl group or aryloxy group that may be substituted with 1 to 5 groups selected from the group consisting of a $C_{1-6}$ alkyl group, a $C_{1-6}$ haloalkyl group, a $C_{1-6}$ alkoxy group, an aralkyloxy group, a nitro group and a halogen atom, a heterocyclic group, a phthalimide group, a $C_{1-6}$ alkanoyloxy group, an aralkyloxy group, a $C_{1-6}$ alkylthio group, an arylthio group, and a group represented by -N($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with a $C_{1-6}$ alkyl group(s), a $C_{1-6}$ alkoxy group(s), $C_{2-6}$ alkoxycarbonyl group(s), or a $C_{1-6}$ haloalkyl group(s) ;
an aralkyloxy group;
a group represented by Formula (II-2)

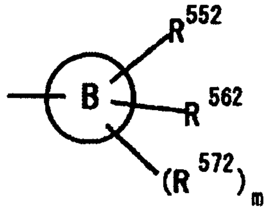

(II-2)

{where
B represents
a $C_{3-10}$ cycloalkyl group;
an aryl group;
a heterocyclic group;
a $C_{2-6}$ cyclic amino group;
a fluorenyl group;
a 2-oxopyrrolidinyl group
a group represented by Formula (III)

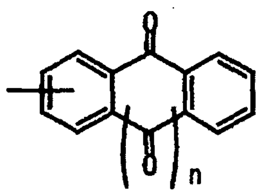

(III)

(where n represents 0 or 1); or
a group represented by Formula (IV-2)

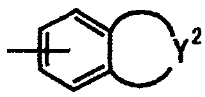

(IV-2)

(where $Y^2$ represents $-(CH_2)p-$, $-CO-CH_2-CH_2-$, $-O-CH_2-CH=CH-$, or $-O-(CH_2)q-O-$, in which p represents an integer of 2 to 4, and q represents an integer of 1 to 3),
$R^{552}$ represents
a hydrogen atom;
a halogen atom;
a $C_{1-10}$ alkyl group that may be substituted with an aryl group(s) that may be substituted with a halogen atom(s); with an aryloxy group(s); or with a group(s) represented by Formula $-N(R^{62})R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ alkylsulfonyl group

an arylthio group that may be substituted with a $C_{1-6}$ alkyl group(s) or a halogen atom(s);
an arylsulfonyl group that may be substituted with a $C_{1-6}$ alkyl group(s) or a halogen atom(s);
a $C_{1-6}$ haloalkoxy group;
a $C_{1-6}$ haloalkylthio group;
a $C_{3-10}$ cycloalkyl group;
a $C_{2-6}$ alkenyl group;
a $C_{1-6}$ alkoxy-$C_{1-6}$ alkoxy group;
an aryl group that may be substituted with 1 to 3 groups selected from the group consisting of : a $C_{1-6}$ alkyl group, a $C_{1-6}$ haloalkyl group, a halogen atom, a $C_{1-6}$ alkoxy group and a nitro group;
a heterocyclic group that may be substituted with a $C_{1-6}$ alkyl group(s) or a $C_{1-6}$ haloalkyl group(s);
an aryloxy group that may be substituted with a halogen atom(s);
a group represented by Formula -N($R^{63}$) $R^{73}$ (where $R^{63}$ and $R^{73}$ each represent a hydrogen atom, a $C_{1-6}$ alkyl group, a $C_{1-6}$ hydroxyalkyl group, a $C_{1-6}$ alkoxy-$C_{1-6}$alkyl group, an aryl group, a $C_{1-6}$ alkanoyl group, or $R^{63}$ and $R^{73}$, in combination with the adjacent nitrogen atom, form a cyclic amino group);
a hydroxyl group;
a cyano group;
a nitro group;
a carboxyl group;
a $C_{2-6}$ alkoxycarbonyl group;
a $C_{2-6}$ cyclic amino group that may be substituted with an aralkyl group(s) or an aryl group(s);
a group represented by Formula -CON($R^{64}$)$R^{74}$ (where $R^{64}$ and $R^{74}$ each represent a hydrogen atom, a $C_{1-6}$ alkyl group, a $C_{1-6}$ alkoxy-$C_{1-6}$ alkyl group, or a heterocyclic group that may be substituted with a $C_{1-6}$ alkyl group(s), or $R^{64}$ and $R^{74}$, in combination with the adjacent nitrogen atom, form a cyclic amino group);
a group represented by Formula -$SO_2$N($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ alkylsulfonyl group that may be substituted with a halogen atom(s);
an arylsulfonyl group that may be substituted with a halogen atom(s); or
a 2-oxa-3-oxobicyclo[2.2.1]heptyl group,
$R^{562}$ represents
a hydrogen atom;
a halogen atom;
a $C_{1-10}$ alkyl group;
a $C_{1-6}$ haloalkyl group; or
a $C_{1-6}$ alkoxy group,
$R^{572}$ represents
a hydrogen atom;
a $C_{1-10}$ alkyl group
a $C_{1-6}$ haloalkyl group;
a halogen atom; or
a $C_{1-6}$ alkoxy group;
m represents an integer of 1 to 3}
X represents an oxygen atom or a sulfur atom;
W represents CO or $SO_2$],
or a pharmaceutically acceptable salt thereof, as an active ingredient.

**23.** A cannabinoid-receptor agonist comprising the imine compound or the pharmaceutically acceptable salt thereof according to claim 22, as an active ingredient, wherein X is a sulfur atom.

**24.** A cannabinoid-receptor agonist comprising the imine compound or the pharmaceutically acceptable salt thereof according to claim 23, as an active ingredient, wherein
$R^{12}$ represents
a hydrogen atom, a halogen atom, a $C_{1-10}$ alkyl group, a carboxyl group, a $C_{2-6}$ alkoxycarbonyl group, a group represented by Formula -CON($R^{61}$)$R^{71}$ (where $R^{61}$ and $R^{71}$ each represent a hydrogen atom, or a $C_{1-6}$ alkyl group that may be substituted with a cyclic amino group(s), or $R^{61}$ and $R^{71}$, in combination with the adjacent nitrogen atom, form a cyclic amino group), or an aryl group,
$R^{22}$ represents
a hydrogen atom, a $C_{1-10}$ alkyl group, a $C_{1-6}$ haloalkyl group, or an aryl group, or
$R^{11}$ and $R^{22}$, in combination with the adjacent carbon atom, form a benzene ring, a pyridine ring or a cyclohexenyl

ring that may be substituted with a $C_{1-6}$ alkyl group (s) or a halogen atom(s).

**25.** A cannabinoid-receptor agonist comprising the imine compound or the pharmaceutically acceptable salt thereof according to claim 24, as an active ingredient, wherein
$R^{42}$ represents
a $C_{1-10}$ alkyl group or $C_{2-6}$ alkenyl group that may be substituted with: a halogen atom(s), a cyano group(s), a carboxyl group(s), a $C_{2-6}$ alkoxycarbonyl group(s), a $C_{3-10}$ cycloalkyl group(s), an aryl group(s) that may be substituted with a $C_{1-6}$ haloalkylthio group(s), a carboxyl group(s), or a $C_{2-6}$ alkoxycarbonyl group(s), an arylthio group(s), a $C_{1-6}$ alkoxy group(s) or a group represented by Formula -CON($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group); or
a $C_{2-6}$ alkynyl group,
$R^{52}$ represents
a $C_{1-6}$ alkoxy group;
a $C_{1-6}$ haloalkyl group;
a $C_{1-10}$ alkyl group or $C_{2-6}$ alkenyl group that may be substituted with 1 to 3 groups selected from the group consisting of: a $C_{3-10}$ cycloalkyl group, a $C_{2-6}$ alkoxycarbonyl group, a $C_{1-6}$ alkoxy group, an aryl group or aryloxy group that may be substituted with 1 to 5 groups selected from the group consisting of a $C_{1-6}$ alkyl group, a $C_{1-6}$ haloalkyl group, a $C_{1-6}$ alkoxy group, an aralkyloxy group, a nitro group and a halogen atom, a heterocyclic group, a $C_{1-6}$ alkylthio group, an arylthio group, and a group represented by Formula -N($R^{62}$)$R^{72}$ (where $R^{62}$ and $R^{12}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with a $C_{1-6}$ alkoxy group(s), $C_{2-6}$ alkoxycarbonyl group(s), or a $C_{1-6}$ haloalkyl group(s); or
a group represented by Formula (II), where
B represents
a $C_{3-10}$ cycloalkyl group;
an aryl group; or
a heterocyclic group,
$R^{552}$ represents
a hydrogen atom;
a halogen atom;
a $C_{1-10}$ alkyl group that may be substituted with an aryl group (s) , or a group(s) represented by Formula -N($R^{62}$) $R^{72}$ ($R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ alkylsulfonyl group;
an arylthio group;
an arylsulfonyl group that may be substituted with a halogen atom(s);
a $C_{1-6}$ haloalkoxy group;
a $C_{1-6}$ haloalkylthio group;
a $C_{1-6}$ alkoxy-$C_{1-6}$alkoxy group;
an aryl group that may be substituted with 1 to 3 groups selected from the group consisting of: a $C_{1-6}$ haloalkyl group, a halogen atom, and a nitro group;
a heterocyclic group that may be substituted with a $C_{1-6}$ haloalkyl group(s);
an aryloxy group that may be substituted with a halogen atom(s);
a group represented by Formula -N($R^{63}$)$R^{73}$ (where $R^{63}$ and $R^{73}$ each represent a hydrogen atom, a $C_{1-6}$ alkyl group, a $C_{1-6}$ hydroxyalkyl group, a $C_{1-6}$ alkoxy-$C_{1-6}$ alkyl group, or a benzoyl group, or $R^{63}$ and $R^{73}$, in combination with the adjacent nitrogen atom, form a cyclic amino group);
a hydroxyl group;
a cyano group;
a nitro group;
a carboxyl group;
a $C_{2-6}$ alkoxycarbonyl group; or a group represented by Formula -CON($R^{64}$)$R^{74}$(where $R^{64}$ and $R^{74}$ each represent a hydrogen atom, a $C_{1-6}$ alkyl group, a $C_{1-6}$ alkoxy-$C_{1-6}$ alkyl group or a heterocyclic group that may be substituted with a $C_{1-6}$ alkyl group(s), or $R^{64}$ and $R^{74}$, in combination with the adjacent nitrogen atom, form a cyclic amino group),

$R^{562}$ represents
a hydrogen atom;
a halogen atom;
a $C_{1-10}$ alkyl group;
a $C_{1-6}$ haloalkyl group; or
a $C_{1-10}$ alkoxy group,
$R^{572}$ represents
a hydrogen atom;
a halogen atom;
$C_{1-10}$ alkyl group; or
a $C_{1-6}$ alkoxy group.

**26.** A cannabinoid-receptor agonist comprising the imine compound or the pharmaceutically acceptable salt thereof according to claim 25, as an active ingredient, wherein
$R^{52}$ represents a group represented by Formula (II-2) where
B represents a phenyl group or a pyridyl group;
$R^{552}$ represents
a hydrogen atom;
a halogen atom;
a $C_{1-10}$ alkyl group ;
a $C_{1-6}$ haloalkyl group; or
a $C_{1-10}$ alkoxy group,
$R^{562}$ represents
a hydrogen atom;
a halogen atom;
a $C_{1-10}$ alkyl group; or
a $C_{1-6}$ alkoxy group,
$R^{572}$ represents
a hydrogen atom;
a halogen atom; or
a $C_{1-6}$ alkoxy group.

**27.** A cannabinoid-receptor agonist comprising the imine compound or the pharmaceutically acceptable salt thereof according to claim 26, as an active ingredient, wherein
$R^{42}$ represents
a $C_{1-10}$ alkyl group that may be substituted with: a halogen atom(s), a cyano group(s), a carboxyl group(s), a $C_{2-6}$ alkoxycarbonyl group(s), a $C_{3-10}$ cycloalkyl group(s), an aryl group(s) that may be substituted with a $C_{1-6}$ haloalkylthio group(s), a carboxyl group(s) or a $C_{2-6}$ alkoxycarbonyl group(s), an arylthio group(s), a $C_{1-6}$ alkoxy group(s), or a group(s) represented by Formula -CON($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group).

**28.** The cannabinoid-receptor agonist according to any one of claims 22 to 27, being a cannabinoid type-1 receptor agonist or a cannabinoid type-2 receptor agonist.

**29.** The cannabinoid-receptor agonist according to any one of claims 22 to 27, being a therapeutic drug or prophylactic drug for pain.

**30.** The cannabinoid-receptor agonist according to any one of claims 22 to 27, being a therapeutic drug or prophylactic drug for autoimmune disease.

**31.** An imine compound represented by Formula (I-2)

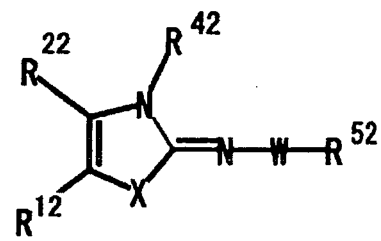

(I-2)

where
W is CO,
$R^{12}$ represents
a halogen atom;
a $C_{1-6}$ alkyl group;
a $C_{1-6}$ haloalkyl group;
a $C_{1-6}$ alkoxy group;
a carboxyl group;
a $C_{2-6}$ alkoxycarbonyl group;
a hydroxy-$C_{1-6}$ alkyl group; or an aryl group that may be substituted with 1 to 3 halogen atoms; or a group represented by Formula -CON($R^{61}$)$R^{71}$ (where $R^{61}$ and $R^{71}$ each represent a hydrogen atom or a $C_{1-6}$ alkyl group that may be substituted with a cyclic amino group(s), or $R^{61}$ and $R^{71}$, in combination with the adjacent nitrogen atom, form a cyclic amino group),
$R^{22}$ represents
a hydrogen atom:
a halogen atom;
a $C_{1-10}$ alkyl group;
a $C_{1-6}$ haloalkyl group; or
an aryl group, or
$R^{12}$ and $R^{22}$, in combination with the adjacent carbon atom, form a benzene ring, a pyridine ring or a cyclohexenyl ring that may be substituted with a $C_{1-6}$ alkyl group(s) or a halogen atom(s),
$R^{42}$ represents
a $C_{1-10}$ alkyl group or $C_{2-6}$ alkenyl group substituted with a $C_{3-10}$ cycloalkyl group(s) or a $C_{1-6}$ alkoxy group(s), and
X is the same as those as defined regarding $R^{52}$, or a pharmaceutically acceptable salt thereof.

**32.** The imine compound or the pharmaceutically acceptable salt thereof according to claim 31, wherein X is a sulfur atom.

**33.** The imine compound or the pharmaceutically acceptable salt thereof according to claim 32, wherein $R^{12}$ represents a halogen atom or a $C_{1-10}$ alkyl group; and $R^{22}$ represents a $C_{1-10}$ alkyl group or a $C_{1-6}$ haloalkyl group.

**34.** The imine compound or the pharmaceutically acceptable
salt thereof according to claim 33, wherein
$R^{52}$ represents
a $C_{1-6}$ alkoxy group;
a $C_{1-6}$ haloalkyl group;
a $C_{1-10}$ alkyl group or $C_{2-6}$ alkenyl group that may be
substituted with 1 to 3 groups selected from the group consisting of: a $C_{3-10}$ cycloalkyl group, a $C_{2-6}$ alkoxycarbonyl group, a $C_{1-6}$ alkoxy group, an aryl group or aryloxy group that may be substituted with 1 to 5 groups selected from the group consisting of a $C_{1-6}$ alkyl group, a $C_{1-6}$ haloalkyl group, a $C_{1-6}$ alkoxy group, an aralkyloxy group, a nitro group and a halogen atom, a heterocyclic group, a $C_{1-6}$ alkylthio group, an arylthio group, and a group represented by Formula -N($R^{62}$)$R^{72}$ (where $R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
an aryloxy group that may be substituted with a $C_{1-6}$ alkoxy group(s), $C_{2-6}$ alkoxycarbonyl group(s), or a $C_{1-6}$ haloalkyl group(s); or
a group represented by Formula (II-2) where

B represents
a $C_{3-10}$ cycloalkyl group;
an aryl group; or
a heterocyclic group,
$R^{552}$ represents
a hydrogen atom;
a halogen atom;
a $C_{1-10}$ alkyl group that may be substituted with an aryl group(s), or a group(s) represented by Formula $-N(R^{62})R^{72}$ ($R^{62}$ and $R^{72}$ each represent a hydrogen atom or a $C_{1-6}$ alkyl group, or $R^{62}$ and $R^{72}$, in combination with the adjacent nitrogen atom, form a cyclic amino group);
a $C_{1-6}$ haloalkyl group;
a $C_{1-10}$ alkoxy group;
a $C_{1-6}$ alkylthio group;
a $C_{1-6}$ alkylsulfonyl group;
an arylthio group
an arylsulfonyl group that may be substituted with a halogen atom(s);
a $C_{1-6}$ haloalkoxy group
a $C_{1-6}$ haloalkylthio group;
a $C_{1-6}$ alkoxy-$C_{1-6}$alkoxy group;
an aryl group that may be substituted with 1 to 3 groups selected from the group consisting of: a $C_{1-6}$ haloalkyl group, a halogen atom, and a nitro group;
a heterocyclic group that may be substituted with a $C_{1-6}$ haloalkyl group(s);
an aryloxy group that may be substituted with a halogen atom(s);
a hydroxyl group;
a cyano group;
a nitro group;
a carboxyl group; or
a $C_{2-6}$ alkoxycarbonyl group,
$R^{562}$ represents
a hydrogen atom;
a halogen atom;
a $C_{1-10}$ alkyl group; or
a $C_{1-10}$ alkoxy group,
$R^{572}$ represents
a hydrogen atom;
a halogen atom;
a $C_{1-10}$ alkyl group; or a $C_{1-10}$ alkoxy group.

**35.** The imine compound or the pharmaceutically acceptable salt thereof according to claim 34, wherein
$R^{52}$ represents a group represented by Formula (II-2) where
B represents a phenyl group or a pyridyl group
$R^{552}$ represents
a hydrogen atom;
a halogen atom;
a $C_{1-10}$ alkyl group;
a $C_{1-6}$ haloalkyl group;
a $C_{1-10}$ alkoxy group; or
a $C_{1-6}$ haloalkoxy group,
$R^{562}$ represents
a hydrogen atom;
a halogen atom;
a $C_{1-10}$ alkyl group; or
a $C_{1-10}$ alkoxy group,
$R^{572}$ represents
a hydrogen atom;
a halogen atom;
a $C_{1-10}$ alkyl group; or a $C_{1-6}$ alkoxy group.

**36.** The imine compound or the pharmaceutically acceptable salt thereof according to any one of claims 31 to 35, wherein the double bond represented by >C=N-CO- in Formula (I-2) is in (Z) configuration.

**37.** A cannabinoid-receptor agonist comprising an imine compound represented by Formula (I-3)

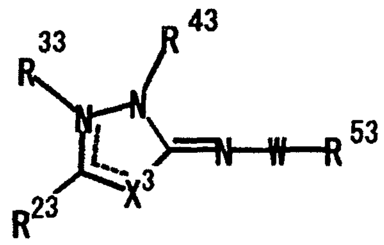

(I-3)

[where the broken line indicates that one of the bonds is a double bond,
$X^3$ represents $C(R^{13})$, S or O,
$R^{13}$, $R^{23}$ and $R^{33}$ each represent
a hydrogen atom;
a $C_{1-10}$ alkyl group that may be substituted with aryl group(s) substituted with a halogen atom(s);
a $C_{1-6}$ haloalkyl group;
a $C_{3-10}$ cycloalkyl group; or an aryl group or aralkyl group that may be substituted with 1 to 3 halogen atoms, or in the case where $X^3$ is $C(R^{13})$, $R^{13}$ and $R^{23}$ together represent a group represented by $-CH_2-S-CH_2-$ (with the proviso that, $R^{33}$ is not substituted in the case where $X^3$ is S or O),
$R^{43}$ represents
a 1,1-dioxothiolanyl group; or
a $C_{1-10}$ alkyl group or $C_{2-6}$ alkenyl group that may be substituted with a group(s) selected from the group consisting of: a $C_{3-10}$ cycloalkyl group, a $C_{1-6}$ haloalkyl group and a $C_{1-6}$ alkoxy group; or
an aryl group,
$R^{53}$ represents
a hydrogen atom;
a $C_{1-10}$ alkoxy group;
a $C_{1-6}$ alkoxy-$C_{1-6}$alkoxy group;
a $C_{1-6}$ haloalkyl group;
a $C_{1-10}$ alkyl group or $C_{2-6}$ alkenyl group that may be substituted with 1 to 3 groups selected from the group consisting of: a $C_{1-6}$ alkoxy group, a $C_{3-10}$ cycloalkyl group, a $C_{2-6}$ alkoxycarbonyl group, an aryl group or aryloxy group that may be substituted with 1 to 5 groups selected from the group consisting of a $C_{1-6}$ alkoxy group and a halogen atom, a heterocyclic group, a $C_{1-6}$ alkanoyloxy group, an aralkyloxy group, and a $C_{1-6}$ alkylthio group;
a group represented by Formula

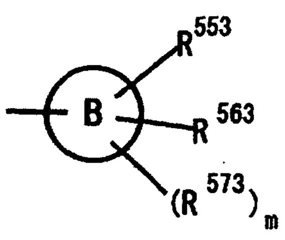

(II-3)

{where B represents
a $C_{3-10}$ cycloalkyl group;
an aryl group;
a heterocyclic group;

a $C_{2-6}$ cyclic amino group;
a group represented by Formula (III)

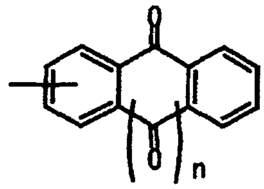

(III)

(where n represents 0 or 1); or
a group represented by Formula (IV-3)

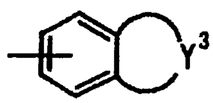

(IV-3)

(where $Y^3$ represents $-O-CH_2-CH=CH-$ or $-O-(CH_2)q-O-$, in which q represents an integer of 1 to 3),
$R^{553}$ represents
a hydrogen atom;
a halogen atom;
an aryl group;
a $C_{1-10}$ alkyl group;
a $C_{1-6}$ alkanoyloxy-$C_{1-6}$ alkyl group;
a $C_{1-6}$ haloalkyl group;
a $C_{1-10}$ alkoxy group;
a $C_{1-6}$ alkylthio group;
a $C_{2-6}$ alkenyloxy group;
a $C_{2-6}$ alkenylthio group;
a $C_{1-6}$ haloalkoxy group;
a $C_{1-6}$ haloalkylthio group;
an aryl group that may be substituted with 1 to 3 halogen atoms or cyano groups;
a heterocyclic group;
an aryloxy group or arylthio group that may be substituted with a halogen atom(s) or a $C_{1-6}$ alkyl group(s);
a group represented by Formula $-N(R^{633})R^{733}$ (where $R^{633}$ and $R^{733}$ each represent a hydrogen atom, a $C_{1-6}$ alkyl group, a $C_{1-6}$ alkanoyl group, a di-$C_{1-6}$ alkylamino-$C_{2-6}$ alkanoyl group, or a heterocyclic group that may be substituted with a $C_{1-6}$ alkyl group (s), or $R^{633}$ and $R^{733}$, in combination with the adjacent nitrogen atom, form a cyclic amino group);
a cyano group;
a nitro group; or
a $C_{2-6}$ alkoxycarbonyl group,
$R^{563}$ represents
a hydrogen atom;
a halogen atom;
a $C_{1-10}$ alkyl group; or
a $C_{1-6}$ haloalkyl group,
$R^{573}$ represents
a hydrogen atom;
a $C_{1-10}$ alkyl group;

a halogen atom; or
a $C_{1-10}$ alkoxy group,
m represents an integer of 1 to 3},
and
W represents -CO-, -CO-CO-, -CO-NH-, -CS-NH- or -$SO_2$-] or a pharmaceutically acceptable salt thereof, as an active ingredient.

**38.** A cannabinoid-receptor agonist comprising the imine compound or the pharmaceutically acceptable salt thereof according to claim 37, as an active ingredient, wherein
$R^{13}$ represents a hydrogen atom or a phenyl group; and $R^{23}$ and $R^{33}$ each represent a $C_{1-6}$ alkyl group, a $C_{1-6}$ haloalkyl group, or a $C_{3-10}$ cycloalkyl group,
$R^{43}$ represents
a $C_{1-10}$ alkyl group that may be substituted with a group(s) selected from the group consisting of: a $C_{3-10}$ cycloalkyl group, a $C_{1-6}$ haloalkyl group and a $C_{1-6}$ alkoxy group,
$R^{53}$ represents
a $C_{1-10}$ alkoxy group;
a $C_{1-6}$ alkoxy-$C_{1-6}$alkoxy group;
a $C_{1-6}$ haloalkyl group;
a $C_{1-10}$ alkyl group or $C_{2-6}$ alkenyl group that may be
substituted with 1 to 3 groups selected from the group consisting of: a $C_{1-6}$ alkoxy group, a $C_{3-10}$ cycloalkyl group, a $C_{2-6}$ alkoxycarbonyl group, an aryl group or aryloxy group that may be substituted with 1 to 5 groups selected from the group consisting of a $C_{1-6}$ alkoxy group and a halogen atom, a heterocyclic group, a $C_{1-6}$ alkanoyloxy group, an aralkyloxy group, and a $C_{1-6}$ alkylthio group;
a group represented by Formula (II-3);
where B represents
an aryl group, a furyl group, a thienyl group, pyrazolyl group, isoxazolyl group, pyridyl group, a group represented by Formula (III), or a group represented by Formula (IV-3).

**39.** A cannabinoid-receptor agonist comprising the imine compound or the pharmaceutically acceptable salt thereof according to claim 38, as an active ingredient, wherein W is -CO-.

**40.** A cannabinoid-receptor agonist comprising the imine compound or the pharmaceutically acceptable salt thereof according to claim 39, as an active ingredient, wherein
$X^3$ represents $C(R^{13})$,
$R^{53}$ represents
a $C_{1-10}$ alkoxy group;
a $C_{1-6}$ haloalkyl group;
a $C_{1-10}$ alkyl group that may be substituted with 1 to 3 groups selected from the group consisting of: a $C_{1-6}$ alkoxy group, a $C_{3-10}$ cycloalkyl group, an aryl group that may be substituted with 1 to 5 groups selected from the group consisting of a $C_{1-6}$ alkoxy group and a halogen atom; or a group represented by Formula (II-3) where B represents a phenyl group.

**41.** A cannabinoid-receptor agonist comprising the imine compound or the pharmaceutically acceptable salt thereof according to claim 40, as an active ingredient, wherein
$R^{553}$ represents
a halogen atom;
a $C_{1-10}$ alkyl group;
a $C_{1-6}$ haloalkyl group;
a $C_{1-10}$ alkoxy group;
a $C_{1-6}$ haloalkoxy group;
an aryl group that may be substituted with 1 to 3 halogen atoms;
an aryloxy group that may be substituted with a halogen atom(s);
a group represented by Formula -$N(R^{633})R^{733}$(where $R^{633}$ and $R^{733}$ each represent a hydrogen atom or a $C_{1-6}$ alkyl group);
a cyano group;
a nitro group; or
a $C_{2-6}$ alkoxycarbonyl group,

$R^{563}$ represents
a hydrogen atom;
a halogen atom;
a $C_{1-10}$ alkyl group;
a $C_{1-6}$ alkoxy group; or
a $C_{1-6}$ haloalkyl group,
$R^{573}$ represents
a hydrogen atom;
a $C_{1-10}$ alkyl group;
a halogen atom; or a $C_{1-10}$ alkoxy group.

**42.** A cannabinoid-receptor agonist comprising the imine compound or the pharmaceutically acceptable salt thereof according to claim 39, as an active ingredient, wherein $X^3$ is a sulfur atom; and $R^{53}$ is a group represented by Formula (II-3)
where B is a phenyl group.

**43.** A cannabinoid-receptor agonist comprising the imine compound or the pharmaceutically acceptable salt thereof according to claim 42, as an active ingredient, wherein
$R^{553}$ represents
a halogen atom;
a $C_{1-10}$ alkyl group;
a $C_{1-6}$ haloalkyl group; or
a $C_{1-10}$ alkoxy group;
$R^{563}$ represents
a hydrogen atom;
a halogen atom;
a $C_{1-10}$ alkyl group;
a $C_{1-6}$ alkoxy group; or
a $C_{1-6}$ haloalkyl group,
$R^{573}$ represents a hydrogen atom.

**44.** A cannabinoid-receptor agonist comprising the imine compound or the pharmaceutically acceptable salt thereof according to claim 41 or 43, as an active ingredient, where $R^{43}$ is a $C_{1-10}$ alkyl group substituted with a $C_{3-10}$ cycloalkyl group(s).

**45.** The cannabinoid-receptor agonist according to any one of claims 37 to 44, being a cannabinoid type-1 receptor agonist or a cannabinoid type-2 receptor agonist.

**46.** The cannabinoid-receptor agonist according to any one of claims 37 to 44, being a therapeutic drug or prophylactic drug for pain.

**47.** The cannabinoid-receptor agonist according to any one of claims 37 to 44, being a therapeutic drug or prophylactic drug for autoimmune disease.

**48.** An imine compound represented by Formula (1-3)

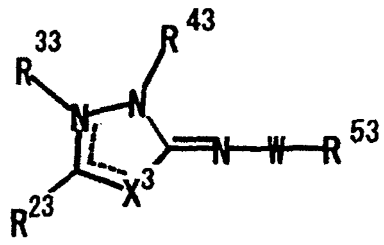

(I-3)

[where a broken line indicates that one of the bonds is a double bond,
$X^3$ represents $C(R^{13})$, S or O,
$R^{13}$, $R^{23}$ and $R^{33}$ each represent
a hydrogen atom;
a $C_{1-10}$ alkyl group that may be substituted with aryl group(s) substituted with a halogen atom(s);
a $C_{1-6}$ haloalkyl group;
a $C_{3-10}$ cycloalkyl group; or
an aryl group or aralkyl group that may be substituted with 1 to 3 halogen atoms, or
in the case where $X^3$ is $C(R^{13})$, $R^{13}$ and $R^{23}$ together represent a group represented by $-CH_2-S-CH_2-$ (with the proviso that, $R^{33}$ is not substituted in the case where $X^3$ is S or O),
$R^{43}$ represents
a 1,1-dioxothiolanyl group;
a $C_{1-10}$ alkyl group or $C_{2-6}$ alkenyl group that may be substituted with a group(s) selected from the group consisting of: a $C_{3-10}$ cycloalkyl group, a $C_{1-6}$ haloalkyl group and a $C_{1-6}$ alkoxy group; or
an aryl group,
$R^{53}$ represents
a hydrogen atom;
a $C_{1-10}$ alkoxy group;
a $C_{1-6}$ alkoxy-$C_{1-6}$alkoxy group;
a $C_{1-6}$ haloalkyl group;
a $C_{1-10}$ alkyl group or $C_{2-6}$ alkenyl group that may be substituted with 1 to 3 groups selected from the group consisting of: a $C_{1-6}$ alkoxy group, a $C_{3-10}$ cycloalkyl group, a $C_{2-6}$ alkoxycarbonyl group, an aryl group or aryloxy group that may be substituted with 1 to 5 groups selected from the group consisting of a $C_{1-6}$ alkoxy group and a halogen atom, a heterocyclic group, a $C_{1-6}$ alkanoyloxy group, an aralkyloxy group, and a $C_{1-6}$ alkylthio group;
a group represented by Formula (II-3)

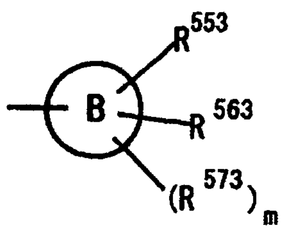

(II-3)

{where B represents
a $C_{3-10}$ cycloalkyl group;
an aryl group;
a heterocyclic group;
a $C_{2-6}$ cyclic amino group;
a group represented by Formula (III)

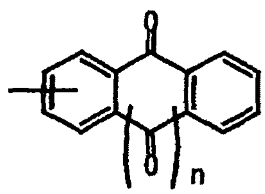

(III)

(where n represents 0 or 1); or
a group represented by Formula (IV-3)

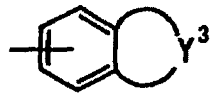

(IV-3)

(where $Y^3$ represents $-O-CH_2-CH=CH-$ or $-O-(CH_2)q-O-$, where q represents an integer of 1 to 3),
$R^{553}$ represents
a hydrogen atom;
a halogen atom;
an aryl group;
a $C_{1-10}$ alkyl group;
a $C_{1-6}$ alkanoyloxy-$C_{1-6}$alkyl group;
a $C_{1-6}$ haloalkyl group;
a $C_{1-10}$ alkoxy group;
a $C_{1-6}$ alkylthio group;
a $C_{2-6}$ alkenyloxy group;
a $C_{2-6}$ alkenylthio group;
a $C_{1-6}$ haloalkoxy group;
a $C_{1-6}$ haloalkylthio group, an aryl group that may be substituted with 1 to 3 halogen atoms or cyano groups;
a heterocyclic group;
an aryloxy group or arylthio group that may be substituted with a halogen atom(s) or a $C_{1-6}$ alkyl group(s);
a group represented by Formula $-N(R^{633})R^{733}$(where $R^{633}$ and $R^{733}$ each represent a hydrogen atom, a $C_{1-6}$ alkyl group, a $C_{1-6}$ alkanoyl group, a di-$C_{1-6}$ alkylamino-$C_{2-6}$ alkanoyl group, or a heterocyclic group that may be substituted with a $C_{1-6}$ alkyl group(s), or $R^{633}$ and $R^{733}$, in combination with the adjacent nitrogen atom, form a cyclic amino group);
a cyano group;
a nitro group; or
a $C_{2-6}$ alkoxycarbonyl group,
$R^{563}$ represents
a hydrogen atom;
a halogen atom;
a $C_{1-10}$ alkyl group; or
a $C_{1-6}$ haloalkyl group,
$R^{573}$ represents
a hydrogen atom;
a $C_{1-10}$ alkyl group;
a halogen atom; or
a $C_{1-10}$ alkoxy group, and
m represents an integer of 1 to 3},
and
W represents -CO-, -CO-CO-, -CO-NH-, -CS-NH- or $-SO_2-$]
or a pharmaceutically acceptable salt thereof.

**49.** The imine compound or the pharmaceutically acceptable salt thereof according to claim 48, wherein $R^{13}$ represents a hydrogen atom or a phenyl group; and $R^{23}$ and $R^{33}$ each represent a $C_{1-6}$ alkyl group, a $C_{1-6}$ haloalkyl group or a $C_{3-10}$ cycloalkyl group,
$R^{43}$ represents
a $C_{1-10}$ alkyl group that may be substituted with a group(s) selected from the group consisting of: a $C_{3-10}$ cycloalkyl group and a $C_{1-6}$ alkoxy group; or
a $C_{1-6}$ haloalkyl group,
$R^{53}$ represents
a $C_{1-6}$ alkoxy group;
a $C_{1-6}$ alkoxy-$C_{1-6}$alkoxy group;
a $C_{1-6}$ haloalkyl group;
a $C_{1-10}$ alkyl group or $C_{2-6}$ alkenyl group that may be

substituted with 1 to 3 groups selected from the group consisting of: a $C_{1-6}$ alkoxy group, a $C_{3-10}$ cycloalkyl group, a $C_{2-6}$ alkoxycarbonyl group, an aryl group or aryloxy group that may be substituted with 1 to 5 groups selected from the group consisting of a $C_{1-6}$ alkoxy group and a halogen atom, a heterocyclic group, a $C_{1-6}$ alkanoyloxy group, an aralkyloxy group, and a $C_{1-6}$ alkylthio group; or
a group represented by Formula (II-3), where
B represents
an aryl group, a furyl group, a thienyl group, pyrazolyl group, isoxazolyl group, pyridyl group, a group represented by Formula (III), or a group represented by Formula (IV-3).

**50.** The imine compound or the pharmaceutically acceptable salt thereof according to claim 49, wherein W is -CO-.

**51.** The imine compound or the pharmaceutically acceptable salt thereof according to claim 50, wherein
$X^3$ represents $C(R^{13})$ ;
$R^{53}$ represents
a $C_{1-10}$ alkoxy group;
a $C_{1-6}$ haloalkyl group;
a $C_{1-10}$ alkyl group that may be substituted with 1 to 3 groups selected from the group consisting of: a $C_{1-6}$ alkoxy group, a $C_{3-10}$ cycloalkyl group, and an aryl group that may be substituted with 1 to 5 groups selected from the group consisting of a $C_{1-6}$ alkoxy group and a halogen atom; or a group represented by Formula (II-3), where B represents a phenyl group.

**52.** The imine compound or the pharmaceutically acceptable salt thereof according to claim 51, wherein
$R^{553}$ represents
a halogen atom;
a $C_{1-10}$ alkyl group;
a $C_{1-6}$ haloalkyl group;
a $C_{1-10}$ alkoxy group;
a $C_{1-6}$ haloalkoxy group;
an aryl group that may be substituted with 1 to 3 halogen atoms;
an aryloxy group that may be substituted with a halogen atom(s);
a group represented by Formula -N $(R^{633})$ $R^{733}$ (where $R^{633}$ and $R^{733}$ each represent a hydrogen atom or a $C_{1-6}$ alkyl group;
a cyano group;
a nitro group; or
a $C_{2-6}$ alkoxycarbonyl group,
$R^{563}$ represents
a hydrogen atom;
a halogen atom;
a $C_{1-10}$ alkyl group; or
a $C_{1-6}$ haloalkyl group,
$R^{573}$ represents
a hydrogen atom;
a $C_{1-10}$ alkyl group;
a halogen atom; or
a $C_{1-10}$ alkoxy group.

**53.** The imine compound or the pharmaceutically acceptable salt thereof according to claim 52, wherein $R^{43}$ represents a $C_{1-10}$ alkyl group substituted with a $C_{3-10}$ cycloalkyl group(s).

**54.** The imine compound or the pharmaceutically acceptable salt thereof according to claim 49, wherein $X^3$ is a sulfur atom and W is -CO- or $-SO_2-$.

**55.** The imine compound or the pharmaceutically acceptable salt thereof according to claim 54, wherein
$R^{23}$ represents a $C_{1-6}$ alkyl group; $R^{43}$ represents a $C_{1-10}$ alkyl group substituted with a $C_{3-10}$ cycloalkyl group;
$R^{53}$ represents a group represented by Formula (II-3), where
B represents an aryl group, $R^{553}$ represents a $C_{1-6}$ haloalkyl group; and $R^{563}$ represents a hydrogen atom, a halogen atom, a $C_{1-10}$ alkyl group or a $C_{1-10}$ alkoxy group; $R^{573}$ represents a hydrogen atom.

**56.** The imine compound or the pharmaceutically acceptable salt thereof according to any one of claims 50 to 55, wherein the double bond represented by >C=N-CO- in Formula (I-3) is in (Z) configuration.

[Figure 1]

Figure 1 Test for neurogenic pain in rat

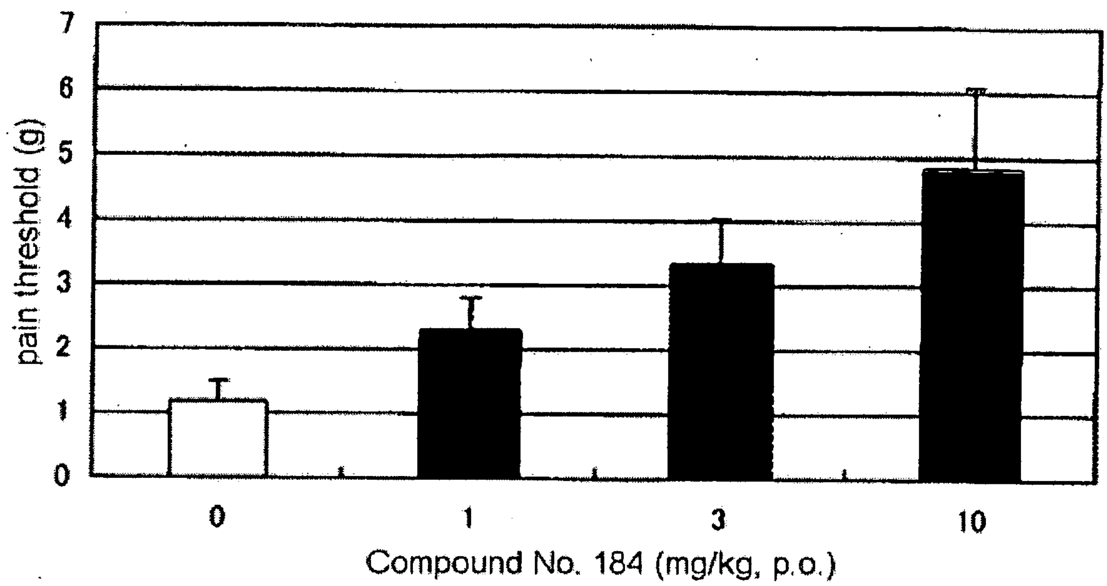

**INTERNATIONAL SEARCH REPORT**

International application No.
PCT/JP2005/019977

A. CLASSIFICATION OF SUBJECT MATTER
***A61K31/426***(2006.01), ***A61K31/415***(2006.01), ***A61K31/4155***(2006.01), ***A61K31/42***(2006.01), ***A61K31/421***(2006.01), ***A61K31/422***(2006.01), ***A61K31/4245***(2006.01), ***A61K31/425***(2006.01), ***A61K31/427***(2006.01), ***A61K31/428***(2006.01),
According to International Patent Classification (IPC) or to both national classification and IPC

B. FIELDS SEARCHED

Minimum documentation searched (classification system followed by classification symbols)
A61K31/426, A61K31/415, A61K31/4155, A61K31/42, A61K31/421, A61K31/422, A61K31/4245, A61K31/425, A61K31/427, A61K31/428, A61K31/429, A61K31/433, A61K31/4365, A61K31/437, A61K31/4375, A61K31/44, A61K31/4409, A61K31/4418,

Documentation searched other than minimum documentation to the extent that such documents are included in the fields searched

Electronic data base consulted during the international search (name of data base and, where practicable, search terms used)
BIOSIS(STN), CAOLD(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2003/070277 A1 (Shionogi & Co., Ltd.), 28 August, 2003 (28.08.03), & EP 1477186 A1 & US 2005/101590 A1 | 1-53,56 |
| A | WO 2004/029204 A2 (MERCK & Co., Inc.), 08 April, 2004 (08.04.04), Example 34 & WO 2004/029204 A3 & CA 2499497 A1 & EP 1546115 A2 & US 2005/245554 A1 | 1-53,56 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 January, 2006 (24.01.06) | 07 February, 2006 (07.02.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-515470 A (Bayer AG.),<br>18 September, 2001 (18.09.01),<br>Example 112<br>& DE 19740785 A1 & CA 2281929 A1<br>& CA 2470183 A1 & WO 98/37061 A1<br>& AU 9863965 A1 & AU 735137 B2<br>& EP 966436 A1 & EP 966436 B1<br>& TR 9902012 T2 & BR 9807848 A<br>& AT 229502 E & PT 966436 T<br>& RU 2203272 C2 & ES 2189142 T3<br>& IL 131010 A1 & TW 527343 B<br>& ZA 9801419 A & BG 63915 B1<br>& NO 9904014 A & NO 314141 B1<br>& MX 9907687 A & US 6262112 B1<br>& US 2002/072529 A1 & US 6573278 B2 | 1-53,56 |
| A | JP 2001-026541 A (SANOFI-SANTE LABO.),<br>30 January, 2001 (30.01.01),<br>Full text<br>& US 5624941 A & FR 2692575 A1<br>& FR 2692575 B1 & FR 2713224 A1<br>& FR 2713224 B1 & FR 2713225 A1<br>& FR 2713225 B1 & ZA 9409342 A | 1-53,56 |
| X<br>A | JP 2003-192591 A (Sumitomo Pharmaceuticals Co., Ltd.),<br>09 July, 2003 (09.07.03),<br>Full text; particularly, CAS RN: 389144-81-4, 389147-66-4,553684-05-2, compounds<br>(Family: none) | 1,2,8,10<br>3-7,9,11-53, 56 |
| X<br>A | JP 01-149781 A (Pfizer Inc.),<br>12 June, 1989 (12.06.89),<br>Full text; particularly, CAS RN: 124812-43-7, compound<br>& EP 314329 A1 & EP 314329 B1<br>& WO 89/03682 A1 & HU 50816 A2<br>& HU 203094 B & AT 71376 E<br>& ES 2038767 T3 & IL 88114 A1<br>& ZA 8807928 A & CA 1294276 A1<br>& AU 8824326 A1 & AU 590485 B2<br>& DK 8805916 A & JP 06-041465 B2<br>& FI 8903111 A & NO 8902638 A<br>& US 5081118 A | 1,2,8,9<br>3-7,10-53,56 |
| X<br>A | JP 57-171986 A (Taiho Pharmaceutical Co., Ltd.),<br>22 October, 1982 (22.10.82),<br>Full text; particularly, CAS RN: 85105-42-6, 85105-44-8, 85105-45-9, 85105-46-0, 85119-07-9, compounds<br>& JP 02-006354 B2 | 1,2,8,9<br>3-7,10-53,56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | JP 57-031665 A (Roussel Uclaf), 20 February, 1982 (20.02.82), Full text; particularly, CAS RN:80777-30-6, compound<br>& EP 40573 A2 & EP 40573 A3<br>& EP 40573 B1 & FR 2482596 A1<br>& FR 2482596 B1 & US 4397856 A<br>& CA 1184558 A1 & DK 8102172 A<br>& DK 152212 B & DK 152212 C<br>& FI 8101529 A & FI 77030 B<br>& FI 77030 C & AU 8170689 A1<br>& AU 543580 B2 & ES 502264 A1<br>& ZA 8103293 A & HU 26727 O<br>& HU 184853 B & JP 63-040430 B2<br>& AT 8783 E & EP 143123 A2<br>& EP 143123 A3 & EP 143123 B1<br>& AT 54913 E & US 4518775 A | 1,2,8,9, 22-24,28,29<br>3-7,10-21, 25-27,30-53, 56 |
| X<br><br>A | US 3455932 A (SMITH KLINE AND FRENCH LABORATORIES), 15 July, 1969 (15.07.69), Full text; particularly, CAS RN:23790-10-5, 23790-11-6, 23790-13-8, 23790-15-0, 23845-04-7, compounds<br>(Family: none) | 1-4,8,9,11, 12<br>5-7,10, 13-53,56 |
| X<br>A | SONDHI, S.M. et al., Synthesis and anti-inflammatory activity evaluation of some acridinylaminoantipyrine, acridinylaminoanthraquinone, acridinothiourea and thiazolinothiourea derivatives, Phosphorus, Sulfur and Silicon and the Related Elements, 2000, Vol.156, pages 21 to 33. (abstract) CAplus [online]; CAplus Accession No.2000: 530801. particularly, CAS RN: 297177-09-4, 297177-18-5, 297177-20-9, compounds | 1,2,8,9<br>3-7,10-53, 56 |
| X<br><br>A | NICOLAS, Colette et al., New Quaternary Ammonium Oxicam Derivatives Targeted toward Cartilage: Synthesis, Pharmacokinetic Studies, and Antiinflammatory Potency, Journal of Medicinal Chemistry, 1999, Vol.42, No.25, pages 5235 to 5240, full text, particularly, compound 6a | 1-3,5,8,11, 13<br>4,6,7,9,10, 12,14-53,56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | ANDREANI, Aldo et al., Ring-opened analogs of indomethacin affecting human neutrophil functions, Collection of Czechoslovak Chemical Communications, 1999, Vol.64, No.2, pages 299 to 312. (abstract) CAplus [online]; CAplus Accession No.1999:218957. particularly, CAS RN: 226905-02-8, 226905-49-3, 226905-50-6, 226905-51-7, 226905-52-8, 226905-57-3, 118214-99-6, 127801-97-2, 226905-11-9, 226905-18-6, 226905-41-5, 226905-42-6, 226905-43-7, compounds | 1,2,8,22-28<br>3-7,9-21, 29-53,56 |
| X<br>A | ROBERT, J.M.H. et al., Nonacidic antiinflammatory compounds. II. Synthesis and activity of 6-amino-2,4-lutidine derivatives, European Journal of Medicinal Chemistry, 1994, Vol.29, No.11, p.841-54, full text, particularly, compounds 44 to 49 | 1-8,11-13<br>9,10,14-53, 56 |
| X<br>A | DAIDONE, G. et al., Synthesis and pharmacological activity of new salicylic acid derivatives, Farmaco, Edizione Scientifica, 1985, Vol.40, No.3, p.178-89. (abstract) CAplus [online]; CAplus Accession No.1985:416394. particularly, CAS RN: 79442-83-4, 88059-46-5, 96935-41-0, 96935-35-2, 96935-36-3, 96935 -37-4, compouds | 1,2,8,9<br>3-7,10-53, 56 |
| X<br><br>A | PAPADOPOULOU-DAIFOTIS, Z. et al., Synthesis and pharmacological activity of some 2-imino-3-alkylthiazoline derivatives, Praktika tes Akademias Athenon, 1979, Vol.53, No.A-D, p.469-84 . (abstract) CAplus [online]; CAplus Accession No.1981:30616. particularly, CAS RN: 71590-66-4, 71590-73-3, 76048-82-3, 76125-59-2, 71590-54-0, 71590-57-3, 76048-70-9, 76048-74-3, 71590-48-2, 71590-49-3, 71590-55-1, 71590-56-2, 71590-58-4, 71590-59-5, 71590-60-8, 71590-61-9, 71590-62-0, 71590-63-1, 71590-64-2, 71590-65-3, 71590-67-5, 71590-68-6, 71590-69-7, 71590-70-0, 71590-71-1, 71590-72-2, 71590-74-4, 71590-75-5, 76048-71-0, 76048-72-1, 76048-73-2, 76048-75-4, 76048-76-5, 76048-77-6, 76048-78-7, 76048-79-8, 76048-80-1, 76048-81-2, 76048-83-4, 76048-84-5, 76048-85-6, 76048-86-7, 76048-87-8, compounds | 1,2,8,22-25, 28<br>3-7,9-21,26, 27,29-53,56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | PAPADOPOULOU-DAIFOTIS, Z. et al., Synthesis and pharmacological activity of some 2-imino-3-alkyl thiazoline derivatives, European Journal of Medicinal Chemistry, 1979, Vol.14, No.3, p.253-6 . (abstract) CAplus [online]; CAplus Accession No.1979:557647. particularly, CAS RN: 71590-48-2, 71590-49-3, 71590-54-0, 71590-55-1, 71590-56-2, 71590-57-3, 71590-58-4, 71590-59-5, 71590-60-8, 71590-61-9, 71590-62-0, 71590-63-1, 71590-64-2, 71590-65-3, 71590-66-4, 71590-67-5, 71590-68-6, 71590-69-7, 71590-70-0, 71590-71-1, 71590-72-2, 71590-73-3, 71590-74-4, 71590-75-5, compounds | 1,2,8,22-25, 28<br>3-7,9-21,26, 27,29-53,56 |
| X<br>A | VASIL'EVA, V.F. et al., Synthesis and properties of 2-imino-3-benzyl-5-phenyl-1,3, 4-oxadiazoline, Khimiya Geterotsiklicheskikh Soedinenii, 1970, No.2, p.155-8. (abstract) CAplus [online]; CAplus Accession No.1970: 121444. particularly, CAS RN: 27643-07-8, 27643-09-0, 27643-10-3, compounds | 1,2,8,9<br>3-7,10-53,56 |
| X<br>A | HANKOVSZKY, H.O. et al., Preparation of some ω-(pyridylalkyl)benzamide and benzoate derivatives, Journal of Medicinal Chemistry, 1966, Vol.9, No.1, p.151-3, full text, particularly, compounds 18, 20 | 1-8,11-13<br>9,10,14-53, 56 |
| X<br>A | JP 03-190859 A (Bayer AG.), 20 August, 1991 (20.08.91), CAS RN:136267-73-7, compound<br>& EP 432600 A2 & EP 432600 A3<br>& DE 4021439 A1 & US 5250498 A<br>& CA 2032046 A & AU 9067999 A1<br>& AU 632880 B2 | 11-13,21<br>1-10,14-20, 22-53,56 |
| X<br>A | US 3655895 A (CIBA LTD.), 11 April, 1972 (11.04.72), CAS RN: 30896-95-8, compound<br>& DE 2031920 A & US 3627889 A<br>& BR 6915374 A0 & US 3719678 A<br>& ZA 7004095 A & FR 2054617 A1<br>& BE 753061 A & NL 7009971 A<br>& AT 297005 B & AT 299962 B<br>& AT 299963 B & AT 300819 B<br>& AT 303047 B & US 3772291 A<br>& US 3758473 A | 11-13,21<br>1-10,14-20, 22-53,56 |
| X<br>A | US 3253913 A (EASTMAN KODAK CO.), 31 May, 1966 (31.05.66), CAS RN:101956-88-1, compound<br>& GB 999776 A | 11,13,21<br>1-10,12, 14-20,22-53, 56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | US 3149990 A (MINNESOTA MINING AND MANUFG. CO.), 22 September, 1964 (22.09.64), CAS RN: 101812-93-5, 101813-38-1, 102290-33-5, 102290-35-7, compounds (Family: none) | 11-13,21<br>1-10,14-20, 22-53,56 |
| X<br>A | PETKO, K.I. et al., Difluoromethylation of Sulfonamides, Russian Journal of Organic Chemistry, 2002, Vol.38, No.7, pages 1030 to 1034. (abstract) CAplus [online]; CAplus Accession No.2002:817633. CAS RN: 504416-39-1, 504416-40-4, compounds | 11,12<br>1-10,13-53, 56 |
| X<br>A | BOBERG, Friedrich et al., Reaction of thioxo compounds with N-chloramidines. VI. Reaction of thioquinolone, dihydrothiazolethione and dihydroisothiazole thione with sodium N-chlorobenzenesulfonamides, Phosphorus, Sulfur and Silicon and the Related Elements, 1996, Vol.108, No.1-4, pages 203 to 220. (abstract) CAplus [online]; CAplus Accession No.1996:420288. CAS RN: 180625-57-4, 180625-96-1, 180625-97-2, 180625-98-3, 180625-99-4, compounds | 11,12<br>1-10,13-53, 56 |
| X<br>A | BOBERG, Friedrich et al., Reaction of thioxoheterocycles with N-chloroamides. II. N-substituted 2- or 4-thiopyridone and sodium N-chlorobenzenesulfonamide, Phosphorus, Sulfur and Silicon and the Related Elements, 1989, Vol.44, No.3-4, p.267-84. (abstract) CAplus [online]; CAplus Accession No.1990: 216635. particularly, CAS RN: 52814-22-9, 126957-86-6, 126957-87-7, compounds | 11,12<br>1-10,13-53, 56 |
| X<br>A | DE Benedetti, P.G. et al., Structure-activity relationships: electronic features of sulfanilamides and p-aminobenzoate in relation to their competitive affinities in bacterial growth, Farmaco, Edizione Scientifica, 1979, Vol.34, No.12, p.1005-14. (abstract) CAplus [online]; CAplus Accession No.1980:88490. CAS RN:72792-59-7, compound | 11,12<br>1-10,13-53, 56 |
| X<br>A | RASTELLI, Augusto et al., Experimental investigations on the mechanisms of intramolecular interactions in sulfanilamides, Journal of Chemical Research, Synopses, 1977, No.4, p.90-1. (abstract) CAplus [online]; CAplus Accession No.1977:483969. CAS RN: 58944-05-1, 58944-07-3, 58944-08-4, 58944-09-5, compounds | 11,12<br>1-10,13-53, 56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | L'ABBE, Gerrit et al., Trapping of thiaziridinimines with imines and nitriles, Journal of Organic Chemistry, 1976, Vol.41, No.21, p.3403-6, compound 5a | 11,12<br>1-10,13-53, 56 |
| X<br>A | MELEGARI, M. et al., Preparation and spectroscopic behavior of acetyl derivatives of sulfonamides, Farmaco, Edizione Scientifica, 1976, Vol.31, No.3, p.183-93 . (abstract) CAplus [online]; CAplus Accession No.1976:180162. CAS RN:59224-70-3, compound | 11,12<br>1-10,13-53, 56 |
| X<br>A | BAILEY, A. Sydney et al., Reactions of dihydroquinolines and dihydroisoquinolines with arenesulfonyl azides, Journal of the Chemical Society, Perkin Transactions 1: Organic and Bio-Organic Chemistry, 1975, No.5, p.420-4, compound VI | 11,12<br>1-10,13-53, 56 |
| X<br>A | BAILEY, A. Sydney et al., Reactions of p-toluenesulfonyl azide with derivatives of cyclohept- and cyclooctindole, Journal of the Chemical Society, Perkin Transactions 1: Organic and Bio-Organic Chemistry, 1974, No.7, p.763-70, compound XVII | 11,12<br>1-10,13-53, 56 |
| X<br>A | BOSIN, Talmage R. et al., Routes of functionalized guanidines. Synthesis of guanidino diesters, Journal of Organic Chemistry, 1973, Vol.38, No.8, p.1591-600, compound 21a | 11,12<br>1-10,13-53, 56 |
| X<br>A | BAILEY, A.S. et al., Reactions of arenesulfonyl azides with some di- and trisubstituted indoles, Journal of the Chemical Society [Section] C: Organic, 1971, No.22, p.3769-78. (abstract) CAplus [online]; CAplus Accession No.1972:14245. CAS RN: 34614-01-2, compound | 11,12<br>1-10,13-53, 56 |
| X<br>A | SCHOLTAN, W., The binding of sulfonamides with proteins, Arzneimittel-Forschung, 1964, Vol.14, p.348-56 . (abstract) CAplus [online]; CAplus Accession No.1964:435373. CAS RN:93003-79-3, compound | 11,12<br>1-10,13-53, 56 |
| X<br>A | SCHUBERT, W.M. et al., The acidity dependence of the carbon protonation of phloroglucinol and its methyl ethers, Journal of the American Chemical Society, 1963, Vol.85, p.1278-84. (abstract) CAplus [online]; CAplus Accession No.1963:80950. CAS RN: 1494-86-6, 65986-33-6, 91397-61-4, 91398-14-0, 91570-81-9, 92644-41-2, 93044-35-0, 93427-47-5, 94209-72-0, compounds | 11,12<br>1-10,13-53, 56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | MASTRYUKOVA, T.A. et al., Application of the Hammett equation to the theory of tautomeric equilibrium. II. Tautomerism of α -arylsulfoaminopyridines, Tetrahedron, 1963, Vol.19, p.357-72 . (abstract) CAplus [online]; CAplus Accession No.1963:80949. CAS RN: 93898-09-0, compound | 11,12<br>1-10,13-53, 56 |
| X<br>A | KANAOKA, Matao, Synthesis of related compounds of thiosemicarbazides. VI. Alkylation of 2-(p-toluenesulfonamido)-1,3,4-thiadiazole, Yakugaku Zasshi, 1961, Vol.81, p.1378-80 . (abstract) CAplus [online]; CAplus Accession No.1962:38480. particularly, CAS RN: 94072-20-5, 95348-24-6, 96877-22-4, 93485-36-0, 96877-23-5, compounds | 11,12,48<br>1-10,13-47, 49-53,56 |
| X<br>A | SHEINKER, Yu. N. et al., Tautomerism of derivatives of heterocyclic compounds. X. Tautomerism of acylated heterocyclic amines, Zhurnal Fizicheskoi Khimii, 1959, Vol.33, pages 2096-2109. (abstract) CAplus [online]; CAplus Accession No.1960:62765. particularly, CAS RN: 99844-29-8, compound | 11,12<br>1-10,13-53, 56 |
| X<br>A | GOERDELER, Joachim et al., 1,2,4-Thiadiazoles. II. N-Methyl derivatives of the aminothiadiazoles and iminothiadiazolines, Chemische Berichte, 1954, Vol.87, pages 68 to 78. (abstract) CAplus [online]; CAplus Accession No.1955:32380. particularly, CAS RN: 857488-38-1, 858501-40-3, compounds | 11-13,21<br>1-10,14-20, 22-53,56 |
| X<br>A | SHEPHERD, Robert G. et al., Hydrolysis and aminolysis of certain methoxypyrimidines, Journal of Organic Chemistry, 1947, Vol.12, p.446-55. (abstract) CAplus [online]; CAplus Accession No.1947:27500. particularly, CAS RN: 857011-33-7, 857990-73-9, compounds | 11,12<br>1-10,13-53, 56 |
| X<br>A | RAJAGOPALAN, S., Bacterial chemotherapy. III. Synthesis of possible lipophilic chemotherapeuticals of the sulfanilamide group, Proceedings - Indian Academy of Sciences, Section A, 1943, Vol.18A, p.108-12. (abstract) CAplus [online]; CAplus Accession No.1944: 8285. CAS RN: 855210-27-4, 861024-59-1, compounds | 11,12<br>1-10,13-53, 56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | SHEPHERD, Robert G. et al., Properties of the nitrogen-carbon-nitrogen system in N1-heterocyclic sulfanilamides, Journal of the American Chemical Society, 1942, Vol.64, p.2532-7. (abstract) CAplus [online]; CAplus Accession No.1943:3566. particularly, CAS RN: 66507-93-5, 782499-09-6, compounds | 11,12<br>1-10,13-53,<br>56 |
| X<br>A | POLYAKOVA, I.M. et al., Preparation of sulfidine and certain new derivatives of streptocide, Zhurnal Prikladnoi Khimii, 1940, Vol.13, p.1215-19 (in French, 1219). (abstract) CAplus [online]; CAplus Accession No.1941:13409. CAS RN: 855876-90-3, 857622-30-1, 858808-78-3, 858810-23-8, 860542-28-5, compouns | 11,12<br>1-10,13-53,<br>56 |
| X<br>A | CHICHIBABIN, A.E. et al., Benzoylation products of α -aminopyridine, Ber., 1922, Vol.55B, pages 998 to 1002. (abstract) CAplus [online]; CAplus Accession No.1922:21367. particularly, CAS RN: 102290-32-4, compound | 11-13,21<br>1-10,14-20,<br>22-53,56 |
| X<br>A | WO 2003/097605 A1 (BAYER CROPSCIENCE GMBH),<br>27 November, 2003 (27.11.03),<br>Table 5, compounds E-1, E-11<br>& BR 2003010055 A & EP 1507762 A1<br>& JP 2005-536468 A & US 2005/227970 A1 | 48<br>1-47,49-53,<br>56 |
| X<br>A | JP 2003-292485 A (Yamanouchi Pharmaceutical Co., Ltd.),<br>15 October, 2003 (15.10.03),<br>Table 7, compound No.93<br>(Family: none) | 48<br>1-47,49-53,<br>56 |
| X<br>A | JP 60-233067 A (Mitsui Toatsu Chemicals, Inc.),<br>19 November, 1985 (19.11.85),<br>Compounds 1 to 6, 9 to 17, 19 to 36<br>& JP 05-064145 B2 | 48<br>1-47,49-53,<br>56 |
| X<br>A | JP 58-080634 A (Konishiroku Shashin Kogyo Kabushiki Kaisha),<br>14 May, 1983 (14.05.83),<br>Compound (8)<br>(Family: none) | 48<br>1-47,49-53,<br>56 |
| X<br>A | JP 55-162765 A (CIBA-Geigy AG.),<br>18 December, 1980 (18.12.80),<br>CAS RN: 77458-91-4, 77459-00-8, 77459-01-9, compounds<br>& EP 20304 A1 & US 4342764 A<br>& FI 8001691 A & ES 491871 A1<br>& DD 151167 C & SU 1003752 A3<br>& DK 8002295 A & NO 8001586 A | 48<br>1-47,49-53,<br>56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | RACKHAM, David M. et al., Carbon-13 NMR spectra of 1,3,4-thiadiazole/thiadiazoline isomeric pairs, Organic Magnetic Resonance, 1980, Vol.14, No.6, p.515-16. (abstract) CAplus [online]; CAplus Accession No.1981:441647. CAS RN: 78258-09-0, compound | 48<br>1-47,49-53,<br>56 |
| X<br>A | NEITZEL, Michael et al., Reaction of cyanates with N- and N'-substituted carboxylic hydrazides, Archiv der Pharmazie, 1980, Vol.313, No.10, p.867-78. (abstract) CAplus [online]; CAplus Accession No.1981:65569. CAS RN: 76441-32-2, 76441-33-3, 76441-34-4, 76441-35-5, compounds | 48<br>1-47,49-53,<br>56 |
| X<br>A | LEPPARD, D. et al., Methylation of 2-acylamido-1,3,4-thiadiazoles. An infrared, proton and carbon NMR study, Journal of Heterocyclic Chemistry, 1980, Vol.17, No.7, p.1469-72, compounds 3, 6, 9, 12 | 48<br>1-47,49-53,<br>56 |
| X<br>A | ALEMAGNA, Andreina et al., N-Alkyl thiadiazolium salts: reactions with bases, Rendiconti - Istituto Lombardo Accademia di Scienze e Lettere, A: Scienze Matematiche, Fisiche, Chimiche e Geologiche, 1979, Volume Date 1978, Vol.112, No.1, pages 67 to 77. (abstract) CAplus [online]; CAplus Accession No.1980:408093. CAS RN: 73606-71-0, compound | 48<br>1-47,49-53,<br>56 |
| X<br>A | FUKUTANI, Yasuhide et al., Molecular structures of adducts of benzoyl isothiocyanate and hydrazones determined by the x-ray diffraction method, Bulletin of the Chemical Society of Japan, 1979, Vol.52, No.8, p.2223-8, compound 2 | 48<br>1-47,49-53,<br>56 |
| X<br>A | WERBER, Giuseppe et al., Reactivity of 2-amino-1,3,4-thiadiazoles. Nucleophilic behavior of some 2-amino-1,3,4-thiadiazoles: model compounds, Journal of Heterocyclic Chemistry, 1977, Vol.14, No.7, p.1263-5 . (abstract) CAplus [online]; CAplus Accession No.1978:73799. CAS RN: 58537-53-4, compound | 48<br>1-47,49-53,<br>56 |
| X<br>A | WERBER, G. et al., Nucleophilic behavior of some 1,3,4-oxadiazoles in benzoylation, nitrosation, acetylation, and methylation reactions, Journal of Heterocyclic Chemistry, 1972, Vol.9, No.1, p.107-9. (abstract) CAplus [online]; CAplus Accession No.1972:99563. CAS RN: 35369-06-3, compound | 48<br>1-47,49-53,<br>56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| X<br>A | GEHLEN, Heinz et al., 2-Amino-1,3,4-oxadiazoles. 34. Reaction of 3,5-disubstituted 2-imino-1,3,4-oxadiazolines with mustard oils, Pharmazie, 1969, Vol.24, No.12, p.728-30 . (abstract) CAplus [online]; CAplus Accession No.1970:121461. CAS RN: 16932-70-0, 27305-00-6, 27305-08-4, compounds | 48<br>1-47,49-53, 56 |
| X<br>A | GEHLEN, H. et al., 2-Amino-1,3,4-oxadiazoles. XXIII. Preparation and reactions of 2-amino-1,3,4-oxadiazoles from aromatic dihydroxy-, di- and trialkoxycarboxylic acid hydrazides and the effect of phenyl isocyanate on 2-imino-1,3,4-oxadiazolines, Journal fuer Praktische Chemie, 1968, Vol.38, No.3-4, p.150-61 . (abstract) CAplus [online]; CAplus Accession No.1968:496593. CAS RN: 19938-63-7, 19938-65-9, compounds | 48<br>1-47,49-53, 56 |
| X<br>A | KRESZE, Guenter et al., Reactions with N-sulfinyl compounds. VII. Reactions with N-acyl thioureas, dithiocarboxylic acid esters, and trithiones, Chemische Berichte, 1967, Vol.100, No.5, p.1655-60 . (abstract) CAplus [online]; CAplus Accession No.1967:421872. CAS RN: 16151-49-8, compound | 48<br>1-47,49-53, 56 |
| X<br>A | SEYDEL, Joachim et al., Sulfanilamidopyrazole derivatives, Arzneimittel-Forschung, 1964, Vol.14, No.12, pages 1294 to 1301 . (abstract) CAplus [online]; CAplus Accession No.1965:82505. CAS RN: 893-08-3, 901-81-5, 904-30-3, compounds | 48<br>1-47,49-53, 56 |
| X<br>A | SEYDEL, Joachim, 5-Sulfanilamido-1-phenylpyrazole, Naturwissenschaften, 1963, Vol.50, No.21, page 663 . (abstract) CAplus [online]; CAplus Accession No.1964:23364. CAS RN: 98342-23-5, compound | 48<br>1-47,49-53, 56 |
| X<br>A | SHEINKER, Yu. N. et al., Tautomerism of some derivatives of heterocyclic compounds. IV. Spectra and the structure of benzene sulfonamides and the thiozole and thiodiazole sulfonamides, Zhurnal Fizicheskoi Khimii, 1957, Vol.31, p.1745-55. (abstract) CAplus [online]; CAplus Accession No.1958:33499. particularly, CAS RN: 75746-24-6, 114098-33-8, compounds | 48<br>1-47,49-53, 56 |
| X<br>A | PELLIZZARI, G., Action of cyanogen halides on phenylhydrazine. X. Derivatives of diazole, Gaze.chim.ital., 1926, Vol.56, pages 695 to 700. (abstract) CAplus [online]; CAplus Accession No.1927:7474. particularly, CAS RN: 858016-28-1, compound | 48<br>1-47,49-53, 56 |

Form PCT/ISA/210 (continuation of second sheet) (April 2005)

**INTERNATIONAL SEARCH REPORT**

International application No.
PCT/JP2005/019977

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2005/075435 A1 (VERTEX PHARMACEUTICALS INC.), 18 August, 2005 (18.08.05), Full text; particularly, Table 1, compounds 70, 71 (Family: none) | 1,2,8 |
| P,X | WO 2005/000825 A1 (Sumitomo Pharmaceuticals Co., Ltd.), 06 January, 2005 (06.01.05), Full text; particularly, examples 1, 2, 4, 5 to 7, 12, 13 (Family: none) | 1,2,8,22-25, 28 |

Form PCT/ISA/210 (continuation of second sheet) (April 2005)

**INTERNATIONAL SEARCH REPORT**

International application No.
PCT/JP2005/019977

Continuation of A. CLASSIFICATION OF SUBJECT MATTER
(International Patent Classification (IPC))

***A61K31/429***(2006.01), ***A61K31/433***(2006.01), ***A61K31/4365***(2006.01), ***A61K31/437***(2006.01), ***A61K31/4375***(2006.01), ***A61K31/44***(2006.01), ***A61K31/4409***(2006.01), ***A61K31/4418***(2006.01), ***A61K31/4427***(2006.01), ***A61K31/443***(2006.01), ***A61K31/4433***(2006.01), ***A61K31/4436***(2006.01), ***A61K31/4439***(2006.01), ***A61K31/444***(2006.01), ***A61K31/454***(2006.01), ***A61K31/47***(2006.01), ***A61K31/4709***(2006.01), ***A61K31/472***(2006.01), ***A61K31/4725***(2006.01), ***A61K31/496***(2006.01), ***A61K31/497***(2006.01), ***A61K31/498***(2006.01), ***A61K31/501***(2006.01), ***A61K31/502***(2006.01), ***A61K31/5025***(2006.01), ***A61K31/505***(2006.01), ***A61K31/506***(2006.01), ***A61K31/5377***(2006.01), ***A61K31/538***(2006.01), ***A61K31/541***(2006.01), ***A61K31/55***(2006.01), ***A61P25/04***(2006.01), ***A61P29/00***(2006.01), ***A61P37/06***(2006.01), ***A61P43/00***(2006.01), ***C07D213/75***(2006.01), ***C07D213/76***(2006.01), ***C07D213/84***(2006.01), ***C07D215/38***(2006.01), ***C07D217/22***(2006.01), ***C07D231/38***(2006.01), ***C07D231/40***(2006.01), ***C07D239/42***(2006.01), ***C07D261/14***(2006.01), ***C07D263/48***(2006.01), ***C07D271/10***(2006.01), ***C07D275/02***(2006.01), ***C07D277/20***(2006.01), ***C07D277/46***(2006.01), ***C07D277/56***(2006.01), ***C07D277/82***(2006.01), ***C07D285/12***(2006.01), ***C07D401/12***(2006.01), ***C07D405/12***(2006.01), ***C07D409/12***(2006.01), ***C07D409/14***(2006.01), ***C07D413/12***(2006.01), ***C07D417/06***(2006.01), ***C07D417/12***(2006.01), ***C07D417/14***(2006.01), ***C07D471/04***(2006.01), ***C07D487/04***(2006.01), ***C07D495/04***(2006.01), ***C07D513/04***(2006.01)

(According to International Patent Classification (IPC) or to both national classification and IPC)

Continuation of B. FIELDS SEARCHED
Minimum documentation searched (International Patent Classification (IPC))

A61K31/4427, A61K31/443, A61K31/4433, A61K31/4436, A61K31/4439, A61K31/444, A61K31/454, A61K31/47, A61K31/4709, A61K31/472, A61K31/4725, A61K31/496, A61K31/497, A61K31/498, A61K31/501, A61K31/502, A61K31/5025, A61K31/505, A61K31/506, A61K31/5377, A61K31/538, A61K31/541, A61K31/55, C07D213/75, C07D213/76, C07D213/84, C07D215/38, C07D217/22, C07D231/38, C07D231/40, C07D239/42, C07D261/14, C07D263/48, C07D271/10, C07D275/02, C07D277/20, C07D277/46, C07D277/56, C07D277/82, C07D285/12, C07D401/12, C07D405/12, C07D409/12, C07D409/14, C07D413/12, C07D417/06, C07D417/12, C07D417/14, C07D471/04, C07D487/04, C07D495/04, C07D513/04

Minimum documentation searched (classification system followed by classification symbols)

Form PCT/ISA/210 (extra sheet) (April 2005)

**INTERNATIONAL SEARCH REPORT**

International application No.
PCT/JP2005/019977

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 54 and 55
because they relate to parts of the international application that do not comply with the prescribed requirements to such an extent that no meaningful international search can be carried out, specifically:
Claims 54 and 55 are an invention relating to a compound in which $X^3$ is sulfur. However, the compound of claim 49, which is cited in these claims, excludes a compound in which $X^3$ is sulfur according to the proviso in claim 48, which
(continued to extra sheet)

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

Form PCT/ISA/210 (continuation of first sheet (2)) (April 2005)

**INTERNATIONAL SEARCH REPORT**

International application No.
PCT/JP2005/019977

Continuation of Box No.II-2 of continuation of first sheet(2)

is cited in claim 49. Consequently, the statements in claims 54 and 55 are inconsistent with the statement in claim 49.

Claims 1, 3, 11, 37, and 48 involve a large number of compounds. However, the compounds which are supported by the description in the meaning of Article 6 of the PCT and are disclosed in the meaning of Article 5 of the PCT are limited to an extremely small part of the compounds disclosed in the claims.

Therefore, in this international search report, a search was made for the part supported by and disclosed in the description, i.e., with respect mainly to Examples.

As a result of the search, many prior-art documents were found. It is difficult to show all the prior-art documents. Only part of the prior-art documents found were hence shown in this international search report.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9215564 A
- EP 432600 A
- DE 1036326
- WO 2001055139 A
- WO 2000063207 A
- JP 2003292485 B
- WO 2002002542 A
- WO 2003097605 A
- JP 2003192591 B
- WO 2000017196 A
- WO 9842703 A
- JP 02250874 A
- JP 62004277 B
- EP 40573 A
- JP 63203672 B
- JP 08081449 B
- WO 9703058 A
- WO 9404516 A
- JP 02229164 A


### Non-patent literature cited in the description


- *Exp. Opin. Ther. Patent,* 2002, vol. 12 (10), 1475-1489
- *Exp. Opin. Ther. Patent,* 2004, vol. 14 (10), 1435-1452
- *European Journal of Medicinal Chmistry,* 1994, vol. 29 (11), 841-854
- *Journal of Medicinal Chmistry,* 1966, vol. 9 (1), 151-153
- IzVestiya Akademii Nauk SSSR. *Seriya Kimicheskaya,* 1953, 154-162
- **FARMACO.** *Edizione Scientifica,* 1985, vol. 40 (3), 178-189
- *Journal of Heterocyclic Chemistry,* 1983, vol. 20 (5), 1153-1154
- *Journal of Heterocyclic Chemistry,* 1981, vol. 18 (4), 745-750
- **MUNRO et al.** *Nature,* 1993, vol. 365, 61-65
- **FUTAKI N et al.** *Gen Pharmacol.,* 1993, vol. 24 (1), 105-110
- **SELTZERZ.** *Pain,* 1990, vol. 43 (2), 205-218